# EUROPEAN PATENT APPLICATION

(11) **EP 4 851 169 A1**
(43) Date of publication of application: **09.12.2026**
(21) Application number: 25747842.0
(22) Date of filing: 24.01.2025
(51) Int. Cl.: C07D 495/14, C07D 491/14, C07D 487/14, C07D 498/14, A61K 31/335, A61K 31/38, A61K 31/395, A61P 35/00

(54) **AROMATIC AMIDE DERIVATIVE, AND PREPARATION METHOD THEREFOR AND USE THEREOF**

(30) Priority: 29.01.2024 CN 202410117462; 26.09.2024 CN 202411350230
(71) Applicant: Zhejiang Hisun Pharmaceutical Co., Ltd., Taizhou, Zhejiang 318000 (CN); Shanghai Aryl Pharmtech Co., Ltd., Songjiang, Shanghai 201612 (CN)
(72) Inventor: QIU, Zongxing, Shanghai 201612 (CN); CAO, Hai, Shanghai 201612 (CN); PENG, Jilei, Shanghai 201612 (CN); WANG, Zefeng, Shanghai 201612 (CN); YE, Cheng, Shanghai 201612 (CN); SHI, Yaqiong, Shanghai 201612 (CN); ZHANG, Heng, Shanghai 201612 (CN); YU, Mingfeng, Shanghai 201612 (CN); ZHOU, Houjiang, Zhejiang 318000 (CN)
(74) Representative: Dehns
(86) International application number: PCT/CN2025/074749
(87) International publication number: WO 2025/162274

(57) **Abstract**

The present disclosure relates to an aromatic amide derivative, a preparation method therefor, and the use of a pharmaceutical composition containing the derivative in medicine. Specifically, the present disclosure relates to an aromatic amide derivative as shown in formula (I), a preparation method therefor and a pharmaceutically acceptable salt thereof, and the use thereof as a therapeutic agent, in particular a DHX9 inhibitor, wherein the definition of each substituent in formula (I) is the same as defined in the specification.

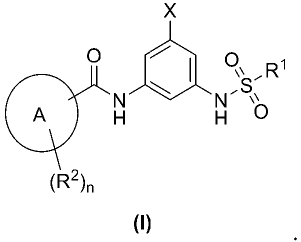

## Description

### TECHNICAL FIELD

The present disclosure relates to an aromatic amide derivative, a preparation method therefor, a pharmaceutical composition containing the derivative and the use thereof as a therapeutic agent, particularly the use as a DHX9 inhibitor.

### BACKGROUD

DExH-box Helicase 9 (DHX9) is a member of DExD/H-box helicase family, and is an NTP-dependent helicase protein, which may unwind RNA, DNA and aberrant polynucleotide structures, thereby being also known as nuclear DNA Helicase II (NDH II) and RNA Helicase A (RHA). A core structure domain of helicase in the family has a conserved Asp-Glu-Ile-His (DEIH) sequence, which is a core region for NTP binding and hydrolysis.

Due to the lack of base selectivity, the DHX9 can meet energy requirements thereof by utilizing all four types of NTPs, and is a multi-domain and multi-functional protein, which plays vital roles in biological development, DNA replication, transcription, translation, repair, cleavage and editing, RNA processing, transportation, remodeling, maintenance of genomic stability, and the like.

Due to the regulatory functions in transcription and maintenance of genomic stability, DHX9 has been proved to be a key regulatory factor in multiple cancer types, which participates in gene regulation associated with sustained proliferative signal transduction, evasion of growth inhibition factor, evasion of apoptosis, angiogenesis and metastasis, all of which are hallmarks of cancer. Specifically, microsatellite instability cancers, such as microsatellite instability (MSI) colorectal cancer, and tumors with defective mismatch repair (MMR), exhibit strong dependence on DHX9.

In addition to the oncogenic roles, DHX9 is also associated with other diseases related to gene replication, translation and regulation. The diseases include viral infection and autoimmune diseases.

No novel DHX9-targeted inhibitor drugs have been approved for marketing to date, and there has been no information about clinical compounds. As a novel anti-tumor target, the DHX9 target has enormous research potential, so that the development of DHX9-targeted inhibitors is of great necessity.

### SUMMARY

Aiming at the above technical problem, the present disclosure provides an aromatic amide derivative as shown in formula (I), or a stereoisomer thereof, a tautomer thereof or a pharmaceutically acceptable salt thereof: 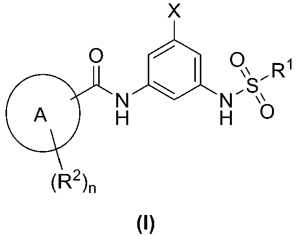 wherein:
ring A is selected from tricyclic heteroaryl or a tricyclic fused ring;
X is selected from halogen;
R¹ is selected from C₁₋₆ alkyl or 3-5 membered cycloalkyl; wherein the alkyl or cycloalkyl is optionally further substituted with one or more substituents selected from halogen, hydroxyl, cyano, C₁₋₆ alkyl or C₁₋₆ alkoxy;
each R² is independently selected from a hydrogen atom, cyano, halogen, alkyl, alkenyl, alkynyl, cycloalkyl, heterocyclyl, aryl, heteroaryl, SF₅, -O(CH₂)ₜR^{A}, -C(O)R³, -C(O)OR³, -NHC(O)R³, -NHC(O)OR³, -NR⁴R⁵, -C(O)NR⁴R⁵, -S(O)₂NR⁴R⁵, -CH₂NHC(O)OR³, -CH₂NR⁴R⁵ or -S(O)ᵣR³; wherein the alkyl, alkenyl, alkynyl, cycloalkyl, heterocyclyl, aryl or heteroaryl is optionally further substituted with one or more substituents selected from a deuterium atom, halogen, nitro, cyano, alkyl, haloalkyl, cycloalkyl, heterocyclyl, aryl, heteroaryl, =O, -OR^{A}, -C(O)R⁶, -C(O)OR⁶, -OC(O)R⁶, -NR⁷R⁸, -C(O)NR⁷R⁸, -SO₂NR⁷R⁸ or -NR⁷C(O)R⁸;
or, two R², together with the same carbon atom to which the groups are attached, form a -C(O) or cyclopropyl;
each R³ is independently selected from a hydrogen atom, alkyl, cycloalkyl, heterocyclyl, aryl or heteroaryl, wherein the alkyl, cycloalkyl, heterocyclyl, aryl or heteroaryl is optionally further substituted with one or more substituents selected from hydroxyl, halogen, nitro, cyano, alkyl, alkoxy, haloalkyl, haloalkoxy, cycloalkyl, heterocyclyl, aryl, heteroaryl, =O, -C(O)R⁶, -C(O)OR⁶, -OC(O)R⁶, -NR⁷R⁸, -C(O)NR⁷R⁸, -SO₂NR⁷R⁸ or -NR⁷C(O)R⁸;
R^{A} is selected from a hydrogen atom, alkyl, cycloalkyl, heterocyclyl, aryl or heteroaryl, wherein the alkyl, cycloalkyl, heterocyclyl, aryl or heteroaryl is optionally further substituted with one or more substituents selected from hydroxyl, halogen, nitro, cyano, alkyl, alkoxy, haloalkyl, haloalkoxy, cycloalkyl, heterocyclyl, aryl, heteroaryl, =O, -C(O)R⁶, -C(O)OR⁶, -OC(O)R⁶, -NR⁷R⁸, -C(O)NR⁷R⁸, -SO₂NR⁷R⁸ or -NR⁷C(O)R⁸;
R⁴ and R⁵ are each independently selected from a hydrogen atom, hydroxyl, alkyl, alkoxy, cycloalkyl, heterocyclyl, aryl or heteroaryl, wherein the alkyl, alkoxy, cycloalkyl, heterocyclyl, aryl or heteroaryl is optionally further substituted with one or more substituents selected from hydroxyl, halogen, nitro, cyano, alkyl, alkoxy, cycloalkyl, heterocyclyl, aryl, heteroaryl, =O, -C(O)R⁶, -C(O)OR⁶, -OC(O)R⁶, -NR⁷R⁸, -C(O)NR⁷R⁸, -SO₂NR⁷R⁸ or -NR⁷C(O)R⁸;
or, R⁴ and R⁵, together with atoms to which the groups are attached, form a 4-8 membered heterocyclyl, wherein the 4-8 membered heterocyclyl contains one or more N, O or S(O)ᵣ, and the 4-8 membered heterocyclyl is optionally further substituted with one or more substituents selected from hydroxyl, halogen, nitro, cyano, alkyl, alkoxy, cycloalkyl, heterocyclyl, aryl, heteroaryl, =O, -C(O)R⁶, -C(O)OR⁶, -OC(O)R⁶, -NR⁷R⁸, -C(O)NR⁷R⁸, -SO₂NR⁷R⁸ or -NR⁷C(O)R⁸;
R⁶, R⁷ and R⁸ are each independently selected from a hydrogen atom, alkyl, amino, cycloalkyl, heterocyclyl, aryl or heteroaryl, wherein the alkyl, cycloalkyl, heterocyclyl, aryl or heteroaryl is optionally further substituted with one or more substituents selected from hydroxyl, halogen, nitro, amino, cyano, alkyl, alkoxy, cycloalkyl, heterocyclyl, aryl, heteroaryl, carboxyl or a carboxylate group;
t is selected from 0 or 1;
n is selected from 0, 1, 2, 3, 4, 5, 6, 7 or 8; and
each r is independently 0, 1 or 2.

A preferred solution of the present disclosure provides the compound as shown in formula (I), or the stereoisomer thereof, the tautomer thereof or the pharmaceutically acceptable salt thereof, wherein ring A is selected from a 12-18 membered tricyclic fused ring or 12-18 membered tricyclic heteroaryl, preferably a 12-16 membered tricyclic fused ring or 12-14 membered tricyclic heteroaryl.

A preferred solution of the present disclosure provides the compound as shown in formula (I), or the stereoisomer thereof, the tautomer thereof or the pharmaceutically acceptable salt thereof, wherein R² is selected from a hydrogen atom, halogen, cyano, C₁₋₆ alkyl, amino, SF₅, -O(CH₂)ₜR^{A}, -C(O)OR³, -NHC(O)R³ or -C(O)R³, wherein the C₁₋₆ alkyl is optionally further substituted with one or more substituents selected from a deuterium atom, halogen, cyano, C₁₋₆ alkyl, -C(O)OR⁶ or -OR^{A};
or, two R², together with the same carbon atom to which the groups are attached, form a -C(O) or cyclopropyl;
R^{A} is selected from a hydrogen atom, C₁₋₆ alkyl, C₃₋₈ cycloalkyl, C₆₋₁₀ aryl, 5-6 membered heteroaryl or 5-6 membered heterocyclyl, wherein the C₁₋₆ alkyl, C₃₋₈ cycloalkyl, C₆₋₁₀ aryl, 5-6 membered heteroaryl or 5-6 membered heterocyclyl is optionally further substituted with one or more substituents selected from hydroxyl, halogen, cyano, -NR⁷R⁸, C₁₋₆ alkyl, C₁₋₆ haloalkyl or C₁₋₆ alkoxy;
R³ is selected from a hydrogen atom, C₃₋₈ cycloalkyl, C₆₋₁₀ aryl or C₁₋₆ alkyl, wherein the C₃₋₈ cycloalkyl, C₆₋₁₀ aryl or C₁₋₆ alkyl is optionally further substituted with one or more substituents selected from halogen, hydroxyl, cyano, C₁₋₆ alkyl or C₁₋₆ alkoxy;
R⁶ is selected from a hydrogen atom or C₁₋₆ alkyl;
R⁷ and R⁸ are each independently selected from a hydrogen atom or C₁₋₆ alkyl;
t is selected from 0 or 1.

A preferred solution of the present disclosure provides the compound as shown in formula (I), or the stereoisomer thereof, the tautomer thereof or the pharmaceutically acceptable salt thereof, wherein R² is selected from a hydrogen atom, halogen, C₁₋₆ alkyl, hydroxyl, cyano, C₁₋₆ alkoxy, SF₅, C₁₋₆ haloalkyl or C₁₋₆ haloalkoxy;
or, two R², together with the same carbon atom to which the groups are attached, form a -C(O) group.

A preferred solution of the present disclosure provides the compound as shown in formula (I), or the stereoisomer thereof, the tautomer thereof or the pharmaceutically acceptable salt thereof, which is a compound as shown in formula (IIA), or a stereoisomer thereof, a tautomer thereof or a pharmaceutically acceptable salt thereof: 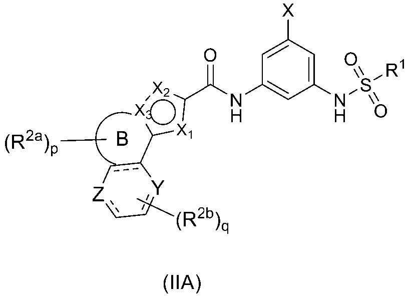 wherein: 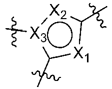
is 5-membered heteroaryl;
X₁ and X₂ are each independently selected from CH, S or N, and X₁ and X₂ are not S at the same time;
X₃ is selected from C or N; provided that when X₃ is C, X₁ and X₂ are not CH at the same time;
ring B is selected from 5-8 membered heterocyclyl, 6-10 membered aryl or 5-6 membered heteroaryl, wherein the heterocyclyl contains 1 or 2 double bonds;
-̅ -̅ -̅ is selected from a single bond or a double bond as required, so that each atom thereof is present in a normal valence state;
Y and Z are each independently selected from CR^{a}, CHR^{a}, O, N or NR^{b};
R^{2a} is selected from a hydrogen atom, C₃₋₈ cycloalkyl, C₁₋₆ alkyl or -C(O)R³, wherein the C₁₋₆ alkyl is optionally further substituted with one or more substituents selected from a deuterium atom, halogen, hydroxyl, cyano, C₁₋₆ alkyl, C₁₋₆ alkoxy or -C(O)OR⁶;
or, two R^{2a}, together with the same carbon atom to which the groups are attached, form a -C(O) or cyclopropyl;
each R^{2b} is independently selected from a hydrogen atom, C₁₋₆ alkyl, C₁₋₆ alkoxy or -C(O)OR³; wherein the C₁₋₆ alkyl or C₁₋₆ alkoxy is optionally further substituted with one or more substituents selected from hydroxyl, halogen or cyano;
R^{a} and R^{b} are each independently selected from a hydrogen atom, halogen, cyano, C₁₋₆ alkyl, amino, -NHC(O)R³ or -O(CH₂)ₜR^{A}, wherein the C₁₋₆ alkyl is optionally further substituted with one or more substituents selected from halogen, cyano, C₁₋₆ alkyl or -OR^{A};
each R^{A} is independently selected from a hydrogen atom, C₁₋₆ alkyl, C₃₋₈ cycloalkyl, C₆₋₁₀ aryl, 5-6 membered heteroaryl or 5-6 membered heterocyclyl, wherein the C₁₋₆ alkyl, C₃₋₈ cycloalkyl, C₆₋₁₀ aryl, 5-6 membered heteroaryl or 5-6 membered heterocyclyl is optionally further substituted with one or more substituents selected from hydroxyl, halogen, cyano, -NR⁷R⁸, C₁₋₆ alkyl, C₁₋₆ haloalkyl or C₁₋₆ alkoxy;
R³ is selected from a hydrogen atom, C₆₋₁₀ aryl, C₃₋₈ cycloalkyl or C₁₋₆ alkyl, wherein the C₆₋₁₀ aryl, C₃₋₈ cycloalkyl or C₁₋₆ alkyl is optionally further substituted with one or more substituents selected from halogen, hydroxyl, cyano, C₁₋₆ alkyl or C₁₋₆ alkoxy;
R⁶ is selected from a hydrogen atom or C₁₋₆ alkyl;
R⁷ and R⁸ are each independently selected from a hydrogen atom or C₁₋₆ alkyl;
t is selected from 0 or 1;
each p is independently selected from 0, 1, 2, 3, 4 or 5;
each q is independently selected from 0, 1 or 2; and
R¹ and X are as defined in formula (I).

A preferred solution of the present disclosure provides the compound as shown in formula (IIA), or the stereoisomer thereof, the tautomer thereof or the pharmaceutically acceptable salt thereof, which is a compound as shown in formula (IIIA) or (IIIB), or a stereoisomer thereof, a tautomer thereof or a pharmaceutically acceptable salt thereof: 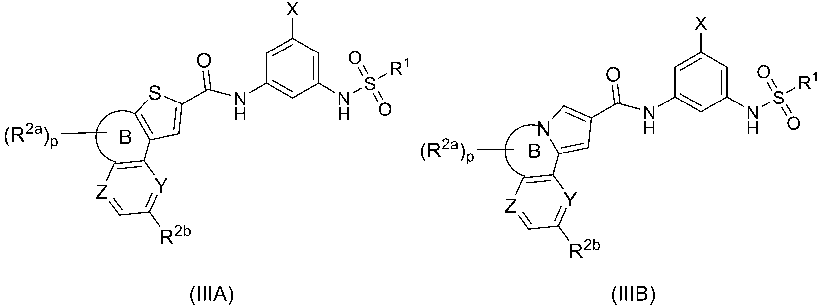 wherein:
ring B is selected from 5-8 membered heterocyclyl, wherein the heterocyclyl contains 1 or 2 double bonds;
Y and Z are each independently selected from CR^{a} or N;
R^{2a} is selected from a hydrogen atom, C₁₋₆ alkyl or -C(O)R³, wherein the C₁₋₆ alkyl is optionally further substituted with one or more substituents selected from halogen, hydroxyl, cyano, C₁₋₆ alkyl, C₁₋₆ alkoxy or -C(O)OR⁶;
or, two R^{2a}, together with the same carbon atom to which the groups are attached, form a -C(O) or cyclopropyl;
each R^{2b} is independently selected from a hydrogen atom, C₁₋₆ alkyl, C₁₋₆ alkoxy or -C(O)OR³;
each R^{a} is independently selected from a hydrogen atom, halogen, cyano, C₁₋₆ alkyl, amino, -NHC(O)R³ or -O(CH₂)ₜR^{A}, wherein the C₁₋₆ alkyl is optionally further substituted with one or more substituents selected from halogen, cyano, C₁₋₆ alkyl or -OR^{A};
each R^{A} is independently selected from a hydrogen atom, C₁₋₆ alkyl, C₃₋₈ cycloalkyl, C₆₋₁₀ aryl, 5-6 membered heteroaryl or 5-6 membered heterocyclyl, wherein the C₁₋₆ alkyl, C₃₋₈ cycloalkyl, C₆₋₁₀ aryl, 5-6 membered heteroaryl or 5-6 membered heterocyclyl is optionally further substituted with one or more substituents selected from hydroxyl, halogen, cyano, -NR⁷R⁸, C₁₋₆ alkyl, C₁₋₆ haloalkyl or C₁₋₆ alkoxy;
R³ is selected from a hydrogen atom, C₆₋₁₀ aryl or C₁₋₆ alkyl, wherein the C₆₋₁₀ aryl or C₁₋₆ alkyl is optionally further substituted with one or more substituents selected from halogen, hydroxyl, cyano, C₁₋₆ alkyl or C₁₋₆ alkoxy;
R⁶ is selected from a hydrogen atom or C₁₋₆ alkyl;
R⁷ and R⁸ are each independently selected from a hydrogen atom or C₁₋₆ alkyl;
t is selected from 0 or 1;
each p is independently selected from 0, 1, 2, 3, 4 or 5;
R¹ and X are as defined in formula (IIA).

A preferred solution of the present disclosure provides the compound as shown in formula (IIA), or the stereoisomer thereof, the tautomer thereof or the pharmaceutically acceptable salt thereof, which is a compound as shown in formula (IIIC), or a stereoisomer thereof, a tautomer thereof or a pharmaceutically acceptable salt thereof: 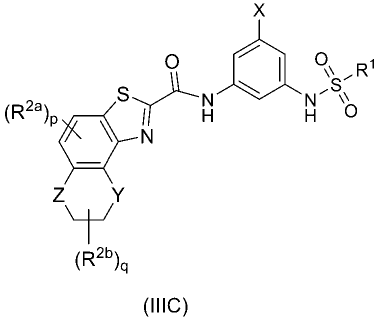 wherein:
Y and Z are each independently selected from CHR^{a}, O or NR^{b};
R^{2a} is selected from a hydrogen atom or C₁₋₆ alkyl, wherein the C₁₋₆ alkyl is optionally further substituted with one or more substituents selected from halogen, hydroxyl, cyano, C₁₋₆ alkyl or C₁₋₆ alkoxy;
each R^{2b} is independently selected from a hydrogen atom or C₁₋₆ alkyl;
R^{a} and R^{b} are each independently selected from a hydrogen atom, halogen, hydroxyl, cyano or C₁₋₆ alkyl, wherein the C₁₋₆ alkyl is optionally further substituted with one or more substituents selected from halogen, hydroxyl, cyano, C₁₋₆ alkyl or C₁₋₆ alkoxy;
each p is independently selected from 0, 1 or 2;
each q is independently selected from 0, 1 or 2; and
R¹ and X are as defined in formula (IIA).

A preferred solution of the present disclosure provides the compound as shown in formula (I), or the stereoisomer thereof, the tautomer thereof or the pharmaceutically acceptable salt thereof, which is a compound as shown in formula (II), or a stereoisomer thereof, a tautomer thereof or a pharmaceutically acceptable salt thereof: 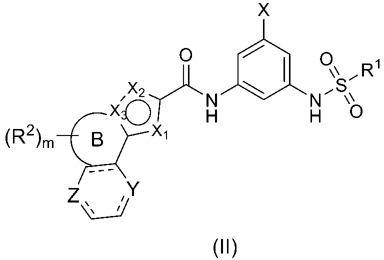 wherein: 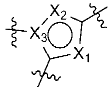
is 5-membered heteroaryl;
X₁ and X₂ are each independently selected from CH, S or N, and X₁ and X₂ are not S at the same time;
X₃ is selected from C or N; provided that when X₃ is C, X₁ and X₂ are not CH at the same time;
ring B is selected from 5-8 membered heterocyclyl, 6-10 membered aryl or 5-6 membered heteroaryl, wherein the heterocyclyl contains 1 or 2 double bonds; 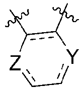
is 6-membered heteroaryl or 6-membered heterocyclyl;
-̅ -̅ -̅ is selected from a single bond or a double bond as required, so that each atom thereof is present in a normal valence state;
Y and Z are each independently selected from CR^{a}, O or NR^{b};
R^{a} is selected from a hydrogen atom, halogen, hydroxyl, cyano, C₁₋₆ alkyl or C₁₋₆ alkoxy, wherein the alkyl or alkoxy is optionally further substituted with one or more substituents selected from halogen, hydroxyl, cyano, C₁₋₆ alkyl or C₁₋₆ alkoxy;
R^{b} is selected from a hydrogen atom or C₁₋₆ alkyl, wherein the alkyl is optionally further substituted with one or more substituents selected from halogen, hydroxyl, cyano, C₁₋₆ alkyl or C₁₋₆ alkoxy;
m is selected from 0, 1, 2 or 3; and
R¹, R² and X are as defined in formula (I).

A preferred solution of the present disclosure provides the compound as shown in formula (I), or the stereoisomer thereof, the tautomer thereof or the pharmaceutically acceptable salt thereof, which is a compound as shown in formula (III), or a stereoisomer thereof, a tautomer thereof or a pharmaceutically acceptable salt thereof: 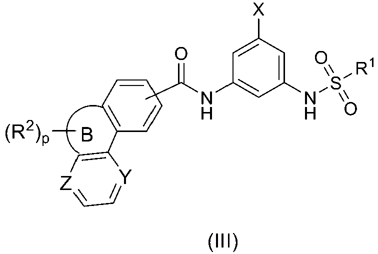 wherein:
ring B is selected from 5-8 membered heterocyclyl or 5-6 membered heteroaryl, wherein the heterocyclyl contains 1 or 2 double bonds;
Y and Z are each independently selected from CR^{a} or N;
R^{a} is selected from a hydrogen atom, halogen, hydroxyl, cyano, C₁₋₆ alkyl or C₁₋₆ alkoxy, wherein the C₁₋₆ alkyl or C₁₋₆ alkoxy is optionally further substituted with one or more substituents selected from halogen, hydroxyl, cyano, C₁₋₆ alkyl or C₁₋₆ alkoxy;
p is selected from 0, 1, 2 or 3; and
R¹, R² and X are as defined in formula (I).

A preferred solution of the present disclosure provides the compound as shown in formula (I), or the stereoisomer thereof, the tautomer thereof or the pharmaceutically acceptable salt thereof, wherein 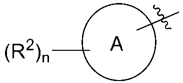 is selected from the following groups: 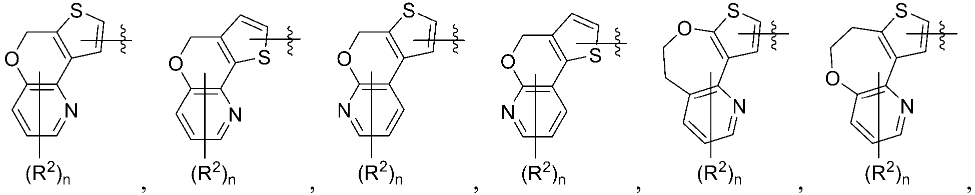 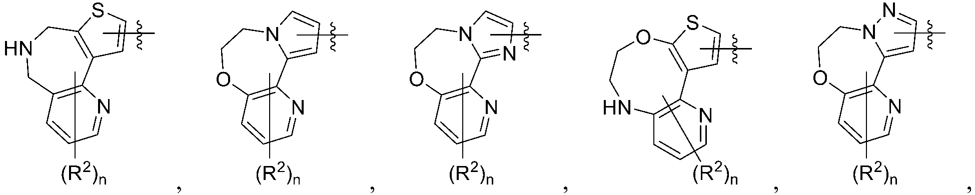 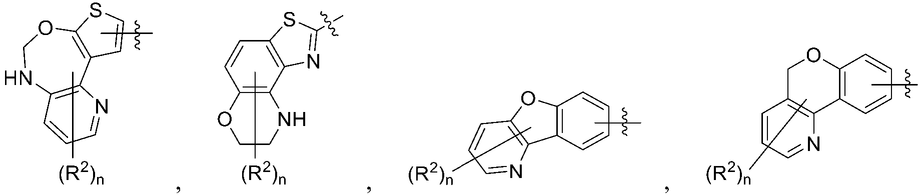 or 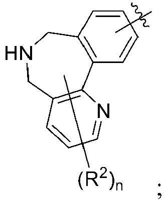 and
R² and n are as defined in formula (I).

A preferred solution of the present disclosure provides the compound as shown in formula (I), or the stereoisomer thereof, the tautomer thereof or the pharmaceutically acceptable salt thereof, wherein 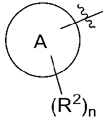 is selected from the following groups: 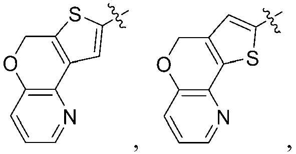 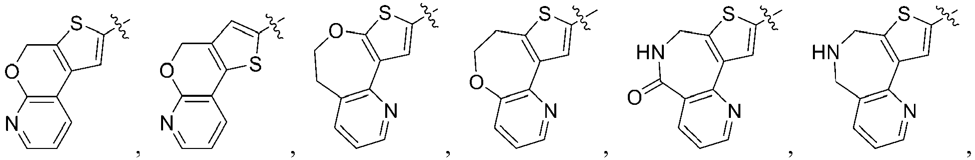 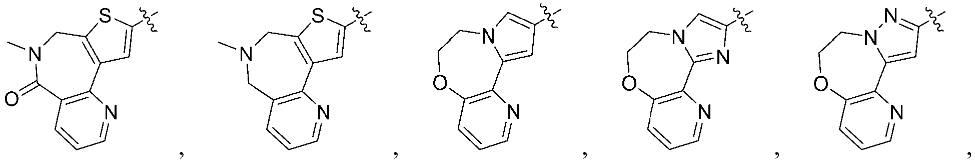 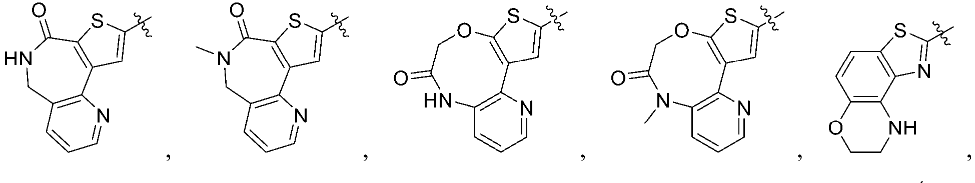 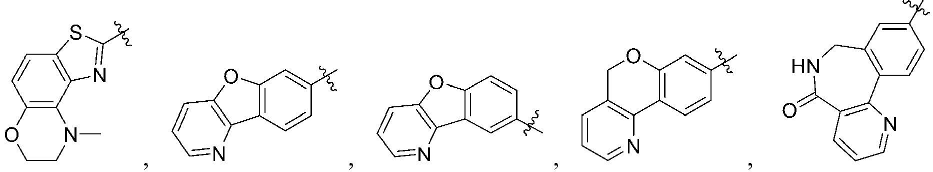 or

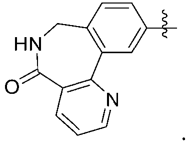

A preferred solution of the present disclosure provides the compound as shown in formula (IIA), or the stereoisomer thereof, the tautomer thereof or the pharmaceutically acceptable salt thereof, wherein 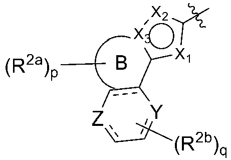 is selected from the following groups: 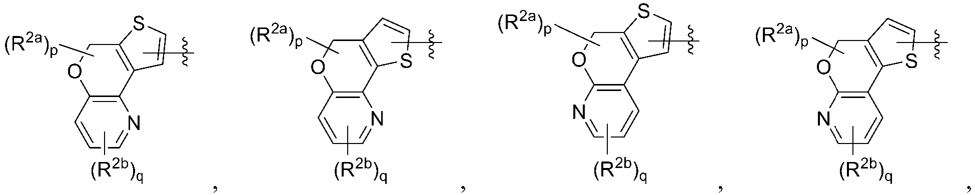 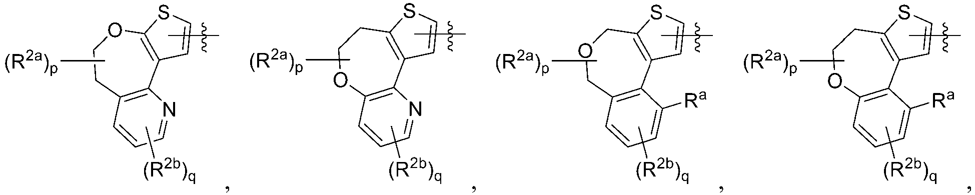 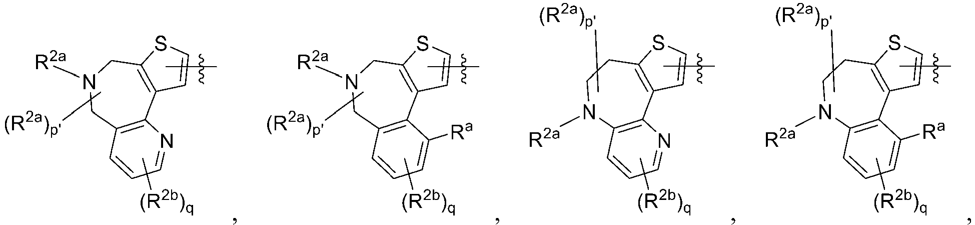 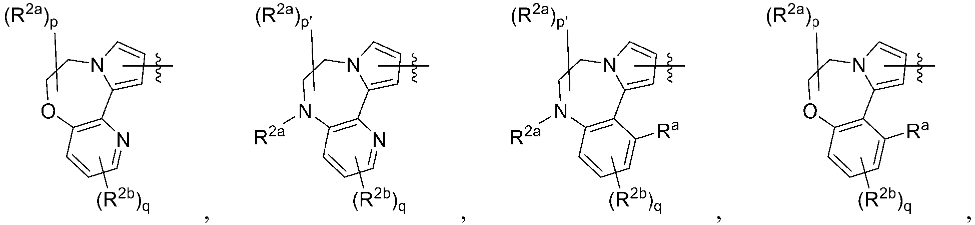 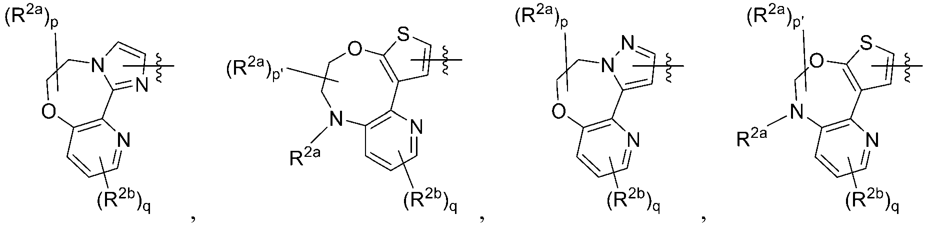 or 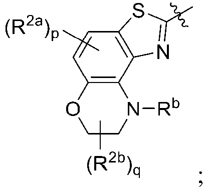
R^{2a} is selected from a hydrogen atom, C₁₋₆ alkyl, C₃₋₈ cycloalkyl or -C(O)R³, wherein the C₁₋₆ alkyl is optionally further substituted with one or more substituents selected from a deuterium atom, halogen, hydroxyl, cyano, C₁₋₆ alkyl, C₁₋₆ alkoxy or -C(O)OR⁶;
or, two R^{2a}, together with the same carbon atom to which the groups are attached, form a -C(O) or cyclopropyl;
each R^{2b} is independently selected from a hydrogen atom, C₁₋₆ alkyl, C₁₋₆ alkoxy or -C(O)OR³;
each R^{a} is independently selected from a hydrogen atom, halogen, cyano, C₁₋₆ alkyl, amino, -NHC(O)R³ or -O(CH₂)ₜR^{A}, wherein the C₁₋₆ alkyl is optionally further substituted with one or more substituents selected from halogen, cyano, C₁₋₆ alkyl or -OR^{A};
R^{b} is selected from a hydrogen atom or C₁₋₆ alkyl, wherein the C₁₋₆ alkyl is optionally further substituted with one or more halogens;
R^{A} is selected from a hydrogen atom, C₁₋₆ alkyl, C₃₋₈ cycloalkyl, C₆₋₁₀ aryl, 5-6 membered heteroaryl or 5-6 membered heterocyclyl, wherein the C₁₋₆ alkyl, C₃₋₈ cycloalkyl, C₆₋₁₀ aryl, 5-6 membered heteroaryl or 5-6 membered heterocyclyl is optionally further substituted with one or more substituents selected from hydroxyl, halogen, cyano, -NR⁷R⁸, C₁₋₆ alkyl, C₁₋₆ haloalkyl or C₁₋₆ alkoxy;
R³ is selected from a hydrogen atom, C₆₋₁₀ aryl, C₃₋₈ cycloalkyl or C₁₋₆ alkyl, wherein the C₆₋₁₀ aryl, C₃₋₈ cycloalkyl or C₁₋₆ alkyl is optionally further substituted with one or more substituents selected from halogen, hydroxyl, cyano, C₁₋₆ alkyl or C₁₋₆ alkoxy;
R⁶ is selected from a hydrogen atom or C₁₋₆ alkyl;
R⁷ and R⁸ are each independently selected from a hydrogen atom or C₁₋₆ alkyl;
t is selected from 0 or 1;
each p is independently selected from 0, 1, 2, 3, 4 or 5;
each p' is independently selected from 0, 1, 2, 3 or 4; and
each q is independently selected from 0, 1 or 2.

A preferred solution of the present disclosure provides the compound as shown in formula (IIA), or the stereoisomer thereof, the tautomer thereof or the pharmaceutically acceptable salt thereof, wherein R^{2a} is selected from a hydrogen atom, methyl, ethyl, isopropyl, 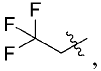 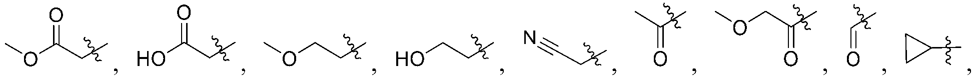 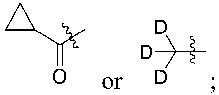 or, two R^{2a}, together with the same carbon atom to which the groups are attached, form a -C(O) or cyclopropyl.

A preferred solution of the present disclosure provides the compound as shown in formula (IIA), or the stereoisomer thereof, the tautomer thereof or the pharmaceutically acceptable salt thereof, wherein R^{a} is selected from a hydrogen atom, hydroxyl, cyano, methyl, methoxy, trifluoromethyl, trifluoromethoxy, amino,

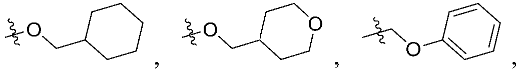

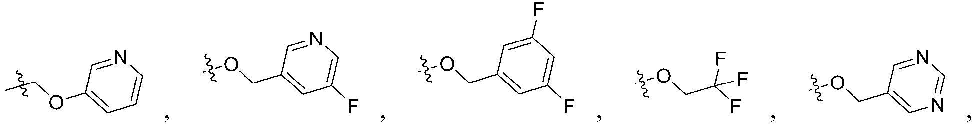

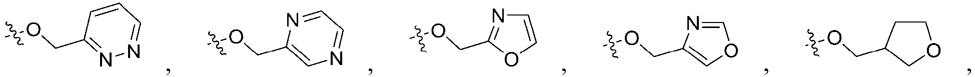

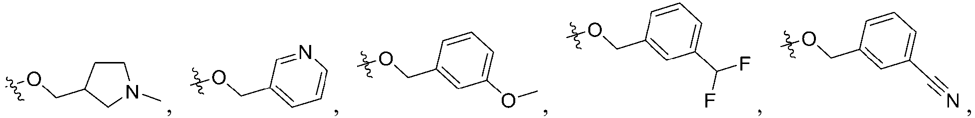

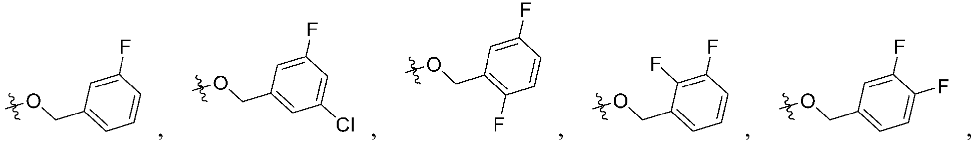

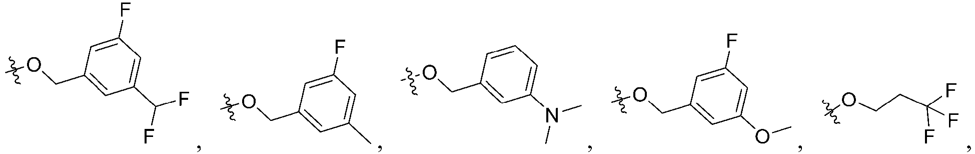

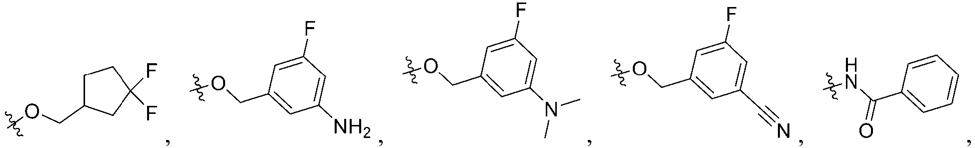

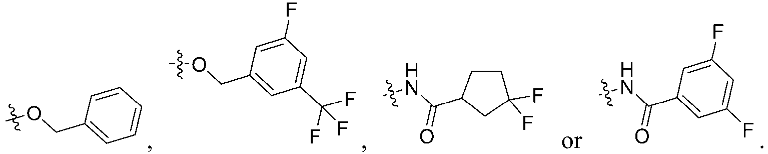

A preferred solution of the present disclosure provides the compound as shown in formula (IIA), or the stereoisomer thereof, the tautomer thereof or the pharmaceutically acceptable salt thereof, wherein q is 0.

A preferred solution of the present disclosure provides the compound as shown in formula (IIA), or the stereoisomer thereof, the tautomer thereof or the pharmaceutically acceptable salt thereof, wherein 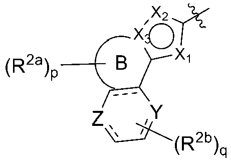 is selected from the following groups: 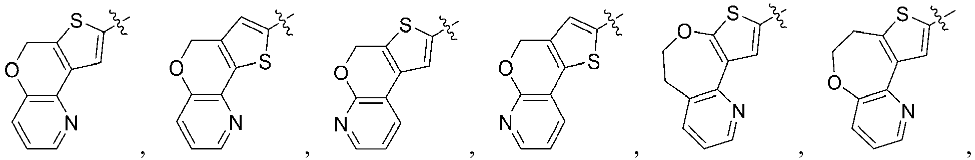 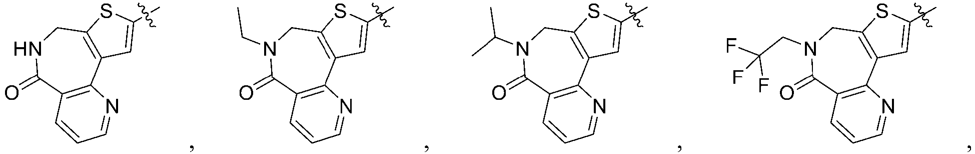 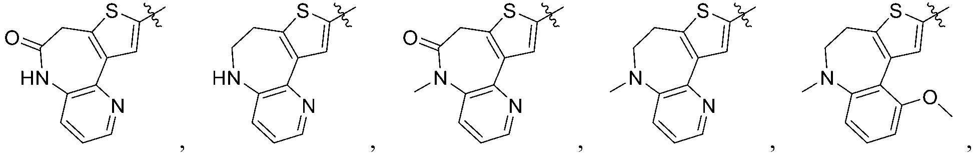 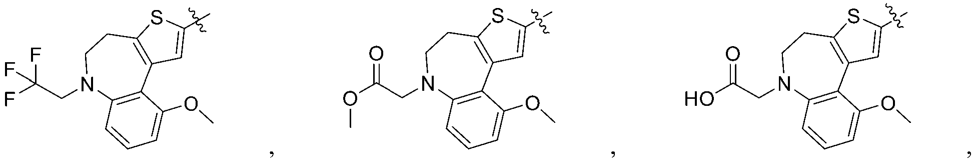 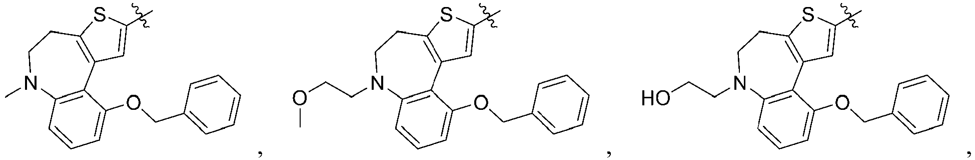 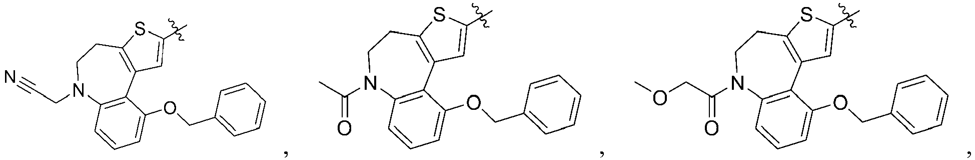 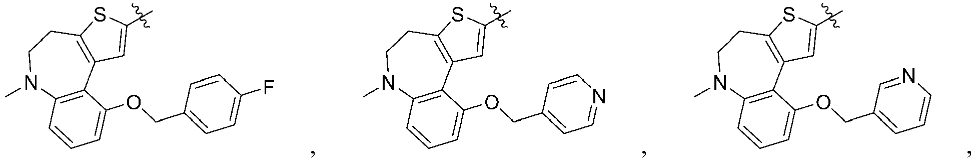 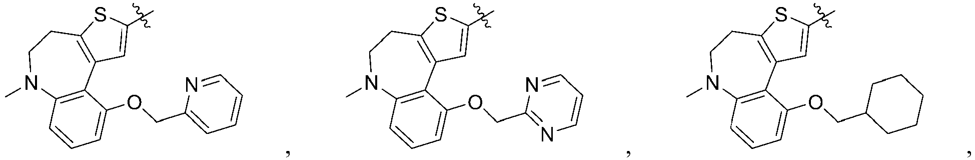 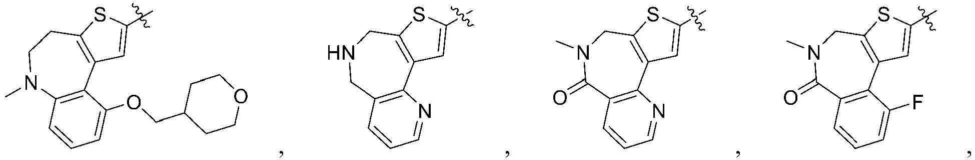 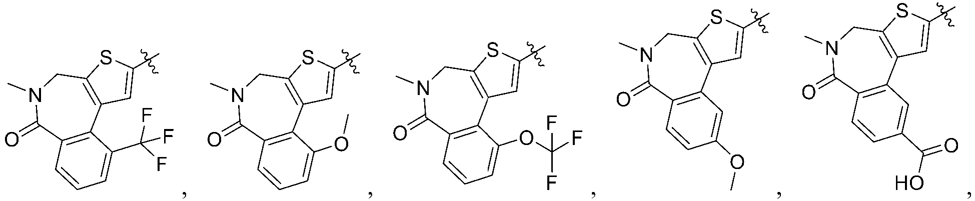 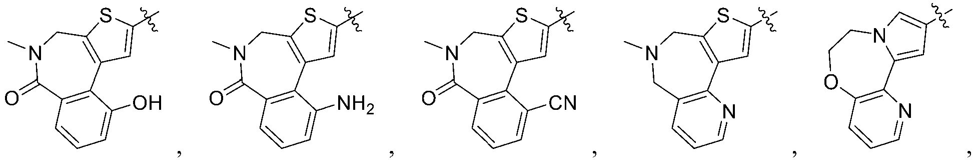 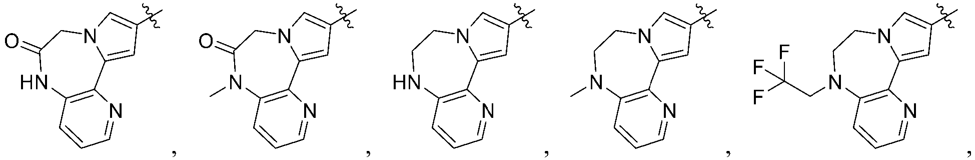 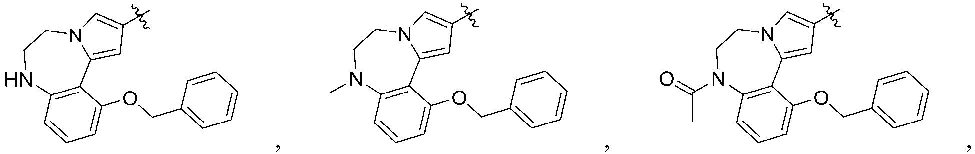 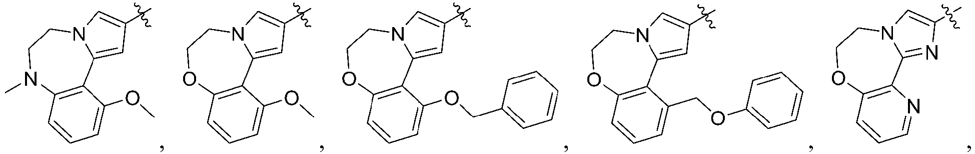 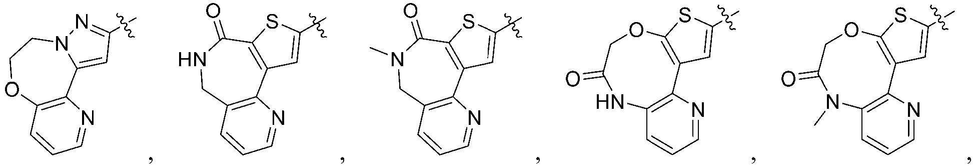 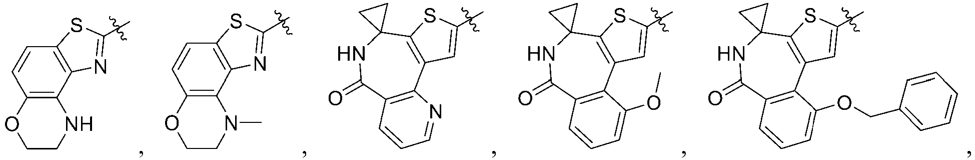 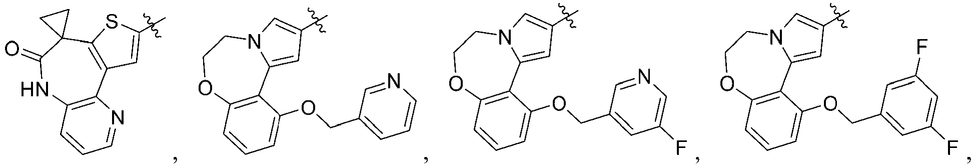 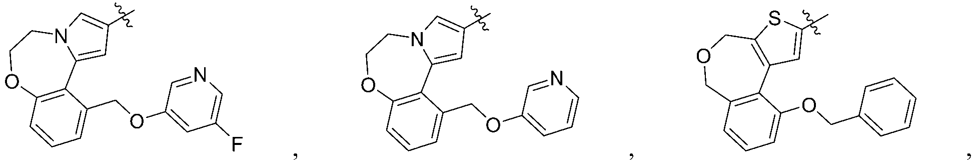 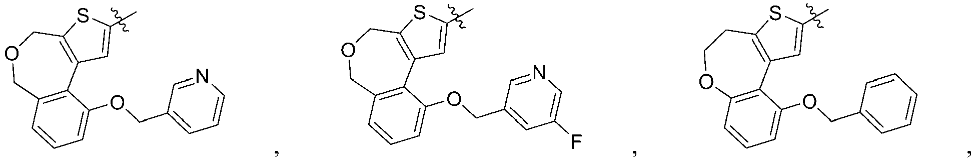 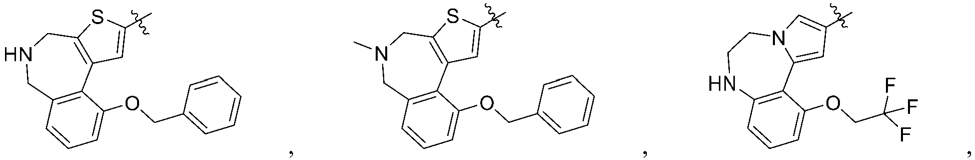 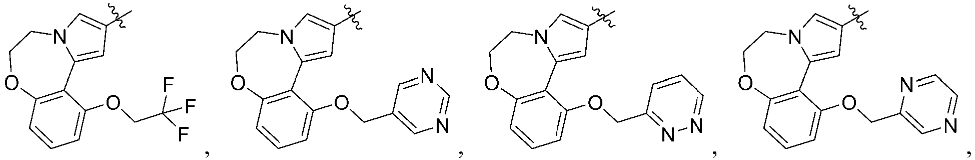 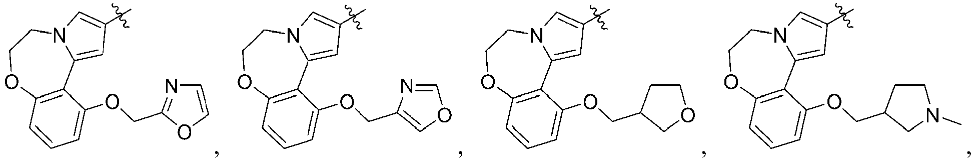 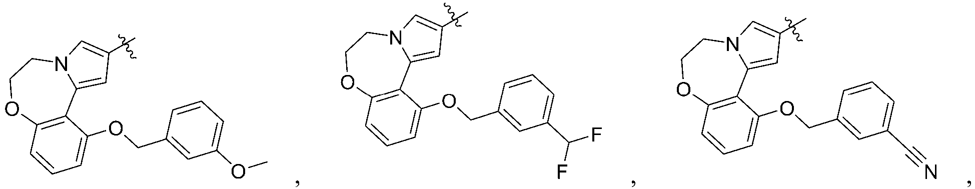 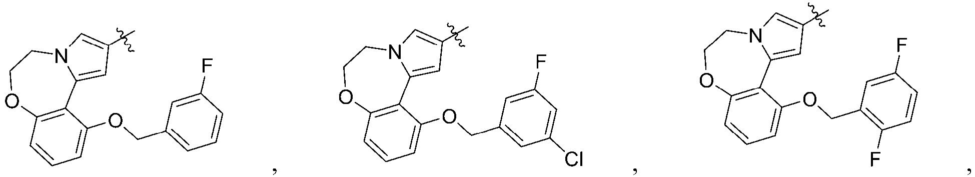 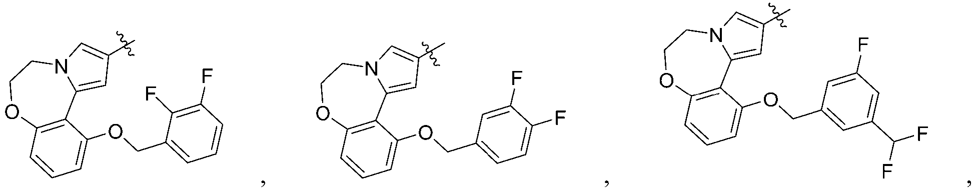 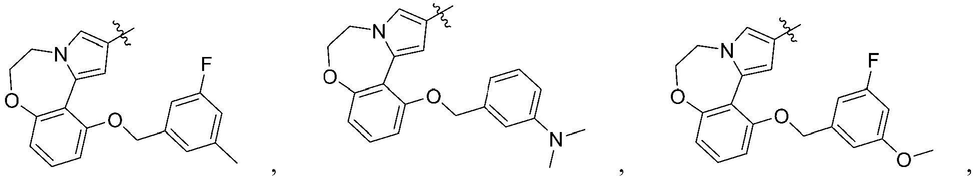 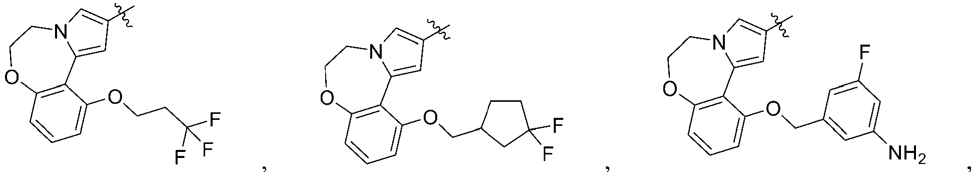 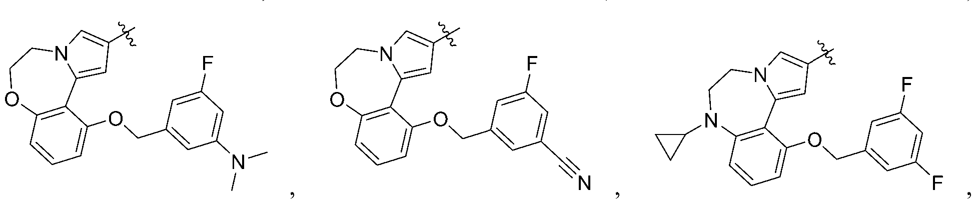 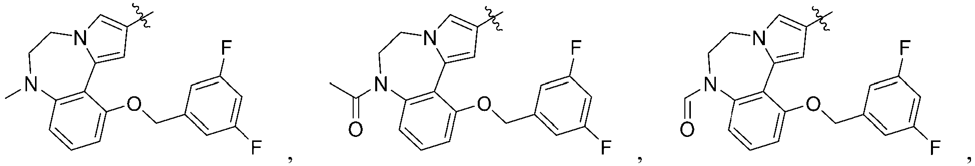 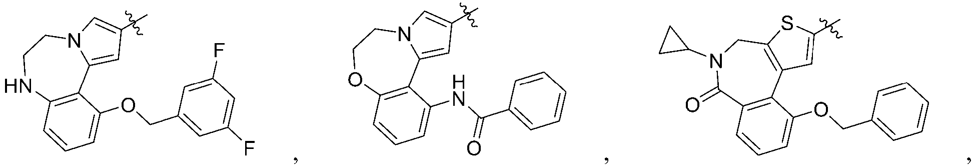 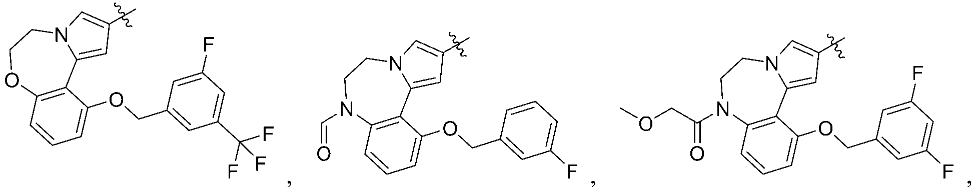 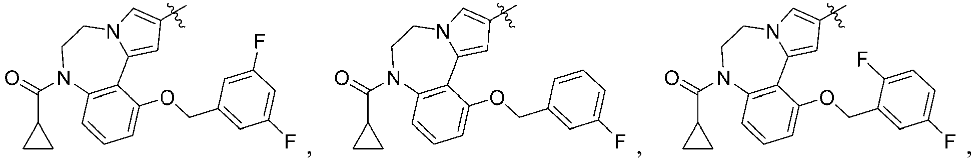 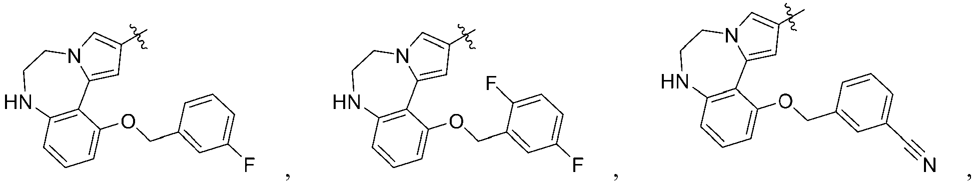 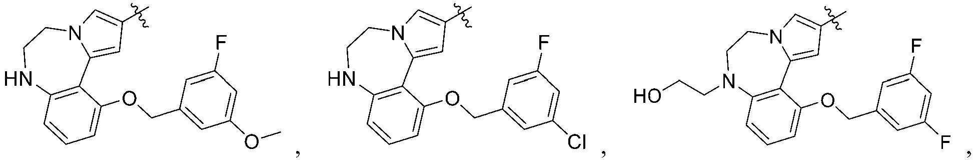 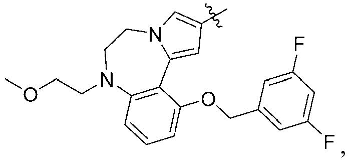 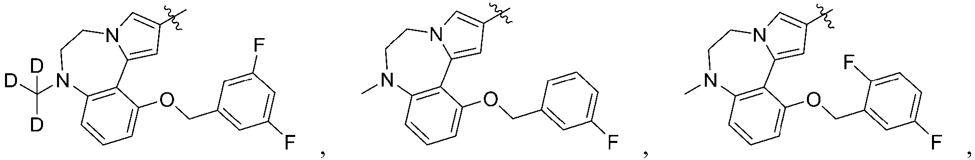 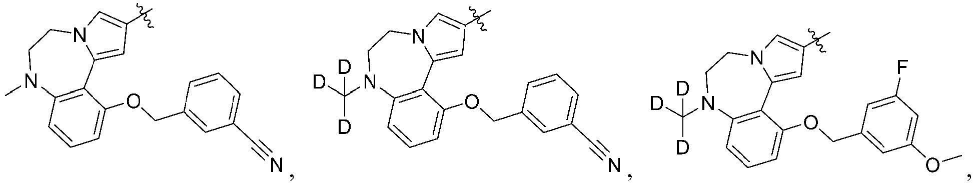 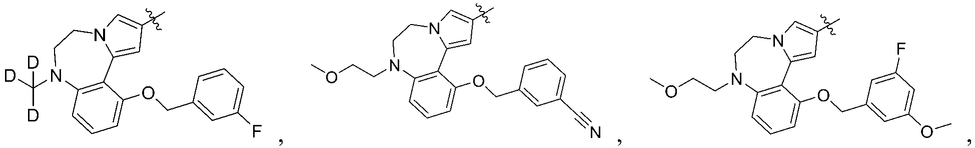 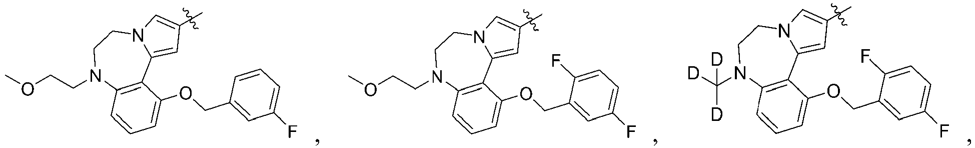 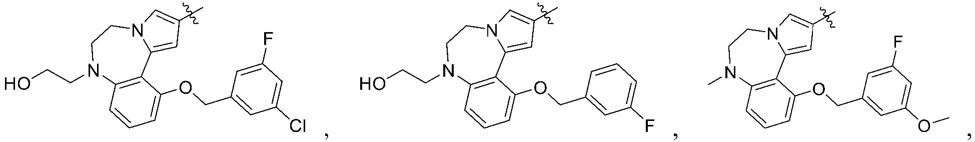 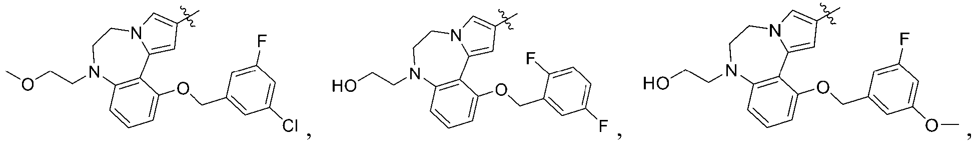 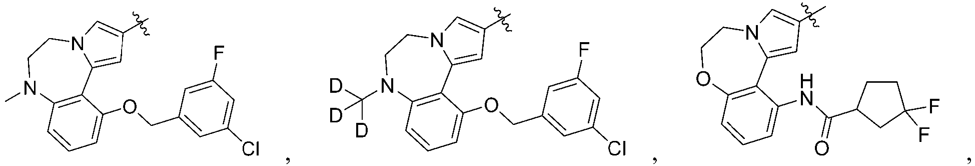 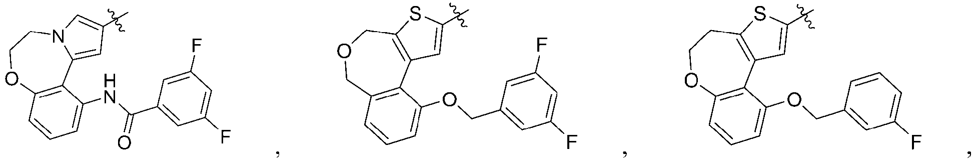 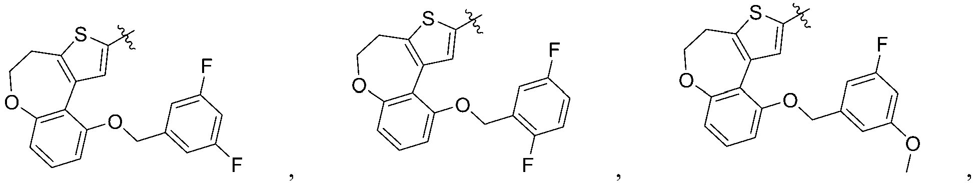 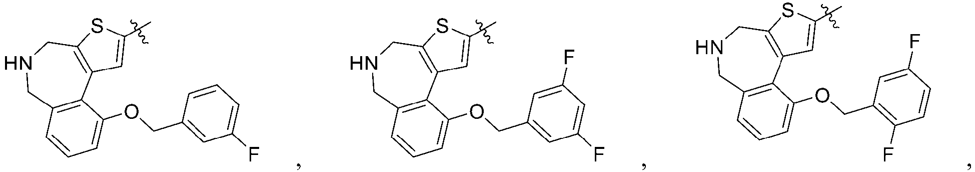 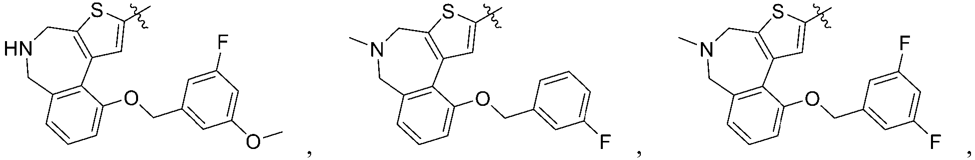 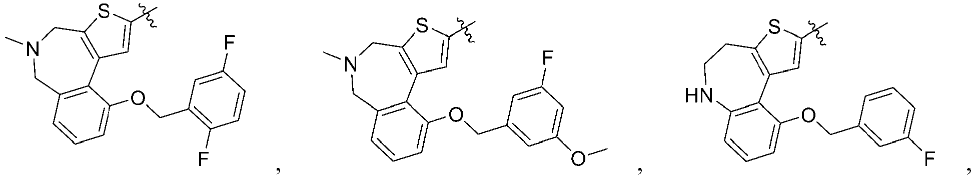 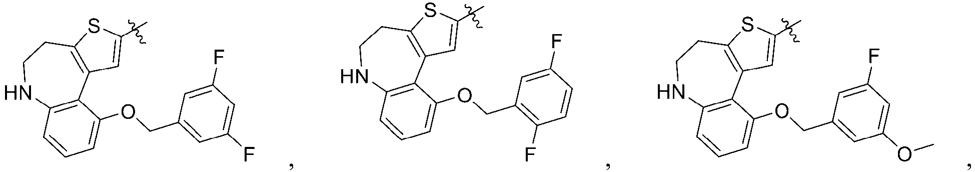 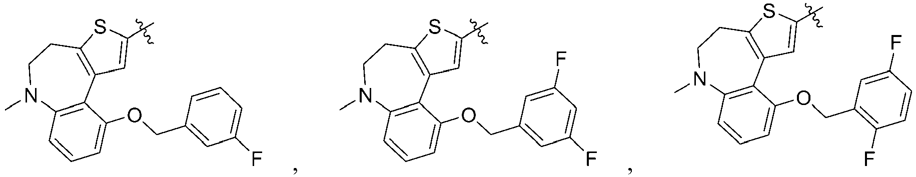 or

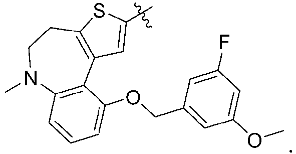

A preferred solution of the present disclosure provides the compound as shown in formula (I), (II), (IIA), (III), (IIIA), (IIIB) or (IIIC), or the stereoisomer thereof, the tautomer thereof or the pharmaceutically acceptable salt thereof, wherein X is selected from Cl or Br, preferably Cl.

A preferred solution of the present disclosure provides the compound as shown in formula (I), (II), (IIA), (III), (IIIA), (IIIB) or (IIIC), or the stereoisomer thereof, the tautomer thereof or the pharmaceutically acceptable salt thereof, wherein R¹ is selected from methyl or ethyl.

In a preferred solution of the present disclosure, the compound as shown in the formula is selected from:

| Serial number of compound | **Structure** | Name |
|---|---|---|
| **Example 1** | 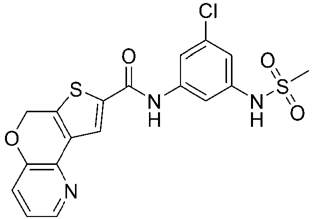 | N-(3-chloro-5-(methylsulfonamido)phen yl)-6H-thieno[3',2':4,5]pyrano[3,2-b]pyr idine-8-carboxamide |
| **Example 2** | 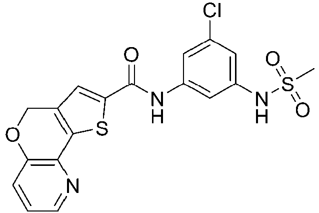 | N-(3-chloro-5-(methylsulfonamido)phen yl)-4H-thieno[2',3':4,5]pyrano[3,2-b]pyr idine-2-carboxamide |
| **Example 3** | 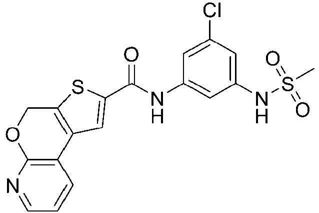 | N-(3-chloro-5-(methylsulfonamido)phen yl)-4H-thieno[3',2':4,5]pyrano[2,3-b]pyr idine-2-carboxamide |
| **Example 4** | 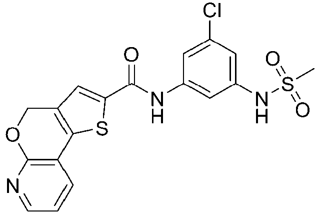 | N-(3-chloro-5-(methylsulfonamido)phen yl)-4H-thieno[2',3':4,5]pyrano[2,3-b]pyr idine-2-carboxamide |
| **Example 5** | 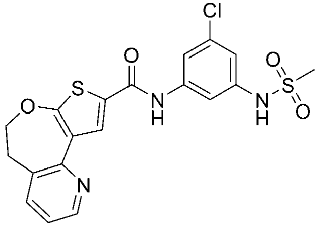 | N-(3-chloro-5-(methylsulfonamido)phen yl)-5,6-dihydrothieno[2',3':2,3]oxepino[ 4,5-b]pyridine-9-carboxamide |
| **Example 6** | 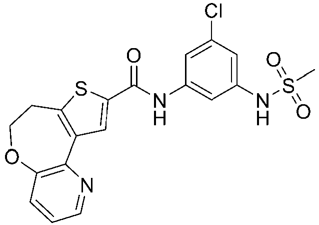 | N-(3-chloro-5-(methylsulfonamido)phen yl)-6,7-dihydrothieno[3',2':4,5]oxepino[ 3,2-b]pyridine-9-carboxamide |
| **Example 7** | 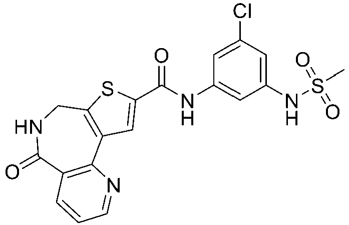 | N-(3-chloro-5-(methylsulfonamido)phen yl)-5-oxo-6,7-dihydro-5H-pyrido[3,2-c]t hieno[3,2-e]azepine-9-carboxamide |
| **Example 8** | 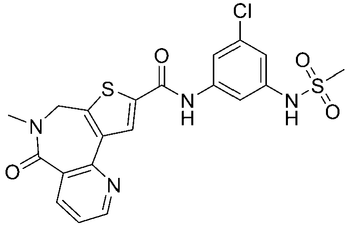 | N-(3-chloro-5-(methylsulfonamido)phen yl)-6-methyl-5-oxo-6,7-dihydro-5H-pyri do[3,2-c]thieno[3,2-e]azepine-9-carboxa mide |
| **Example 9** | 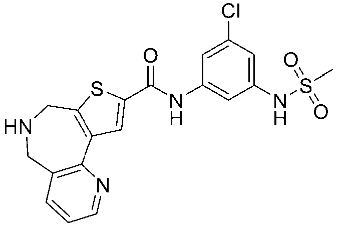 | N-(3-chloro-5-(methylsulfonamido)phen yl)-6,7-dihydro-5H-pyrido[3,2-c]thieno[ 3,2-e]azepine-9-carboxamide |
| **Example 10** | 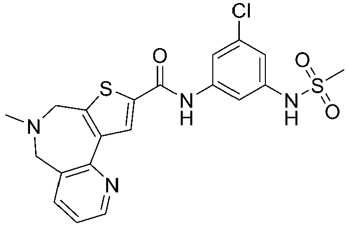 | N-(3-chloro-5-(methylsulfonamido)phen yl)-6-methyl-6,7-dihydro-5H-pyrido[3,2-c]thieno[3,2-e]azepine-9-carboxamide |
| **Example 11** | 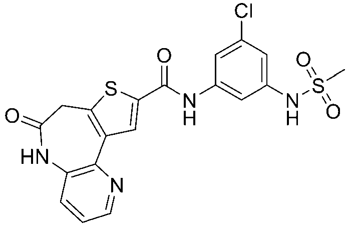 | N-(3-chloro-5-(methylsulfonamido)phen yl)-6-oxo-6,7-dihydro-5H-pyrido[3,2-b]t hieno[3,2-d]azepine-9-carboxamide |
| **Example 12** | 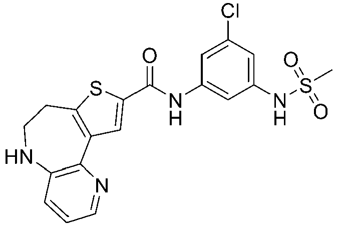 | N-(3-chloro-5-(methylsulfonamido)phen yl)-6,7-dihydro-5H-pyrido[3,2-b]thieno[ 3,2-d]azepine-9-carboxamide |
| **Example 13** | 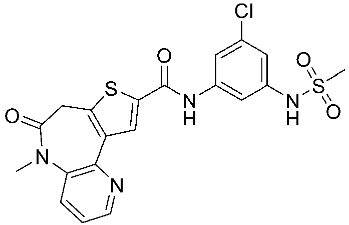 | N-(3-chloro-5-(methylsulfonamido)phen yl)-5-methyl-6-oxo-6,7-dihydro-5H-pyri do[3,2-b]thieno[3,2-d]azepine-9-carboxa mide |
| **Example 14** | 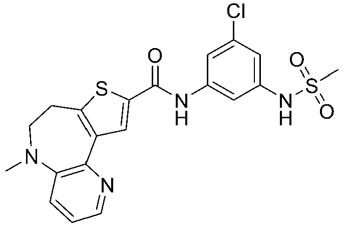 | N-(3-chloro-5-(methylsulfonamido)phen yl)-5-methyl-6,7-dihydro-5H-pyrido[3,2-b]thieno[3,2-d]azepine-9-carboxamide |
| **Example 15** | 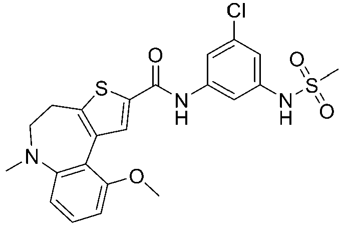 | N-(3-chloro-5-(methylsulfonamido)phen yl)-10-methoxy-6-methyl-5,6-dihydro-4 H-benzo[b]thieno[3,2-d]azepine-2-carbo xamide |
| **Example 16** | 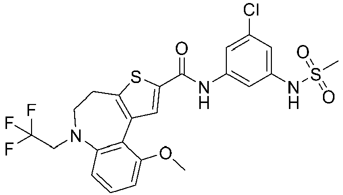 | N-(3-chloro-5-(methylsulfonamido)phen yl)-10-methoxy-6-(2,2,2-trifluoroethyl)-5 ,6-dihydro-4H-benzo[b]thieno[3,2-d]aze pine-2-carboxamide |
| **Example 17** | 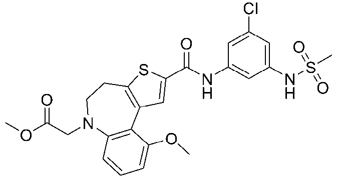 | methyl 2-(2-((3-chloro-5-(methylsulfonamido)p henyl)carbamoyl)-10-methoxy-4,5-dihyd ro-6H-benzo[b]thieno[3,2-d]azepin-6-yl) acetate |
| **Example 18** | 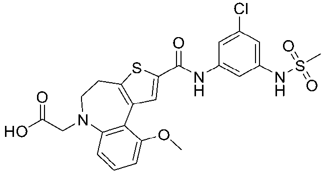 | 2-(2-((3-chloro-5-(methylsulfonamido)p henyl)carbamoyl)-10-methoxy-4,5-dihyd ro-6H-benzo[b]thieno[3,2-d]azepin-6-yl) acetic acid |
| **Example 19** | 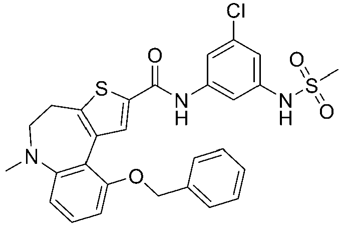 | 10-(benzyloxy)-N-(3-chloro-5-(methylsu lfonamido)phenyl)-6-methyl-5,6-dihydro -4H-benzo[b]thieno[3,2-d]azepine-2-carb oxamide |
| **Example 20** | 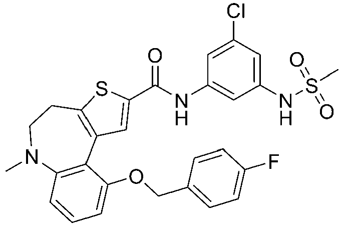 | N-(3-chloro-5-(methylsulfonamido)phen yl)-10-((4-fluorobenzyl)oxy)-6-methyl-5, 6-dihydro-4H-benzo[b]thieno[3,2-d]azep ine-2-carboxamide |
| **Example 20** | 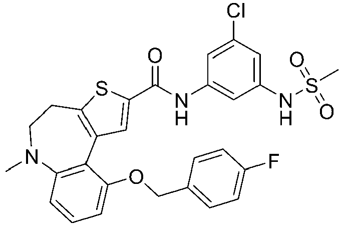 | N-(3-chloro-5-(methylsulfonamido)phen yl)-10-((4-fluorobenzyl)oxy)-6-methyl-5, 6-dihydro-4H-benzo[b]thieno[3,2-d]azep ine-2-carboxamide |
| **Example 21** | 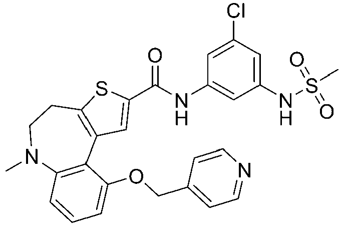 | N-(3-chloro-5-(methylsulfonamido)phen yl)-6-methyl-10-(pyridin-4-ylmethoxy)-5 ,6-dihydro-4H-benzo[b]thieno[3,2-d]aze pine-2-carboxamide |
| **Example 22** | 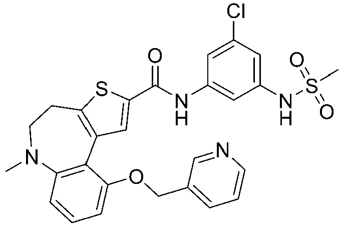 | N-(3-chloro-5-(methylsulfonamido)phen yl)-6-methyl-10-(pyridin-3-ylmethoxy)-5 ,6-dihydro-4H-benzo[b]thieno[3,2-d]aze pine-2-carboxamide |
| **Example 23** | 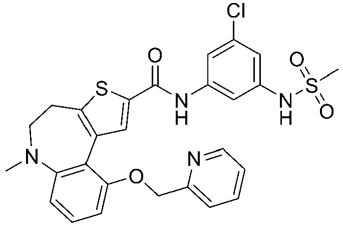 | N-(3-chloro-5-(methylsulfonamido)phen yl)-6-methyl-10-(pyridin-2-ylmethoxy)-5 ,6-dihydro-4H-benzo[b]thieno[3,2-d]aze pine-2-carboxamide |
| **Example 24** | 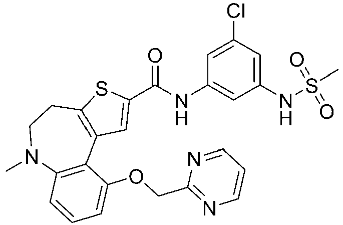 | N-(3-chloro-5-(methylsulfonamido)phen yl)-6-methyl-10-(pyrimidin-2-ylmethoxy )-5,6-dihydro-4H-benzo[b]thieno[3,2-d]a zepine-2-carboxamide |
| **Example 25** | 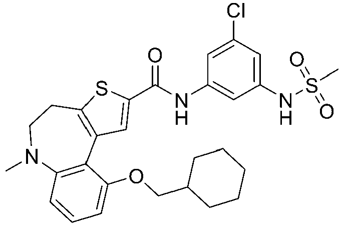 | N-(3-chloro-5-(methylsulfonamido)phen yl)-10-(cyclohexylmethoxy)-6-methyl-5, 6-dihydro-4H-benzo[b]thieno[3,2-d]azep ine-2-carboxamide |
| **Example 26** | 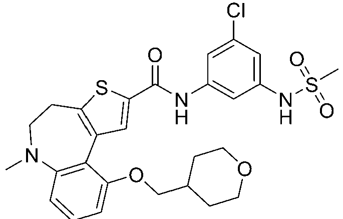 | N-(3-chloro-5-(methylsulfonamido)phen yl)-6-methyl-10-((tetrahydro-2H-pyran-4 -yl)methoxy)-5,6-dihydro-4H-benzo[b]th ieno[3,2-d]azepine-2-carboxamide |
| **Example 27** | 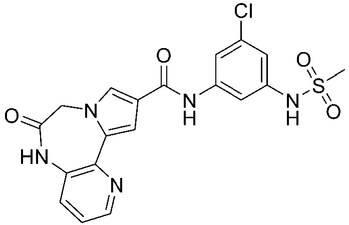 | N-(3-chloro-5-(methylsulfonamido)phen yl)-6-oxo-6,7-dihydro-5H-pyrido[2,3-f]p yrrolo[1,2-d][1,4]diazepine-10-carboxam ide |
| **Example 28** | 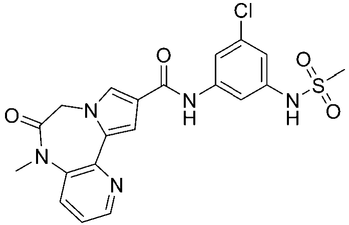 | N-(3-chloro-5-(methylsulfonamido)phen yl)-5-methyl-6-oxo-6,7-dihydro-5H-pyri do[2,3-f]pyrrolo[1,2-d][1,4]diazepine-10 -carboxamide |
| **Example 29** | 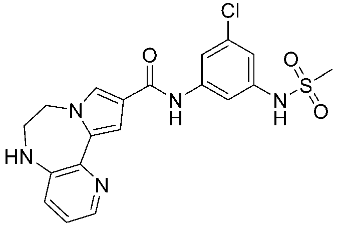 | N-(3-chloro-5-(methylsulfonamido)phen yl)-6,7-dihydro-5H-pyrido[2,3-f]pyrrolo[ 1,2-d][1,4]diazepine-10-carboxamide |
| **Example 30** | 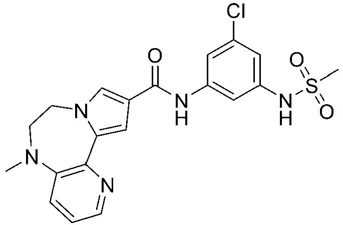 | N-(3-chloro-5-(methylsulfonamido)phen yl)-5-methyl-6,7-dihydro-5H-pyrido[2,3-f]pyrrolo[1,2-d][1,4]diazepine-10-carbox amide |
| **Example 31** | 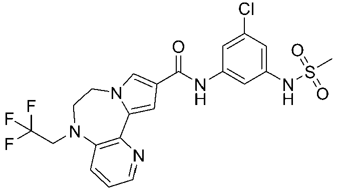 | N-(3-chloro-5-(methylsulfonamido)phen yl)-5-(2,2,2-trifluoroethyl)-6,7-dihydro-5 H-pyrido[2,3-f]pyrrolo[1,2-d][1,4]diazep ine-10-carboxamide |
| **Example 32** | 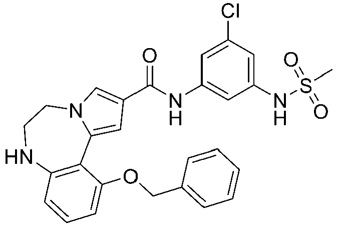 | 11-(benzyloxy)-N-(3-chloro-5-(methylsu lfonamido)phenyl)-6,7-dihydro-5H-benz o[f]pyrrolo[1,2-d][1,4]diazepine-2-carbo xamide |
| **Example 33** | 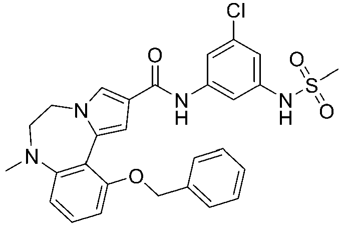 | 11-(benzyloxy)-N-(3-chloro-5-(methylsu lfonamido)phenyl)-7-methyl-6,7-dihydro -5H-benzo[f]pyrrolo[1,2-d][1,4]diazepin e-2-carboxamide |
| **Example 34** | 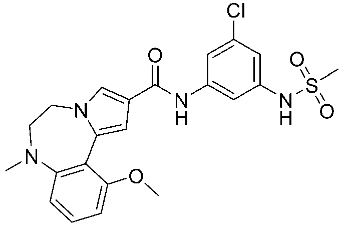 | N-(3-chloro-5-(methylsulfonamido)phen yl)-11-methoxy-7-methyl-6,7-dihydro-5 H-benzo[f]pyrrolo[1,2-d][1,4]diazepine-2-carboxamide |
| **Example 35** | 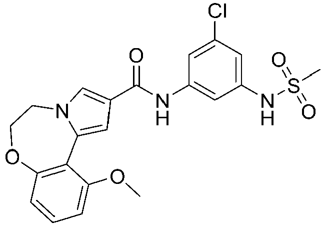 | N-(3-chloro-5-(methylsulfonamido)phen yl)-11-methoxy-5,6-dihydrobenzo[f]pyrr olo[1,2-d][1,4]oxazepine-2-carboxamide |
| **Example 36** | 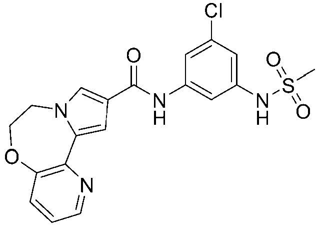 | N-(3-chloro-5-(methylsulfonamido)phen yl)-6,7-dihydropyrido[2,3-f]pyrrolo[1,2-d][1,4]oxazepine-10-carboxamide |
| **Example 37** | 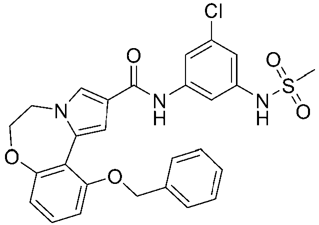 | 11-(benzyloxy)-N-(3-chloro-5-(methylsu lfonamido)phenyl)-5,6-dihydrobenzo[f]p yrrolo[1,2-d][1,4]oxazepine-2-carboxami de |
| **Example 38** | 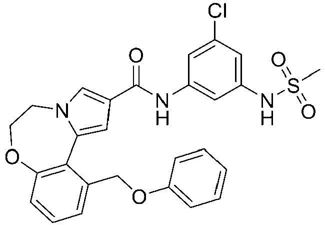 | N-(3-chloro-5-(methylsulfonamido)phen yl)-11-(phenoxymethyl)-5,6-dihydrobenz o[f]pyrrolo[1,2-d][1,4]oxazepine-2-carbo xamide |
| **Example 39** | 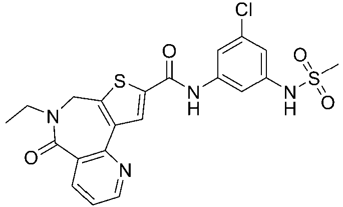 | N-(3-chloro-5-(methylsulfonamido)phen yl)-6-ethyl-5-oxo-6,7-dihydro-5H-pyrido [3,2-c]thieno[3,2-e]azepine-9-carboxami de |
| **Example 40** | 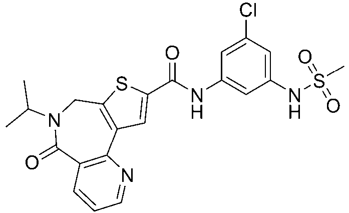 | N-(3-chloro-5-(methylsulfonamido)phen yl)-6-isopropyl-5-oxo-6,7-dihydro-5H-py rido[3,2-c]thieno[3,2-e]azepine-9-carbox amide |
| **Example 41** | 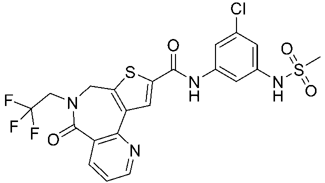 | N-(3-chloro-5-(methylsulfonamido)phen yl)-5-oxo-6-(2,2,2-trifluoroethyl)-6,7-dih ydro-5H-pyrido[3,2-c]thieno[3,2-e]azepi ne-9-carboxamide |
| **Example 42** | 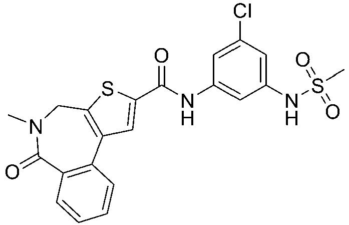 | N-(3-chloro-5-(methylsulfonamido)phen yl)-5-methyl-6-oxo-5,6-dihydro-4H-benz o[c]thieno[3,2-e]azepine-2-carboxamide |
| **Example 43** | 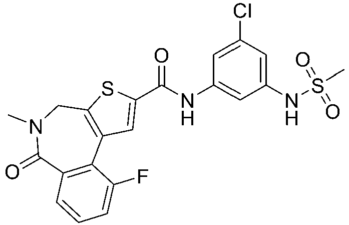 | N-(3-chloro-5-(methylsulfonamido)phen yl)-10-fluoro-5-methyl-6-oxo-5,6-dihydr o-4H-benzo[c]thieno[3,2-e]azepine-2-car boxamide |
| **Example 44** | 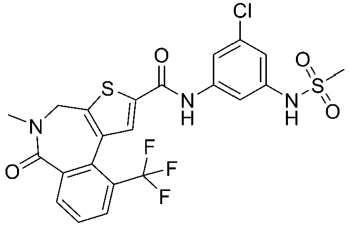 | N-(3-chloro-5-(methylsulfonamido)phen yl)-5-methyl-6-oxo-10-(trifluoromethyl)-5,6-dihydro-4H-benzo[c]thieno[3,2-e]aze pine-2-carboxamide |
| **Example 45** | 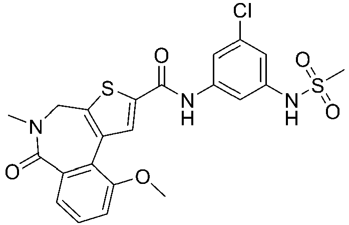 | N-(3-chloro-5-(methylsulfonamido)phen yl)-10-methoxy-5-methyl-6-oxo-5,6-dihy dro-4H-benzo[c]thieno[3,2-e]azepine-2-c arboxamide |
| **Example 46** | 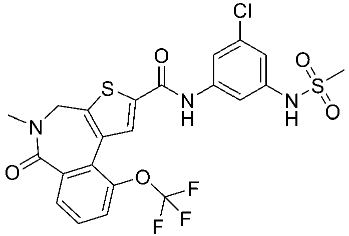 | N-(3-chloro-5-(methylsulfonamido)phen yl)-5-methyl-6-oxo-10-(trifluoromethoxy )-5,6-dihydro-4H-benzo[c]thieno[3,2-e]a zepine-2-carboxamide |
| **Example 47** | 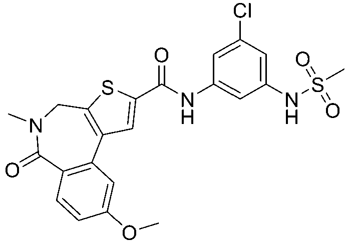 | N-(3-chloro-5-(methylsulfonamido)phen yl)-9-methoxy-5-methyl-6-oxo-5,6-dihyd ro-4H-benzo[c]thieno[3,2-e]azepine-2-ca rboxamide |
| **Example 48** | 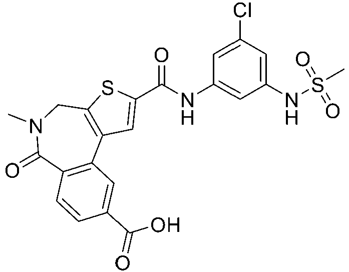 | 2-((3-chloro-5-(methylsulfonamido)phen yl)carbamoyl)-5-methyl-6-oxo-5,6-dihyd ro-4H-benzo[c]thieno[3,2-e]azepine-9-ca rboxylic acid |
| **Example 49** | 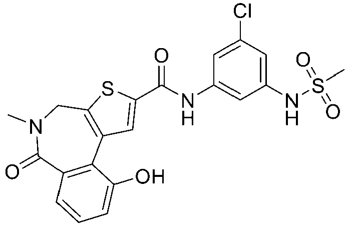 | N-(3-chloro-5-(methylsulfonamido)phen yl)-10-hydroxy-5-methyl-6-oxo-5,6-dihy dro-4H-benzo[c]thieno[3,2-e]azepine-2-c arboxamide |
| **Example 50** | 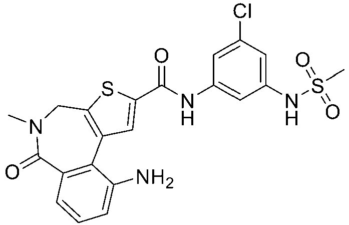 | 10-amino-N-(3-chloro-5-(methylsulfona mido)phenyl)-5-methyl-6-oxo-5,6-dihydr o-4H-benzo[c]thieno[3,2-e]azepine-2-car boxamide |
| **Example 51** | 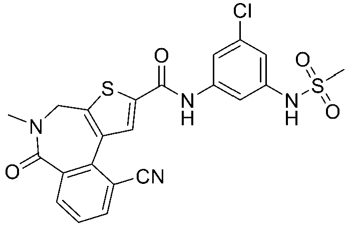 | N-(3-chloro-5-(methylsulfonamido)phen yl)-10-cyano-5-methyl-6-oxo-5,6-dihydr o-4H-benzo[c]thieno[3,2-e]azepine-2-car boxamide |
| **Example 52** | 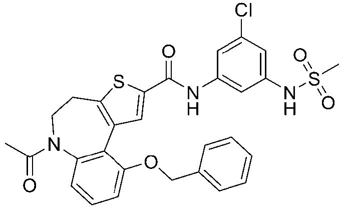 | 6-acetyl-10-(benzyloxy)-N-(3-chloro-5-( methylsulfonamido)phenyl)-5,6-dihydro-4H-benzo[b]thieno[3,2-d]azepine-2-carb oxamide |
| **Example 53** | 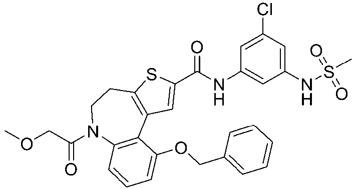 | 10-(benzyloxy)-N-(3-chloro-5-(methylsu lfonamido)phenyl)-6-(2-methoxyacetyl)-5,6-dihydro-4H-benzo[b]thieno[3,2-d]az epine-2-carboxamide |
| **Example 54** | 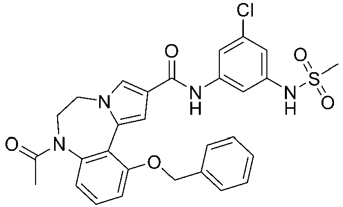 | 7-acetyl-11-(benzyloxy)-N-(3-chloro-5-( methylsulfonamido)phenyl)-6,7-dihydro-5H-benzo[f]pyrrolo[1,2-d][1,4]diazepine -2-carboxamide |
| **Example 55** | 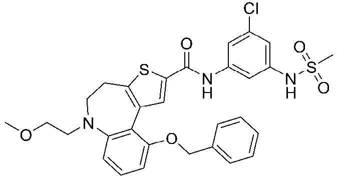 | 10-(benzyloxy)-N-(3-chloro-5-(methylsu lfonamido)phenyl)-6-(2-methoxyethyl)-5 ,6-dihydro-4H-benzo[b]thieno[3,2-d]aze pine-2-carboxamide |
| **Example 56** | 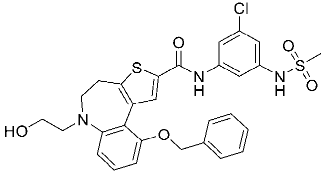 | 10-(benzyloxy)-N-(3-chloro-5-(methylsu lfonamido)phenyl)-6-(2-hydroxyethyl)-5, 6-dihydro-4H-benzo[b]thieno[3,2-d]azep ine-2-carboxamide |
| **Example 57** | 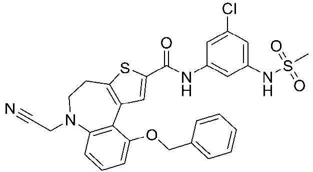 | 10-(benzyloxy)-N-(3-chloro-5-(methylsu lfonamido)phenyl)-6-(cyanomethyl)-5,6-dihydro-4H-benzo[b]thieno[3,2-d]azepin e-2-carboxamide |
| **Example 58** | 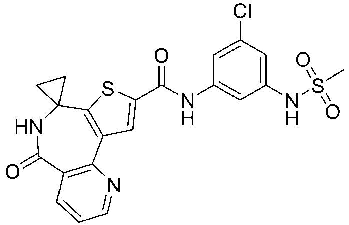 | N-(3-chloro-5-(methylsulfonamido)phen yl)-5'-oxo-5',6'-dihydrospiro[cyclopropa ne-1,7'-pyrido[3,2-c]thieno[3,2-e]azepin e]-9'-carboxamide |
| **Example 59** | 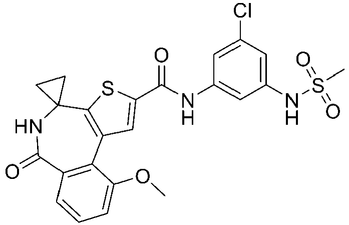 | N-(3-chloro-5-(methylsulfonamido)phen yl)-10-methoxy-6-oxo-5,6-dihydrospiro[ benzo[c]thieno[3,2-e]azepine-4,1'-cyclo propane]-2-carboxamide |
| **Example 60** | 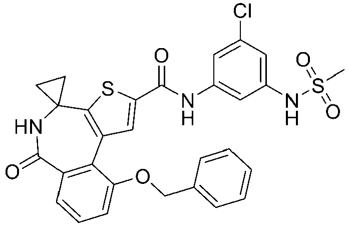 | 10-(benzyloxy)-N-(3-chloro-5-(methylsu lfonamido)phenyl)-6-oxo-5,6-dihydrospi ro[benzo[c]thieno[3,2-e]azepine-4,1'-cyc lopropane]-2-carboxamide |
| **Example 61** | 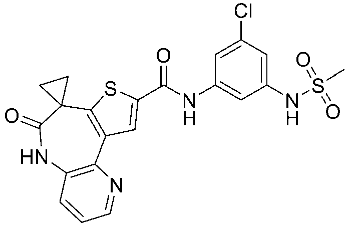 | N-(3-chloro-5-(methylsulfonamido)phen yl)-6'-oxo-5',6'-dihydrospiro[cyclopropa ne-1,7'-pyrido[3,2-b]thieno[3,2-d]azepin e]-9'-carboxamide |
| **Example 62** | 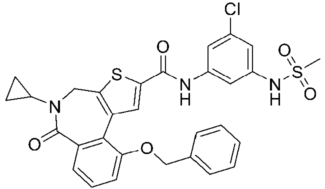 | 10-(benzyloxy)-N-(3-chloro-5-(methylsu lfonamido)phenyl)-5-cyclopropyl-6-oxo-5,6-dihydro-4H-benzo[c]thieno[3,2-e]aze pine-2-carboxamide |
| **Example 63** | 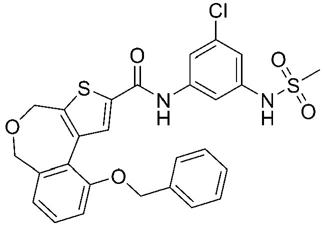 | 10-(benzyloxy)-N-(3-chloro-5-(methylsu lfonamido)phenyl)-4,6-dihydrobenzo[c]t hieno[3,2-e]oxepine-2-carboxamide |
| **Example 64** | 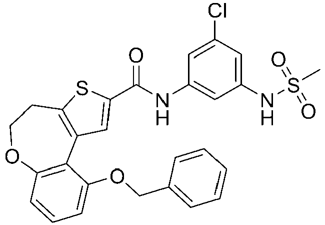 | 10-(benzyloxy)-N-(3-chloro-5-(methylsu lfonamido)phenyl)-4,5-dihydrobenzo[b]t hieno[3,2-d]oxepine-2-carboxamide |
| **Example 65** | 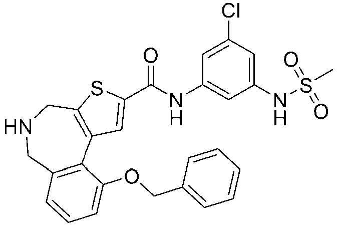 | 10-(benzyloxy)-N-(3-chloro-5-(methylsu lfonamido)phenyl)-5,6-dihydro-4H-benz o[c]thieno[3,2-e]azepine-2-carboxamide |
| **Example 66** | 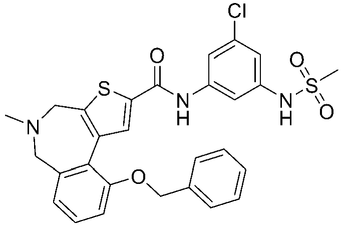 | 10-(benzyloxy)-N-(3-chloro-5-(methylsu lfonamido)phenyl)-5-methyl-5,6-dihydro -4H-benzo[c]thieno[3,2-e]azepine-2-carb oxamide |
| **Example 67** | 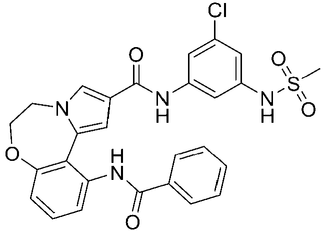 | 11-benzamido-N-(3-chloro-5-(methylsulf onamido)phenyl)-5,6-dihydrobenzo[f]pyr rolo[1,2-d][1,4]oxazepine-2-carboxamide |
| **Example 68** | 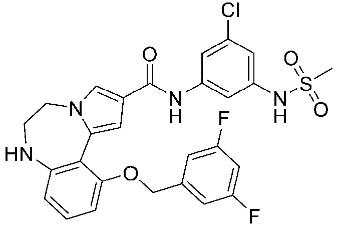 | N-(3-chloro-5-(methylsulfonamido)phen yl)-11-((3,5-difluorobenzyl)oxy)-6,7-dih ydro-5H-benzo[f]pyrrolo[1,2-d][1,4]diaz epine-2-carboxamide |
| **Example 69** | 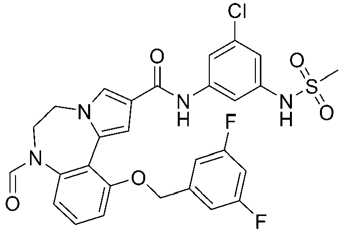 | N-(3-chloro-5-(methylsulfonamido)phen yl)-11-((3,5-difluorobenzyl)oxy)-7-form yl-6,7-dihydro-5H-benzo[f]pyrrolo[1,2-d ][1,4]diazepine-2-carboxamide |
| **Example 70** | 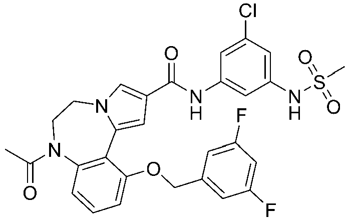 | 7-acetyl-N-(3-chloro-5-(methylsulfonami do)phenyl)-11-((3,5-difluorobenzyl)oxy) -6,7-dihydro-5H-benzo[f]pyrrolo[1,2-d][ 1,4]diazepine-2-carboxamide |
| **Example 71** | 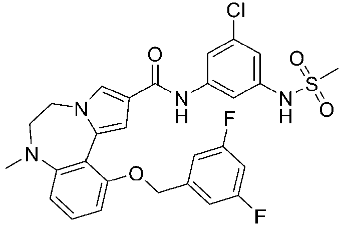 | N-(3-chloro-5-(methylsulfonamido)phen yl)-11-((3,5-difluorobenzyl)oxy)-7-meth yl-6,7-dihydro-5H-benzo[f]pyrrolo[1,2-d ][1,4]diazepine-2-carboxamide |
| **Example 72** | 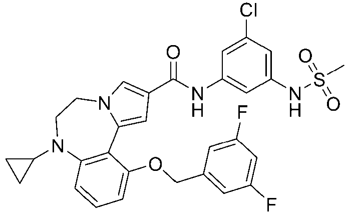 | N-(3-chloro-5-(methylsulfonamido)phen yl)-7-cyclopropyl-11-((3,5-difluorobenzy l)oxy)-6,7-dihydro-5H-benzo[f]pyrrolo[1 ,2-d][1,4]diazepine-2-carboxamide |
| **Example 73** | 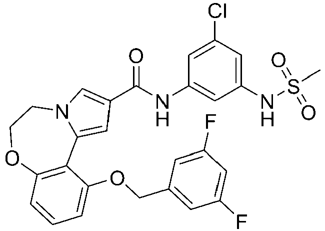 | N-(3-chloro-5-(methylsulfonamido)phen yl)-11-((3,5-difluorobenzyl)oxy)-5,6-dih ydrobenzo[f]pyrrolo[1,2-d][1,4]oxazepin e-2-carboxamide |
| **Example 74** | 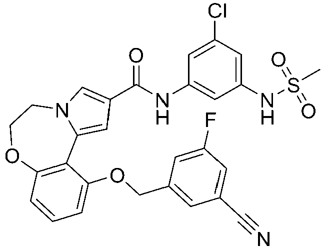 | N-(3-chloro-5-(methylsulfonamido)phen yl)-11-((3-cyano-5-fluorobenzyl)oxy)-5, 6-dihydrobenzo[f]pyrrolo[1,2-d][1,4]oxa zepine-2-carboxamide |
| **Example 75** | 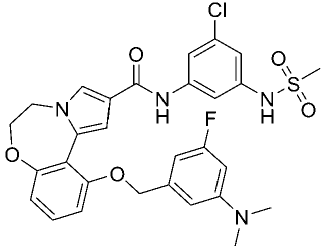 | N-(3-chloro-5-(methylsulfonamido)phen yl)-11-((3-(dimethylamino)-5-fluorobenz yl)oxy)-5,6-dihydrobenzo[f]pyrrolo[1,2-d][1,4]oxazepine-2-carboxamide |
| **Example 76** | 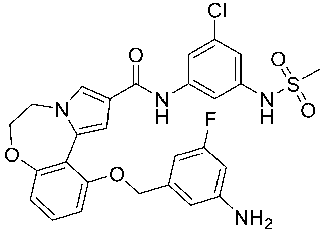 | 11-((3-amino-5-fluorobenzyl)oxy)-N-(3-chloro-5-(methylsulfonamido)phenyl)-5, 6-dihydrobenzo[f]pyrrolo[1,2-d][1,4]oxa zepine-2-carboxamide |
| **Example 77** | 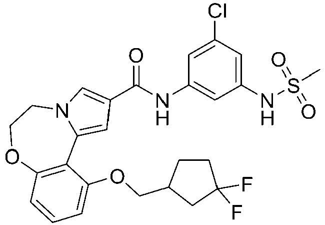 | N-(3-chloro-5-(methylsulfonamido)phen yl)-11-((3,3-difluorocyclopentyl)methox y)-5,6-dihydrobenzo[f]pyrrolo[1,2-d][1,4 ]oxazepine-2-carboxamide |
| **Example 78** | 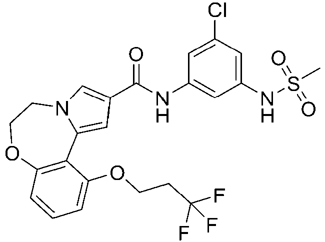 | N-(3-chloro-5-(methylsulfonamido)phen yl)-11-(3,3,3-trifluoropropoxy)-5,6-dihyd robenzo[f]pyrrolo[1,2-d][1,4]oxazepine-2-carboxamide |
| **Example 79** | 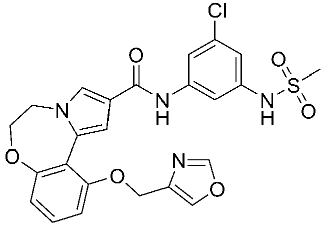 | N-(3-chloro-5-(methylsulfonamido)phen yl)-11-(oxazol-4-ylmethoxy)-5,6-dihydro benzo[f]pyrrolo[1,2-d][1,4]oxazepine-2-carboxamide |
| **Example 80** | 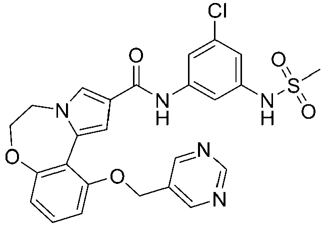 | N-(3-chloro-5-(methylsulfonamido)phen yl)-11-(pyrimidin-5-ylmethoxy)-5,6-dihy drobenzo[f]pyrrolo[1,2-d][1,4]oxazepine -2-carboxamide |
| **Example 81** | 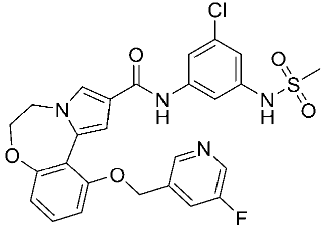 | N-(3-chloro-5-(methylsulfonamido)phen yl)-11-((5-fluoropyridin-3-yl)methoxy)-5 ,6-dihydrobenzo[f]pyrrolo[1,2-d][1,4]oxa zepine-2-carboxamide |
| **Example 82** | 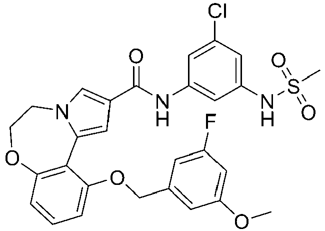 | N-(3-chloro-5-(methylsulfonamido)phen yl)-11-((3-fluoro-5-methoxybenzyl)oxy)-5,6-dihydrobenzo[f]pyrrolo[1,2-d][1,4]o xazepine-2-carboxamide |
| **Example 83** | 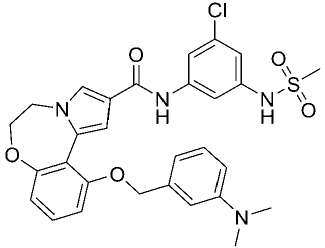 | N-(3-chloro-5-(methylsulfonamido)phen yl)-11-((3-(dimethylamino)benzyl)oxy)-5 ,6-dihydrobenzo[f]pyrrolo[1,2-d][1,4]oxa zepine-2-carboxamide |
| **Example 84** | 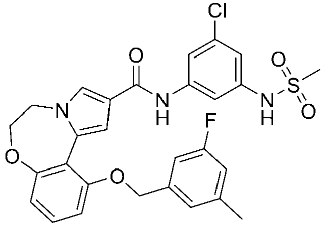 | N-(3-chloro-5-(methylsulfonamido)phen yl)-11-((3-fluoro-5-methylbenzyl)oxy)-5, 6-dihydrobenzo[f]pyrrolo[1,2-d][1,4]oxa zepine-2-carboxamide |
| **Example 85** | 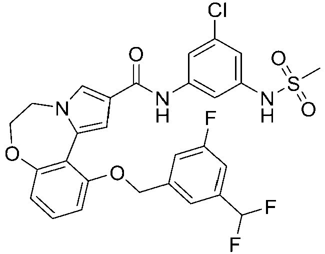 | N-(3-chloro-5-(methylsulfonamido)phen yl)-11-((3-(difluoromethyl)-5-fluorobenz yl)oxy)-5,6-dihydrobenzo[f]pyrrolo[1,2-d][1,4]oxazepine-2-carboxamide |
| **Example 86** | 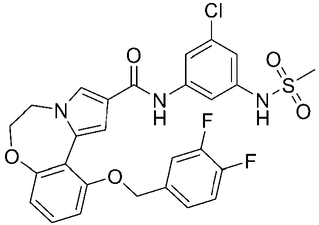 | N-(3-chloro-5-(methylsulfonamido)phen yl)-11-((3,4-difluorobenzyl)oxy)-5,6-dih ydrobenzo[f]pyrrolo[1,2-d][1,4]oxazepine -2-carboxamide |
| **Example 87** | 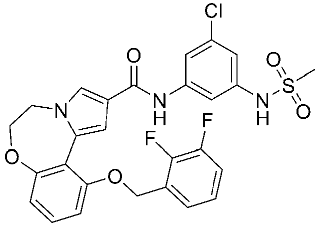 | N-(3-chloro-5-(methylsulfonamido)phen yl)-11-((2,3-difluorobenzyl)oxy)-5,6-dih ydrobenzo[f]pyrrolo[1,2-d][1,4]oxazepin e-2-carboxamide |
| **Example 88** | 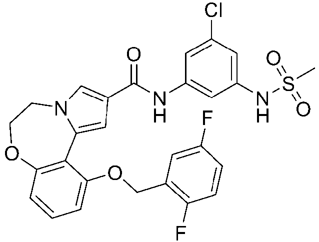 | N-(3-chloro-5-(methylsulfonamido)phen yl)-11-((2,5-difluorobenzyl)oxy)-5,6-dih ydrobenzo[f]pyrrolo[1,2-d][1,4]oxazepin e-2-carboxamide |
| **Example 89** | 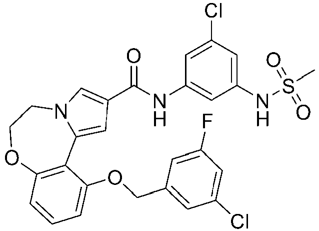 | N-(3-chloro-5-(methylsulfonamido)phen yl)-11-((3-chloro-5-fluorobenzyl)oxy)-5, 6-dihydrobenzo[f]pyrrolo[1,2-d][1,4]oxa zepine-2-carboxamide |
| **Example 90** | 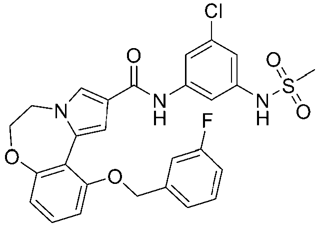 | N-(3-chloro-5-(methylsulfonamido)phen yl)-11-((3-fluorobenzyl)oxy)-5,6-dihydro benzo[f]pyrrolo[1,2-d][1,4]oxazepine-2-carboxamide |
| **Example 91** | 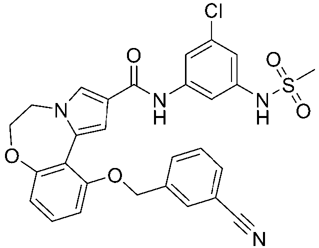 | N-(3-chloro-5-(methylsulfonamido)phen yl)-11-((3-cyanobenzyl)oxy)-5,6-dihydro benzo[f]pyrrolo[1,2-d][1,4]oxazepine-2-carboxamide |
| **Example 92** | 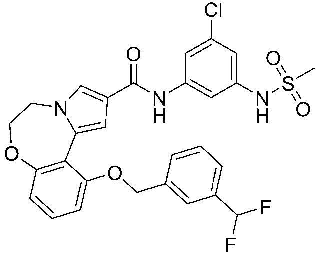 | N-(3-chloro-5-(methylsulfonamido)phen yl)-11-((3-(difluoromethyl)benzyl)oxy)-5 ,6-dihydrobenzo[f]pyrrolo[1,2-d][1,4]oxa zepine-2-carboxamide |
| **Example 93** | 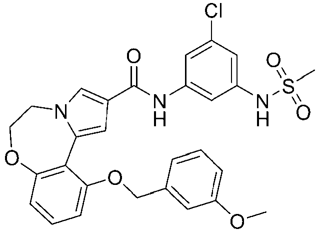 | N-(3-chloro-5-(methylsulfonamido)phen yl)-11-((3-methoxybenzyl)oxy)-5,6-dihy drobenzo[f]pyrrolo[1,2-d][1,4]oxazepine -2-carboxamide |
| **Example 94** | 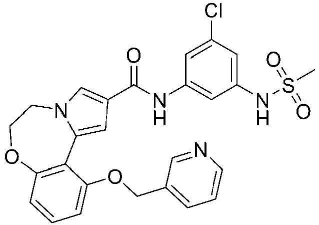 | N-(3-chloro-5-(methylsulfonamido)phen yl)-11-(pyridin-3-ylmethoxy)-5,6-dihydr obenzo[f]pyrrolo[1,2-d][1,4]oxazepine-2 -carboxamide |
| **Example 95** | 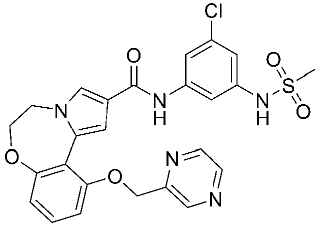 | N-(3-chloro-5-(methylsulfonamido)phen yl)-11-(pyrazin-2-ylmethoxy)-5,6-dihydr obenzo[f]pyrrolo[1,2-d][1,4]oxazepine-2 -carboxamide |
| **Example 96** | 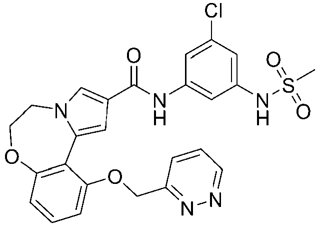 | N-(3-chloro-5-(methylsulfonamido)phen yl)-11-(pyridazin-3-ylmethoxy)-5,6-dihy drobenzo[f]pyrrolo[1,2-d][1,4]oxazepine -2-carboxamide |
| **Example 97** | 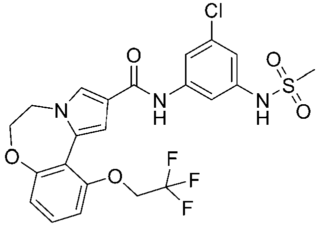 | N-(3-chloro-5-(methylsulfonamido)phen yl)-11-(2,2,2-trifluoroethoxy)-5,6-dihydr obenzo[f]pyrrolo[1,2-d][1,4]oxazepine-2 -carboxamide |
| **Example 98** | 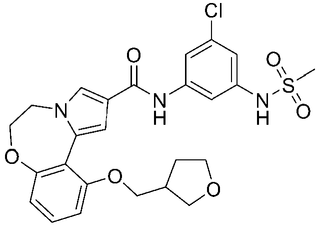 | N-(3-chloro-5-(methylsulfonamido)phen yl)-11-((tetrahydrofuran-3-yl)methoxy)-5 ,6-dihydrobenzo[f]pyrrolo[1,2-d][1,4]oxa zepine-2-carboxamide |
| **Example 99** | 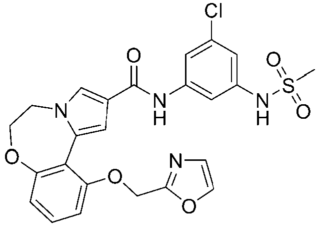 | N-(3-chloro-5-(methylsulfonamido)phen yl)-11-(oxazol-2-ylmethoxy)-5,6-dihydro benzo[f]pyrrolo[1,2-d][1,4]oxazepine-2-carboxamide |
| **Example 100** | 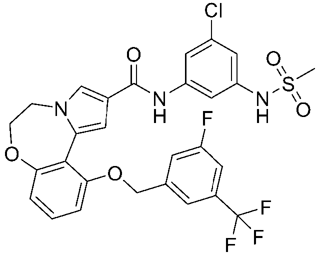 | N-(3-chloro-5-(methylsulfonamido)phen yl)-11-((3-fluoro-5-(trifluoromethyl)benz yl)oxy)-5,6-dihydrobenzo[f]pyrrolo[1,2-d][1,4]oxazepine-2-carboxamide |
| **Example 101** | 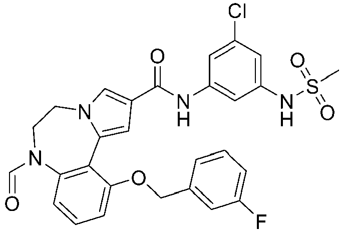 | N-(3-chloro-5-(methylsulfonamido)phen yl)-11-((3-fluorobenzyl)oxy)-7-formyl-6, 7-dihydro-5H-benzo[f]pyrrolo[1,2-d][1,4 ]diazepine-2-carboxamide |
| **Example 102** | 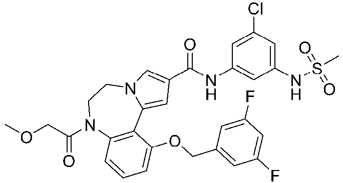 | N-(3-chloro-5-(methylsulfonamido)phen yl)-11-((3,5-difluorobenzyl)oxy)-7-(2-me thoxyacetyl)-6,7-dihydro-5H-benzo[f]pyr rolo[1,2-d][1,4]diazepine-2-carboxamide |
| **Example 103** | 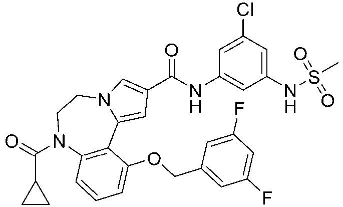 | N-(3-chloro-5-(methylsulfonamido)phen yl)-7-(cyclopropanecarbonyl)-11-((3,5-di fluorobenzyl)oxy)-6,7-dihydro-5H-benzo [f]pyrrolo[1,2-d][1,4]diazepine-2-carbox amide |
| **Example 104** | 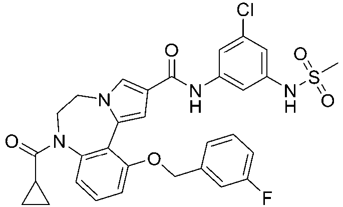 | N-(3-chloro-5-(methylsulfonamido)phen yl)-7-(cyclopropanecarbonyl)-11-((3-fluo robenzyl)oxy)-6,7-dihydro-5H-benzo[f]p yrrolo[1,2-d][1,4]diazepine-2-carboxami de |
| **Example 105** | 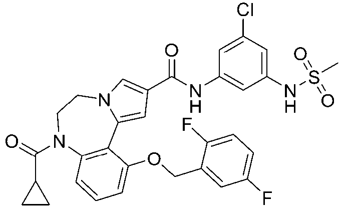 | N-(3-chloro-5-(methylsulfonamido)phen yl)-7-(cyclopropanecarbonyl)-11-((2,5-di fluorobenzyl)oxy)-6,7-dihydro-5H-benzo [f]pyrrolo[1,2-d][1,4]diazepine-2-carbox amide |
| **Example 106** | 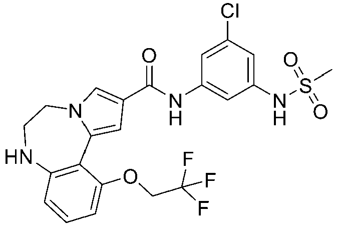 | N-(3-chloro-5-(methylsulfonamido)phen yl)-11-(2,2,2-trifluoroethoxy)-6,7-dihydr o-5H-benzo[f]pyrrolo[1,2-d][1,4]diazepi ne-2-carboxamide |
| **Example 107** | 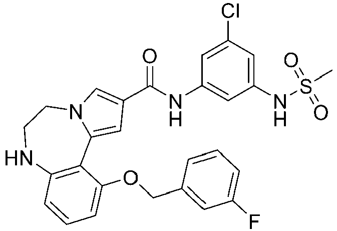 | N-(3-chloro-5-(methylsulfonamido)phen yl)-11-((3-fluorobenzyl)oxy)-6,7-dihydro -5H-benzo[f]pyrrolo[1,2-d][1,4]diazepin e-2-carboxamide |
| **Example 108** | 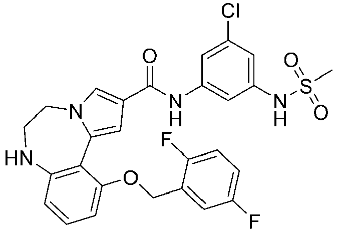 | N-(3-chloro-5-(methylsulfonamido)phen yl)-11-((2,5-difluorobenzyl)oxy)-6,7-dih ydro-5H-benzo[f]pyrrolo[1,2-d][1,4]diaz epine-2-carboxamide |
| **Example 109** | 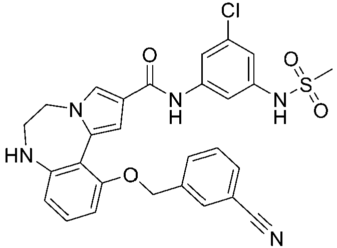 | N-(3-chloro-5-(methylsulfonamido)phen yl)-11-((3-cyanobenzyl)oxy)-6,7-dihydro -5H-benzo[f]pyrrolo[1,2-d][1,4]diazepin e-2-carboxamide |
| **Example 110** | 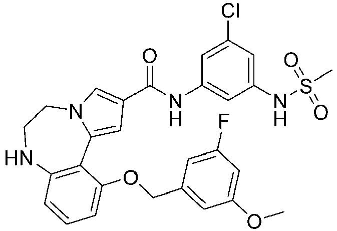 | N-(3-chloro-5-(methylsulfonamido)phen yl)-11-((3-fluoro-5-methoxybenzyl)oxy)-6,7-dihydro-5H-benzo[f]pyrrolo[1,2-d][1 ,4]diazepine-2-carboxamide |
| **Example 111** | 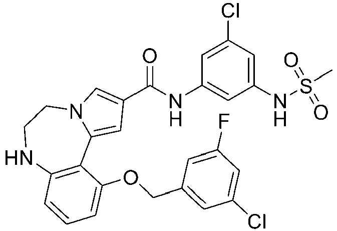 | N-(3-chloro-5-(methylsulfonamido)phen yl)-11-((3-chloro-5-fluorobenzyl)oxy)-6, 7-dihydro-5H-benzo[f]pyrrolo[1,2-d][1,4 ]diazepine-2-carboxamide |
| **Example 112** | 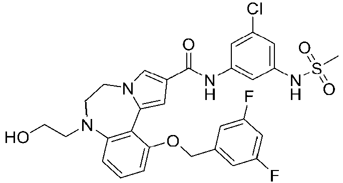 | N-(3-chloro-5-(methylsulfonamido)phen yl)-11-((3,5-difluorobenzyl)oxy)-7-(2-hy droxyethyl)-6,7-dihydro-5H-benzo[f]pyrr olo[1,2-d][1,4]diazepine-2-carboxamide |
| **Example 113** | 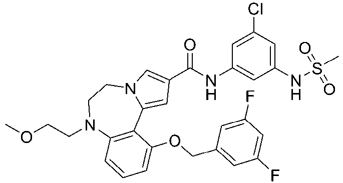 | N-(3-chloro-5-(methylsulfonamido)phen yl)-11-((3,5-difluorobenzyl)oxy)-7-(2-me thoxyethyl)-6,7-dihydro-5H-benzo[f]pyrr olo[1,2-d][1,4]diazepine-2-carboxamide |
| **Example 114** | 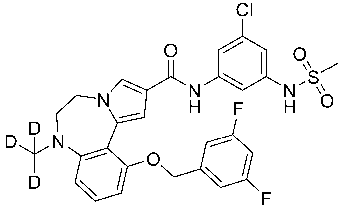 | N-(3-chloro-5-(methylsulfonamido)phen yl)-11-((3,5-difluorobenzyl)oxy)-7-(meth yl-d3)-6,7-dihydro-5H-benzo[f]pyrrolo[1 ,2-d][1,4]diazepine-2-carboxamide |
| **Example 115** | 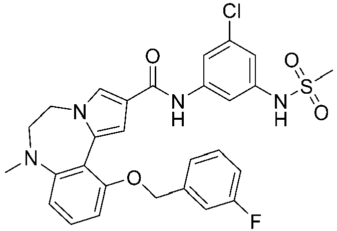 | N-(3-chloro-5-(methylsulfonamido)phen yl)-11-((3-fluorobenzyl)oxy)-7-methyl-6, 7-dihydro-5H-benzo[f]pyrrolo[1,2-d][1,4 ]diazepine-2-carboxamide |
| **Example 116** | 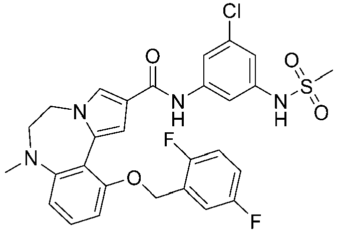 | N-(3-chloro-5-(methylsulfonamido)phen yl)-11-((2,5-difluorobenzyl)oxy)-7-meth yl-6,7-dihydro-5H-benzo[f]pyrrolo[1,2-d ][1,4]diazepine-2-carboxamide |
| **Example 117** | 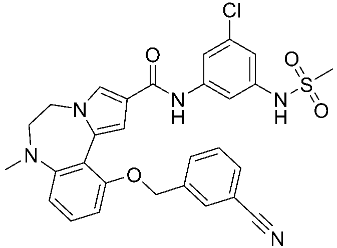 | N-(3-chloro-5-(methylsulfonamido)phen yl)-11-((3-cyanobenzyl)oxy)-7-methyl-6, 7-dihydro-5H-benzo[f]pyrrolo[1,2-d][1,4 ]diazepine-2-carboxamide |
| **Example 118** | 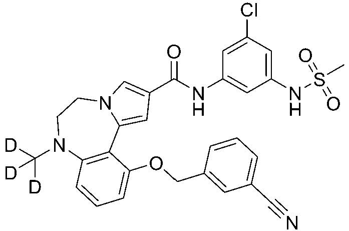 | N-(3-chloro-5-(methylsulfonamido)phen yl)-11-((3-cyanobenzyl)oxy)-7-(methyl-d 3)-6,7-dihydro-5H-benzo[f]pyrrolo[1,2-d ][1,4]diazepine-2-carboxamide |
| **Example 119** | 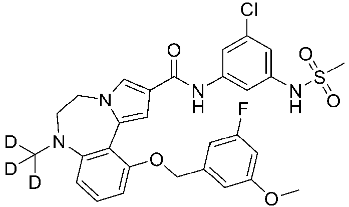 | N-(3-chloro-5-(methylsulfonamido)phen yl)-11-((3-fluoro-5-methoxybenzyl)oxy)-7-(methyl-d3)-6,7-dihydro-5H-benzo[f]p yrrolo[1,2-d][1,4]diazepine- 2-carboxami de |
| **Example 120** | 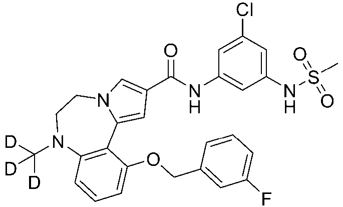 | N-(3-chloro-5-(methylsulfonamido)phen yl)-11-((3-fluorobenzyl)oxy)-7-(methyl-d 3)-6,7-dihydro-5H-benzo[f]pyrrolo[1,2-d ][1,4]diazepine-2-carboxamide |
| **Example 121** | 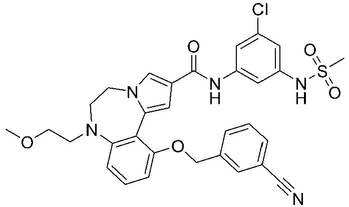 | N-(3-chloro-5-(methylsulfonamido)phen yl)-11-((3-cyanobenzyl)oxy)-7-(2-metho xyethyl)-6,7-dihydro-5H-benzo[f]pyrrolo [1,2-d][1,4]diazepine-2-carboxamide |
| **Example 122** | 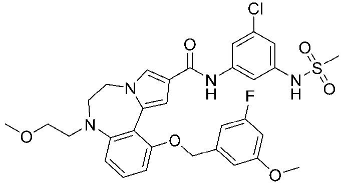 | N-(3-chloro-5-(methylsulfonamido)phen yl)-11-((3-fluoro-5-methoxybenzyl)oxy)-7-(2-methoxyethyl)-6,7-dihydro-5H-benz o[f]pyrrolo[1,2-d][1,4]diazepine-2-carbo xamide |
| **Example 123** | 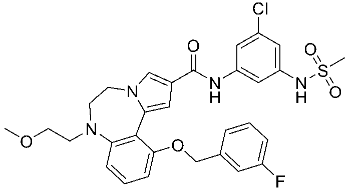 | N-(3-chloro-5-(methylsulfonamido)phen yl)-11-((3-fluorobenzyl)oxy)-7-(2-metho xyethyl)-6,7-dihydro-5H-benzo[f]pyrrolo [1,2-d][1,4]diazepine-2-carboxamide |
| **Example 124** | 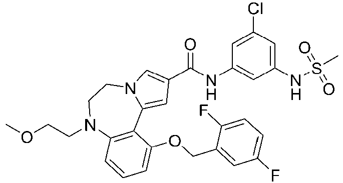 | N-(3-chloro-5-(methylsulfonamido)phen yl)-11-((2,5-difluorobenzyl)oxy)-7-(2-me thoxyethyl)-6,7-dihydro-5H-benzo[f]pyrr olo[1,2-d][1,4]diazepine-2-carboxamide |
| **Example 125** | 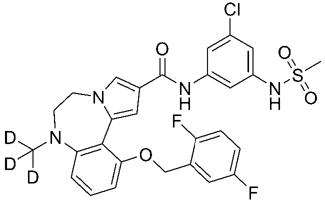 | N-(3-chloro-5-(methylsulfonamido)phen yl)-11-((2,5-difluorobenzyl)oxy)-7-(meth yl-d3)-6,7-dihydro-5H-benzo[f]pyrrolo[1 ,2-d][1,4]diazepine-2-carboxamide |
| **Example 126** | 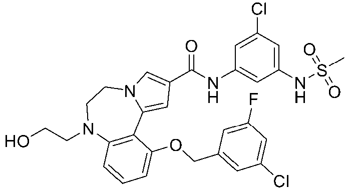 | N-(3-chloro-5-(methylsulfonamido)phen yl)-11-((3-chloro-5-fluorobenzyl)oxy)-7-(2-hydroxyethyl)-6,7-dihydro-5H-benzo[ f]pyrrolo[1,2-d][1,4]diazepine-2-carboxa mide |
| **Example 127** | 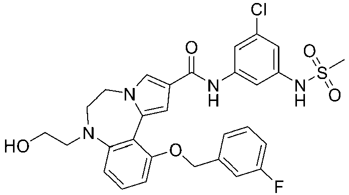 | N-(3-chloro-5-(methylsulfonamido)phen yl)-11-((3-fluorobenzyl)oxy)-7-(2-hydro xyethyl)-6,7-dihydro-5H-benzo[f]pyrrolo [1,2-d][1,4]diazepine-2-carboxamide |
| **Example 128** | 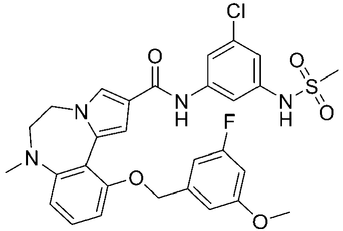 | N-(3-chloro-5-(methylsulfonamido)phen yl)-11-((3-fluoro-5-methoxybenzyl)oxy)-7-methyl-6,7-dihydro-5H-benzo[f]pyrrol o[1,2-d][1,4]diazepine-2-carboxamide |
| **Example 129** | 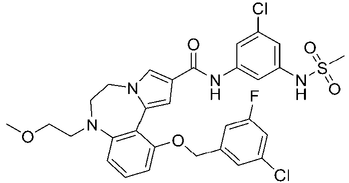 | N-(3-chloro-5-(methylsulfonamido)phen yl)-11-((3-chloro-5-fluorobenzyl)oxy)-7-(2-methoxyethyl)-6,7-dihydro-5H-benzo[ f]pyrrolo[1,2-d][1,4]diazepine-2-carboxa mide |
| **Example 130** | 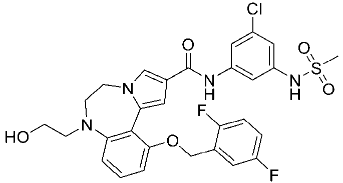 | N-(3-chloro-5-(methylsulfonamido)phen yl)-11-((2,5-difluorobenzyl)oxy)-7-(2-hy droxyethyl)-6,7-dihydro-5H-benzo[f]pyrr olo[1,2-d][1,4]diazepine-2-carboxamide |
| **Example 131** | 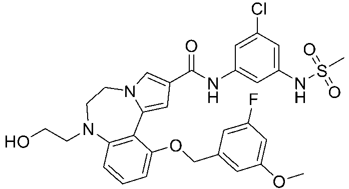 | N-(3-chloro-5-(methylsulfonamido)phen yl)-11-((3-fluoro-5-methoxybenzyl)oxy)-7-(2-hydroxyethyl)-6,7-dihydro-5H-benz o[f]pyrrolo[1,2-d][1,4]diazepine-2-carbo xamide |
| **Example 132** | 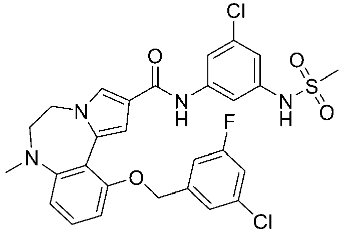 | N-(3-chloro-5-(methylsulfonamido)phen yl)-11-((3-chloro-5-fluorobenzyl)oxy)-7-methyl-6,7-dihydro-5H-benzo[f]pyrrolo[ 1,2-d][1,4]diazepine-2-carboxamide |
| **Example 133** | 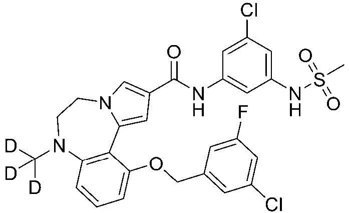 | N-(3-chloro-5-(methylsulfonamido)phen yl)-11-((3-chloro-5-fluorobenzyl)oxy)-7-(methyl-d3)-6,7-dihydro-5H-benzo[f]pyr rolo[1,2-d][1,4]diazepine-2-carboxamide |
| **Example 134** | 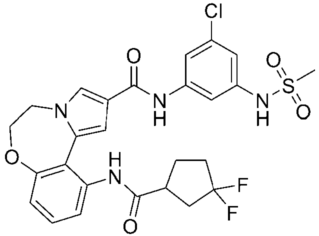 | N-(3-chloro-5-(methylsulfonamido)phen yl)-11-(3,3-difluorocyclopentane-1-carbo xamido)-5,6-dihydrobenzo[f]pyrrolo[1,2-d][1,4]oxazepine-2-carboxamide |
| **Example 135** | 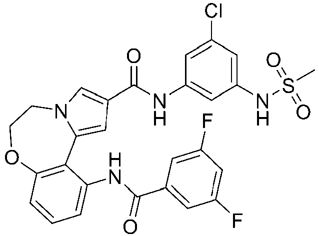 | N-(3-chloro-5-(methylsulfonamido)phen yl)-11-(3,5-difluorobenzamido)-5,6-dihy drobenzo[f]pyrrolo[1,2-d][1,4]oxazepine -2-carboxamide |
| **Example 136** | 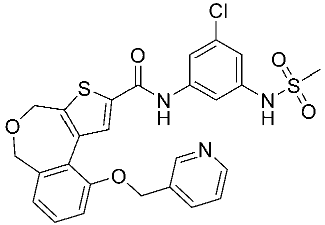 | N-(3-chloro-5-(methylsulfonamido)phen yl)-10-(pyridin-3-ylmethoxy)-4,6-dihydr obenzo[c]thieno[3,2-e]oxepine-2-carbox amide |
| **Example 137** | 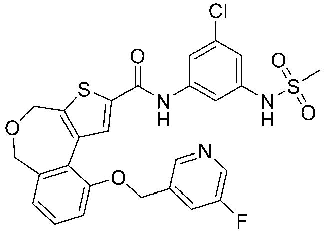 | N-(3-chloro-5-(methylsulfonamido)phen yl)-10-((5-fluoropyridin-3-yl)methoxy)-4 ,6-dihydrobenzo[c]thieno[3,2-e]oxepine-2-carboxamide |
| **Example 138** | 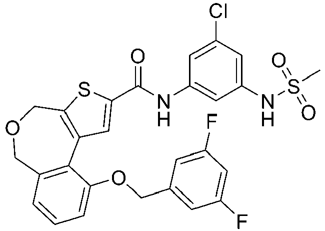 | N-(3-chloro-5-(methylsulfonamido)phen yl)-10-((3,5-difluorobenzyl)oxy)-4,6-dih ydrobenzo[c]thieno[3,2-e]oxepine-2-carb oxamide |
| **Example 139** | 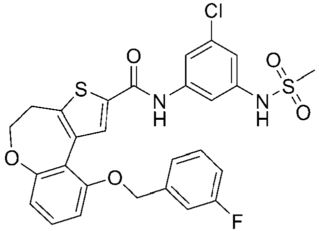 | N-(3-chloro-5-(methylsulfonamido)phen yl)-10-((3-fluorobenzyl)oxy)-4,5-dihydro benzo[b]thieno[3,2-d]oxepine-2-carboxa mide |
| **Example 140** | 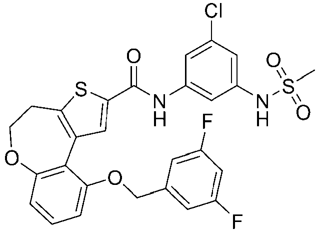 | N-(3-chloro-5-(methylsulfonamido)phen yl)-10-((3,5-difluorobenzyl)oxy)-4,5-dih ydrobenzo[b]thieno[3,2-d]oxepine-2-car boxamide |
| **Example 141** | 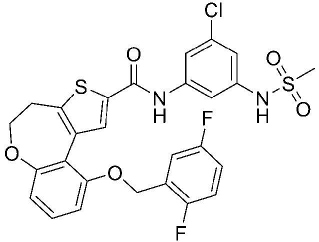 | N-(3-chloro-5-(methylsulfonamido)phen yl)-10-((2,5-difluorobenzyl)oxy)-4,5-dih ydrobenzo[b]thieno[3,2-d]oxepine-2-car boxamide |
| **Example 142** | 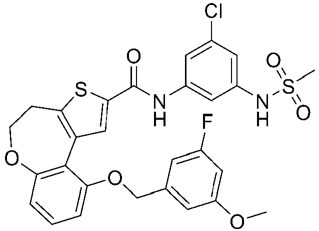 | N-(3-chloro-5-(methylsulfonamido)phen yl)-10-((3-fluoro-5-methoxybenzyl)oxy)-4,5-dihydrobenzo[b]thieno[3,2-d]oxepin e-2-carboxamide |
| **Example 143** | 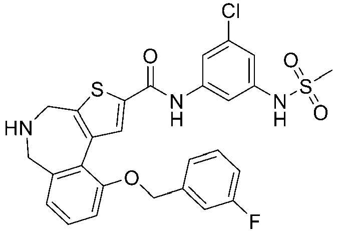 | N-(3-chloro-5-(methylsulfonamido)phen yl)-10-((3-fluorobenzyl)oxy)-5,6-dihydro -4H-benzo[c]thieno[3,2-e]azepine-2-carb oxamide |
| **Example 144** | 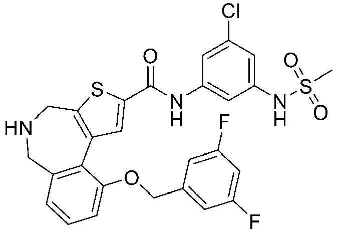 | N-(3-chloro-5-(methylsulfonamido)phen yl)-10-((3,5-difluorobenzyl)oxy)-5,6-dih ydro-4H-benzo[c]thieno[3,2-e]azepine-2-carboxamide |
| **Example 145** | 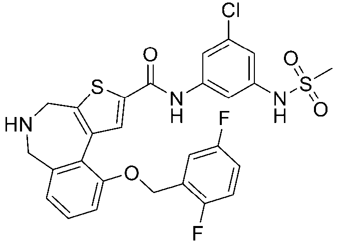 | N-(3-chloro-5-(methylsulfonamido)phen yl)-10-((2,5-difluorobenzyl)oxy)-5,6-dih ydro-4H-benzo[c]thieno[3,2-e]azepine-2-carboxamide |
| **Example 146** | 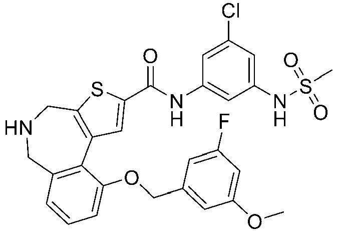 | N-(3-chloro-5-(methylsulfonamido)phen yl)-10-((3-fluoro-5-methoxybenzyl)oxy)-5,6-dihydro-4H-benzo[c]thieno[3,2-e]aze pine-2-carboxamide |
| **Example 147** | 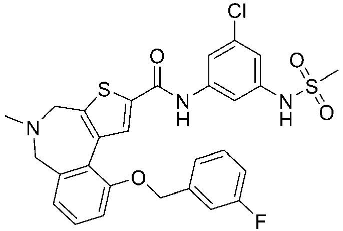 | N-(3-chloro-5-(methylsulfonamido)phen yl)-10-((3-fluorobenzyl)oxy)-5-methyl-5, 6-dihydro-4H-benzo[c]thieno[3,2-e]azepi ne-2-carboxamide |
| **Example 148** | 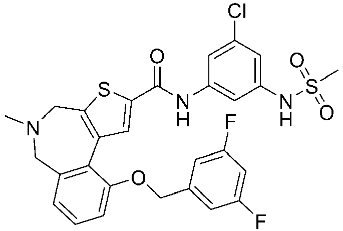 | N-(3-chloro-5-(methylsulfonamido)phen yl)-10-((3,5-difluorobenzyl)oxy)-5-meth yl-5,6-dihydro-4H-benzo[c]thieno[3,2-e] azepine-2-carboxamide |
| **Example 149** | 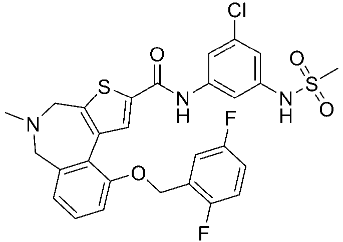 | N-(3-chloro-5-(methylsulfonamido)phen yl)-10-((2,5-difluorobenzyl)oxy)-5-meth yl-5,6-dihydro-4H-benzo[c]thieno[3,2-e] azepine-2-carboxamide |
| **Example 150** | 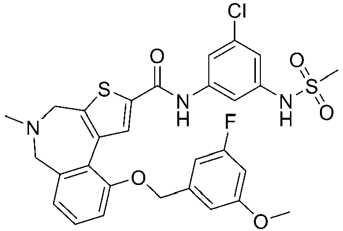 | N-(3-chloro-5-(methylsulfonamido)phen yl)-10-((3-fluoro-5-methoxybenzyl)oxy)-5-methyl-5,6-dihydro-4H-benzo[c]thieno [3,2-e]azepine-2-carboxamide |
| **Example 151** | 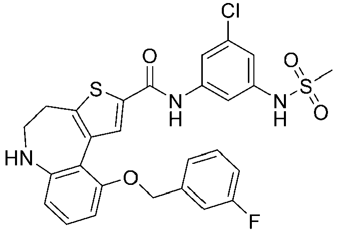 | N-(3-chloro-5-(methylsulfonamido)phen yl)-10-((3-fluorobenzyl)oxy)-5,6-dihydro -4H-benzo[b]thieno[3,2-d]azepine-2-carb oxamide |
| **Example 152** | 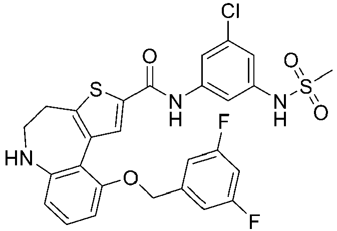 | N-(3-chloro-5-(methylsulfonamido)phen yl)-10-((3,5-difluorobenzyl)oxy)-5,6-dih ydro-4H-benzo[b]thieno[3,2-d]azepine-2 -carboxamide |
| **Example 153** | 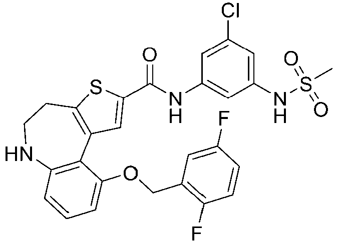 | N-(3-chloro-5-(methylsulfonamido)phen yl)-10-((2,5-difluorobenzyl)oxy)-5,6-dih ydro-4H-benzo[b]thieno[3,2-d]azepine-2 -carboxamide |
| **Example 154** | 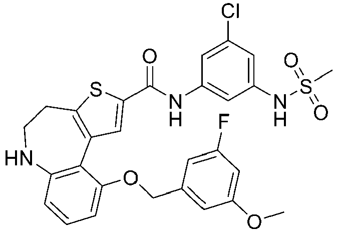 | N-(3-chloro-5-(methylsulfonamido)phen yl)-10-((3-fluoro-5-methoxybenzyl)oxy)-5,6-dihydro-4H-benzo[b]thieno[3,2-d]az epine-2-carboxamide |
| **Example 155** | 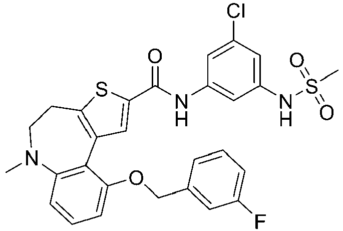 | N-(3-chloro-5-(methylsulfonamido)phen yl)-10-((3-fluorobenzyl)oxy)-6-methyl-5, 6-dihydro-4H-benzo[b]thieno[3,2-d]azep ine-2-carboxamide |
| **Example 156** | 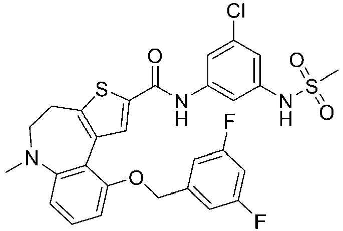 | N-(3-chloro-5-(methylsulfonamido)phen yl)-10-((3,5-difluorobenzyl)oxy)-6-meth yl-5,6-dihydro-4H-benzo[b]thieno[3,2-d] azepine-2-carboxamide |
| **Example 157** | 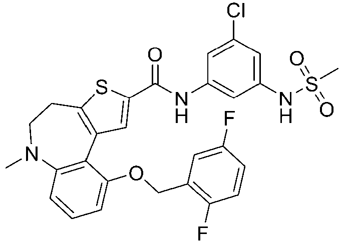 | N-(3-chloro-5-(methylsulfonamido)phen yl)-10-((2,5-difluorobenzyl)oxy)-6-meth yl-5,6-dihydro-4H-benzo[b]thieno[3,2-d] azepine-2-carboxamide |
| **Example 158** | 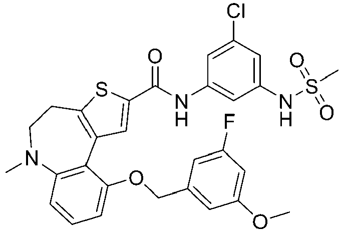 | N-(3-chloro-5-(methylsulfonamido)phen yl)-10-((3-fluoro-5-methoxybenzyl)oxy)-6-methyl-5,6-dihydro-4H-benzo[b]thieno [3,2-d]azepine-2-carboxamide |

or the stereoisomer thereof, the tautomer thereof or the pharmaceutically acceptable salt thereof.

Note: if there is a difference between the drawn structure and the given name of the structure, the drawn structure shall prevail.

Further, the present disclosure provides a pharmaceutical composition, wherein the pharmaceutical composition contains an effective dose of the compound as shown in formula (I), (II), (IIA), (III), (IIIA), (IIIB) or (IIIC), or the stereoisomer thereof, the tautomer thereof or the pharmaceutically acceptable salt thereof, and a pharmaceutically acceptable carrier, an excipient or a combination thereof.

The present disclosure provides the use of the compound as shown in formula (I), (II), (IIA), (III), (IIIA), (IIIB) or (IIIC), or the stereoisomer thereof, the tautomer thereof or the pharmaceutically acceptable salt thereof, or the pharmaceutical composition thereof (such as the pharmaceutical composition described in the above technical solution) as a medicament (which refers to the use for treatment).

The present disclosure provides the use of the compound as shown in formula (I), (II), (IIA), (III), (IIIA), (IIIB) or (IIIC), or the stereoisomer thereof, the tautomer thereof or the pharmaceutically acceptable salt thereof, or the pharmaceutical composition thereof (such as the pharmaceutical composition described in the above technical solution) in the preparation of a DHX9 inhibitor.

The present disclosure provides the use of the compound as shown in formula (I), (II), (IIA), (III), (IIIA), (IIIB) or (IIIC), or the stereoisomer thereof, the tautomer thereof or the pharmaceutically acceptable salt thereof, or the pharmaceutical composition thereof (such as the pharmaceutical composition described in the above technical solution), in the manufacture of a medicament for the treatment of a DHX9-mediated disease, wherein the DHX9-mediated disease is preferably cancer, viral infection or autoimmune disease, more preferably cancer; wherein, the cancer is selected from colorectal cancer, endometrial cancer, ovarian cancer, gastric cancer, hematopoietic system cancer, breast cancer, brain cancer, skin cancer, lung cancer, blood cancer, prostate cancer, head and neck cancer, pancreatic cancer, bladder cancer, bone cancer, soft tissue cancer, renal cancer and liver cancer, preferably colorectal cancer, endometrial cancer, ovarian cancer, hematopoietic system cancer and gastric cancer.

The present disclosure provides the use of the compound as shown in formula (I), (II), (IIA), (III), (IIIA), (IIIB) or (IIIC), or the stereoisomer thereof, the tautomer thereof or the pharmaceutically acceptable salt thereof, or the pharmaceutical composition thereof (such as the pharmaceutical composition described in the above technical solution), in the manufacture of a medicament for the treatment of a DHX9-mediated disease, wherein the DHX9-mediated disease is preferably cancer, and the cancer is microsatellite instability (MSI) cancer, preferably high microsatellite instability (MSI-H) cancer, and further preferably high microsatellite instability (MSI-H) colorectal cancer.

The present disclosure provides the use of the compound as shown in formula (I), (II), (IIA), (III), (IIIA), (IIIB) or (IIIC), or the stereoisomer thereof, the tautomer thereof or the pharmaceutically acceptable salt thereof, or the pharmaceutical composition thereof (such as the pharmaceutical composition described in the above technical solution), in the manufacture of a medicament for the treatment of cancer, viral infection or autoimmune disease.

The present disclosure provides the use of the compound as shown in formula (I), (II), (IIA), (III), (IIIA), (IIIB) or (IIIC), or the stereoisomer thereof, the tautomer thereof or the pharmaceutically acceptable salt thereof, or the pharmaceutical composition thereof (such as the pharmaceutical composition described in the above technical solution), in the manufacture of a medicament for the treatment of colorectal cancer, endometrial cancer, ovarian cancer, gastric cancer, hematopoietic system cancer, breast cancer, brain cancer, skin cancer, lung cancer, blood cancer, prostate cancer, head and neck cancer, pancreatic cancer, bladder cancer, bone cancer, soft tissue cancer, renal cancer and liver cancer, preferably the use in the manufacture of the medicament for the treatment of colorectal cancer, endometrial cancer, ovarian cancer, hematopoietic system cancer and gastric cancer.

The present disclosure provides the use of the compound as shown in formula (I), (II), (IIA), (III), (IIIA), (IIIB) or (IIIC), or the stereoisomer thereof, the tautomer thereof or the pharmaceutically acceptable salt thereof, or the pharmaceutical composition thereof (such as the pharmaceutical composition described in the above technical solution), in the manufacture of a medicament for the treatment of microsatellite instability (MSI) cancer, preferably the use in the manufacture of a medicament for the treatment of high microsatellite instability (MSI-H) cancer, and further preferably the use in the manufacture of a medicament for the treatment of high microsatellite instability (MSI-H) colorectal cancer.

The present disclosure further provides a method for preventing and/or treating a DHX9-mediated disease, which comprises administering to a patient a therapeutically effective amount of the compound as shown in formula (I), (II), (IIA), (III), (IIIA), (IIIB) or (IIIC), or the stereoisomer thereof, the tautomer thereof or the pharmaceutically acceptable salt thereof, or the pharmaceutical composition thereof (such as the pharmaceutical composition described in the above technical solution); wherein the DHX9-mediated disease is preferably cancer, viral infection or autoimmune disease, more preferably cancer; wherein the cancer is selected from colorectal cancer, endometrial cancer, ovarian cancer, gastric cancer, hematopoietic system cancer, breast cancer, brain cancer, skin cancer, lung cancer, blood cancer, prostate cancer, head and neck cancer, pancreatic cancer, bladder cancer, bone cancer, soft tissue cancer, renal cancer and liver cancer, preferably colorectal cancer, endometrial cancer, ovarian cancer, hematopoietic system cancer and gastric cancer; or the cancer is microsatellite instability (MSI) cancer, preferably high microsatellite instability (MSI-H) cancer, and further preferably high microsatellite instability (MSI-H) colorectal cancer.

The present disclosure further provides a method for preventing and/or treating cancer, viral infection or autoimmune disease, which comprises administering to a patient a therapeutically effective amount of the compound as shown in formula (I), (II), (IIA), (III), (IIIA), (IIIB) or (IIIC), or the stereoisomer thereof, the tautomer thereof or the pharmaceutically acceptable salt thereof, or the pharmaceutical composition thereof (such as the pharmaceutical composition described in the above technical solution); wherein the cancer is selected from colorectal cancer, endometrial cancer, ovarian cancer, gastric cancer, hematopoietic system cancer, breast cancer, brain cancer, skin cancer, lung cancer, blood cancer, prostate cancer, head and neck cancer, pancreatic cancer, bladder cancer, bone cancer, soft tissue cancer, renal cancer and liver cancer, preferably colorectal cancer, endometrial cancer, ovarian cancer, hematopoietic system cancer and gastric cancer; or the cancer is microsatellite instability (MSI) cancer, preferably high microsatellite instability (MSI-H) cancer, and further preferably high microsatellite instability (MSI-H) colorectal cancer.

### Detailed description of the invention

Unless stated to the contrary, some terms used in the specification and the claims of the present disclosure are defined as follows.

"Alkyl" refers to an aliphatic hydrocarbon group comprising a C₁-C₂₀ straight chain or having a branched chain when taken as one group or a part of one group. Preferably, the alkyl is C₁-C₁₀ alkyl, and more preferably, the alkyl is C₁-C₆ alkyl. Examples of an alkyl group comprise, but are not limited to, methyl, ethyl, n-propyl, isopropyl, n-butyl, isobutyl, tert-butyl, sec-butyl, n-pentyl, 1,1-dimethyl propyl, 1,2-dimethyl propyl, 2,2-dimethyl propyl, 1-ethyl propyl, 2-methyl butyl, 3-methyl butyl, n-hexyl, 1-ethyl-2-methyl propyl, 1,1,2-trimethyl propyl, 1,1-dimethyl butyl, 1,2-dimethyl butyl, 2,2-dimethyl butyl, 1,3-dimethyl butyl, 2-ethyl butyl, 2-methyl pentyl, 3-methyl pentyl, 4-methyl pentyl, 2,3-dimethyl butyl, and the like. The alkyl may be substituted or unsubstituted.

"Alkenyl" refers to the alkyl as defined above consisting of at least two carbon atoms and at least one carbon-carbon double bond, and representative examples comprise, but are not limited to, vinyl, 1-propenyl, 2-propenyl, 1-,2- or 3-butenyl, 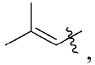 and the like. Preferably, the alkenyl is C₂-C₄ alkenyl. The alkenyl may be optionally substituted or unsubstituted.

"Alkynyl" refers to an aliphatic hydrocarbon group containing one carbon-carbon triple bond, and may be a straight chain or have a branched chain. Preferably, the alkynyl is C₂-C₁₀ alkynyl, more preferably, the alkynyl is C₂-C₆ alkynyl, and most preferably, the alkynyl is C₂-C₄ alkynyl. Examples of an alkynyl group comprise, but are not limited to, ethynyl, 1-propynyl, 2-propynyl, 1-, 2- or 3-butynyl, and the like. The alkynyl may be substituted or unsubstituted.

"Alkylene" refers to a saturated C₁-C₂₀ straight or branched aliphatic hydrocarbon group, which has 2 residues derived by removing two hydrogen atoms from the same carbon or two different carbon atoms of the parent alkane, and is preferably C₁-C₁₀ alkylene, and more preferably C₁-C₆ alkylene. Examples of an alkylene group comprise, but are not limited to, methylene, 1,1-ethylidene, 1,2-ethylidene, 1,1-propylidene, 1,2-propylidene, 1,3-propylidene, 1,4-butylidene, and the like. The alkylene may be substituted or unsubstituted.

"Cycloalkyl" refers to a non-aromatic cyclic alkyl group, wherein one or more ring-forming atoms are carbon atoms, comprising a monocyclic ring, a polycyclic ring, a fused ring, a bridged ring and a spirocyclic ring, and preferably having a 5- to 7-membered monocyclic ring, or a 7- to 10-membered bicyclic or tricyclic ring. Examples of the "cycloalkyl" comprise, but are not limited to, cyclopropyl, cyclopentyl, cyclobutyl, and the like. The cycloalkyl may be substituted or unsubstituted.

"Spiroalkyl" refers to a 5- to 18-membered polycyclic group with two or more cyclic structures, and single rings sharing one carbon atom (called a spiro atom) with each other, and the ring contains one or more double bonds, but no ring has a fully conjugated π electron aromatic system. Preferably, the spiroalkyl is 6- to 14-membered, and more preferably, the spiroalkyl is 7- to 10-membered. The spiroalkyl is divided into mono-, di- or multi-spiroalkyl according to a number of shared spiro-atoms between rings, is preferably mono- and di-spiroalkyl, and is preferably 4-membered/5-membered, 4-membered/6-membered, 5-membered/5-membered or 5-membered/6-membered. Non-limiting examples of the "spiroalkyl" comprise, but are not limited to, spiro[4.5]decyl, spiro[4.4]nonyl, spiro[3.5]nonyl, spiro[2.4]heptyl, and the like.

"Fused cycloalkyl" refers to a 5- to 18-membered full-carbon polycyclic group with two or more cyclic structures sharing one pair of carbon atoms with each other, and one or more rings may contain one or more double bonds, but no ring has a fully conjugated π electron aromatic system. Preferably, the fused cycloalkyl is 6- to 12-membered, and more preferably, the fused cycloalkyl is 7- to 10-membered. The fused cycloalkyl may be divided into bicyclic, tricyclic, tetracyclic or polycyclic fused cycloalkyl according to a number of constituent rings, is preferably bicyclic or tricyclic fused cycloalkyl, and is more preferably 5-membered/5-membered or 5-membered/6-membered bicycloalkyl. Non-limiting examples of the "fused cycloalkyl" comprise, but are not limited to, bicyclo[3.1.0]hexyl, bicyclo[3.2.0]heptyl-1-alkenyl, bicyclo[3.2.0]heptyl, decahydronaphthyl, tetrahydrophenyl, and the like.

"Bridged cycloalkyl" refers to a 5- to 18-membered full-carbon polycyclic group with two or more cyclic structures sharing two carbon atoms not directly linked with each other, and one or more rings may contain one or more double bonds, but no ring has a fully conjugated π electron aromatic system. Preferably, the bridged cycloalkyl is 6- to 12-membered, and more preferably, the bridged cycloalkyl is 7- to 10-membered. Preferably, the bridged cycloalkyl is 6-to 14-membered, and more preferably, the bridged cycloalkyl is 7- to 10-membered The bridged cycloalkyl may be divided into bicyclic, tricyclic, tetracyclic or polycyclic bridged cycloalkyl according to a number of constituent rings, is preferably bicyclic, tricyclic or tetracyclic bridged cycloalkyl, and is more preferably bicyclic or tricyclic bridged cycloalkyl. Non-limiting examples of the "bridged cycloalkyl" comprise, but are not limited to, (1s,4s)-bicyclo [2.2.1] heptyl, bicyclo [3.2.1] octyl, (1s,5s)-bicyclo [3.3.1] nonyl, bicyclo [2.2.2] octyl, (1r,5r)-bicyclo [3.3.2] decyl, and the like.

"Heterocyclyl", "heterocycloalkyl", "heterocycle" or "heterocyclic" may be used interchangeably in the present application, each refers to non-aromatic heterocyclyl, and the ring may contain 0, 1 or more double bonds, wherein one or more ring-forming atoms are selected from heteroatoms of nitrogen, oxygen or S(O)ᵣ (wherein r is selected from 0, 1 or 2), the non-aromatic heterocyclyl comprises a monocyclic ring, a polycyclic ring, a fused ring, a bridged ring and a spirocyclic ring. Preferably, the heterocyclyl is a 5- to 8-membered monocyclic ring or a 7- to 10-membered bicyclic or tricyclic ring, which may contain 1, 2 or 3 atoms selected from nitrogen, oxygen and/or sulfur. Examples of the "heterocyclyl" comprise, but are not limited to, morpholinyl, oxetanyl, thiomorpholinyl, tetrahydrofuranyl, tetrahydropyranyl, 1,1-dioxo-thiomorpholinyl, piperidinyl, 2-oxopiperidinyl, pyrrolidinyl, 2-oxopyrrolidinyl, piperazine-2-one, 8-oxa-3-aza-bicyclo [3.2.1] octyl, piperazinyl, hexahydropyrimidine, 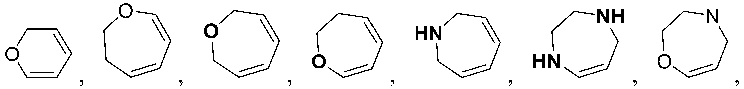 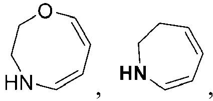 and the like. The heterocyclyl may be substituted or unsubstituted.

"Spiro-heterocyclyl" refers to a 5- to 18-membered polycyclic group with two or more cyclic structures, and single rings sharing one atom with each other, and the ring may contain 0, 1 or more double bonds, but no ring has a fully conjugated π electron aromatic system, wherein one or more ring atoms are selected from heteroatoms of nitrogen, oxygen or S(O)ᵣ (wherein r is selected from 0, 1 or 2), and the remaining ring atoms are carbon. Preferably, the spiro-heterocyclyl is 6- to 14-membered, and more preferably, the spiro-heterocyclyl is 7- to 10-membered. The spiro-heterocyclyl is divided into mono-, di- or multi-spiro-heterocyclyl according to a number of shared spiro atoms between rings, is preferably the mono- and di-spiro-heterocyclyl, and is more preferably 4-membered/4-membered, 4-membered/5-membered, 3-membered/5-membered, 3-membered/6-membered, 4-membered/6-membered, 5-membered/ 5-membered or 5-membered/6-membered mono-spiro-heterocyclyl. Non-limiting examples of the "spiro-heterocyclyl" comprise, but are not limited to, 1,7-dioxane [4.5] decyl, 2-oxa-7-azaspiro [4.4] nonyl, 7-oxaspiro [3.5], 5-oxaspiro [2.4] heptyl, and the like.

"Fused heterocyclyl" refers to a full carbon polycyclic group with two or more cyclic structures sharing one pair of atoms with each other, and one or more rings may contain 0, 1 or more double bonds, but no ring has a fully conjugated π electron aromatic system, wherein one or more ring atoms are selected from heteroatoms: nitrogen, oxygen or S(O)ᵣ (wherein r is selected from 0, 1 or 2), and the remaining ring atoms are carbon. Preferably, the fused heterocyclyl is 6- to 14-membered, and more preferably, the fused heterocyclyl is 7- to 10-membered The fused heterocyclyl may be divided into bicyclic, tricyclic, tetracyclic or polycyclic fused heterocyclyl according to a number of constituent rings, is preferably bicyclic or tricyclic fused heterocyclyl, and is more preferably 5-membered/5-membered or 5-membered/6-membered bicyclic fused heterocyclyl. Non-limiting examples of the "fused heterocyclyl" comprise, but are not limited to, octahydropyrrolo [3,4-c] pyrrolyl, octahydro-1H-isoindolyl, 3- zabicyclo [3.1.0] hexyl, octahydrobenzo [b][1,4] dioxine, and the like.

"Bridged heterocyclyl" refers to a 5- to 14-membered and 5- to 18-membered polycyclic group with two or more cyclic structures sharing two atoms not directly linked to each other, and one or more rings may contain 0, 1 or more double bonds, but no ring has a fully conjugated π electron aromatic system, wherein one or more ring atoms are selected from heteroatoms: nitrogen, oxygen or S(O)ₜ (wherein t is selected from 0, 1 or 2), and the remaining ring atoms are carbon. Preferably, the bridged heterocyclyl is 6- to 14-membered, and more preferably, the bridged heterocyclyl is 7- to 10-membered The bridged heterocyclyl may be divided into bicyclic, tricyclic, tetracyclic or polycyclic bridged heterocyclyl according to a number of constituent rings, is preferably bicyclic, tricyclic or tetracyclic bridged heterocyclyl, and is more preferably bicyclic or tricyclic bridged heterocyclyl. Non-limiting examples of the "bridged heterocyclyl" comprise, but are not limited to, 2-azabicyclo[2.2.1]heptyl, 2-azabicyclo[2.2.2]octyl, 2-azabicyclo[3.3.2]decyl, and the like.

"Aryl" refers to a carbocyclic aromatic system containing one or two rings, wherein the rings may be linked together in a fused manner. The term "aryl" comprises monocyclic or bicyclic aryl, such as aromatic groups: phenyl, naphthyl and tetrahydronaphthyl. Preferably, the aryl is C₆-C₁₀ aryl, more preferably, the aryl is phenyl and naphthyl, and most preferably, the aryl is naphthyl. The aryl may be substituted or unsubstituted.

"Heteroaryl" refers to an aromatic 5- to 6-membered monocyclic ring, an 8- to 10-membered bicyclic ring, or a 12- to 14-membered tricyclic ring, which may contain 1 to 4 heteroatoms selected from nitrogen, oxygen or S(O)ᵣ (wherein r is selected from 0, 1 or 2). Examples of the "heteroaryl" comprise, but are not limited to, furyl, pyridyl, 2-oxo-1,2-dihydropyridyl, pyridazinyl, pyrimidinyl, pyrazinyl, thienyl, isoxazolyl, oxazolyl, oxadiazolyl, imidazolyl, pyrrolyl, pyrazolyl, triazolyl, tetrazolyl, thiazolyl, isothiazolyl, 1,2,3-thiadiazolyl, benzodioxolyl, benzothiophenyl, benzimidazolyl, indolyl, isoindolyl, 1,3-dioxo-isoindolyl, quinolyl, indazolyl, benzisothiazolyl, benzoxazolyl, benzisoxazolyl, isothiazolyl, 1H-1,2,4-triazolyl, 4H-1,2,4-triazolyl, pyridyl, pyrimidinyl, pyrazine-2(1H)-keto, pyrimidine-4(3H)-keto, pyridazine-3(2H)-keto, 1H-indolyl, 1H-benzo[d]imidazolyl, 1H-pyrrolo[2,3-c]pyridyl, 3H-imidazo[4,5-c]pyridyl, isoquinolinyl, quinazolinyl, 2H-isoindolyl, furan[3,2-b]pyridyl, furan[2,3-c]pyridyl, thieno[2,3-c]pyridyl, benzofuryl, benzo[b]thienyl, 1H-pyrrolo[3,2-b]pyridyl, 2H-pyrrolo[3,4-c]pyridyl, 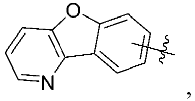 and the like. The heteroaryl may be substituted or unsubstituted.

"Fused ring" refers to a polycyclic group with two or more cyclic structures sharing one pair of atoms with each other, wherein at least one ring has a fully conjugated π electron aromatic system. Meanwhile, one or more rings may contain 0, 1 or more double bonds, but at least one ring does not have the fully conjugated π electron aromatic system, wherein 0, 1 or more ring atoms are selected from heteroatoms: nitrogen, oxygen or S(O)ᵣ (wherein r is selected from 0, 1 or 2), and the remaining ring atoms are carbon. The fused ring preferably comprises a bicyclic or tricyclic fused ring, wherein the bicyclic fused ring is preferably a fused ring of aryl or heteroaryl and monocyclic heterocyclyl or monocyclic cycloalkyl. Preferably, the fused ring is 6- to 16-membered, and more preferably, the fused ring is an 8- to 10-membered bicyclic fused ring or 12- to 17-membered tricyclic fused ring. Examples of "fused ring" comprise, but are not limited to: 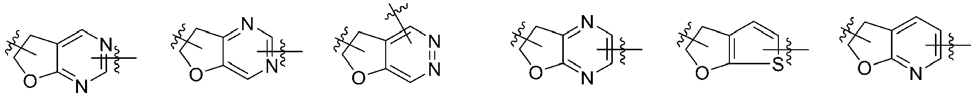 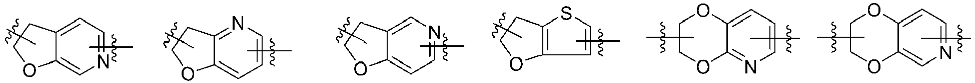 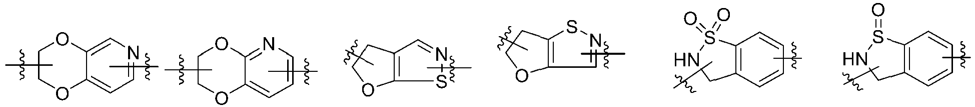 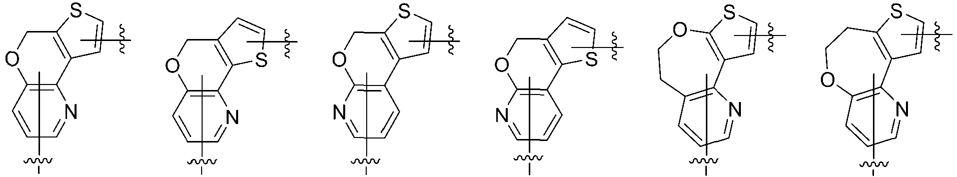 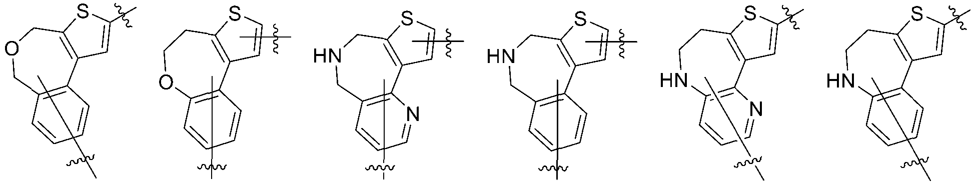 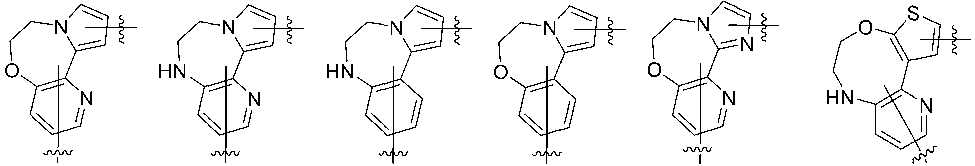 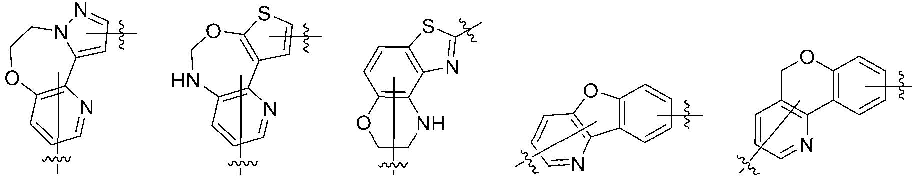 or

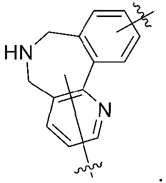

"Alkoxy" refers to a group of (alkyl-O-), wherein the alkyl is as defined herein. C₁-C₆ alkoxy is prefered. Examples of the alkoxy comprise, but are not limited to, methoxy, ethoxy, n-propoxy, isopropoxy, n-butoxy, isobutoxy, tert-butoxy, and the like.

"Nitro" refers to a -NO₂ group.

"Hydroxyl" refers to a -OH group.

"Halogen" refers to fluorine, chlorine, bromine and iodine.

"Amino" refers to -NH₂.

"Cyano" refers to -CN.

"Benzyl" refers to -CH₂-phenyl.

"Carboxyl" refers to -C(O)OH.

"Carboxylate group" refers to -C(O)O-alkyl or -C(O)O-cycloalkyl, wherein the alkyl and the cycloalkyl are as defined above.

"Hydroxyalkyl" refers to hydroxyl-substituted alkyl, wherein the alkyl is as defined above.

"Aminoalkyl" refers to amino-substituted alkyl, wherein the alkyl is as defined above.

"Haloalkyl" refers to halogen-substituted alkyl, wherein the alkyl is as defined above.

"Haloalkoxy" refers to halogen-substituted alkoxy, wherein the alkoxy is as defined above.

"DMSO" refers to dimethyl sulfoxide.

"BOC" refers to tert-butoxycarbonyl.

"Bn" refers to benzyl.

"THP" refers to 2-tetrahydropyranyl.

"TFA" refers to trifluoroacetic acid

"Ts" refers to p-toluenesulfonyl.

"SF₅" refers to pentafluorosulfanyl.

"Leaving group" is an atom or functional group detached from a larger molecule in a chemical reaction, which is a term used in a nucleophilic substitution reaction and an elimination reaction. In the nucleophilic substitution reaction, a reactant attacked by a nucleophilic reagent is called a substrate, and an atom or an atomic group broken together with a pair of electrons from a substrate molecule is called the leaving group. A group that accepts electrons easily and bears negative charges strongly is a good leaving group. When pKa of a conjugated acid of the leaving group is smaller, the leaving group is easier to be separated from other molecules. It is because that, when the pKa of the conjugated acid is smaller, the corresponding leaving group does not need to be combined with other atoms, and the trend of existence in the form of anion (or electrically neutral leaving group) is also enhanced. Common leaving groups comprise, but are not limited to, halogen, methanesulfonyl, -OTs or -OH.

"Substituted" refers to that one or more hydrogen atoms, preferably at most 5 hydrogen atoms, and more preferably 1 to 3 hydrogen atoms, in a group, are independently substituted with a corresponding number of substituents. It goes without saying that the substituents are only in their possible chemical positions, and those skilled in the art can determine (by experiment or theory) possible or impossible substitutions without excessive efforts. For example, amino or hydroxyl with free hydrogen may be unstable when combined with carbon atoms with unsaturated (such as olefinic) bonds.

"Substitution" or "substituted" in the specification, unless otherwise specified, refers to that the group may be substituted with one or more groups selected from: alkyl, alkenyl, alkynyl, alkoxy, alkylthio, alkylamino, halogen, SF₅, mercapto, hydroxyl, nitro, cyano, cycloalkyl, heterocyclyl, aryl, heteroaryl, cycloalkoxy, heterocycloalkoxy, cycloalkylthio, heterocycloalkylthio, amino, haloalkyl, hydroxyalkyl, carboxyl, carboxylate group, =O, -O(CH₂)ₘR^{A}, -C(O)R³, -C(O)OR³, -NHC(O)R³, -NHC(O)OR³, -NR⁴R⁵, -C(O)NR⁴R⁵, -S(O)₂NR⁴R⁵, -CH₂NHC(O)OR³, -CH₂NR⁴R⁵ or -S(O)ᵣR³;
or, two substituents, together with the same carbon atom to which the substituents are attached, form a cyclopropyl;
R^{A} is selected from a hydrogen atom, alkyl, cycloalkyl, heterocyclyl, aryl or heteroaryl, wherein the alkyl, cycloalkyl, heterocyclyl, aryl or heteroaryl is optionally further substituted with one or more substituents selected from hydroxyl, halogen, nitro, cyano, alkyl, alkoxy, haloalkyl, haloalkoxy, cycloalkyl, heterocyclyl, aryl, heteroaryl, =O, -C(O)R⁶, -C(O)OR⁶, -OC(O)R⁶, -NR⁷R⁸, -C(O)NR⁷R⁸, -SO₂NR⁷R⁸ or -NR⁷C(O)R⁸;
each R³ is independently selected from a hydrogen atom, alkyl, cycloalkyl, heterocyclyl, aryl and heteroaryl, wherein the alkyl, cycloalkyl, heterocyclyl, aryl or heteroaryl is optionally further substituted with one or more substituents selected from hydroxyl, halogen, nitro, cyano, alkyl, alkoxy, haloalkyl, haloalkoxy, cycloalkyl, heterocyclyl, aryl, heteroaryl, =O, -C(O)R⁶, -C(O)OR⁶, -OC(O)R⁶, -NR⁷R⁸, -C(O)NR⁷R⁸, -SO₂NR⁷R⁸ or -NR⁷C(O)R⁸.
R⁴ and R⁵ are each independently selected from a hydrogen atom, hydroxyl, alkyl, alkoxy, cycloalkyl, heterocyclyl, aryl or heteroaryl, wherein the alkyl, alkoxy, cycloalkyl, heterocyclyl, aryl or heteroaryl is optionally further substituted with one or more substituents selected from hydroxyl, halogen, nitro, cyano, alkyl, alkoxy, cycloalkyl, heterocyclyl, aryl, heteroaryl, =O, -C(O)R⁶, -C(O)OR⁶, -OC(O)R⁶, -NR⁷R⁸, -C(O)NR⁷R⁸, -SO₂NR⁷R⁸ or -NR⁷C(O)R⁸;
or, R⁴ and R⁵, together with atoms to which the groups are attached, form a 4-8 membered heterocyclyl, wherein the 4-8 membered heterocyclyl contains one or more N, O or S(O)ᵣ, and the 4-8 membered heterocyclyl is optionally further substituted with one or more substituents selected from hydroxyl, halogen, nitro, cyano, alkyl, alkoxy, cycloalkyl, heterocyclyl, aryl, heteroaryl, =O, -C(O)R⁶, -C(O)OR⁶, -OC(O)R⁶, -NR⁷R⁸, -C(O)NR⁷R⁸, -SO₂NR⁷R⁸ or -NR⁷C(O)R⁸;
R⁶, R⁷ and R⁸ are each independently selected from a hydrogen atom, alkyl, amino, cycloalkyl, heterocyclyl, aryl or heteroaryl, wherein the alkyl, cycloalkyl, heterocyclyl, aryl or heteroaryl is optionally further substituted with one or more substituents selected from hydroxyl, halogen, nitro, amino, cyano, alkyl, alkoxy, cycloalkyl, heterocyclyl, aryl, heteroaryl, carboxyl or a carboxylate group;
r is selected from 0, 1 or 2; and
m is selected from 0 or 1.

The compound of the present disclosure may contain an asymmetric center or a chiral center, thus existing in different stereoisomeric forms. It is expected that all stereoisomeric forms of the compound of the present disclosure comprise, but are not limited to, a diastereomer, an enantiomer, and an atropisomer and a geometric (conformational) isomer and a mixture thereof, such as a racemic mixture, which are all within the scope of the present disclosure.

Unless otherwise specified, the structure described in the present disclosure further comprises all isomers of this structure (such as forms of a diastereomer, an enantiomer, and an atropisomer and a geometric (conformational) isomer; such as R and S configurations of asymmetric centers, (Z) and (E) double-bond isomers, and (Z) and (E) conformational isomers). Therefore, a single stereoisomer, and an enantiomeric mixture, a diastereomeric mixture and a geometric (conformational) isomer mixture of the compound of the present disclosure are all within the scope of the present disclosure.

"Pharmaceutically acceptable salt" refers to some salts of the above compounds capable of maintaining original biological activity and suitable for medical use. The pharmaceutically acceptable salt of the compound as shown in formula (I) may be a metal salt or an amine salt formed with a suitable acid.

"Pharmaceutical composition" refers to a mixture containing one or more compounds described herein or their physiologically acceptable salts or prodrugs and other chemical components, and other components such as physiologically acceptable carriers and excipients. The purpose of the pharmaceutical composition is to promote administration to an organism, which is beneficial for the absorption of active ingredients, thereby exerting biological activity.

### Synthesis method for compound of the present disclosure

In order to achieve the objective of the present disclosure, the following technical solution is used in the present disclosure.

The present disclosure provides a preparation method for a compound as shown in formula (I), or a stereoisomer thereof, a tautomer thereof or a pharmaceutically acceptable salt thereof, and the method comprises: 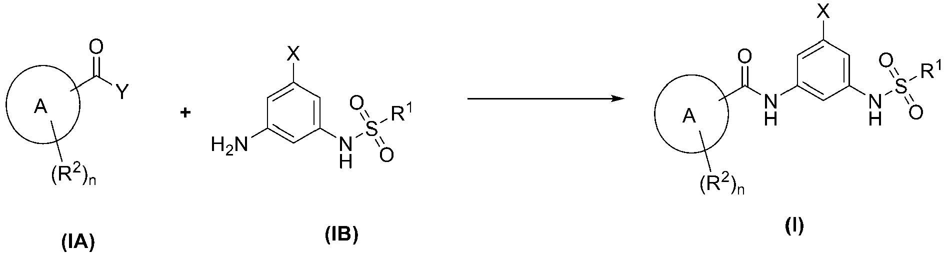
carrying out a condensation reaction on a compound as shown in formula (IA) and a compound as shown in formula (IB), and optionally further carrying out a substitution reaction to obtain the compound as shown in formula (I),
wherein:
   Y is selected from hydroxyl or chlorine; and
   ring A, X, R¹, R² and n are as defined in formula (I).

### DESCRIPTION OF EXEMPLARY EMBODIMENTS

The present disclosure is further described hereinafter with reference to the embodiments, but these embodiments are not intended to limit the scope of the present disclosure.

### Embodiments

The preparation of a representative compound as shown in formula (I) and related structural identification data are given in the embodiments. It must be noted that the following embodiments are used to illustrate the present disclosure and are not intended to limit the present disclosure.

A ¹H NMR spectrum is measured by a Bruker instrument (400 MHz), and a chemical shift is represented by ppm. Tetramethylsilane is used as an internal standard (0.00 ppm). In a representation method of ¹H NMR: s = single peak, d = double peak, t = triple peak, m = multiple peak, br = broadening, dd = double peak of double peak, and dt = double peak of triple peak. When a coupling constant is provided, the unit of the coupling constant is Hz.

A mass spectrum is measured by a LC/MS instrument, and an ionization mode may be ESI or APCI.

A silica gel plate used for thin-layer chromatography is a Yantai Huanghai HSGF254 or Qingdao GF254 silica gel plate, the silica gel plate used for thin-layer chromatography (TLC) adopts a specification of 0.15 mm-0.2 mm, and a product separated and purified by thin-layer chromatography adopts a specification of 0.4 mm-0.5 mm.

200-300-mesh silica gel of Yantai Huanghai silica gel is generally used as a carrier in column chromatography.

In the following embodiments, unless otherwise specified, all temperatures are Celsius temperatures. Unless otherwise specified, various starting materials and reagents are commercially available or synthesized according to known methods, and the commercially available raw materials and reagents are directly used without further purification. Unless otherwise specified, manufacturers of the commercially available raw materials and reagents comprise, but are not limited to, Aldrich Chemical Company, ABCR GmbH & Co.KG, Acros Organics, Guangzan Chemical Technology Co., Ltd., Jingyan Chemical Technology Co., Ltd., etc.
CD₃OD: deuterated methanol.
CDCl₃: deuterated chloroform.
DMSO-*d*₆: deuterated dimethyl sulfoxide.

Argon atmosphere means that a reaction bottle is connected with an argon balloon with a volume of about 1 L.

Unless otherwise specified in the embodiments, a solution in the reaction refers to an aqueous solution.

The compound is purified by silica gel column chromatography and reversed-phase column chromatography, wherein an eluent system is selected from A: petroleum ether and ethyl acetate system; B: dichloromethane and methanol system; C: dichloromethane: ethyl acetate; and D: aqueous trifluoroacetic acid solution and acetonitrile system. A volume ratio of a solvent varies according to the polarity of the compound, and may also be adjusted by adding a small amount of acidic or alkaline reagent, such as acetic acid or triethylamine.

### Example 1

### N-(3-chloro-5-(methylsulfonamido)phenyl)-6H-thieno[3',2':4,5]pyrano[3,2-b]pyridine-8-carbox amide

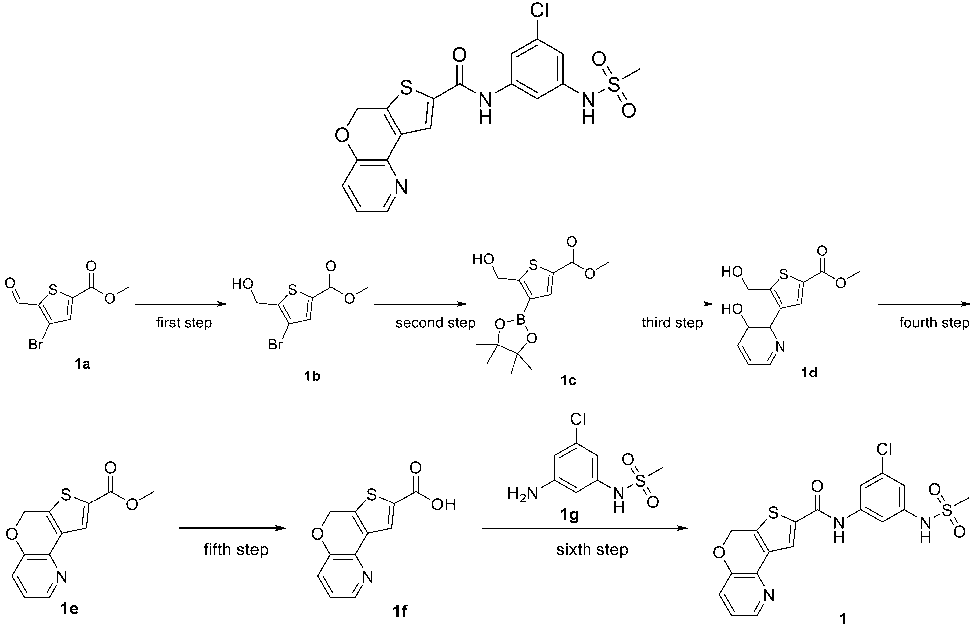

### First step

### methyl 4-bromo-5-(hydroxymethyl)thiophene-2-carboxylate

Methyl 4-bromo-5-formylthiophene-2-carboxylate **1a** (800 mg, 3.21 mmol, commercially available) was dissolved in methanol (10 mL), sodium acetoxyborohydride (2.04 g, 9.64 mmol) was added, and the mixture was stirred at 25°C for 2 hours. The reaction solution was diluted with water and extracted with ethyl acetate (30 mL × 3). The combined organic phase was washed with a saturated sodium chloride solution, dried with anhydrous sodium sulfate, filtered, and concentrated under reduced pressure to obtain methyl 4-bromo-5-(hydroxymethyl)thiophene-2-carboxylate **1b** (800 mg), with a yield of 99.2%.

MS m/z (ESI): 251.0 [M+1].

### Second step

### methyl

### 5-(hydroxymethyl)-4-(4,4,5,5-tetramethyl-1,3,2-dioxaborolan-2-yl)thiophene-2-carboxylate

Methyl 4-bromo-5-(hydroxymethyl)thiophene-2-carboxylate **1b** (400 mg, 1.59 mmol), bis(pinacolato)diboron (606.79 mg, 2.39 mmol) and potassium acetate (312.68 mg, 3.19 mmol) were added into dioxane (5 mL). The reaction solution was subjected to nitrogen purging for three times. Under nitrogen protection, [1,1'-bis(diphenylphosphino)ferrocene]dichloropalladium(II) (116.56 mg, 159.30 µmol) was added to the reaction solution. The mixture was heated to 90°C and stirred for 2 hours. The reaction solution was filtered to obtain methyl 5-(hydroxymethyl)-4-(4,4,5,5-tetramethyl-1,3,2-dioxaborolan-2-yl)thiophene-2-carboxylate **1c** (473 mg), which was used directly in the next step of reaction.

MS m/z (ESI): 299.0 [M+1].

### Third step

### methyl 5-(hydroxymethyl)-4-(3-hydroxypyridin-2-yl)thiophene-2-carboxylate

Methyl 5-(hydroxymethyl)-4-(4,4,5,5-tetramethyl-1,3,2-dioxaborolan-2-yl)thiophene-2-carboxylate **1c** (473 mg), bis(pinacolato)diboron (204.25 mg, 1.17 mmol) and potassium acetate (486.72 mg, 3.52 mmol) were added to 1,4-dioxane (4 mL) and water (1 mL). The reaction solution was subjected to nitrogen purging for three times. Under nitrogen protection, [1,1'-bis(diphenylphosphino)ferrocene]dichloropalladium(II) (85.89 mg, 117.39 µmol) was added to the reaction solution. The mixture was heated to 90°C and stirred for 2 hours. After the reaction was complete, the reaction solution was diluted with water and extracted with ethyl acetate (30 mL × 3). The combined organic phase was washed with a saturated sodium chloride solution, dried with anhydrous sodium sulfate, filtered, concentrated under reduced pressure, and separated and purified by silica gel column chromatography (eluent: system A) to obtain methyl 5-(hydroxymethyl)-4-(3-hydroxypyridin-2-yl)thiophene-2-carboxylate **1d** (130 mg), with a yield of 41.75 %.

MS m/z (ESI): 266.0 [M+1].

### Fourth step

### methyl 6H-thieno[3',2':4,5]pyrano[3,2-b]pyridine-8-carboxylate

Methyl 5-(hydroxymethyl)-4-(3-hydroxypyridin-2-yl)thiophene-2-carboxylate **1d** (130 mg, 490.04 µmol) and triphenylphosphine (385.59 mg, 1.47 mmol) were dissolved in tetrahydrofuran (6 mL). The reaction solution was subjected to nitrogen purging for three times. Diisopropyl azodicarboxylate (297.27 mg, 1.47 mmol) was added dropwise at 0°C, and the mixture was stirred at room temperature for 1 hour. The reaction solution was concentrated under reduced pressure, and separated and purified by silica gel column chromatography (eluent: system A) to obtain methyl 6H-thieno[3',2':4,5]pyrano[3,2-b]pyridine-8-carboxylate **1e** (50 mg), with a yield of 41.26%.

MS m/z (ESI): 248.0 [M+1].

### Fifth step

### 6H-thieno[3',2':4,5]pyrano[3,2-b]pyridine-8-carboxylic acid

Methyl 6H-thieno[3',2':4,5]pyrano[3,2-b]pyridine-8-carboxylate **1e** (50 mg, 202.21 µmol) was dissolved in tetrahydrofuran (1.5 mL) and water (0.5 mL), lithium hydroxide monohydrate (12.73 mg, 303.31 µmol) was added, and the mixture was stirred at 25°C for 1 hour. After the reaction was complete, the solvent was removed by rotary evaporation to obtain 6H-thieno[3',2':4,5]pyrano[3,2-b]pyridine-8-carboxylic acid **1f** (70 mg), with a yield of 74.21%, which was used directly in the next step of reaction.

MS m/z (ESI): 234.0 [M+1].

### Sixth step

### N-(3-chloro-5-(methylsulfonamido)phenyl)-6H-thieno[3',2':4,5]pyrano[3,2-b]pyridine-8-carbox amide

At room temperature, *O*-(7-azabenzotriazol-1-yl)-*N,N,N',N'*-tetramethyluronium hexafluorophosphate (335.30 mg, 643.11 µmol) and *N*,*N*-diisopropylethylamine (83.12 mg, 643.11 µmol) were added to a solution of 6H-thieno[3',2':4,5]pyrano[3,2-b]pyridine-8-carboxylic acid **1f** (50 mg, 214.37 µmol) and *N*-(3-amino-5-chlorophenyl)methanesulfonamide **1g** (70.96 mg, 321.55 µmol, prepared according to the published patent WO2023154519) in *N*,*N*-dimethylformamide (1 mL). The reaction solution was allowed to react at 25 °C for 3 hours. The reaction solution was diluted with water and extracted with ethyl acetate (30 mL × 3). The combined organic phase was washed with a saturated sodium chloride solution, dried with anhydrous sodium sulfate, and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% NH₄HCO₃+H₂O; and mobile phase B: CH₃CN) to obtain N-(3-chloro-5-(methylsulfonamido)phenyl)-6H-thieno[3',2':4,5]pyrano[3,2-b]pyridine-8-carbox amide **1** (6 mg), with a yield of 6.42%.

MS m/z (ESI): 435.8 [M+1].

¹H NMR (400 MHz, DMSO-*d₆*) δ 10.59 (s, 1H), 10.08 (s, 1H), 8.57 (s, 1H), 8.21 (dd, *J =* 4.8, 1.2 Hz, 1H), 7.70 (t, *J =* 1.6 Hz, 1H), 7.68 (d, *J =* 1.6 Hz, 1H), 7.38 (dd, *J =* 8.0, 1.2 Hz, 1H), 7.26 (dd, *J =* 8.0, 4.8 Hz, 1H), 6.96 (t, *J =* 2.0 Hz, 1H), 5.57 (s, 2H), 3.07 (s, 3H).

### Example 2

### N-(3-chloro-5-(methylsulfonamido)phenyl)-4H-thieno[2',3':4,5]pyrano[3,2-b]pyridine-2-carbox amide

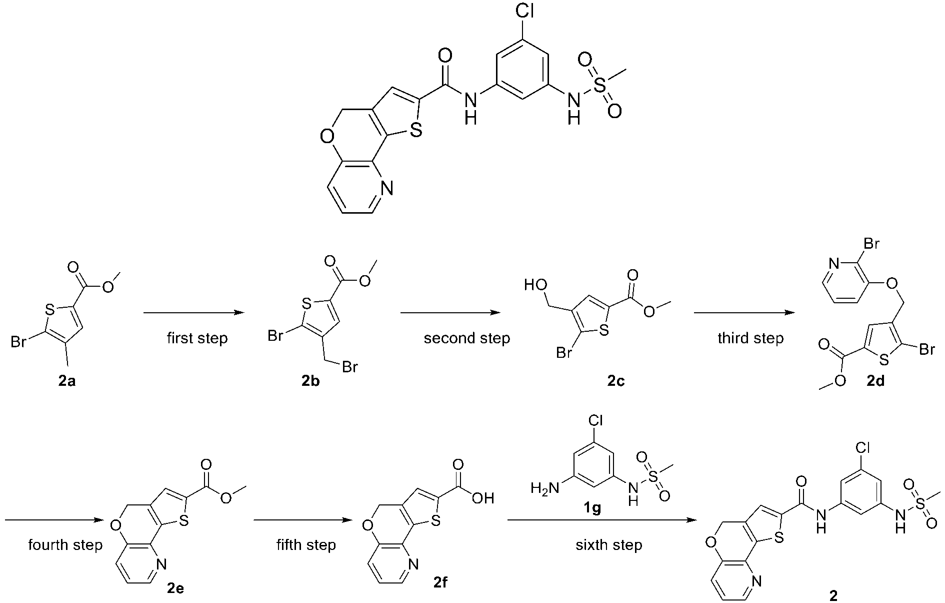

### First step

### methyl 5-bromo-4-(bromomethyl)thiophene-2-carboxylate

Methyl 5-bromo-4-methylthiophene-2-carboxylate **2a** (3 g, 12.76 mmol, commercially available) and N-bromosuccinimide (2.04 g, 11.48 mmol) were added to carbon tetrachloride (30 mL). The reaction solution was subjected to nitrogen purging for three times. Under nitrogen protection, dibenzoyl peroxide (30.91 mg, 127.61 µmol) was added to the reaction solution, and the mixture was heated to 80°C and stirred for 18 hours. The reaction solution was diluted with water and extracted with ethyl acetate (30 mL × 3). The combined organic phase was washed with a saturated sodium chloride solution, dried with anhydrous sodium sulfate, and concentrated under reduced pressure to obtain methyl 5-bromo-4-(bromomethyl)thiophene-2-carboxylate **2b** (4.1 g), which was used directly in the next step of reaction.

MS m/z (ESI): 314.8 [M+1].

### Second step

### methyl 5-bromo-4-(hydroxymethyl)thiophene-2-carboxylate

Methyl 5-bromo-4-(bromomethyl)thiophene-2-carboxylate **2b** (6.7 g, 21.34 mmol) was dissolved in acetonitrile (60 mL) and water (20 mL), potassium carbonate (8.85 g, 64.01 mmol) was added, and the mixture was heated to 60°C and stirred for 48 hours. The reaction solution was concentrated under reduced pressure, and separated and purified by silica gel column chromatography (eluent: system A) to obtain methyl 5-bromo-4-(hydroxymethyl)thiophene-2-carboxylate **2c** (1.3 g), with a yield of 24.26%.

MS m/z (ESI): 252.9 [M+1].

### Third step

### methyl 5-bromo-4-(((2-bromopyridin-3-yl)oxy)methyl)thiophene-2-carboxylate

Methyl 5-bromo-4-(hydroxymethyl)thiophene-2-carboxylate **2c** (350 mg, 1.39 mmol), 2-bromopyridin-3-ol (363.79 mg, 2.09 mmol) and triphenylphosphine (1.10 g, 4.18 mmol) were dissolved in tetrahydrofuran (20 mL). The reaction solution was subjected to nitrogen purging for three times. Diisopropyl azodicarboxylate (845.56 mg, 4.18 mmol) was added dropwise at 0°C, and the mixture was heated to 50°C and stirred for 1 hour. The reaction solution was concentrated under reduced pressure, and separated and purified by silica gel column chromatography (eluent: system A) to obtain methyl 5-bromo-4-(((2-bromopyridin-3-yl)oxy)methyl)thiophene-2-carboxylate **2d** (600 mg), with a yield of 82.69%.

MS m/z (ESI): 407.9 [M+1].

### Fourth step

### methyl 4H-thieno[2',3':4,5]pyrano[3,2-b]pyridine-2-carboxylate

Compound **2d** (750 mg, 1.84 mmol), bis(pinacolato)diboron (1.40 g, 5.53 mmol), di(1-adamantyl)-butylphosphine (264.23 mg, 736.96 µmol) and potassium carbonate (1.27 g, 9.21 mmol) were dissolved in 1,2-dimethoxyethane (10 mL) and water (1 mL). The reaction solution was subjected to nitrogen puring for three times. Under nitrogen protection, palladium(II) acetate (82.73 mg, 368.48 µmol) was added, and the mixture was heated to 80°C and stirred for 2 hours. The reaction solution was concentrated under reduced pressure, and separated and purified by silica gel column chromatography (eluent: system A) to obtain methyl 4H-thieno[2',3':4,5]pyrano[3,2-b]pyridine-2-carboxylate **2e** (300 mg), with a yield of 65.82%.

MS m/z (ESI): 247.9 [M+1].

### Fifth step

### 4H-thieno[2',3':4,5]pyrano[3,2-b]pyridine-2-carboxylic acid

Methyl 4H-thieno[2',3':4,5]pyrano[3,2-b]pyridine-2-carboxylate **2e** (420 mg, 1.70 mmol) was dissolved in tetrahydrofuran (12 mL) and water (4 mL), lithium hydroxide monohydrate (71.28 mg, 1.70 mmol) was added, and the mixture was stirred at 25°C for 1 hour. After the reaction was complete, 1 M hydrochloric acid solution was added to adjust pH to be slightly acidic. The mixture was then extracted with dichloromethane (20 mL × 3), dried wit anhydrous sodium sulfate, and concentrated under reduced pressure to obtain 4H-thieno[2',3':4,5]pyrano[3,2-b]pyridine-2-carboxylic acid **2f** (270 mg), with a yield of 68.15%.

MS m/z (ESI): 233.9 [M+1].

### Sixth step

### N-(3-chloro-5-(methylsulfonamido)phenyl)-4H-thieno[2',3':4,5]pyrano[3,2-b]pyridine-2-carbox amide

At room temperature, *O*-(7-azabenzotriazol-1-yl)-*N,N,N',N'*-tetramethyluronium hexafluorophosphate (1.48 g, 2.83 mmol) and *N*,*N*-diisopropylethylamine (365.71 mg, 2.83 mmol) were added to a solution of 4H-thieno[2',3':4,5]pyrano[3,2-b]pyridine-2-carboxylic acid **2f** (220 mg, 943.22 µmol) and *N*-(3-amino-5-chlorophenyl)methanesulfonamide **1g** (312.22 mg, 1.41 mmol) in *N*,*N*-dimethylformamide (1 mL). The reaction solution was allowed to react at 25 °C for 3 hours. The reaction solution was diluted with water and extracted with ethyl acetate (30 mL × 3). The combined organic phase was washed with a saturated sodium chloride solution, dried with anhydrous sodium sulfate, and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% NH₄HCO₃+H₂O; and mobile phase B: CH₃CN) to obtain N-(3-chloro-5-(methylsulfonamido)phenyl)-4H-thieno[2',3':4,5]pyrano[3,2-b]pyridine-2-carbox amide **2** (15 mg), with a yield of 3.65%.

MS m/z (ESI): 436.0 [M+1].

¹H NMR (400 MHz, DMSO-*d₆*) δ 10.55 (s, 1H), 10.09 (s, 1H), 8.16 (dd, *J =* 4.8, 1.4 Hz, 1H), 7.91 (s, 1H), 7.66 (dt, *J =* 21.8, 1.8 Hz, 2H), 7.32 (ddd, *J =* 12.8, 8.2, 3.0 Hz, 2H), 6.97 (t, *J =* 1.8 Hz, 1H), 5.48 (s, 2H), 3.08 (s, 3H).

Examples 3-4 were synthesized according to the synthesis methods in Examples 1-2 of the present disclosure. Spectral parameters of Examples 3-4 were shown in the table below:

| Serial number and structure of example | MS m/z (ESI) | ¹H NMR |
|---|---|---|
| 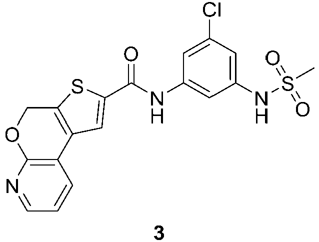 | 436.0 [M+1] | ¹H NMR (400 MHz, DMSO-*d₆*) δ 10.57 (s, 1H), 10.09 (s, 1H), 8.37 (s, 1H), 8.09 (dd, *J =* 4.8, 1.6 Hz, 1H), 7.93 (dd, *J* = 7.2, 1.6 Hz, 1H), 7.68 (s, 1H), 7.62 (s, 1H), 7.15 (dd, *J =* 7.2, 4.8 Hz, 1H), 6.97 (s, 1H), 5.65 (s, 2H), 3.08 (s, 3H). |
| 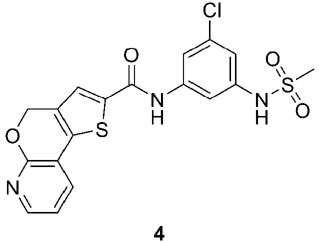 | 435.8 [M+1] | ¹H NMR (500 MHz, DMSO-*d₆*) δ 10.53 (s, 1H), 10.22 - 9.51 (m, 1H), 8.10 (dd, *J =* 4.8, 1.8 Hz, 1H), 7.90 (dd, *J =* 7.0, 2.2 Hz, 2H), 7.66 (s, 1H), 7.58 (s, 1H), 7.08 (dd, *J =* 7.4, 5.0 Hz, 1H), 6.95 (s, 1H), 5.55 (s, 2H), 3.05 (s, 3H). |

### Example 5

### N-(3-chloro-5-(methylsulfonamido)phenyl)-5,6-dihydrothieno[2',3':2,3]oxepino[4,5-b]pyridine-9-carboxamide

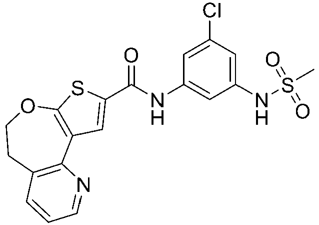

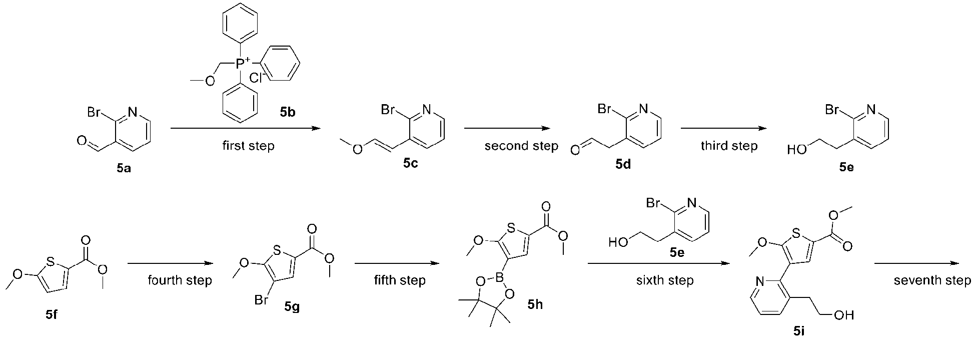

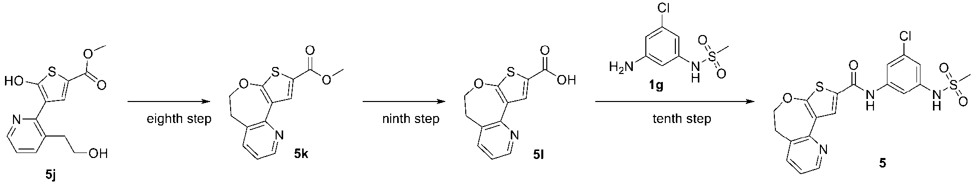

### First step

### (E)-2-bromo-3-(2-methoxyvinyl)pyridine

(Methoxymethyl)triphenylphosphonium chloride **5b** (13.8 g, 40.3 mmol, commercially available) was added to tetrahydrofuran (50 mL). The reaction solution was subjected to nitrogen purging for three times and then cooled to -78°C. Lithium bis(trimethylsilyl)amide (6.75 g, 40.3 mmol, 1 mol/L in THF) was added dropwise to the reaction solution, and the mixture was stirred at -78°C for 0.5 hours. Subsequently, 2-bromo-3-pyridinecarbaldehyde **5a** (5 g, 26.9 mmol, commercially available) was dissolved in tetrahydrofuran and slowly added dropwise to the reaction solution, and the mixture was stirred for 16 hours and gradually warmed to room temperature. The reaction solution was quenched with methanol at 0°C, concentrated under reduced pressure, and separated and purified by silica gel column chromatography (eluent: system A) to obtain (E)-2-bromo-3-(2-methoxyvinyl)pyridine **5c** (2.1 g), with a yield of 37%.

MS m/z (ESI): 215.9 [M+1].

### Second step

### 2-(2-bromopyridin-3-yl)acetaldehyde

At room temperature, the (E)-2-bromo-3-(2-methoxyvinyl)pyridine **5c** (2.1 g, 9.81 mmol) was added to a formic acid (5 mL) solution. The mixture was heated to 90°C and allowed to react for 16 hours. The reaction solution was concentrated under reduced pressure, and separated and purified by silica gel column chromatography (eluent: system A) to obtain 2-(2-bromopyridin-3-yl)acetaldehyde **5d** (700 mg), with a yield of 36%.

MS m/z (ESI): 201.9 [M+1].

### Third step

### 2-(2-bromopyridin-3-yl)ethan-1-ol

At room temperature, sodium borohydride (265 mg, 7.00 mmol) was added portionwise to a solution of 2-(2-bromopyridin-3-yl)acetaldehyde **5d** (700 mg, 3.50 mmol) in ethanol (10 mL). The reaction solution was allowed to react at room temperature for 16 hours. The reaction solution was quenched with methanol, concentrated under reduced pressure, and separated and purified by silica gel column chromatography (eluent: system A) to obtain 2-(2-bromopyridin-3-yl)ethan-1-ol **5e** (430 mg), with a yield of 61%.

MS m/z (ESI): 204.0 [M+1].

### Fourth step

### methyl 4-bromo-5-methoxythiophene-2-carboxylate

At room temperature, N-bromosuccinimide (2.27 g, 12.8 mmol) was added to a solution of methyl 5-methoxy-2-thiophenecarboxylate **5f** (2 g, 11.6 mmol, commercially available) in dichloromethane (50 mL). The reaction solution was allowed to react at room temperature for 2 hours. The reaction solution was concentrated under reduced pressure, and separated and purified by silica gel column chromatography (eluent: system A) to obtain methyl 4-bromo-5-methoxythiophene-2-carboxylate **5g** (2.8 g), with a yield of 96%.

MS m/z (ESI): 251.0 [M+1].

### Fifth step

### methyl 5-methoxy-4-(4,4,5,5-tetramethyl-1,3,2-dioxaborolan-2-yl)thiophene-2-carboxylate

At room temperature, [1,1'-bis(diphenylphosphino)ferrocene]dichloropalladium(II) (291 mg, 0.4 mmol), bis(pinacolato)diboron (2.63 g, 10.3 mmol), potassium acetate (1.56 g, 15.9 mmol) and methyl 4-bromo-5-methoxythiophene-2-carboxylate **5g** (2 g, 7.97 mmol) were added to dioxane (20 mL). The mixture was subjected to nitrogen purging for three times and allowed to react at 100°C for 2 hours. The mixture was filtered, the filter cake was rinsed with ethyl acetate (150 mL), and the filtrate was concentrated under reduced pressure, and separated and purified by silica gel column chromatography (eluent: system A) to obtain methyl 5-methoxy-4-(4,4,5,5-tetramethyl-1,3,2-dioxaborolan-2-yl)thiophene-2-carboxylate **5h** (1.0 g), with a yield of 42%.

MS m/z (ESI): 299.2 [M+1].

### Sixth step

### methyl 4-(3-(2-hydroxyethyl)pyridin-2-yl)-5-methoxythiophene-2-carboxylate

At room temperature, [1,1'-bis(diphenylphosphino)ferrocene]dichloropalladium(II) (156 mg, 0.21 mmol), 2-(2-bromopyridin-3-yl)ethan-1-ol **5e** (430 mg, 2.13 mmol), potassium carbonate (588 mg, 4.26 mmol) and methyl 5-methoxy-4-(4,4,5,5-tetramethyl-1,3,2-dioxaborolan-2-yl)thiophene-2-carboxylate **5h** (825 mg, 2.77 mmol) were added to a mixed solution of dioxane (5 mL) and water (1 mL). The mixture was subjected to nitrogen purging for three times, heated to 100°C and stirred for 16 hours. The mixture was filtered, the filter cake was rinsed with ethyl acetate (50 mL), and the filtrate was concentrated under reduced pressure, and separated and purified by silica gel column chromatography (eluent: system A) to obtain methyl 4-(3-(2-hydroxyethyl)pyridin-2-yl)-5-methoxythiophene-2-carboxylate **5i** (500 mg), with a yield of 80%.

MS m/z (ESI): 294.0 [M+1].

### Seventh step

### methyl 5-hydroxy-4-(3-(2-hydroxyethyl)pyridin-2-yl)thiophene-2-carboxylate

At room temperature, aluminum trichloride (2.27 g, 17.1 mmol) was added to a solution of methyl 4-(3-(2-hydroxyethyl)pyridin-2-yl)-5-methoxythiophene-2-carboxylate 5i (500 mg, 1.70 mmol) in dichloromethane (20 mL). The reaction solution was allowed to react at room temperature for 48 hours. The reaction solution was poured into water and extracted with ethyl acetate (100 mL × 3). The combined organic phase was washed with a saturated sodium chloride solution, dried with anhydrous sodium sulfate, concentrated under reduced pressure, and separated and purified by silica gel column chromatography (eluent: system A) to obtain methyl 5-hydroxy-4-(3-(2-hydroxyethyl)pyridin-2-yl)thiophene-2-carboxylate **5j** (300 mg), with a yield of 63%.

MS m/z (ESI): 280.0 [M+1].

¹H NMR (500 MHz, DMSO-*d₆*) δ 10.20 (s, 1H), 8.42 (d, *J =* 4.8 Hz, 1H), 8.10 (d, *J =* 7.2 Hz, 1H), 7.98 (s, 1H), 7.41 - 7.33 (m, 1H), 4.90 (s, 1H), 3.77 (t, *J =* 6.4 Hz, 2H), 3.72 (s, 3H), 3.06 (t, *J =* 6.4 Hz, 2H).

### Eighth step

### methyl 5,6-dihydrothieno[2',3':2,3]oxepino[4,5-b]pyridine-9-carboxylate

At room temperature, diisopropyl azodicarboxylate (586 mg, 2.90 mmol) was added to a solution of methyl 5-hydroxy-4-(3-(2-hydroxyethyl)pyridin-2-yl)thiophene-2-carboxylate **5j** (270 mg, 0.97 mmol) and triphenylphosphine (760 mg, 2.90 mmol) in a tetrahydrofuran (30 mL). The reaction solution was allowed to react at room temperature for 16 hours. The reaction solution was concentrated under reduced pressure, and separated and purified by silica gel column chromatography (eluent: system A) to obtain methyl 5,6-dihydrothieno[2',3':2,3]oxepino[4,5-b]pyridine-9-carboxylate **5k** (230 mg), with a yield of 91%.

MS m/z (ESI): 262.0 [M+1].

### Ninth step

### 5,6-dihydrothieno[2',3':2,3]oxepino[4,5-b]pyridine-9-carboxylic acid

At room temperature, lithium hydroxide monohydrate (111 mg, 2.64 mmol) was added to a mixed solution of methyl 5,6-dihydrothieno[2',3':2,3]oxepino[4,5-b]pyridine-9-carboxylate **5k** (230 mg, 0.88 mmol) in tetrahydrofuran (3 mL), methanol (3 mL) and water (3 mL). The reaction solution was allowed to react at room temperature for 16 hours. Dilute hydrochloric acid was added to the reaction solution to adjust pH to 6, and the mixture was extracted with ethyl acetate (30 mL × 3). The combined organic phase was washed with a saturated sodium chloride solution, dried with anhydrous sodium sulfate, and concentrated under reduced pressure to obtain 5,6-dihydrothieno[2',3':2,3]oxepino[4,5-b]pyridine-9-carboxylic acid **5l** (230 mg), with a yield of 91%.

MS m/z (ESI): 248.0 [M+1].

### Tenth step

### N-(3-chloro-5-(methylsulfonamido)phenyl)-5,6-dihydrothieno[2',3':2,3]oxepino[4,5-b]pyridine-9-carboxamide

5,6-dihydrothieno[2',3':2,3]oxepino[4,5-b]pyridine-9-carboxylic acid **51** (50.0 mg, 0.202 mmol) and *N*-(3-amino-5-chlorophenyl)methanesulfonamide **1g** (89.3 mg, 0.404 mmol) were added to *N*,*N*-dimethylformamide (5 mL) at 25°C. Subsequently, (7-azabenzotriazol-1-yloxy)tripyrrolidinophosphonium hexafluorophosphate (211 mg, 0.404 mmol) and *N*,*N*-diisopropylethylamine (78.4 mg, 0.607 mmol) were added, and the mixture was allowed to react at 65°C for 18 hours. The mixture was poured into water (50 mL) and extracted with ethyl acetate (100 mL×3). The combined organic phase was washed with a saturated sodium chloride solution, dried with anhydrous sodium sulfate, filtered, and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA+H₂O, and mobile phase B: CH₃CN) to obtain N-(3-chloro-5-(methylsulfonamido)phenyl)-5,6-dihydrothieno[2',3':2,3]oxepino[4,5-b]pyridine-9-carboxamide **5** (46 mg), with a yield of 51%.

MS m/z (ESI): 450.2 [M+1].

¹H NMR (400 MHz, DMSO-*d₆*) δ 10.56 (d, *J =* 2.4 Hz, 1H), 8.66 (d, *J =* 2.0 Hz, 1H), 8.58 - 8.47 (m, 1H), 7.73 - 7.61 (m, 3H), 7.28 - 7.20 (m, 1H), 6.92 (s, 1H), 4.62 - 4.48 (m, 2H), 3.26 - 3.18 (m, 2H), 3.067 (s, 3H).

### Example 6

### N-(3-chloro-5-(methylsulfonamido)phenyl)-6,7-dihydrothieno[3',2':4,5]oxepino[3,2-b]pyridine-9-carboxamide

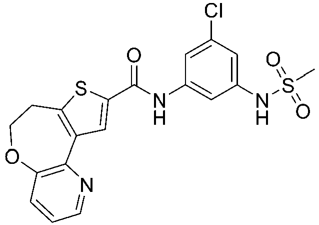

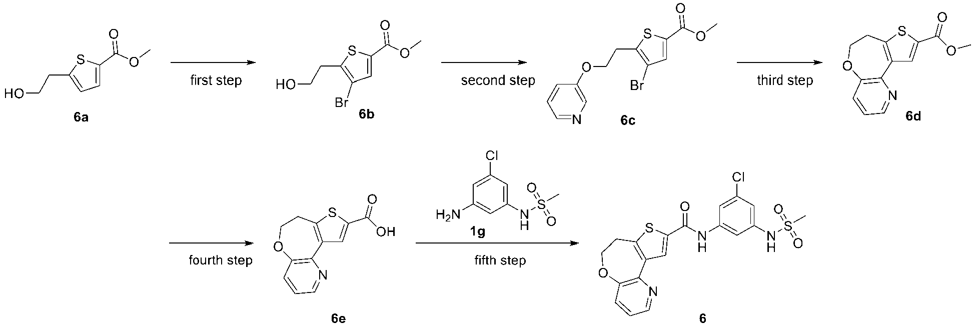

### First step

### methyl 4-bromo-5-(2-hydroxyethyl)thiophene-2-carboxylate

Methyl 5-(2-hydroxyethyl)thiophene-2-carboxylate **6a** (1.3 g, 6.98 mmol, commercially available) was added to N,N-dimethylformamide (15 mL), then N-bromosuccinimide (2.48 g, 13.96 mmol) was added, and the mixture was heated to 50°C and stirred for 12 hours. The reaction solution was concentrated under reduced pressure, and separated and purified by silica gel column chromatography (eluent: system A) to obtain methyl 4-bromo-5-(2-hydroxyethyl)thiophene-2-carboxylate 6b (900 mg), with a yield of 48.36%.

MS (ESI) m/z: 264.9 [M+H].

1H NMR (400 MHz, DMSO-*d₆*) δ 7.70 (s, 1H), 3.81 (s, 3H), 3.63 (d, J = 6.4 Hz, 2H), 2.93 (t, J = 6.4 Hz, 2H).

### Second step

### methyl 4-bromo-5-(2-((2-bromopyridin-3-yl)oxy)ethyl)thiophene-2-carboxylate

Methyl 4-bromo-5-(2-hydroxyethyl)thiophene-2-carboxylate **6b** (700 mg, 2.64 mmol), 2-bromopyridin-3-ol (689.09 mg, 3.96 mmol) and triphenylphosphine (2.08 g, 7.92 mmol) were dissolved in tetrahydrofuran (20 mL). The reaction solution was subjected to nitrogen purging for three times. Diisopropyl azodicarboxylate (1.60 g, 7.92 mmol) was added dropwise at 0°C, and the reaction solution was stirred at 50°C for 1 hour. The reaction solution was concentrated under reduced pressure, and separated by silica gel column chromatography (eluent: system A) to obtain methyl 4-bromo-5-(2-((2-bromopyridin-3-yl)oxy)ethyl)thiophene-2-carboxylate **6c** (1.2 g), with a yield of 78.39%.

MS (ESI) m/z: 421.7 [M+H].

### Third step

### methyl 6,7-dihydrothieno[3',2':4,5]oxepino[3,2-b]pyridine-9-carboxylate

Methyl 4-bromo-5-(2-((2-bromopyridin-3-yl)oxy)ethyl)thiophene-2-carboxylate **6c** (880 mg, 2.09 mmol), bis(pinacolato)diboron (1.59 g, 6.27 mmol), di(1-adamantyl)-butylphosphine (299.70 mg, 835.90 µmol) and potassium carbonate (1.44 g, 10.45 mmol) were dissolved in 1,2-dimethoxyethane (10 mL) and water (1 mL). The reaction solution was subjected to nitrogen purging for three times. Under nitrogen protection, palladium(II) acetate (93.83 mg, 417.95 µmol) was added, and the reaction solution was stirred at 100°C for 2 hours. The reaction solution was concentrated under reduced pressure, and separated by silica gel column chromatography (eluent: system A) to obtain methyl 6,7-dihydrothieno[3',2':4,5]oxepino[3,2-b]pyridine-9-carboxylate **6d** (270 mg), with a yield of 49.45%.

MS (ESI) m/z: 261.9 [M+H].

### Fourth step

### 6,7-dihydrothieno[3',2':4,5]oxepino[3,2-b]pyridine-9-carboxylic acid

Methyl 6,7-dihydrothieno[3',2':4,5]oxepino[3,2-b]pyridine-9-carboxylate **6d** (300 mg, 1.15 mmol) was dissolved in tetrahydrofuran (12 mL) and water (4 mL), a lithium hydroxide monohydrate (53.00 mg, 1.26 mmol) was added, and the mixture was stirred at 25°C for 1 hour. After the reaction was complete, 1 M hydrochloric acid solution was added to adjust pH to be slightly acidic, and then the mixture was extracted with dichloromethane (50 mL × 3). The combined organic phase was washed with a saturated sodium chloride solution, dried with anhydrous sodium sulfate, and concentrated under reduced pressure to obtain 6,7-dihydrothieno[3',2':4,5]oxepino[3,2-b]pyridine-9-carboxylic acid **6e** (230 mg), with a yield of 81.02%.

MS (ESI) m/z: 247.9 [M+H].

### Fifth step

### N-(3-chloro-5-(methylsulfonamido)phenyl)-6,7-dihydrothieno[3',2':4,5]oxepino[3,2-b]pyridine-9-carboxamide

At room temperature, *O*-(7-azabenzotriazol-1-yl)-*N,N,N',N'*-tetramethyluronium hexafluorophosphate (1.14 g, 2.18 mmol) and N,N-diisopropylethylamine (282.25 mg, 2.18 mmol) were added to a solution of 6,7-dihydrothieno[3',2':4,5]oxepino[3,2-b]pyridine-9-carboxylic acid **6e** (180 mg, 727.95 µmol) and N-(3-amino-5-chlorophenyl)methanesulfonamide **1g** (240.96 mg, 1.09 mmol) in N,N-dimethylformamide (1 mL). The reaction solution was allowed to react at 25°C for 3 hours. The reaction solution was diluted with water and extracted with ethyl acetate (30 mL × 3). The combined organic phase was washed with a saturated sodium chloride solution, dried with anhydrous sodium sulfate, and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA+H₂O, and mobile phase B: CH₃CN) to obtain N-(3-chloro-5-(methylsulfonamido)phenyl)-6,7-dihydrothieno[3',2':4,5]oxepino[3,2-b]pyridine-9-carboxamide **6** (12 mg), with a yield of 3.66%.

MS (ESI) m/z: 449.8 [M+H].

¹H NMR (400 MHz, DMSO-*d₆*) δ 10.67 (s, 1H), 10.06 (s, 1H), 8.86 (s, 1H), 8.40 (dd, J = 4.4, 1.6 Hz, 1H), 7.81 - 7.64 (m, 2H), 7.47 (dd, J = 8.0, 1.6 Hz, 1H), 7.31 (dd, J = 8.0, 4.4 Hz, 1H), 6.95 (t, J = 2.0 Hz, 1H), 4.39 (t, J = 4.8 Hz, 2H), 3.46 (t, J = 4.8 Hz, 2H), 3.07 (s, 3H).

### Example 7

### N-(3-chloro-5-(methylsulfonamido)phenyl)-5-oxo-6,7-dihydro-5H-pyrido[3,2-c]thieno[3,2-e]aze pine-9-carboxamide

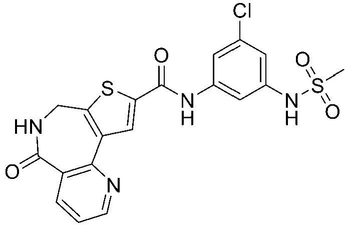

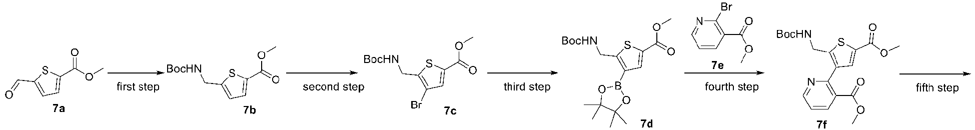

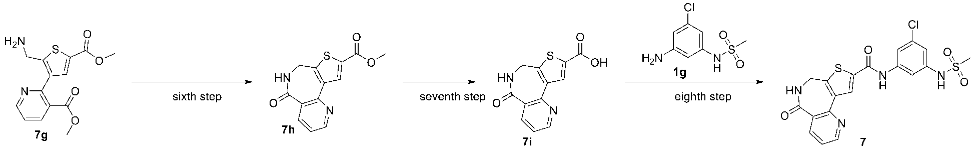

### First step

### methyl 5-(((tert-butoxycarbonyl)amino)methyl)thiophene-2-carboxylate

Triethylsilane (10.3 g, 88.1 mmol), tert-butyl carbamate (10.3 g, 88.1 mmol) and trifluoroacetic acid (6.70 g, 58.8 mmol) were added to a mixed solution of methyl 5-formylthiophene-2-carboxylate **7a** (5 g, 29.4 mmol, commercially available) in dichloromethane (50 mL) and acetonitrile (50 mL) at 25°C, and the mixture was allowed to react at room temperature for 18 hours. The mixture was poured into a saturated aqueous sodium bicarbonate solution (100 mL) and extracted with ethyl acetate (500 mL × 3). The combined organic phase was washed with a saturated sodium chloride solution, dried with anhydrous sodium sulfate, filtered, concentrated under reduced pressure, and separated and purified by silica gel column chromatography (eluent: system A) to obtain methyl 5-(((tert-butoxycarbonyl)amino)methyl)thiophene-2-carboxylate **7b** (7.0 g), with a yield of 88%. MS m/z (ESI): 215.9 [M+1].

### Second step

### methyl 4-bromo-5-(((tert-butoxycarbonyl)amino)methyl)thiophene-2-carboxylate

N-bromosuccinimide (9.84 g, 55.28 mmol) was added to a solution of methyl 5-(((tert-butoxycarbonyl)amino)methyl)thiophene-2-carboxylate **7b** (5.00 g, 18.4 mmol) in N,N-dimethylformamide (30 mL) at 25°C. The mixture was allowed to react at 50°C for 18 hours. The mixture was poured into water (500 mL) and extracted with ethyl acetate (400 mL×3). The combined organic phase was washed with a saturated sodium chloride solution, dried with anhydrous sodium sulfate, filtered, concentrated under reduced pressure, and separated and purified by silica gel column chromatography (eluent: system A) to obtain methyl 4-bromo-5-(((tert-butoxycarbonyl)amino)methyl)thiophene-2-carboxylate **7c** (2.5 g), with a yield of 39%.

MS m/z (ESI): 372.0 [M+1].

¹H NMR (400 MHz, DMSO-*d₆*) δ 7.75 (t, *J =* 6.0 Hz, 1H), 7.71 (s, 1H), 4.24 (d, *J* = 6.0 Hz, 2H), 3.82 (s, 3H), 1.41 (s, 9H).

### Third step

### methyl 5-(((tert-butoxycarbonyl)amino)methyl)-4-(4,4,5,5-tetramethyl-1,3,2-dioxaborolan-2-yl)thiophen e-2-carboxylate

Potassium acetate (420 mg, 4.28 mmol), bis(pinacolato)diboron (725 mg, 2.86 mmol), tris(dibenzylideneacetone)dipalladium (261 mg, 0.286 mmol) and 2-dicyclohexylphosphino-2',4',6'-triisopropylbiphenyl (204 mg, 0.428 mmol) were added to a solution of methyl 4-bromo-5-(((tert-butoxycarbonyl)amino)methyl)thiophene-2-carboxylate **7c** (500 mg, 1.43 mmol) in 1,4-dioxane (10 mL) at 25°C. The mixture was subjected to nitrogen purging for three times and allowed to react at 90°C for 1 hour. The mixture was filtered and concentrated under reduced pressure to obtain methyl 5-(((tert-butoxycarbonyl)amino)methyl)-4-(4,4,5,5-tetramethyl-1,3,2-dioxaborolan-2-yl)thiophen e-2-carboxylate **7d** (560 mg), which was used directly in the next step of reaction.

MS m/z (ESI): 420.2 [M+1].

### Fourth step

### methyl 2-(2-(((tert-butoxycarbonyl)amino)methyl)-5-(methoxycarbonyl)thiophen-3-yl)nicotinate

[1,1'-bis(diphenylphosphino)ferrocene]dichloropalladium(II) (210 mg, 0.287 mmol) and potassium carbonate (595 mg, 4.30 mmol) were added to a mixed solution of methyl 5-(((tert-butoxycarbonyl)amino)methyl)-4-(4,4,5,5-tetramethyl-1,3,2-dioxaborolan-2-yl)thiophen e-2-carboxylate **7d** (570 mg, 1.43 mmol) and methyl 2-bromo-3-pyridinecarboxylate **7e** (620 mg, 2.87 mmol, commercially available) in 1,4-dioxane (10 mL) and water (2 mL) at 25°C, and the mixture was heated to 90°C and allowed to react for 2 hours. The reaction solution was filtered and then concentrated under reduced pressure to obtain methyl 2-(2-(((tert-butoxycarbonyl)amino)methyl)-5-(methoxycarbonyl)thiophen-3-yl)nicotinate **7f** (500 mg), with a yield of 86%.

MS m/z (ESI): 407.1 [M+1].

### Fifth step

### methyl 2-(2-(aminomethyl)-5-(methoxycarbonyl)thiophen-3-yl)nicotinate

Trifluoroacetic acid (2 mL) was added to a solution of methyl 2-(2-(((tert-butoxycarbonyl)amino)methyl)-5-(methoxycarbonyl)thiophen-3-yl)nicotinate **7f** (560 mg, 1.38 mmol) in dichloromethane (4 mL) at 25°C, and the mixture was allowed to react at room temperature for 2 hours. The mixture was concentrated under reduced pressure to obtain methyl 2-(2-(aminomethyl)-5-(methoxycarbonyl)thiophen-3-yl)nicotinate **7g** (420 mg), which was used directly in the next step of reaction.

MS m/z (ESI): 306.9 [M+1].

### Sixth step

### methyl 5-oxo-6,7-dihydro-5H-pyrido[3,2-c]thieno[3,2-e]azepine-9-carboxylate

*N*,*N*-diisopropylethylamine (1.05 g, 8.16 mmol) was added to a solution of methyl 2-(2-(aminomethyl)-5-(methoxycarbonyl)thiophen-3-yl)nicotinate **7g** (500 mg, 1.63 mmol) in toluene (5 mL) at 25°C, and the mixture was allowed to react at 70°C for 18 hours. The mixture was concentrated under reduced pressure, and separated and purified by silica gel column chromatography (eluent: system A) to obtain methyl 5-oxo-6,7-dihydro-5H-pyrido[3,2-c]thieno[3,2-e]azepine-9-carboxylate **7h** (200 mg), with a yield of 45%.

MS m/z (ESI): 275.0 [M+1].

### Seventh step

### 5-oxo-6,7-dihydro-5H-pyrido[3,2-c]thieno[3,2-e]azepine-9-carboxylic acid

Lithium hydroxide monohydrate (59.7 mg, 1.42 mmol) was added to a mixed solution of methyl 5-oxo-6,7-dihydro-5H-pyrido[3,2-c]thieno[3,2-e]azepine-9-carboxylate **7h** (130 mg, 0.474 mmol) in tetrahydrofuran (5 mL) and water (2 mL) at 25°C, and the mixture was allowed to react at 25°C for 2 hours. The mixture was concentrated under reduced pressure to obtain 5-oxo-6,7-dihydro-5H-pyrido[3,2-c]thieno[3,2-e]azepine-9-carboxylic acid 7i (120 mg), with a yield of 65.82%.

MS m/z (ESI): 261.0 [M+1].

### Eighth step

### N-(3-chloro-5-(methylsulfonamido)phenyl)-5-oxo-6,7-dihydro-5H-pyrido[3,2-c]thieno[3,2-e]aze pine-9-carboxamide

5-oxo-6,7-dihydro-5H-pyrido[3,2-c]thieno[3,2-e]azepine-9-carboxylic acid **7i** (130 mg, 0.499 mmol) and *N*-(3-amino-5-chlorophenyl)methanesulfonamide **1g** (220 mg, 0.999 mmol) were added to *N*,*N*-dimethylformamide (5 mL) at 25°C. Subsequently, (7-azabenzotriazol-1-yloxy)tripyrrolidinophosphonium hexafluorophosphate (521 mg, 0.999 mmol) and *N*,*N*-diisopropylethylamine (194 mg, 1.50 mmol) were added, and the mixture was heated to 65°C and allowed to react for 18 hours. The mixture was poured into water (50 mL) and extracted with ethyl acetate (100 mL×3). The combined organic phase was washed with a saturated sodium chloride solution, dried with anhydrous sodium sulfate, filtered, and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA+H₂O, and mobile phase B: CH₃CN) to obtain N-(3-chloro-5-(methylsulfonamido)phenyl)-5-oxo-6,7-dihydro-5H-pyrido[3,2-c]thieno[3,2-e]aze pine-9-carboxamide **7** (50 mg), with a yield of 22%.

MS m/z (ESI): 463.0 [M+1].

¹H NMR (400 MHz, DMSO-*d₆*) δ 10.64 (s, 1H), 10.09 (s, 1H), 8.84 (dd, *J =* 4.8, 2.0 Hz, 1H), 8.77 (s, 1H), 8.62 (d, *J =* 1.6 Hz, 1H), 8.30 (dd, *J =* 8.0, 1.6 Hz, 1H), 7.74 - 7.67 (m, 2H), 7.55 (dd, *J =* 8.0, 4.8 Hz, 1H), 6.97 (t, *J =* 2.0 Hz, 1H), 4.31 (d, *J =* 6.0 Hz, 2H), 3.08 (s, 3H).

### Example 8

### N-(3-chloro-5-(methylsulfonamido)phenyl)-6-methyl-5-oxo-6,7-dihydro-5H-pyrido[3,2-c]thieno [3,2-e]azepine-9-carboxamide

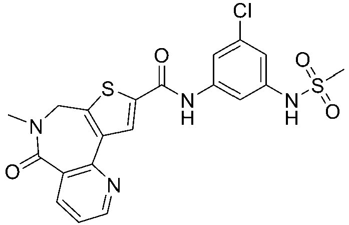

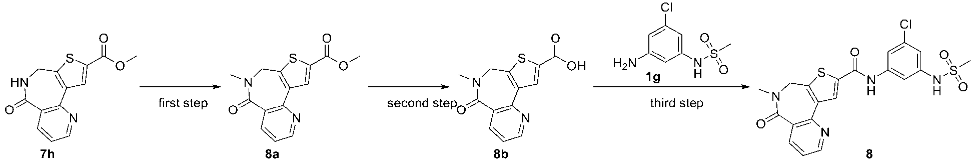

### First step

### methyl 6-methyl-5-oxo-6,7-dihydro-5H-pyrido[3,2-c]thieno[3,2-e]azepine-9-carboxylate

Sodium hydride (45.6 mg, 1.09 mmol, purity 60%) was added to a solution of methyl 5-oxo-6,7-dihydro-5H-pyrido[3,2-c]thieno[3,2-e]azepine-9-carboxylate **7h** (150 mg, 0.547 mmol) in N,N-dimethylformamide (5 mL) at 0°C, and the mixture was allowed to react at 0°C for 0.5 hours. Iodomethane (155 mg, 1.09 mmol) was added to the above solution, and the mixture was allowed to react at 25°C for 1 hour. The mixture was quenched with saturated aqueous ammonium chloride solution (5 mL), then water (30 mL) was added, and the mixture was extracted with ethyl acetate (100 mL × 3). The combined organic phase was washed with a saturated sodium chloride solution, dried with anhydrous sodium sulfate, filtered, concentrated under reduced pressure, and separated and purified by silica gel column chromatography (eluent: system A) to obtain methyl 6-methyl-5-oxo-6,7-dihydro-5H-pyrido[3,2-c]thieno[3,2-e]azepine-9-carboxylate 8a (130 mg), with a yield of 82%.

MS m/z (ESI): 289.0 [M+1].

### Second step

### 6-methyl-5-oxo-6,7-dihydro-5H-pyrido[3,2-c]thieno[3,2-e]azepine-9-carboxylic acid

Lithium hydroxide monohydrate (56.8 mg, 1.35 mmol) was added to a mixed solution of methyl 6-methyl-5-oxo-6,7-dihydro-5H-pyrido[3,2-c]thieno[3,2-e]azepine-9-carboxylate 8a (130 mg, 0.451 mmol) in tetrahydrofuran (5 mL) and water (2 mL) at 25°C, and the mixture was allowed to react at 25°C for 2 hours. The mixture was concentrated under reduced pressure to obtain 6-methyl-5-oxo-6,7-dihydro-5H-pyrido[3,2-c]thieno[3,2-e]azepine-9-carboxylic acid 8b (120 mg), which was used directly in the next step of reaction.

MS m/z (ESI): 275.0 [M+1].

### Third step

### N-(3-chloro-5-(methylsulfonamido)phenyl)-6-methyl-5-oxo-6,7-dihydro-5H-pyrido[3,2-c]thieno [3,2-e]azepine-9-carboxamide

6-methyl-5-oxo-6,7-dihydro-5H-pyrido[3,2-c]thieno[3,2-e]azepine-9-carboxylic acid **8b** (125 mg, 0.456 mmol) and *N*-(3-amino-5-chlorophenyl)methanesulfonamide **1g** (201 mg, 0.911 mmol) were added to *N*,*N*-dimethylformamide (5 mL) at 25°C. Subsequently, (7-azabenzotriazol-1-yloxy)tripyrrolidinophosphonium hexafluorophosphate (475 mg, 0.911 mmol) and *N*,*N*-diisopropylethylamine (177 mg, 1.37 mmol) were added, and the mixture was heated to 65°C and allowed to react for 3 hours. The mixture was poured into water (50 mL) and extracted with ethyl acetate (100 mL × 3). The combined organic phase was washed with a saturated sodium chloride solution, dried with anhydrous sodium sulfate, filtered, and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA+H₂O, and mobile phase B: CH₃CN) to obtain N-(3-chloro-5-(methylsulfonamido)phenyl)-6-methyl-5-oxo-6,7-dihydro-5H-pyrido[3,2-c]thieno [3,2-e]azepine-9-carboxamide **8** (40 mg), with a yield of 18%.

MS m/z (ESI): 477.0 [M+1].

¹H NMR (400 MHz, DMSO-*d₆*) δ 10.64 (s, 1H), 10.08 (s, 1H), 8.82 (dd, *J =* 4.4, 2.0 Hz, 1H), 8.62 (s, 1H), 8.28 (dd, *J =* 8.0, 2.0 Hz, 1H), 7.72 (t, *J =* 2.0 Hz, 1H), 7.68 (t, *J =* 2.0 Hz, 1H), 7.54 (dd, *J =* 8.0, 4.8 Hz, 1H), 6.96 (t, *J =* 2.0 Hz, 1H), 4.59 (s, 2H), 3.14 (s, 3H), 3.08 (s, 3H).

### Example 9

### N-(3-chloro-5-(methylsulfonamido)phenyl)-6,7-dihydro-5H-pyrido[3,2-c]thieno[3,2-e]azepine-9 -carboxamide

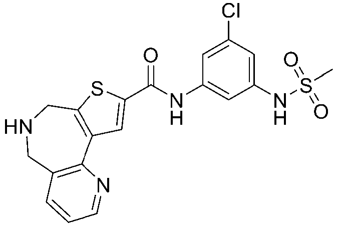

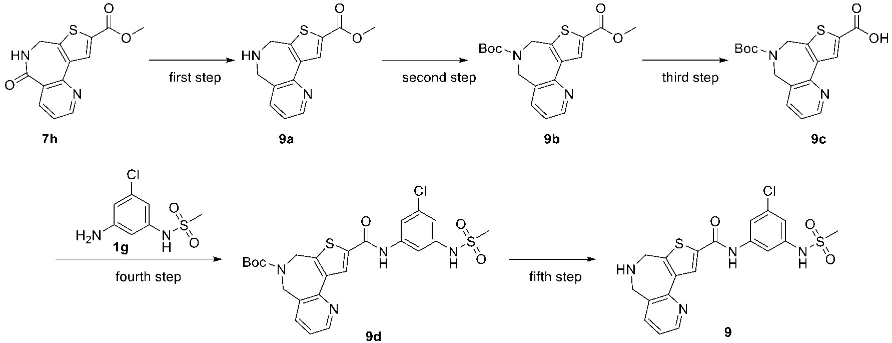

### First step

### methyl 6,7-dihydro-5H-pyrido[3,2-c]thieno[3,2-e]azepine-9-carboxylate

Methyl 5-oxo-6,7-dihydro-5H-pyrido[3,2-c]thieno[3,2-e]azepine-9-carboxylate 7h (500 mg, 1.82 mmol) was added to a solution of borane in tetrahydrofuran (1 M, 10 mL) at 25°C, and the mixture was heated to 65°C and allowed to react for 1 hour. The system was quenched with methanol (20 mL), and the mixture was concentrated under reduced pressure to obtain methyl 6,7-dihydro-5H-pyrido[3,2-c]thieno[3,2-e]azepine-9-carboxylate 9a (470 mg), which was used directly in the next step of reaction.

MS m/z (ESI): 261.0 [M+1].

### Second step

### 6-(tert-butyl) 9-methyl 5,7-dihydro-6H-pyrido[3,2-c]thieno[3,2-e]azepine-6,9-dicarboxylate

N,N-diisopropylethylamine (700 mg, 5.42 mmol), di-tert-butyl dicarbonate (788 mg, 3.61 mmol) and 4-dimethylaminopyridine (22.1 mg, 0.181 mmol) were added to a solution of methyl 6,7-dihydro-SH-pyrido[3,2-c]thieno[3,2-e]azepine-9-carboxylate **9a** (470 mg, 1.81 mmol) in N,N-dimethylformamide (10 mL) at 25°C, and the mixture was stirred at room temperature for 18 hours. The mixture was poured into water (50 mL) and extracted with ethyl acetate (200 mL × 3). The combined organic phase was washed with a saturated sodium chloride solution, dried with anhydrous sodium sulfate, filtered, concentrated under reduced pressure, and separated and purified by silica gel column chromatography (eluent: system A) to obtain 6-(tert-butyl) 9-methyl 5,7-dihydro-6H-pyrido[3,2-c]thieno[3,2-e]azepine-6,9-dicarboxylate **9b** (300 mg), with a yield of 46%.

MS m/z (ESI): 361.2 [M+1].

### Third step

### 6-(tert-butoxycarbonyl)-6,7-dihydro-5H-pyrido[3,2-c]thieno[3,2-e]azepine-9-carboxylic acid

Lithium hydroxide monohydrate (48.9 mg, 1.17 mmol) was added to a solution of 6-(tert-butyl) 9-methyl 5,7-dihydro-6H-pyrido[3,2-c]thieno[3,2-e]azepine-6,9-dicarboxylate **9b** (140 mg, 0.388 mmol) in tetrahydrofuran (2 mL) and water (1 mL) at 25°C, and the mixture was allowed to react at 25°C for 2 hours. The mixture was concentrated under reduced pressure to obtain 6-(tert-butoxycarbonyl)-6,7-dihydro-5H-pyrido[3,2-c]thieno[3,2-e]azepine-9-carboxylic acid **9c** (130 mg), which was used directly in the next step of reaction.

MS m/z (ESI): 347.0 [M+1].

### Fourth step

### tert-butyl 9-((3-chloro-5-(methylsulfonamido)phenyl)carbamoyl)-5,7-dihydro-6H-pyrido[3,2-c]thieno[3,2-e]azepine-6-carboxylate

The 6-(tert-butoxycarbonyl)-6,7-dihydro-5H-pyrido[3,2-c]thieno[3,2-e]azepine-9-carboxylic acid **9c** (130 mg, 0.375 mmol) and *N*-(3-amino-5-chlorophenyl)methanesulfonamide **1g** (166 mg, 0.751 mmol) were added to *N*,*N*-dimethylformamide (5 mL) at 25°C. Subsequently, (7-azabenzotriazol-1-yloxy)tripyrrolidinophosphonium hexafluorophosphate (391 mg, 0.751 mmol) and *N*,*N*-diisopropylethylamine (146 mg, 1.13 mmol) were added, and the mixture was heated to 65°C and stirred for 3 hours. The mixture was poured into water (100 mL) and extracted with ethyl acetate (150 mL×3). The combined organic phase was washed with a saturated sodium chloride solution, dried with anhydrous sodium sulfate, filtered, concentrated under reduced pressure, and separated and purified by silica gel column chromatography (eluent: system A) to obtain tert-butyl 9-((3-chloro-5-(methylsulfonamido)phenyl)carbamoyl)-5,7-dihydro-6H-pyrido[3,2-c]thieno[3,2-e]azepine-6-carboxylate **9d** (70 mg), with a yield of 34%.

MS m/z (ESI): 548.8 [M+1].

### Fifth step

### N-(3-chloro-5-(methylsulfonamido)phenyl)-6,7-dihydro-5H-pyrido[3,2-c]thieno[3,2-e]azepine-9 -carboxamide

Trifluoroacetic acid (1 mL) was added to a solution of tert-butyl 9-((3-chloro-5-(methylsulfonamido)phenyl)carbamoyl)-5,7-dihydro-6H-pyrido[3,2-c]thieno[3,2-e]azepine-6-carboxylate **9d** (50.0 mg, 0.0911 mmol) in dichloromethane (3 mL) at 25°C, and the mixture was allowed to react at 25°C for 2 hours. After the reaction was complete, the mixture was concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-6,7-dihydro-5H-pyrido[3,2-c]thieno[3,2-e]azepine-9 -carboxamide **9** (10 mg), with a yield of 24%.

MS m/z (ESI): 449.0 [M+1].

¹H NMR (400 MHz, DMSO-*d₆*) δ 10.62 (s, 1H), 10.01 (s, 1H), 8.76 (s, 1H), 8.54 (dd, *J =* 4.8, 1.6 Hz, 1H), 7.69 (dt, *J =* 6.0, 2.0 Hz, 2H), 7.63 (dd, *J =* 7.6, 1.6 Hz, 1H), 7.25 (dd, *J =* 7.6, 4.8 Hz, 1H), 6.93 (t, *J =* 2.0 Hz, 1H), 4.34 (s, 2H), 3.95 (s, 2H), 3.09 (s, 3H).

### Example 10

### N-(3-chloro-5-(methylsulfonamido)phenyl)-6-methyl-6,7-dihydro-5H-pyrido[3,2-c]thieno[3,2-e] azepine-9-carboxamide

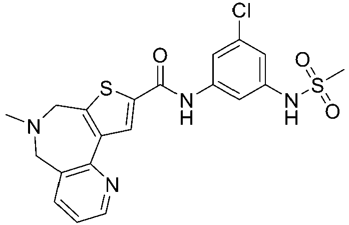

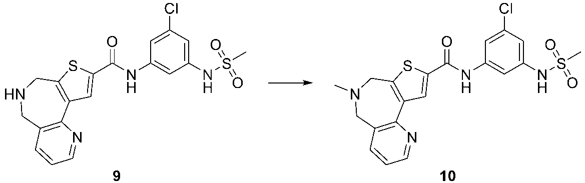

Aqueous formaldehyde solution (37.1 mg, 0.0457 mmol, purity 37%) was added to a solution of N-(3-chloro-5-(methylsulfonamido)phenyl)-6,7-dihydro-5H-pyrido[3,2-c]thieno[3,2-e]azepine-9 -carboxamide **9** (41.0 mg, 0.0913 mmol) in acetonitrile (3 mL) at 25°C, and the mixture was allowed to react at 25°C for 2 hours. The mixture was concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-6-methyl-6,7-dihydro-5H-pyrido[3,2-c]thieno[3,2-e] azepine-9-carboxamide **10** (10 mg), with a yield of 24%.

MS m/z (ESI): 463.0 [M+1].

¹H NMR (400 MHz, DMSO-*d₆*) δ 10.63 (s, 1H), 10.11 (s, 1H), 8.74 (s, 1H), 8.59 (dd, *J=* 4.8, 1.6 Hz, 1H), 7.74 (dd, *J =* 7.6, 1.6 Hz, 1H), 7.70 (dt, *J =* 8.0, 2.0 Hz, 2H), 7.32 (dd, *J =* 7.6, 4.8 Hz, 1H), 6.95 (s, 1H), 4.15 (s, 2H), 3.85 (s, 2H), 3.07 (s, 3H), 2.33 (s, 3H).

### Example 11

### N-(3-chloro-5-(methylsulfonamido)phenyl)-6-oxo-6,7-dihydro-5H-pyrido[3,2-b]thieno[3,2-d]aze pine-9-carboxamide

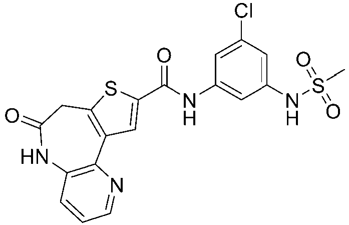

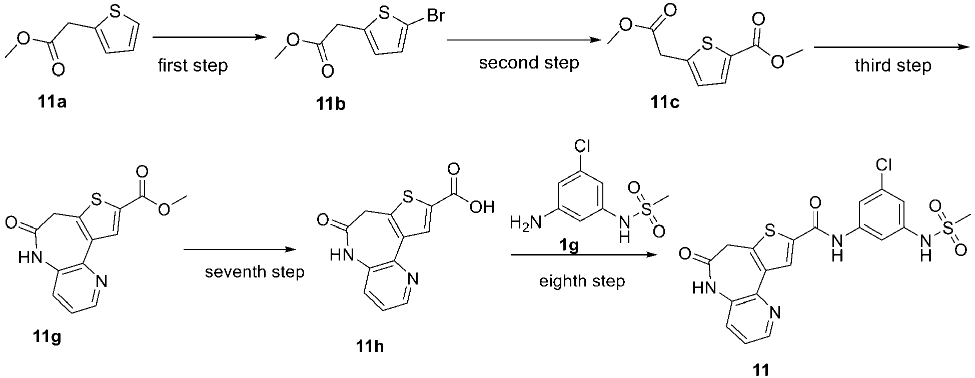

### First step

### methyl 2-(5-bromothiophen-2-yl)acetate

N-bromosuccinimide (13.7 g, 76.8 mmol) was added to a solution of methyl 2-thiopheneacetate **11a** (10.0 g, 64.0 mmol) in N,N-dimethylformamide (50 mL) at 25 °C, and the mixture was allowed to react at 25°C for 1 hour. The mixture was poured into water (500 mL) and extracted with ethyl acetate (400 mL). The organic phase was washed with saturated sodium chloride solution (50 mL), dried with anhydrous sodium sulfate, filtered, and concentrated under reduced pressure. The residue was purified by silica gel column chromatography (eluent: system A) to obtain methyl 2-(5-bromothiophen-2-yl)acetate **11b** (10.0 g), with a yield of 66%.

### Second step

### methyl 5-(2-methoxy-2-oxoethyl)thiophene-2-carboxylate

[1,1'-bis(diphenylphosphino)ferrocene]dichloropalladium(II) (1.14 g, 1.53 mmol) and triethylamine (4.65 g, 45.9 mmol) were added to a solution of methyl 2-(5-bromothiophen-2-yl)acetate **11b** (3.60 g, 15.3 mmol) in methanol (20 mL) at 25°C, and the system was subjected to carbon monoxide purging and allowed to react at 70°C for 18 hours. The mixture was filtered and concentrated under reduced pressure. The residue was purified by silica gel column chromatography (eluent: system A) to obtain methyl 5-(2-methoxy-2-oxoethyl)thiophene-2-carboxylate **11c** (1.5 g), with a yield of 46%.

MS m/z (ESI): 215.0 [M+1].

### Third step

### methyl 4-bromo-5-(2-methoxy-2-oxoethyl)thiophene-2-carboxylate

N-bromosuccinimide (1.94 g, 10.9 mmol) was added to a solution of methyl 5-(2-methoxy-2-oxoethyl)thiophene-2-carboxylate **11c** (1.80 g, 8.40 mmol) in trifluoroacetic acid (10 mL) and acetic acid (1 mL) at 25°C, and the mixture was allowed to react at 25°C for 1 hour. The mixture was diluted with ethyl acetate (100 mL), and the above ethyl acetate solution was added dropwise to a saturated aqueous sodium bicarbonate solution to adjust pH to 8-9. The organic phase was washed with saturated sodium chloride solution (50 mL), dried with anhydrous sodium sulfate, filtered, and concentrated under reduced pressure. The residue was purified by silica gel column chromatography (eluent: system A) to obtain methyl 4-bromo-5-(2-methoxy-2-oxoethyl)thiophene-2-carboxylate **11d** (1.2 g), with a yield of 49%.

MS m/z (ESI): 293.0/295.0 [M+1].

¹H NMR (400 MHz, DMSO-*d₆*) δ 7.75 (s, 1H), 4.02 (s, 2H), 3.85 (s, 3H), 3.68 (s, 3H).

### Fourth step

### methyl

### 5-(2-methoxy-2-oxoethyl)-4-(4,4,5,5-tetramethyl-1,3,2-dioxaborolan-2-yl)thiophene-2-carboxyla te

Potassium acetate (3.72 g, 37.9 mmol), bis(pinacolato)diboron (9.62 g, 37.9 mmol) and [1,1'-bis(diphenylphosphino)ferrocene]dichloropalladium(II) (924 mg, 1.26 mmol) were added to a solution of methyl 4-bromo-5-(2-methoxy-2-oxoethyl)thiophene-2-carboxylate **11d** (3.70 g, 12.6 mmol) in 1,4-dioxane (30 mL) at 25°C. The mixture was subjected to nitrogen purging for three times and allowed to react at 90°C for 4 hours. The mixture was filtered, and the filtrate was concentrated under reduced pressure. The residue was purified by silica gel column chromatography (eluent: system A) to obtain methyl 5-(2-methoxy-2-oxoethyl)-4-(4,4,5,5-tetramethyl-1,3,2-dioxaborolan-2-yl)thiophene-2-carboxyla te **11e** (3.50 g), with a yield of 82%.

MS m/z (ESI): 341.1 [M+1].

### Fifth step

### methyl 4-(3-aminopyridin-2-yl)-5-(2-methoxy-2-oxoethyl)thiophene-2-carboxylate

[1,1'-bis(diphenylphosphino)ferrocene]dichloropalladium(II) (698 mg, 0.955 mmol) and potassium carbonate (3.96 g, 28.7 mmol) were added to a mixed solution of methyl 5-(2-methoxy-2-oxoethyl)-4-(4,4,5,5-tetramethyl-1,3,2-dioxaborolan-2-yl)thiophene-2-carboxyla te **11e** (3.25 g, 9.55 mmol) and 2-bromo-3-aminopyridine (3.31 g, 19.1 mmol) in 1,4-dioxane (30 mL) and water (6 mL) at 25°C, and the mixture was allowed to react at 90°C for 2 hours. The reaction solution was filtered and then concentrated under reduced pressure. The residue was purified by silica gel column chromatography (eluent: system A) to obtain methyl 4-(3-aminopyridin-2-yl)-5-(2-methoxy-2-oxoethyl)thiophene-2-carboxylate **11f** (1.60 g), with a yield of 55%.

MS m/z (ESI): 307.0 [M+1].

### Sixth step

### methyl 6-oxo-6,7-dihydro-5H-pyrido[3,2-b]thieno[3,2-d]azepine-9-carboxylate

A solution of trimethylaluminum in n-hexane (2 M, 2.0 mL) was added to a solution of methyl 4-(3-aminopyridin-2-yl)-5-(2-methoxy-2-oxoethyl)thiophene-2-carboxylate **11f** (300 mg, 0.979 mmol) in tetrahydrofuran (10 mL) at 25°C, and the mixture was allowed to react at 25°C for 1 hour. The system was quenched with methanol, and then the mixture was concentrated under reduced pressure. The residue was purified by silica gel column chromatography (eluent: system A) to obtain methyl 6-oxo-6,7-dihydro-5H-pyrido[3,2-b]thieno[3,2-d]azepine-9-carboxylate **11g** (240 mg), with a yield of 89%.

MS m/z (ESI): 275.0 [M+1].

### Seventh step

### 6-oxo-6,7-dihydro-5H-pyrido[3,2-b]thieno[3,2-d]azepine-9-carboxylic acid

Methyl 6-oxo-6,7-dihydro-5H-pyrido[3,2-b]thieno[3,2-d]azepine-9-carboxylate **11g** (80.0 mg, 0.292 mmol) was added to a mixed solution of tetrahydrofuran (1.5 mL) and water (1.5 mL) at 25°C, then lithium hydroxide monohydrate (36.7 mg, 0.875 mmol) was added, and the mixture was allowed to react at 25°C for 18 hours. The mixture was concentrated under reduced pressure to obtain 6-oxo-6,7-dihydro-5H-pyrido[3,2-b]thieno[3,2-d]azepine-9-carboxylic acid **11h** (75.0 mg), which was used directly in the next step of reaction.

MS m/z (ESI): 261.0 [M+1].

### Eighth step

### N-(3-chloro-5-(methylsulfonamido)phenyl)-6-oxo-6,7-dihydro-5H-pyrido[3,2-b]thieno[3,2-d]aze pine-9-carboxamide

6-oxo-6,7-dihydro-5H-pyrido[3,2-b]thieno[3,2-d]azepine-9-carboxylic acid **11h** (75.0 mg, 0.288 mmol) and *N*-(3-amino-5-chlorophenyl)methanesulfonamide **1g** (127 mg, 0.576 mmol) were added to N,N-dimethylformamide (5 mL) at 25°C, and (7-azabenzotriazole-1-yloxy)tripyrrolidinophosphonium hexafluorophosphate (300 mg, 0.576 mmol) and N,N-diisopropylethylamine (112 mg, 0.864 mmol) were added to the above reaction solution. The mixture was allowed to react at 65°C for 2 hours. The mixture was poured into water (50 mL) and extracted with ethyl acetate (100 mL). The organic phase was washed with saturated sodium chloride solution (50 mL), dried with anhydrous sodium sulfate, filtered, and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-6-oxo-6,7-dihydro-5H-pyrido[3,2-b]thieno[3,2-d]aze pine-9-carboxamide **11** (3.00 mg), with a yield of 2%.

MS m/z (ESI): 463.0 [M+1].

¹H NMR (400 MHz, DMSO-*d₆*) δ 10.57 (s, 1H), 10.39 (s, 1H), 8.57 (s, 1H), 8.50 (dd, J = 4.4, 1.6 Hz, 1H), 7.72 - 7.59 (m, 3H), 7.46 (dd, J = 8.0, 4.4 Hz, 1H), 6.93 (t, J = 2.0 Hz, 1H), 3.75 (s, 2H), 3.03 (s, 3H).

### Example 12

### N-(3-chloro-5-(methylsulfonamido)phenyl)-6,7-dihydro-5H-pyrido[3,2-b]thieno[3,2-d]azepine-9 -carboxamide

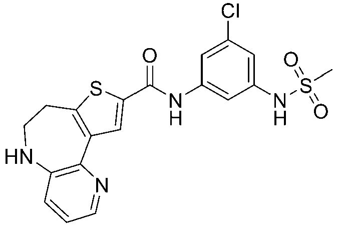

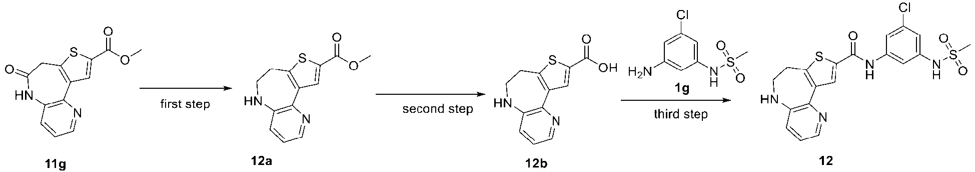

### First step

### methyl 6,7-dihydro-5H-pyrido[3,2-b]thieno[3,2-d]azepine-9-carboxylate

Methyl 6-oxo-6,7-dihydro-5H-pyrido[3,2-b]thieno[3,2-d]azepine-9-carboxylate **11g** (200 mg, 0.729 mmol) was added to a solution of borane in tetrahydrofuran (1 M, 10 mL) at 25°C, and the mixture was allowed to react at 65°C for 1 hour. The system was quenched with methanol (20 mL), and the mixture was concentrated under reduced pressure. The residue was purified by silica gel column chromatography (eluent: system A) to obtain methyl 6,7-dihydro-5H-pyrido[3,2-b]thieno[3,2-d]azepine-9-carboxylate **12a** (160 mg), with a yield of 84%.

MS m/z (ESI): 261.0 [M+1].

### Second step

### 6,7-dihydro-5H-pyrido[3,2-b]thieno[3,2-d]azepine-9-carboxylic acid

Methyl 6,7-dihydro-5H-pyrido[3,2-b]thieno[3,2-d]azepine-9-carboxylate **12a** (40.0 mg, 0.154 mmol) was added to a mixed solution of tetrahydrofuran (1 mL), methanol (2 mL) and water (1 mL) at 25°C, then lithium hydroxide monohydrate (19.3 mg, 0.461 mmol) was added, and the mixture was allowed to react at 25°C for 2 hours. The mixture was concentrated under reduced pressure to obtain 6,7-dihydro-5H-pyrido[3,2-b]thieno[3,2-d]azepine-9-carboxylic acid **12b** (40 mg), which was used directly in the next step of reaction.

MS m/z (ESI): 247.0 [M+1].

### Third step

### N-(3-chloro-5-(methylsulfonamido)phenyl)-6,7-dihydro-5H-pyrido[3,2-b]thieno[3,2-d]azepine-9 -carboxamide

The 6,7-dihydro-5H-pyrido[3,2-b]thieno[3,2-d]azepine-9-carboxylic acid **12b** (40.0 mg, 0.162 mmol) and *N*-(3-amino-5-chlorophenyl)methanesulfonamide **1g** (71.7 mg, 0.325 mmol) were added to *N*,*N*-dimethylformamide (4 mL) at 25°C, then (7-azabenzotriazol-1-yloxy)tripyrrolidinophosphonium hexafluorophosphate (169 mg, 0.325 mmol) and *N*,*N*-diisopropylethylamine (63.0 mg, 0.487 mmol) were added to the above reaction solution, and the mixture was allowed to react at 65°C for 3 hours. The mixture was poured into water (100 mL) and extracted with ethyl acetate (150 mL). The organic phase was washed with saturated sodium chloride solution (50 mL), dried with anhydrous sodium sulfate, filtered, and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-6,7-dihydro-5H-pyrido[3,2-b]thieno[3,2-d]azepine-9 -carboxamide **12** (11.0 mg), with a yield of 15%.

MS m/z (ESI): 449.0 [M+1].

¹H NMR (400 MHz, DMSO-*d₆*) δ 10.60 (s, 1H), 10.07 (s, 1H), 8.84 (s, 1H), 8.03 (dd, *J =* 4.4, 1.6 Hz, 1H), 7.71 - 7.69 (m, 2H), 7.18 (dd, *J =* 8.0, 1.6 Hz, 1H), 7.07 (dd, *J =* 8.0, 4.4 Hz, 1H), 6.94 (t, *J* = 2.0 Hz, 1H), 6.48 (t, *J =* 4.4 Hz, 1H), 3.38 - 3.33 (m, 2H), 3.23 - 3.19 (m, 2H), 3.07 (s, 3H).

### Example 13

### N-(3-chloro-5-(methylsulfonamido)phenyl)-5-methyl-6-oxo-6,7-dihydro-5H-pyrido[3,2-b]thieno [3,2-d]azepine-9-carboxamide

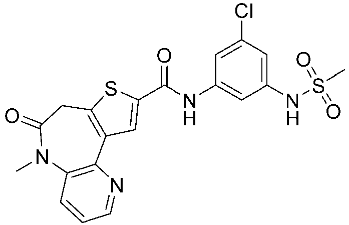

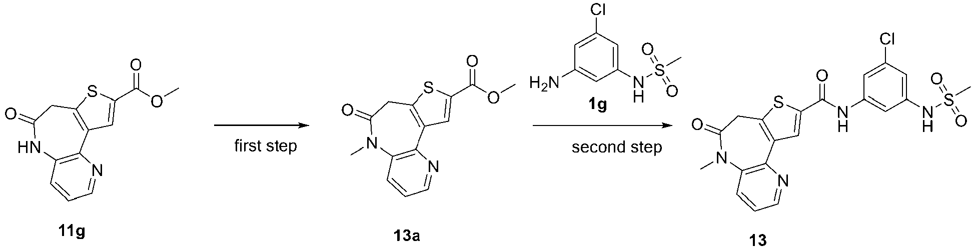

### First step

### methyl 5-methyl-6-oxo-6,7-dihydro-5H-pyrido[3,2-b]thieno[3,2-d]azepine-9-carboxylate

Iodomethane (51.8 mg, 0.365 mmol) and cesium carbonate (178 mg, 0.547 mmol) were added to a solution of methyl 6-oxo-6,7-dihydro-5H-pyrido[3,2-b]thieno[3,2-d]azepine-9-carboxylate **11g** (50.0 mg, 0.183 mmol) in tetrahydrofuran (10 mL) at 25°C, and the mixture was allowed to react at 50°C for 1 hour. The mixture was filtered and concentrated under reduced pressure to obtain methyl 5-methyl-6-oxo-6,7-dihydro-5H-pyrido[3,2-b]thieno[3,2-d]azepine-9-carboxylate **13a** (50.0 mg), which was used directly in the next step.

MS m/z (ESI): 289.0 [M+1].

### Second step

### N-(3-chloro-5-(methylsulfonamido)phenyl)-5-methyl-6-oxo-6,7-dihydro-5H-pyrido[3,2-b]thieno [3,2-d]azepine-9-carboxamide

Methyl 5-methyl-6-oxo-6,7-dihydro-5H-pyrido[3,2-b]thieno[3,2-d]azepine-9-carboxylate **13a** (50.0 mg, 0.173 mmol) and *N*-(3-amino-5-chlorophenyl)methanesulfonamide **1g** (76.5 mg, 0.347 mmol) were added to tetrahydrofuran (10 mL) at 25°C, a solution of trimethylaluminum in n-hexane (2 M, 0.86 mL) was added to the above reaction solution, and the mixture was allowed to react at 60°C for 3 hours. The mixture was poured into methanol (50 mL) and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-5-methyl-6-oxo-6,7-dihydro-5H-pyrido[3,2-b]thieno [3,2-d]azepine-9-carboxamide **13** (10.0 mg), with a yield of 12%.

MS m/z (ESI): 472.2 [M+1].

¹H NMR (400 MHz, DMSO-*d₆*) δ 10.59 (s, 1H), 10.12 (s, 1H), 8.69 - 8.49 (m, 2H), 8.03 (dd, J = 8.4, 1.2 Hz, 1H), 7.71 - 7.64 (m, 2H), 7.55 (dd, J = 8.4, 4.4 Hz, 1H), 6.96 (t, J = 2.0 Hz, 1H), 3.74 (s, 2H), 3.27 (s, 3H), 3.06 (s, 3H).

### Example 14

### N-(3-chloro-5-(methylsulfonamido)phenyl)-5-methyl-6,7-dihydro-5H-pyrido[3,2-b]thieno[3,2-d] azepine-9-carboxamide

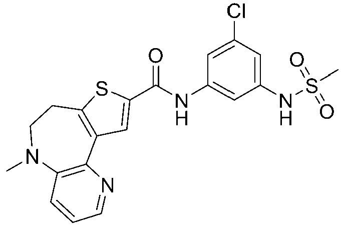

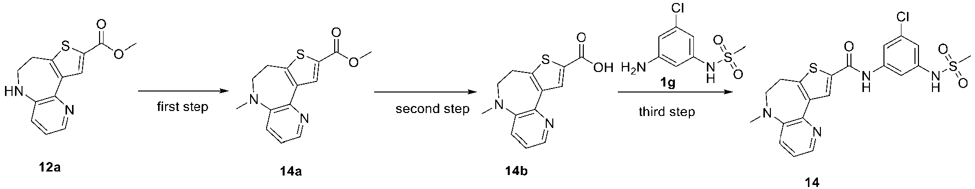

### First step

### methyl 5-methyl-6,7-dihydro-5H-pyrido[3,2-b]thieno[3,2-d]azepine-9-carboxylate

An aqueous formaldehyde solution (156 mg, 1.92 mmol, purity 37%), acetic acid (23.1 mg, 0.384 mmol) and sodium triacetoxyborohydride (244 mg, 1.15 mmol) were added to a solution of methyl 6,7-dihydro-5H-pyrido[3,2-b]thieno[3,2-d]azepine-9-carboxylate **12a** (100 mg, 0.384 mmol) in 1,2-dichloroethane (5 mL) at 25°C, and the mixture was allowed to react at 25°C for 2 hours. The mixture was poured into water (100 mL) and extracted with ethyl acetate (150 mL). The organic phase was washed with a saturated sodium chloride solution (50 mL), dried with anhydrous sodium sulfate, filtered, and concentrated under reduced pressure. The residue was purified by silica gel column chromatography (eluent: system A) to obtain methyl 5-methyl-6,7-dihydro-5H-pyrido[3,2-b]thieno[3,2-d]azepine-9-carboxylate **14a** (100 mg), with a yield of 95%.

MS m/z (ESI): 275.0 [M+1].

### Second step

### 5-methyl-6,7-dihydro-5H-pyrido[3,2-b]thieno[3,2-d]azepine-9-carboxylic acid

Methyl 5-methyl-6,7-dihydro-5H-pyrido[3,2-b]thieno[3,2-d]azepine-9-carboxylate **14a** (85.0 mg, 0.310 mmol) was added to a mixed solution of tetrahydrofuran (3 mL) and water (1.5 mL) at 25°C, then lithium hydroxide monohydrate (39.0 mg, 0.930 mmol) was added, and the mixture was allowed to react at 50°C for 2 hours. The mixture was concentrated under reduced pressure to obtain 5-methyl-6,7-dihydro-5H-pyrido[3,2-b]thieno[3,2-d]azepine-9-carboxylic acid **14b** (80 mg), which was used directly in the next step.

MS m/z (ESI): 261.0 [M+1].

### Third step

### N-(3-chloro-5-(methylsulfonamido)phenyl)-5-methyl-6,7-dihydro-5H-pyrido[3,2-b]thieno[3,2-d] azepine-9-carboxamide

5-methyl-6,7-dihydro-5H-pyrido[3,2-b]thieno[3,2-d]azepine-9-carboxylic acid **14b** (80.0 mg, 0.307 mmol) and *N*-(3-amino-5-chlorophenyl)methanesulfonamide **1g** (136 mg, 0.615 mmol) were added to *N*,*N*-dimethylformamide (5 mL) at 25°C, then (7-azabenzotriazol-1-yloxy)tripyrrolidinophosphonium hexafluorophosphate (641 mg, 1.23 mmol) and *N*,*N*-diisopropylethylamine (238 mg, 1.84 mmol) were added to the above reaction solution, and the mixture was allowed to react at 65°C for 3 hours. The mixture was poured into water (100 mL) and extracted with ethyl acetate (150 mL). The organic phase was washed with saturated sodium chloride solution (50 mL), dried with anhydrous sodium sulfate, filtered, and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-5-methyl-6,7-dihydro-5H-pyrido[3,2-b]thieno[3,2-d] azepine-9-carboxamide **14** (10.0 mg), with a yield of 12%.

MS m/z (ESI): 463.2 [M+1].

¹H NMR (400 MHz, DMSO-*d₆*) δ 10.61 (s, 1H), 10.11 (s, 1H), 8.81 (s, 1H), 8.18 (dd, *J* = 4.4, 1.6 Hz, 1H), 7.72 - 7.69 (m, 2H), 7.37 (dd, *J =* 8.4, 1.6 Hz, 1H), 7.23 (dd, *J =* 8.4, 4.4 Hz, 1H), 6.94 (t, *J =* 2.0 Hz, 1H), 3.34 - 3.30 (m, 2H), 3.30 - 3.26 (m, 2H), 3.07 (s, 3H), 2.99 (s, 3H).

### Example 15

### N-(3-chloro-5-(methylsulfonamido)phenyl)-10-methoxy-6-methyl-5,6-dihydro-4H-benzo[b]thie no[3,2-d]azepine-2-carboxamide

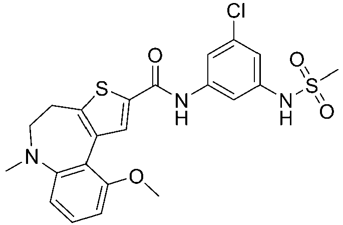

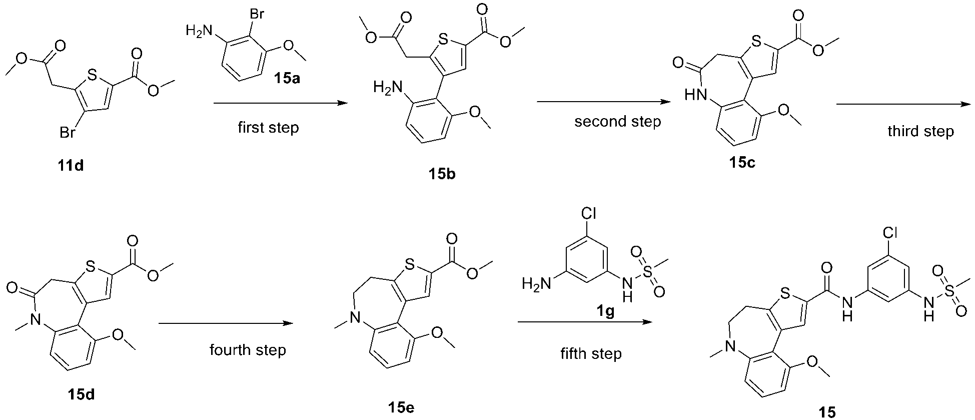

### First step

### methyl 4-(2-amino-6-methoxyphenyl)-5-(2-methoxy-2-oxoethyl)thiophene-2-carboxylate

At room temperature, the methyl 4-bromo-5-(2-methoxy-2-oxoethyl)thiophene-2-carboxylate **11d** (3.00 g, 10.23 mmol) was dissolved in a mixed solution of 1,2-dimethoxyethane (30 mL) and water (3 mL), and then 2-bromo-3-methoxyaniline **15a** (6.20 g, 30.70 mmol), potassium carbonate (4.24 g, 30.70 mmol), bis(pinacolato)diboron (12.99 g, 51.17 mmol), palladium(II) acetate (459.53 mg, 2.05 mmol) and di(1-adamantyl)-n-butylphosphine (1.47 g, 4.09 mmol) were added sequentially. The mixture was subjected to nitrogen purging for three times, and then the reaction was carried out at 80°C for 18 hours. The reaction solution was filtered and concentrated under reduced pressure. The residue was purified by silica gel column chromatography (eluent: system A) to obtain methyl 4-(2-amino-6-methoxyphenyl)-5-(2-methoxy-2-oxoethyl)thiophene-2-carboxylate **15b** (2.80 g), with a yield of 81%.

MS m/z (ESI): 336.0 [M+1].

### Second step

### methyl 10-methoxy-5-oxo-5,6-dihydro-4H-benzo[b]thieno[3,2-d]azepine-2-carboxylate

At room temperature, methyl 4-(2-amino-6-methoxyphenyl)-5-(2-methoxy-2-oxoethyl)thiophene-2-carboxylate **15b** (2.80 g, 8.35 mmol) was dissolved in toluene (15 mL), and N,N-diisopropylethylamine (6.47 g, 50.09 mmol) was added dropwise. The mixture was subjected to nitrogen purging for three times, and heated to 100°C and allowed to react for 18 hours. The reaction solution was concentrated under reduced pressure. The residue was purified by silica gel column chromatography (eluent: system A) to obtain methyl 10-methoxy-5-oxo-5,6-dihydro-4H-benzo[b]thieno[3,2-d]azepine-2-carboxylate 15c (1.30 g), with a yield of 51%.

MS m/z (ESI): 304.0 [M+1].

### Third step

### methyl 10-methoxy-6-methyl-5-oxo-5,6-dihydro-4H-benzo[b]thieno[3,2-d]azepine-2-carboxylate

At room temperature, methyl 10-methoxy-5-oxo-5,6-dihydro-4H-benzo[b]thieno[3,2-d]azepine-2-carboxylate **15c** (280.00 mg, 0.92 mmol) was dissolved in a tetrahydrofuran (3 mL) solution, and then cesium carbonate (902.27 mg, 2.77 mmol) and iodomethane (262.04 mg, 1.85 mmol) were added sequentially. The mixture was subjected to nitrogen purging for three times, and then the reaction was carried out at 25°C for 3 hours. The reaction solution was filtered and concentrated under reduced pressure. The residue was purified by silica gel column chromatography (eluent: system A) to obtain methyl
10-methoxy-6-methyl-5-oxo-5,6-dihydro-4H-benzo[b]thieno[3,2-d]azepine-2-carboxylate **15d** (220 mg), with a yield of 75%.

MS m/z (ESI): 318.0 [M+1].

### Fourth step

### methyl 10-methoxy-6-methyl-5,6-dihydro-4H-benzo[b]thieno[3,2-d]azepine-2-carboxylate

At room temperature, methyl 10-methoxy-6-methyl-5-oxo-5,6-dihydro-4H-benzo[b]thieno[3,2-d]azepine-2-carboxylate **15d** (200.00 mg, 0.63 mmol) was dissolved in a tetrahydrofuran (2 mL) solution, borane-tetrahydrofuran (1 M, 1.89 mmol, 162.48 mg) was added dropwise, and the reaction was carried out at 25°C for 1 hour. The reaction solution was poured into methanol (50 mL), stirred at 50°C for 0.5 hour, and then concentrated under reduced pressure. The residue was purified by silica gel column chromatography (eluent: system A) to obtain methyl 10-methoxy-6-methyl-5,6-dihydro-4H-benzo[b]thieno[3,2-d]azepine-2-carboxylate **15e** (125 mg), with a yield of 65%.

MS m/z (ESI): 304.0 [M+1].

### Fifth step

### N-(3-chloro-5-(methylsulfonamido)phenyl)-10-methoxy-6-methyl-5,6-dihydro-4H-benzo[b]thie no[3,2-d]azepine-2-carboxamide

At room temperature, methyl 10-methoxy-6-methyl-5,6-dihydro-4H-benzo[b]thieno[3,2-d]azepine-2-carboxylate **15e** (125.00 mg, 0.41 mmol) and *N*-(3-amino-5-chlorophenyl)methanesulfonamide **1g** (90.90 mg, 0.41 mmol) were dissolved in a tetrahydrofuran (1 mL) solution, and a solution of trimethylaluminum in n-hexane (1 M, 1.24 mmol, 89.10 mg) was added dropwise. The reaction was carried out at 60°C for 18 hours. The reaction solution was poured into methanol (50 mL) for quenching and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-10-methoxy-6-methyl-5,6-dihydro-4H-benzo[b]thie no[3,2-d]azepine-2-carboxamide **15** (106 mg), with a yield of 52%.

MS m/z (ESI): 492.0 [M+1].

¹H NMR (400 MHz, DMSO-*d₆*) δ 10.31 (s, 1H), 10.17 (s, 1H), 8.11 (s, 1H), 7.64 (dt, *J =* 15.2, 1.8 Hz, 2H), 7.29 (t, *J =* 8.2 Hz, 1H), 6.95 (t, *J =* 1.8 Hz, 1H), 6.81 (t, *J =* 7.8 Hz, 2H), 3.80 (s, 3H), 3.42 (t, *J =* 6.6 Hz, 2H), 3.06 (s, 3H), 2.83 (t, *J =* 6.6 Hz, 2H), 2.69 (s, 3H).

Compounds of Examples 16-18 were prepared according to the preparation method of Example 15 of the present disclosure, and specific structures and structural characterizations were as follows:

| Serial number and structure of example | MS m/z (ESI) | ¹H NMR |
|---|---|---|
| 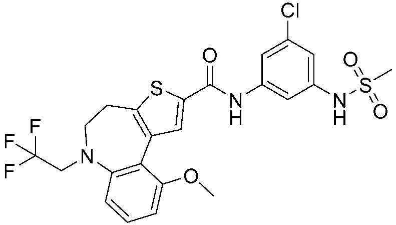 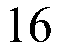 | 560.0[M+1] | ¹H NMR (400 MHz, DMSO-*d₆*) δ 10.36 (s, 1H), 10.09 (s, 1H), 8.17 (s, 1H), 7.70 - 7.59 (m, 2H), 7.30 (t, J = 8.2 Hz, 1H), 7.01 (d, J = 8.2 Hz, 1H), 6.97 - 6.88 (m, 2H), 3.95 (q, J = 9.6 Hz, 2H), 3.81 (s, 3H), 3.62 (t, J = 6.6 Hz, 2H), 3.06 (s, 3H), 2.86 (t, J = 6.6 Hz, 2H). |
| 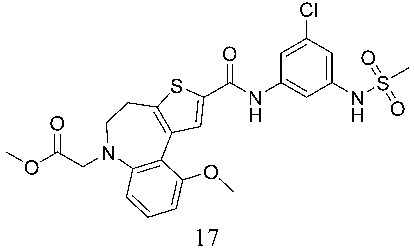 | 550.0[M+1] | ¹H NMR (400 MHz, DMSO-*d₆*) δ 10.34 (s, 1H), 10.04 (s, 1H), 8.14 (s, 1H), 7.67 - 7.61 (m, 2H), 7.22 (t, J = 8.2 Hz, 1H), 6.95 (t, J = 2.0 Hz, 1H), 6.81 (d, J = 8.2 Hz, 1H), 6.68 (d, J = 8.0 Hz, 1H), 3.96 (s, 2H), 3.80 (s, 3H), 3.61 (s, 3H), 3.58 (t, J = 6.4 Hz, 2H), 3.06 (s, 3H), 2.91 (t, J = 6.4 Hz, 2H). |
| 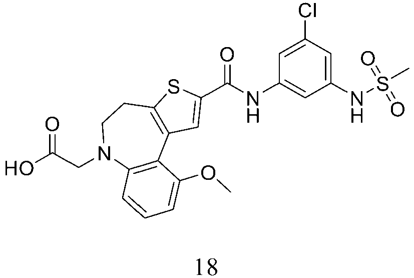 | 536.0[M+1] | ¹H NMR (400 MHz, DMSO-*d₆*) δ 12.47 (s, 1H), 10.33 (s, 1H), 10.06 (s, 1H), 8.13 (s, 1H), 7.64 (dt, J = 11.8, 1.8 Hz, 2H), 7.22 (t, J = 8.2 Hz, 1H), 6.95 (t, J = 2.0 Hz, 1H), 6.79 (d, J = 8.0 Hz, 1H), 6.70 (d, J = 8.0 Hz, 1H), 3.83 (s, 2H), 3.80 (s, 3H), 3.60 (t, J = 6.4 Hz, 2H), 3.06 (s, 3H), 2.91 (t, J = 6.4 Hz, 2H). |

### Example 19

### 10-(benzyloxy)-N-(3-chloro-5-(methylsulfonamido)phenyl)-6-methyl-5,6-dihydro-4H-benzo[b]t hieno[3,2-d]azepine-2-carboxamide

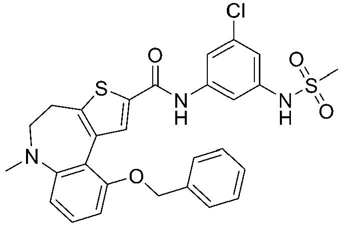

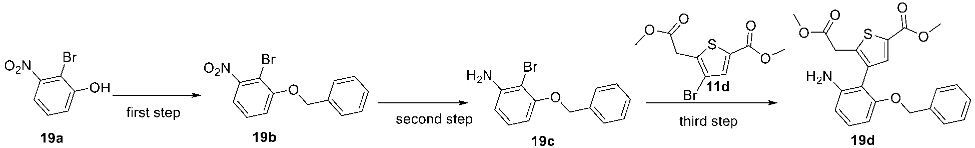

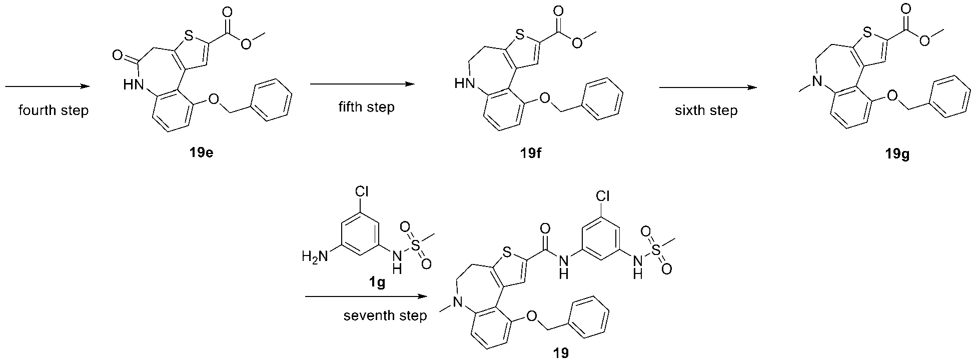

### First step

### 1-(benzyloxy)-2-bromo-3-nitrobenzene

2-bromo-3-nitrophenol **19a** (1 g, 4.59 mmol) was dissolved in tetrahydrofuran (10 mL), cesium carbonate (4.48 g, 13.76 mmol) and benzyl bromide (1.18 g, 6.88 mmol, 817.23 µL) were added to the resulting solution, and the reaction solution was stirred at room temperature for 12 hours. The resulting mixture was filtered under reduced pressure, the filter cake was washed with dichloromethane (5 mL × 3), and the resulting filtrates were concentrated under reduced pressure. The residue was purified by silica gel column chromatography (eluent: system A) to obtain 1-(benzyloxy)-2-bromo-3-nitrobenzene **19b** (1.26 g), with a yield of 89%.

MS m/z (ESI): 308.0 [M+1].

### Second step

### 3-(benzyloxy)-2-bromoaniline

The 1-(benzyloxy)-2-bromo-3-nitrobenzene **19b** (1.26 g, 4.09 mmol) was dissolved in ethanol (12 mL), and iron powder (685.15 mg, 12.27 mmol), ammonium chloride (656.20 mg, 12.27 mmol) and water (1.5 mL) were added to the resulting solution. The reaction solution was subjected to nitrogen purging for three times and stirred at 90°C under nitrogen protection for 2 hours. The resulting mixture was filtered under reduced pressure, the filter cake was washed with dichloromethane (5 mL × 3), and the resulting filtrates were concentrated under reduced pressure. The residue was purified by silica gel column chromatography (eluent: system A) to obtain 3-(benzyloxy)-2-bromoaniline **19c** (1.0 g), with a yield of 88%.

MS m/z (ESI): 278.0 [M+1].

### Third step

### methyl 4-(2-amino-6-(benzyloxy)phenyl)-5-(2-methoxy-2-oxoethyl)thiophene-2-carboxylate

3-(benzyloxy)-2-bromoaniline **19c** (1 g, 3.60 mmol) and methyl 4-bromo-5-(2-methoxy-2-oxoethyl)thiophene-2-carboxylate **11d** (702.60 mg, 2.40 mmol) were dissolved in 1,4-dioxane (20 mL), and bis(pinacolato)diboron (1.83 g, 7.19 mmol), di(1-adamantyl)-n-butylphosphine (343.75 mg, 958.74 µmol), potassium carbonate (993.77 mg, 7.19 mmol), palladium(II) acetate (107.62 mg, 479.37 µmol) and water (2 mL) were added to the resulting solution. The reaction solution was subjected to nitrogen purging for three times and stirred at 80°C under nitrogen protection for 1 hour. The resulting mixture was concentrated under reduced pressure and purified by silica gel column chromatography (eluent: system A) to obtain methyl 4-(2-amino-6-(benzyloxy)phenyl)-5-(2-methoxy-2-oxoethyl)thiophene-2-carboxylate **19d** (900 mg), with a yield of 45%.

MS m/z (ESI): 412.2 [M+1].

### Fourth step

### methyl 10-(benzyloxy)-5-oxo-5,6-dihydro-4H-benzo[b]thieno[3,2-d]azepine-2-carboxylate Methyl

4-(2-amino-6-(benzyloxy)phenyl)-5-(2-methoxy-2-oxoethyl)thiophene-2-carboxylate **19d** (800 mg, 972.12 µmol) was dissolved in tetrahydrofuran (8 mL), a solution of trimethylaluminum in n-hexane (1 M, 9.72 mL) was slowly added dropwise to the resulting solution, and the reaction solution was stirred at 60°C for 1 hour. The reaction solution was slowly added dropwise to methanol (20 mL). The resulting mixture was concentrated under reduced pressure. The residue was purified by silica gel column chromatography (eluent: system A) to obtain methyl 10-(benzyloxy)-5-oxo-5,6-dihydro-4H-benzo[b]thieno[3,2-d]azepine-2-carboxylate **19e** (200 mg), with a yield of 54%.

MS m/z (ESI): 380.0 [M+1].

### Fifth step

### methyl 10-(benzyloxy)-5,6-dihydro-4H-benzo[b]thieno[3,2-d]azepine-2-carboxylate

Methyl 10-(benzyloxy)-5-oxo-5,6-dihydro-4H-benzo[b]thieno[3,2-d]azepine-2-carboxylate **19e** (150 mg, 395.33 µmol) was added slowly to a borane-tetrahydrofuran complex (1 M, 4 mL). The reaction solution was stirred at 60°C for 1 hour, and then the reaction solution was slowly added dropwise to methanol (20 mL) and stirred at 60°C for 1 hour. The resulting mixture was concentrated under reduced pressure. The residue was purified by silica gel column chromatography (eluent: system A) to obtain methyl 10-(benzyloxy)-5,6-dihydro-4H-benzo[b]thieno[3,2-d]azepine-2-carboxylate **19f** (100 mg), with a yield of 69%.

MS m/z (ESI): 336.2 [M+1].

### Sixth step

### methyl 10-(benzyloxy)-6-methyl-5,6-dihydro-4H-benzo[b]thieno[3,2-d]azepine-2-carboxylate

Methyl 10-(benzyloxy)-5,6-dihydro-4H-benzo[b]thieno[3,2-d]azepine-2-carboxylate **19f** (100 mg, 273.64 µmol) was dissolved in 1,2-dichloroethane (4.00 mL), sodium triacetoxyborohydride (347.97 mg, 1.64 mmol), acetic acid (1.64 mg, 27.36 µmol, 1.57 µL) and an aqueous formaldehyde solution (133.24 mg, 1.64 mmol, 122.24 µL, purity 37%) were added to the resulting solution, and the reaction solution was stirred at room temperature for 12 hours. The resulting mixture was concentrated under reduced pressure. The residue was purified by silica gel column chromatography (eluent: system A) to obtain methyl 10-(benzyloxy)-6-methyl-5,6-dihydro-4H-benzo[b]thieno[3,2-d]azepine-2-carboxylate **19g** (90 mg), with a yield of 86%.

MS m/z (ESI): 380.2 [M+1].

### Seventh step

### 10-(benzyloxy)-N-(3-chloro-5-(methylsulfonamido)phenyl)-6-methyl-5,6-dihydro-4H-benzo[b]t hieno[3,2-d]azepine-2-carboxamide

Methyl 10-(benzyloxy)-6-methyl-5,6-dihydro-4H-benzo[b]thieno[3,2-d]azepine-2-carboxylate **19g** (20 mg, 52.70 µmol) and N-(3-amino-5-chlorophenyl)methanesulfonamide **1g** (17.45 mg, 79.06 µmol) were dissolved in tetrahydrofuran (1 mL), and a solution of trimethylaluminum in n-hexane (1 M, 527.05 µL) was slowly added dropwise to the resulting mixture. The reaction solution was stirred at 60°C for 1 hour, and then the reaction solution was slowly added dropwise to methanol (5 mL) to quench the reaction. The resulting mixture was concentrated under reduced pressure. The residue was purified by preparative liquid chromatography (separation column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain 10-(benzyloxy)-N-(3-chloro-5-(methylsulfonamido)phenyl)-6-methyl-5,6-dihydro-4H-benzo[b]t hieno[3,2-d]azepine-2-carboxamide **19** (11 mg), with a yield of 36%.

MS m/z (ESI): 568.2 [M+1].

¹H NMR (400 MHz, DMSO-*d₆*) δ 10.28 (s, 1H), 10.14 (s, 1H), 8.26 (s, 1H), 7.63 (dt, *J* = 4.4, 2.0 Hz, 2H), 7.44 (d, *J =* 6.4 Hz, 2H), 7.30 - 7.22 (m, 4H), 6.95 (t, *J =* 2.0 Hz, 1H), 6.90 (d, *J =* 8.0 Hz, 1H), 6.81 (d, *J =* 8.0 Hz, 1H), 5.14 (s, 2H), 3.42 (t, *J =* 6.4 Hz, 2H), 3.07 (s, 3H), 2.84 (t, *J=* 6.4 Hz, 2H), 2.71 (s, 3H).

Compounds of Examples 20-26 were prepared according to the preparation method of Example 19 of the present disclosure, and specific structures and structural characterizations were as follows:

| Serial number and structure of example | MS m/z (ESI) | ¹H NMR |
|---|---|---|
| 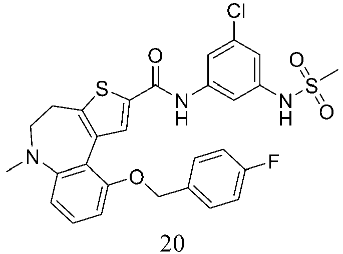 | 586.2[M+1] | ¹H NMR (400 MHz, DMSO-*d₆*) δ 10.26 (s, 1H), 10.10 (s, 1H), 8.22 (s, 1H), 7.64 (dt, J = 13.4, 1.8 Hz, 2H), 7.51-7.44 (m, 2H), 7.28 (t, J = 8.2 Hz, 1H), 7.10 - 7.03 (m, 2H), 6.96 (t, J = 1.8 Hz, 1H), 6.89 (d, J = 8.2 Hz, 1H), 6.81 (d, J = 8.2 Hz, 1H), 5.12 (s, 2H), 3.43 (t, J = 6.6 Hz, 2H), 3.07 (s, 3H), 2.83 (t, J = 6.6 Hz, 2H), 2.71 (s, 3H). |
| 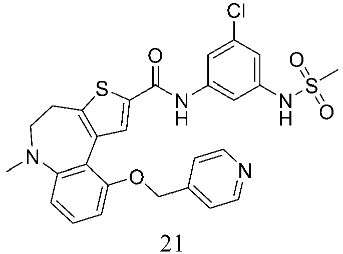 | 569.2[M+1] | ¹H NMR (400 MHz, DMSO-*d₆*) δ 10.30 (s, 1H), 10.05 (S, 1H), 8.45 (dd, J = 4.4, 1.6 Hz, 2H), 8.28 (s, 1H), 7.65 (t, J = 1.6 Hz, 1H), 7.61 (t, J = 1.6 Hz, 1H), 7.40 (d, J = 6.0 Hz, 2H), 7.29 (t, J = 8.0 Hz, 1H), 6.96 (t, J = 2.0 Hz, 1H), 6.85 (t, J = 8.0 Hz, 2H), 5.22 (s, 2H), 3.45 (t, J = 6.4 Hz, 2H), 3.07 (s, 3H), 2.86 (t, J = 6.4 Hz, 2H), 2.72 (s, 3H). |
| 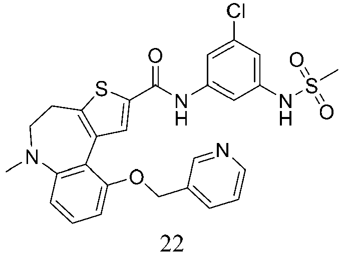 | 569.2[M+1] | ¹H NMR (400 MHz, DMSO-*d₆*) δ 10.25 (s, 1H), 10.09 (s, 1H), 8.62 (d, J = 1.8 Hz, 1H), 8.44 (dd, J = 4.8, 1.5 Hz, 1H), 8.24 (s, 1H), 7.83 (dt, J = 7.8, 1.8 Hz, 1H), 7.63 (dd, J = 3.6, 1.8 Hz, 2H), 7.32 - 7.25 (m, 2H), 6.98 - 6.90 (m, 2H), 6.83 (d, J = 8.2 Hz, 1H), 5.19 (s, 2H), 3.43 (t, J = 6.4 Hz, 2H), 3.08 (s, 3H), 2.83 (t, J = 6.4 Hz, 2H), 2.71 (s, 3H). |
| 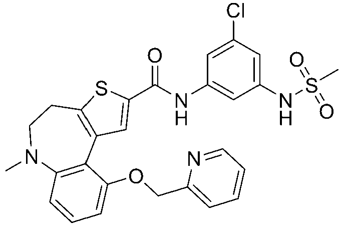 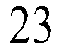 | 569.2[M+1] | ¹H NMR (400 MHz, DMSO-*d₆*) δ 10.28 (s, 1H), 10.06 (s, 1H), 8.54-8.52 (m, 1H), 8.28 (s, 1H), 7.69 (td, J = 7.6, 2.0 Hz, 1H), 7.64 (t, J = 2.0 Hz, 1H), 7.60 (t, J = 2.0 Hz, 1H), 7.48 (d, J = 7.6 Hz, 1H), 7.33 - 7.24 (m, 2H), 6.95 (t, J = 2.0 Hz, 1H), 6.89 (d, J = 8.4 Hz, 1H), 6.83 (d, J = 8.0 Hz, 1H), 5.22 (s, 2H), 3.46 (t, J = 6.4 Hz, 2H), 3.07 (s, 3H), 2.87 (t, J = 6.4 Hz, 2H), 2.72 (s, 3H). |
| 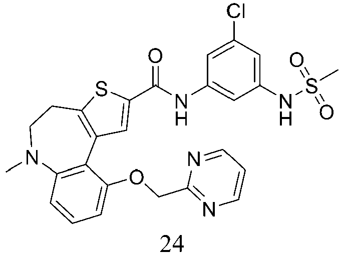 | 570.3[M+1] | ¹H NMR (400 MHz, DMSO-*d₆*) δ 10.28 (s, 2H), 8.77 (d, J = 4.8 Hz, 2H), 8.28 (s, 1H), 7.61 (t, J = 2.0 Hz, 1H), 7.55 (s, 1H), 7.40 (t, J = 4.8 Hz, 1H), 7.21 (t, J = 8.4 Hz, 1H), 6.92 (t, J = 2.0 Hz, 1H), 6.80 -6.76 (m, 2H), 5.31 (s, 2H), 3.44 (t, J = 6.4 Hz, 2H), 3.04 (s, 3H), 2.85 (t, J = 6.4 Hz, 2H), 2.70 (s, 3H). |
| 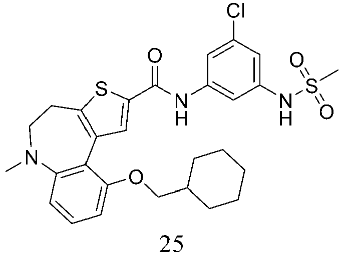 | 574.2[M+1] | ¹H NMR (400 MHz, DMSO-*d₆*) δ 10.28 (s, 1H), 8.17 (s, 1H), 7.62 (d, J = 1.6 Hz, 2H), 7.25 (t, J = 8.0 Hz, 1H), 6.93 (t, J = 1.6 Hz, 1H), 6.79 (s, 1H), 6.77 (s, 1H), 3.79 (d, J = 5.6 Hz, 2H), 3.43 (t, J = 6.4 Hz, 2H), 3.05 (s, 3H), 2.85 (t, J = 6.4 Hz, 2H), 2.71 (s, 3H), 1.72 (d, J = 10.0 Hz, 2H), 1.55 (dd, J = 25.6, 12.0 Hz, 3H), 1.27 - 0.97 (m, 6H). |
| 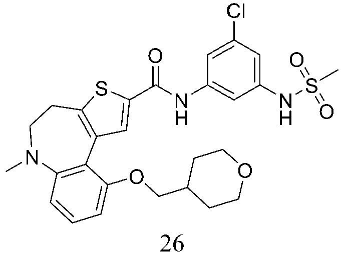 | 576.2[M+1] | ¹H NMR (400 MHz, DMSO-*d₆*) δ 10.27 (s, 1H), 8.17 (s, 1H), 7.64 (t, J = 1.6 Hz, 1H), 7.61 (t, J *=* 1.6 Hz, 1H), 7.26 (t, J = 8.0 Hz, 1H), 6.94 (t, J = 2.0 Hz, 1H), 6.79 (dd, J = 8.0, 5.6 Hz, 2H), 3.85 (d, J = 6.4 Hz, 2H), 3.77 (d, J = 4.4 Hz, 2H), 3.74 (d, J = 3.2 Hz, 2H), 3.22 (t, J = 11.6 Hz, 2H), 3.05 (s, 3H), 2.86 (t, J = 6.4 Hz, 2H), 2.71 (s, 3H), 1.99 (s, 1H), 1.61 (d, J = 12.4 Hz, 2H), 1.35 - 1.27 (m, 2H). |

### Example 27

### N-(3-chloro-5-(methylsulfonamido)phenyl)-6-oxo-6,7-dihydro-5H-pyrido[2,3-f]pyrrolo[1,2-d][1 ,4]diazepine-10-carboxamide

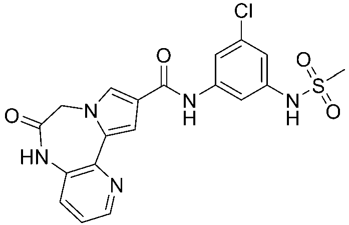

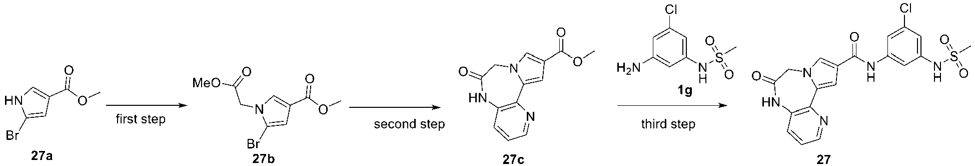

### First step

### methyl 5-bromo-1-(2-methoxy-2-oxoethyl)-1H-pyrrole-3-carboxylate

Methyl 5-bromo-1H-pyrrole-3-carboxylate **27a** (1.5 g, 7.35 mmol) and methyl bromoacetate (1.69 g, 11.03 mmol) were dissolved in acetonitrile (30 mL), then potassium carbonate (3.05 g, 22.06 mmol) was added, and the mixture was allowed to react at room temperature for 12 hours. The reaction solution was filtered through diatomaceous earth and concentrated under reduced pressure. The residue was purified by silica gel column chromatography (eluent: system A) to obtain methyl 5-bromo-1-(2-methoxy-2-oxoethyl)-1H-pyrrole-3-carboxylate **27b** (1.8 g), with a yield of 88%.

MS m/z (ESI): 276.0 [M+1].

### Second step

### methyl 6-oxo-6,7-dihydro-5H-pyrido[2,3-f]pyrrolo[1,2-d][1,4]diazepine-10-carboxylate

Methyl 5-bromo-1-(2-methoxy-2-oxoethyl)-1H-pyrrole-3-carboxylate **27b** (2 g, 2.09 mmol), 2-bromo-3-aminopyridine (3.76 g, 21.73 mmol), bis(pinacolato)diboron (5.52 g, 21.73 mmol), di(1-adamantyl)-butylphosphine (1.04 g, 2.90 mmol) and potassium carbonate (5.01 g, 36.22 mmol) were dissolved in 1,2-dimethoxyethane (20 mL) and water (4 mL). The reaction solution was subjected to nitrogen purging for three times. Under nitrogen protection, palladium(II) acetate (325.28 mg, 1.45 mmol) was added, and the reaction was carried out at 80°C for 12 hours. The reaction solution was diluted with water and extracted with ethyl acetate (100 mL × 3). The extracts were combined, washed with saturated sodium chloride solution, dried with anhydrous sodium sulfate, and concentrated under reduced pressure. The residue was purified by silica gel column chromatography (eluent: system A) to obtain methyl 6-oxo-6,7-dihydro-5H-pyrido[2,3-f]pyrrolo[1,2-d][1,4]diazepine-10-carboxylate **27c** (600 mg), with a yield of 32%.

MS m/z (ESI): 258.2 [M+1].

### Third step

### N-(3-chloro-5-(methylsulfonamido)phenyl)-6-oxo-6,7-dihydro-5H-pyrido[2,3-f]pyrrolo[1,2-d][1 ,4]diazepine-10-carboxamide

N-(3-amino-5-chlorophenyl)methanesulfonamide **1g** (171.57 mg, 777.47 µmol) was dissolved in tetrahydrofuran (1 mL) and toluene (1 mL), a solution of trimethylaluminum in n-hexane (2 M, 583.10 µL) was added dropwise at 0°C, and then the mixture was stirred at 25°C for 0.5 hours. Methyl 6-oxo-6,7-dihydro-5H-pyrido[2,3-f]pyrrolo[1,2-d][1,4]diazepine-10-carboxylate **27c** (100 mg, 388.74 µmol) was added to the reaction solution, and then the mixture was allowed to react at 60°C for 1 hour. After the reaction was complete, the reaction solution was quenched with methanol and evaporated to dryness. The residue was purified by silica gel column chromatography (eluent: system A), and further separated by preparative liquid chromatography (column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-6-oxo-6,7-dihydro-5H-pyrido[2,3-f]pyrrolo[1,2-d][1 ,4]diazepine-10-carboxamide **27** (7 mg), with a yield of 19 %.

MS m/z (ESI): 446.0 [M+1].

¹H NMR (400 MHz, DMSO-*d₆*) δ 10.50 (s, 1H), 10.03 (s, 1H), 9.98 (s, 1H), 8.46 (d, J = 4.4 Hz, 1H), 7.80 (s, 1H), 7.75 (s, 1H), 7.67 (s, 1H), 7.56 (d, J = 8.0 Hz, 1H), 7.45 - 7.35 (m, 2H), 6.90 (s, 1H), 4.81 (s, 2H), 3.07 (s, 3H).

### Example 28

### N-(3-chloro-5-(methylsulfonamido)phenyl)-5-methyl-6-oxo-6,7-dihydro-5H-pyrido[2,3-f]p yrrolo[1,2-d][1,4]diazepine-10-carboxamide

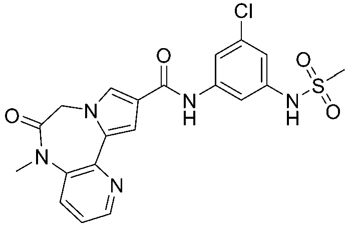

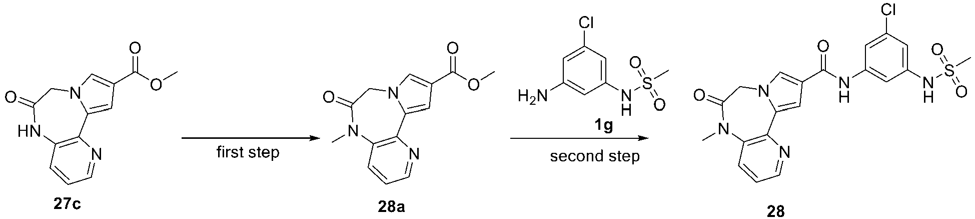

### First step

### methyl 5-methyl-6-oxo-6,7-dihydro-5H-pyrido[2,3-f]pyrrolo[1,2-d][1,4]diazepine-10-carboxylate

Methyl 6-oxo-6,7-dihydro-5H-pyrido[2,3-f]pyrrolo[1,2-d][1,4]diazepine-10-carboxylate **27c** (300 mg, 1.17 mmol) and potassium carbonate (322.35 mg, 2.33 mmol) were dissolved in *N,N*-dimethylformamide (5 mL), iodomethane (248.29 mg, 1.75 mmol, 108.90 µL) was added, and then the mixture was allowed to react at room temperature for 3 hours. The reaction solution was diluted with water and extracted with ethyl acetate (50 mL × 3). The extracts were combined, washed with a saturated sodium chloride solution, dried with anhydrous sodium sulfate, and concentrated under reduced pressure. The residue was purified by silica gel column chromatography (eluent: system A) to obtain methyl 5-methyl-6-oxo-6,7-dihydro-5H-pyrido[2,3-f]pyrrolo[1,2-d][1,4]diazepine-10-carboxylate **28a** (300 mg), with a yield of 95%.

MS m/z (ESI): 272.1 [M+1].

### Second step

### N-(3-chloro-5-(methylsulfonamido)phenyl)-5-methyl-6-oxo-6,7-dihydro-5H-pyrido[2,3-f]p yrrolo[1,2-d][1,4]diazepine-10-carboxamide

Methyl 5-methyl-6-oxo-6,7-dihydro-5H-pyrido[2,3-f]pyrrolo[1,2-d][1,4]diazepine-10-carboxylate **28a** (250 mg, 921.59 µmol) and *N*-(3-amino-5-chlorophenyl)methanesulfonamide **1g** (406.75 mg, 1.84 mmol) were dissolved in tetrahydrofuran (2 mL) and toluene (2 mL), a solution of trimethylaluminum in n-hexane (2 M, 2.76 mL) was added dropwise at 0°C, and then the mixture was allowed to react at 60°C for 3 hours. After the reaction was complete, the reaction solution was quenched with methanol and evaporated to dryness. The residue was purified by silica gel column chromatography (eluent: system A), and further separated by preparative liquid chromatography (column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-5-methyl-6-oxo-6,7-dihydro-5H-pyrido[2,3-f]pyrrol o[1,2-d][1,4]diazepine-10-carboxamide **28** (92 mg), with a yield of 22%.

MS m/z (ESI): 460.0 [M+1].

¹H NMR (400 MHz, DMSO-*d₆*) δ 9.97 (s, 2H), 8.54 (dd, J = 4.8, 1.2 Hz, 1H), 7.97 (dd, J = 8.0, 1.2 Hz, 1H), 7.84 (d, J = 1.6 Hz, 1H), 7.73 (t, J = 1.6 Hz, 1H), 7.65 (s, 1H), 7.50 (dd, J = 8.4, 4.8 Hz, 1H), 7.38 (d, J = 2.0 Hz, 1H), 6.90 (t, J = 2.0 Hz, 1H), 4.78 (s, 2H), 3.28 (s, 3H), 3.05 (s, 3H).

### Example 29

### N-(3-chloro-5-(methylsulfonamido)phenyl)-6,7-dihydro-5H-pyrido[2,3-f]pyrrolo[1,2-d][1,4 ]diazepine-10-carboxamide

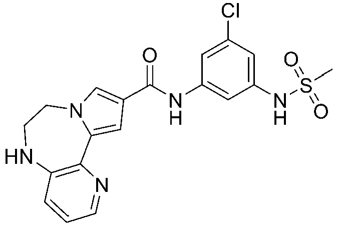

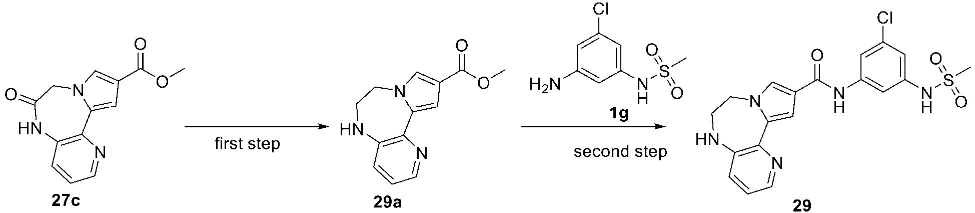

### First step

### methyl 6,7-dihydro-5H-pyrido[2,3-f]pyrrolo[1,2-d][1,4]diazepine-10-carboxylate

Methyl 6-oxo-6,7-dihydro-5H-pyrido[2,3-f]pyrrolo[1,2-d][1,4]diazepine-10-carboxylate **27c** (200 mg, 777.47 µmol) was dissolved in tetrahydrofuran (5 mL), borane dimethyl sulfide (2 M, 9.72 mL) was added dropwise at 0°C, and then the mixture was allowed to react at room temperature for 1 hour. The reaction solution was quenched with methanol and concentrated under reduced pressure. The residue was purified by silica gel column chromatography (eluent: system A) to obtain methyl 6,7-dihydro-5H-pyrido[2,3-f]pyrrolo[1,2-d][1,4]diazepine-10-carboxylate **29a** (110 mg), with a yield of 58%.

MS m/z (ESI): 244.0 [M+1].

### Second step

### N-(3-chloro-5-(methylsulfonamido)phenyl)-6,7-dihydro-5H-pyrido[2,3-f]pyrrolo[1,2-d][1,4 ]diazepine-10-carboxamide

Methyl 6,7-dihydro-5H-pyrido[2,3-f]pyrrolo[1,2-d][1,4]diazepine-10-carboxylate **29a** (90 mg, 369.97 µmol) and N-(3-amino-5-chlorophenyl)methanesulfonamide **1g** (163.29 mg, 739.95 µmol) were dissolved in tetrahydrofuran (2 mL) and toluene (2 mL), trimethylaluminum (2 M, 554.96 µL) was added dropwise at 0°C, and then the mixture was allowed to react at 60°C for 3 hours. After the reaction was complete, the reaction solution was quenched with methanol and evaporated to dryness. The residue was purified by silica gel column chromatography (eluent: system A), and further separated by preparative liquid chromatography (column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-6,7-dihydro-5H-pyrido[2,3-f]pyrrolo[1,2-d][1,4]diaz epine-10-carboxamide **29** (50 mg), with a yield of 31%.

MS m/z (ESI): 432.1 [M+1].

¹H NMR (400 MHz, DMSO-*d₆*) δ 9.99 (s, 1H), 9.89 (s, 1H), 7.94 (dd, J = 4.0, 1.2 Hz, 1H), 7.76 (t, J = 2.0 Hz, 1H), 7.70 (t, J = 1.6 Hz, 1H), 7.56 (d, J = 2.0 Hz, 1H), 7.52 (d, J = 2.0 Hz, 1H), 7.11 (dd, J = 8.4, 1.6 Hz, 1H), 7.00 (dd, J = 8.4, 4.4 Hz, 1H), 6.89 (t, J = 1.6 Hz, 1H), 6.51 (t, J = 4.0 Hz, 1H), 4.42 - 4.21 (m, 2H), 3.46 (dd, J = 7.2, 3.6 Hz, 2H), 3.07 (s, 3H).

Compounds of Examples 30-34 were prepared according to the preparation method of Example 29 of the present disclosure, and specific structures and structural characterizations were as follows:

| Serial number and structure of example | MS m/z (ESI) | ¹H NMR |
|---|---|---|
| 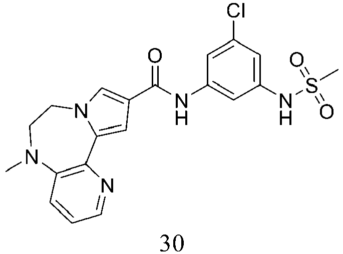 | 446.2[M+1] | ¹H NMR (400 MHz, DMSO-*d₆*) δ 9.99 (s, 1H), 9.91 (s, 1H), 8.09 (dd, *J* = 4.4, 1.2 Hz, 1H), 7.75 (t, *J* = 2.0 Hz, 1H), 7.69 (t, J = 1.6 Hz, 1H), 7.66 (d, *J* = 2.4 Hz, 1H), 7.44 (d, *J* = 2.0 Hz, 1H), 7.31 (dd, *J* = 8.4, 1.2 Hz, 1H), 7.17 (dd, *J* = 8.4, 4.4 Hz, 1H), 6.89 (t, *J* = 2.0 Hz, 1H), 4.39 - 4.22 (m, 2H), 3.60 - 3.45 (m, 2H), 3.06 (s, 3H), 2.95 (s, 3H). |
| 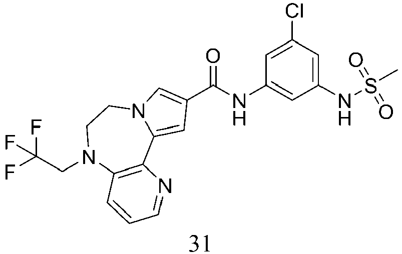 | 514.2 [M+1] | ¹H NMR (400 MHz, DMSO-*d₆*) δ 10.08 (s, 1H), 9.93 (s, 1H), 8.17 (dd, J = 4.3, 1.2 Hz, 1H), 7.75 (t, J = 1.8 Hz, 1H), 7.70 - 7.65 (m, 2H), 7.60-7.55 (m, 2H), 7.17 (dd, J = 8.4, 4.3 Hz, 1H), 6.89 (t, J = 1.8 Hz, 1H), 4.40 - 4.29 (m, 4H), 3.08-3.64 (m, 2H), 3.06 (s, 3H). |
| 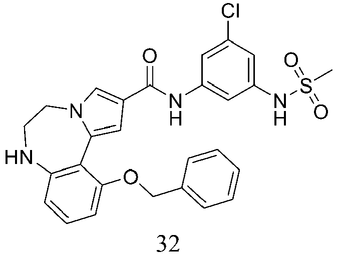 | 537.2 [M+1] | ¹H NMR (400 MHz, DMSO-*d₆*) δ 9.74 (s, 1H), 7.71 (t, *J* = 1.6 Hz, 1H), 7.63 (t, *J* = 1.6 Hz, 1H), 7.61 (d,*J* = 2.0 Hz, 1H), 7.45 (d, *J* = 6.8 Hz, 2H), 7.31 (dt, *J* = 18.4, 7.2 Hz, 3H), 7.01 - 6.96 (m, 2H), 6.89 (t, *J* = 2.0 Hz, 1H), 6.50 (dd, *J* = 14.4, 8.0 Hz, 2H), 5.67 (s, 1H), 5.10 (s, 2H), 4.14 (t, *J* = 5.2 Hz, 2H), 3.53 (s, 2H), 3.06 (s, 3H). |
| 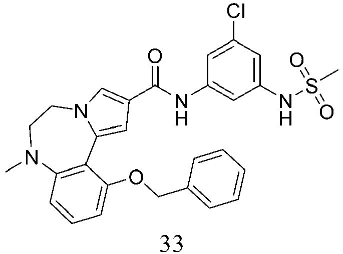 | 551.2 [M+1] | ¹H NMR (400 MHz, DMSO-*d₆*) δ 10.00 (s, 1H), 9.77 (s, 1H), 7.72 (t, *J* = 1.6 Hz, 1H), 7.69 (d, *J* = 1.6 Hz, 1H), 7.63 (t, *J* = 2.0 Hz, 1H), 7.42 (d, *J* = 7.2 Hz, 2H), 7.36 - 7.23 (m, 4H), 6.90 - 6.84 (m, 3H), 6.75 (d, *J* = 8.0 Hz, 1H), 5.14 (s, 2H), 4.01 (t, *J* = 6.0 Hz, 2H), 3.35 (t, *J* = 5.6 Hz, 2H), 3.06 (s, 3H), 2.70 (s, 3H). |

### Example 34

### N-(3-chloro-5-(methylsulfonamido)phenyl)-11-methoxy-7-methyl-6,7-dihydro-5H-benzo[f]pyrr olo[1,2-d][1,4]diazepine-2-carboxamide

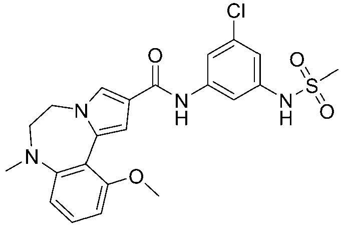

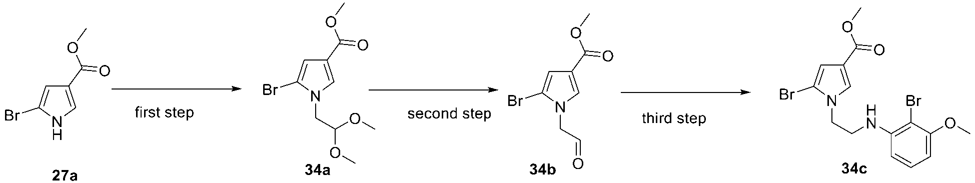

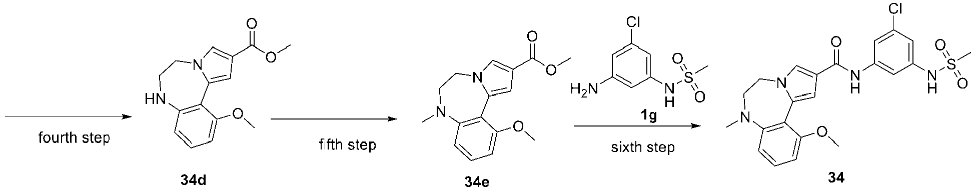

### First step

### methyl 5-bromo-1-(2,2-dimethoxyethyl)-1H-pyrrole-3-carboxylate

Methyl 5-bromo-1H-pyrrole-3-carboxylate **27a** (5.00 g, 24.5 mmol), 2-bromo-1,1-dimethoxyethane (8.28 g, 49.0 mmol) and cesium carbonate (24.0 g, 73.5 mmol) were added to acetonitrile (50 mL) at 25°C, and the mixture was allowed to react at 80°C for 18 hours. The reaction solution was filtered, and the filtrate was concentrated under reduced pressure. The residue was purified by silica gel column chromatography (eluent: system A) to obtain methyl 5-bromo-1-(2,2-dimethoxyethyl)-1H-pyrrole-3-carboxylate **34a** (5.0 g), with a yield of 70%.

MS m/z (ESI): 292.0/294 [M+1].

### Second step

### methyl 5-bromo-1-(2-oxoethyl)-1H-pyrrole-3-carboxylate

Methyl 5-bromo-1-(2,2-dimethoxyethyl)-1H-pyrrole-3-carboxylate **34a** (1.00 g, 3.42 mmol) was added to trifluoroacetic acid (10 mL) at 25°C, and the mixture was allowed to react at 25°C for 0.5 hours. The system was concentrated under reduced pressure to obtain methyl 5-bromo-1-(2-oxoethyl)-1H-pyrrole-3-carboxylate **34b** (800 mg), which was used directly in the next step.

MS m/z (ESI): 264.0/266 [M+1].

### Third step

### methyl 5-bromo-1-(2-((2-bromo-3-methoxyphenyl)amino)ethyl)-1H-pyrrole-3-carboxylate

Methyl 5-bromo-1-(2-oxoethyl)-1H-pyrrole-3-carboxylate **34b** (700 mg, 2.84 mmol) was added to 1,2-dichloroethane (10 mL) at 25°C, sodium acetoxyborohydride (1.81 g, 8.53 mmol), acetic acid (17.1 mg, 0.284 mmol) and 2-bromo-3-methoxyaniline (575 mg, 2.84 mmol) were added to the above reaction solution, and the mixture was allowed to react at 25°C for 18 hours. Methanol (20 mL) was added to the system, and then the mixture was concentrated under reduced pressure. The residue was purified by silica gel column chromatography (eluent: system A) to obtain methyl 5-bromo-1-(2-((2-bromo-3-methoxyphenyl)amino)ethyl)-1H-pyrrole-3-carboxylate **34c** (150 mg), with a yield of 12%.

MS m/z (ESI): 430.9/432.9/434.9 [M+1].

### Fourth step

### methyl 11-methoxy-6,7-dihydro-5H-benzo[f]pyrrolo[1,2-d][1,4]diazepine-2-carboxylate

Methyl 5-bromo-1-(2-((2-bromo-3-methoxyphenyl)amino)ethyl)-1H-pyrrole-3-carboxylate **34c** (140 mg, 0.324 mmol) was added to a mixed solution of 1,4-dioxane (5 mL) and water (1 mL) at 25°C, and palladium(II) acetate (14.5 mg, 0.0648 mmol), bis(pinacolato)diboron (247 mg, 0.972 mmol), potassium carbonate (134 mg, 0.972 mmol) and di(1-adamantyl)-n-butylphosphine (46.5 mg, 0.130 mmol) were added to the above reaction solution. The mixture was heated to 80°C under nitrogen protection and allowed to react for 18 hours. The system was filtered and then concentrated under reduced pressure. The residue was purified by silica gel column chromatography (eluent: system A) to obtain methyl 11-methoxy-6,7-dihydro-5H-benzo[f]pyrrolo[1,2-d][1,4]diazepine-2-carboxylate **34d** (80 mg), with a yield of 91%.

MS m/z (ESI): 273.1 [M+1].

### Fifth step

### methyl 11-methoxy-7-methyl-6,7-dihydro-5H-benzo[f]pyrrolo[1,2-d][1,4]diazepine-2-carboxylate

Methyl 11-methoxy-6,7-dihydro-5H-benzo[f]pyrrolo[1,2-d][1,4]diazepine-2-carboxylate **34d** (80 mg, 0.294 mmol) was dissolved in methanol (5 mL) at 25°C, and an aqueous formaldehyde solution (298 mg, 3.67 mmol, 0.27 mL, purity 37%) and palladium on carbon (40.0 mg, 10% Pd content, 55% w/w water content) were added to the above reaction solution. The mixture was allowed to react at 25°C for 2 hours under a hydrogen atmosphere. The system was filtered, and then the filtrate was concentrated under reduced pressure to obtain methyl 11-methoxy-7-methyl-6,7-dihydro-5H-benzo[f]pyrrolo[1,2-d][1,4]diazepine-2-carboxylate **34e** (80.0 mg), which was used directly in the next step.

MS m/z (ESI): 287.1 [M+1].

### Sixth step

### N-(3-chloro-5-(methylsulfonamido)phenyl)-11-methoxy-7-methyl-6,7-dihydro-5H-benzo[f]pyrr olo[1,2-d][1,4]diazepine-2-carboxamide

Methyl 11-methoxy-7-methyl-6,7-dihydro-5H-benzo[f]pyrrolo[1,2-d][1,4]diazepine-2-carboxylate **34e** (50.0 mg, 0.175 mmol) and *N*-(3-amino-5-chlorophenyl)methanesulfonamide **1g** (57.8 mg, 0.262 mmol) were added to tetrahydrofuran (10 mL) at 25°C, a solution of trimethylaluminum in n-hexane (1 M, 0.87 mL) was added to the above reaction solution, and the mixture was allowed to react at 60°C for 18 hours. The mixture was poured into methanol (50 mL) and then concentrated under reduced pressure. The residue was purified by silica gel column chromatography (eluent: system A), and further separated by preparative liquid chromatography (column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-11-methoxy-7-methyl-6,7-dihydro-5H-benzo[f]pyrr olo[1,2-d][1,4]diazepine-2-carboxamide **34** (16 mg), with a yield of 19 %.

MS m/z (ESI): 475.2 [M+1].

¹H NMR (400 MHz, DMSO-*d*₆) δ 10.06 (s, 1H), 9.75 (s, 1H), 7.71 (t, *J* = 2.0 Hz, 1H), 7.67 (d, *J* = 2.0 Hz, 1H), 7.62 (t, *J* = 2.0 Hz, 1H), 7.27 (t, *J* = 8.0 Hz, 1H), 6.88 (t, *J* = 2.0 Hz, 1H), 6.83 - 6.77 (m, 2H), 6.74 (d, *J* = 8.0 Hz, 1H), 4.01 (t, *J* = 6.0 Hz, 2H), 3.78 (s, 3H), 3.35 (d, *J* = 6.0 Hz, 2H), 3.05 (s, 3H), 2.70 (s, 3H).

### Example 35

### N-(3-chloro-5-(methylsulfonamido)phenyl)-11-methoxy-5,6-dihydrobenzo[f]pyrrolo[1,2-d][1,4] oxazepine-2-carboxamide

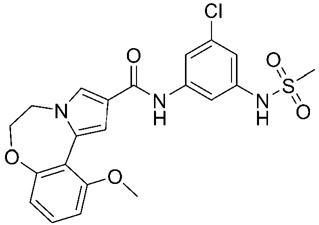

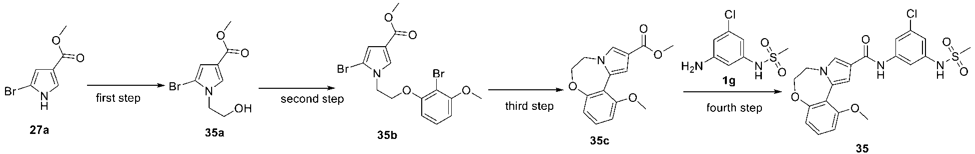

### First step

### methyl 5-bromo-1-(2-hydroxyethyl)-1H-pyrrole-3-carboxylate

Methyl 5-bromo-1H-pyrrole-3-carboxylate **27a** (1.00 g, 4.90 mmol), 2-bromoethanol (9.19 g, 73.5 mmol) and cesium carbonate (24.0 g, 73.5 mmol) were added to acetonitrile (50 mL) at 25°C, and the mixture was allowed to react at 80°C for 18 hours. The system was filtered, and then the filtrate was concentrated under reduced pressure. The residue was purified by silica gel column chromatography (eluent: system A) to obtain methyl 5-bromo-1-(2-hydroxyethyl)-1H-pyrrole-3-carboxylate **35a** (900 mg), with a yield of 74%.

MS m/z (ESI): 247.9/249.9 [M+1].

### Second step

### methyl 5-bromo-1-(2-(2-bromo-3-methoxyphenoxy)ethyl)-1H-pyrrole-3-carboxylate

Methyl 5-bromo-1-(2-hydroxyethyl)-1H-pyrrole-3-carboxylate **35a** (400 mg, 1.61 mmol), cyanomethylene tributyl phosphorane (973 mg, 4.03 mmol) and 2-bromo-3-methoxyphenol (491 mg, 2.42 mmol) were added to toluene (5 mL) at 25°C, and the mixture was allowed to react at 80°C for 2 hours. The system was concentrated under reduced pressure. The residue was purified by silica gel column chromatography (eluent: system A) to obtain methyl 5-bromo-1-(2-(2-bromo-3-methoxyphenoxy)ethyl)-1H-pyrrole-3-carboxylate **35b** (400 mg), with a yield of 57%.

MS m/z (ESI): 431.9/433.9/435.9 [M+1].

### Third step

### methyl 11-methoxy-5,6-dihydrobenzo[f]pyrrolo[1,2-d][1,4]oxazepine-2-carboxylate

Methyl 5-bromo-1-(2-(2-bromo-3-methoxyphenoxy)ethyl)-1H-pyrrole-3-carboxylate **35b** (400 mg, 0.927 mmol) was added to a mixed solution of 1,4-dioxane (10 mL) and water (2 mL) at 25°C, and palladium(II) acetate (62.2 mg, 0.277 mmol), bis(pinacolato)diboron (1.06 g, 4.16 mmol), potassium carbonate (574 mg, 4.16 mmol) and di(1-adamantyl)-n-butylphosphine (199 mg, 0.554 mmol) were added to the above reaction solution. The mixture was allowed to react at 80°C under nitrogen protection for 18 hours. The system was filtered and then concentrated under reduced pressure. The residue was purified by silica gel column chromatography (eluent: system A) to obtain methyl 11-methoxy-5,6-dihydrobenzo[f]pyrrolo[1,2-d][1,4]oxazepine-2-carboxylate **35c** (200 mg), with a yield of 79%.

MS m/z (ESI): 274.0 [M+1].

### Fourth step

### N-(3-chloro-5-(methylsulfonamido)phenyl)-11-methoxy-5,6-dihydrobenzo[f]pyrrolo[1,2-d][1,4] oxazepine-2-carboxamide

Methyl 11-methoxy-5,6-dihydrobenzo[f]pyrrolo[1,2-d][1,4]oxazepine-2-carboxylate **35c** (200 mg, 0.732 mmol) and N-(3-amino-5-chlorophenyl)methanesulfonamide **1g** (242 mg, 1.10 mmol) were added to tetrahydrofuran (10 mL) at 25°C, a solution of trimethylaluminum in n-hexane (2 M, 1.8 mL) was added to the above reaction solution, and the mixture was allowed to react at 60°C for 18 hours. The mixture was poured into methanol (50 mL) and then concentrated under reduced pressure. The residue was purified by silica gel column chromatography (eluent: system A), and further separated by preparative liquid chromatography (column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-11-methoxy-5,6-dihydrobenzo[f]pyrrolo[1,2-d][1,4] oxazepine-2-carboxamide **35** (60 mg), with a yield of 18%.

MS m/z (ESI): 462.0 [M+1].

¹H NMR (400 MHz, DMSO-*d*₆) δ 10.07 (s, 1H), 9.82 (s, 1H), 7.78 - 7.68 (m, 2H), 7.63 (t, *J* = 2.0 Hz, 1H), 7.28 (t, *J* = 8.0 Hz, 1H), 6.97 - 6.92 (m, 2H), 6.89 (t, *J* = 2.0 Hz, 1H), 6.78 (dd, *J* = 8.0, 1.2 Hz, 1H), 4.42 (t, *J* = 6.0 Hz, 2H), 4.15 (t, *J* = 6.0 Hz, 2H), 3.83 (s, 3H), 3.05 (s, 3H).

Compounds of Examples 36-37 were prepared according to the preparation method of Example 35 of the present disclosure, and specific structures and structural characterizations were as follows:

| Serial number and structure of example | MS m/z (ESI) | ¹H NMR |
|---|---|---|
| 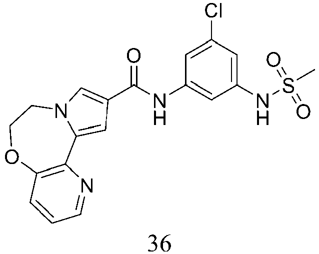 | 433.2[M+1] | ¹H NMR (400 MHz, DMSO-*d*₆) δ 9.96 (s, 1H), 8.30 (dd, *J* = 4.4, 1.6 Hz, 1H), 7.74 (t, *J* = 2.0 Hz, 1H), 7.71 - 7.68 (m, 2H), 7.67 (t, *J* = 2.0 Hz, 1H), 7.41 (dd, *J* = 8.0, 1.6 Hz, 1H), 7.21 (dd, *J* = 8.0, 4.4 Hz, 1H), 6.89 (t, *J* = 2.0 Hz, 1H), 4.57 - 4.51 (m, 2H), 4.50 - 4.42 (m, 2H), 3.05 (s, 3H). |
| 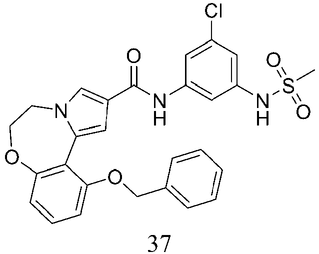 | 538.2 [M+1] | ¹H NMR (400 MHz, DMSO-*d₆*) δ 10.21 (s, 1H), 9.81 (s, 1H), 7.75 (d, J = 1.6 Hz, 1H), 7.69 (s, 1H), 7.59 (s, 1H), 7.46 (d, J = 7.2 Hz, 2H), 7.36 (t, J = 7.2 Hz, 2H), 7.31 - 7.23 (m, 2H), 7.04-6.98 (m, 2H), 6.88 (t, J = 1.6 Hz, 1H), 6.79 (d, J = 7.8 Hz, 1H), 5.20 (s, 2H), 4.43 (t, J = 5.6 Hz, 2H), 4.15 (t, J = 5.6 Hz, 2H), 3.04 (s, 3H). |

### Example 38

### N-(3-chloro-5-(methylsulfonamido)phenyl)-11-(phenoxymethyl)-5,6-dihydrobenzo[f]pyrrolo[1,2 -d][1,4]oxazepine-2-carboxamide

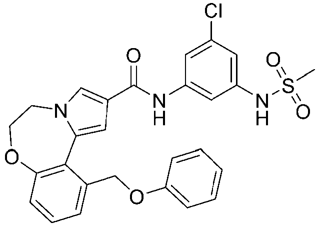

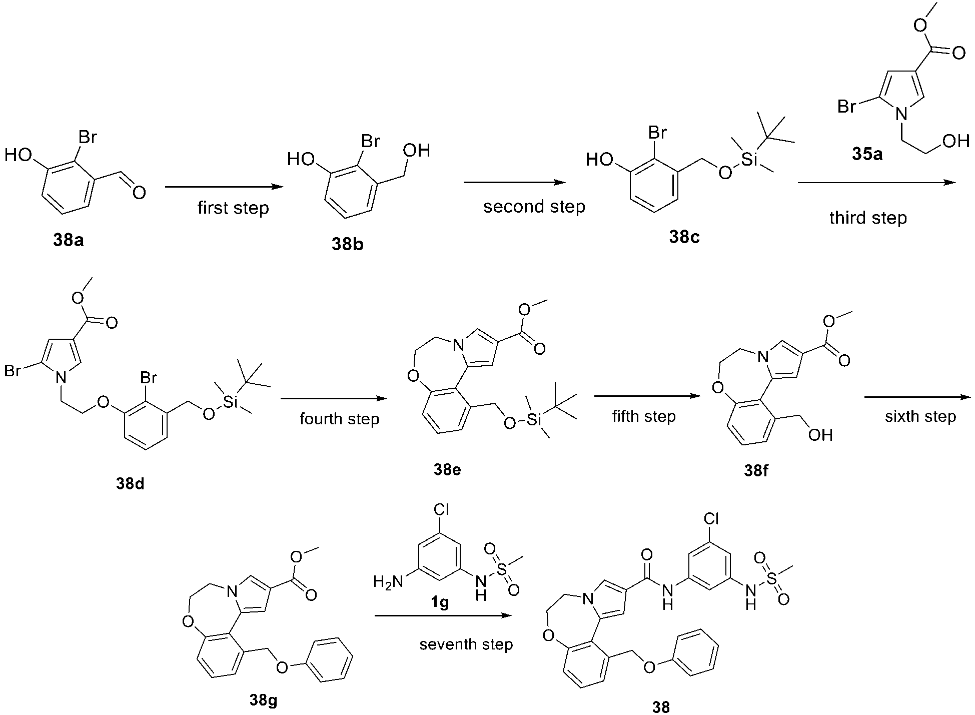

### First step

### 2-bromo-3-(hydroxymethyl)phenol

At room temperature, 2-bromo-3-hydroxybenzaldehyde **38a** (2.00 g, 9.95 mmol) was dissolved in methanol (30 mL), and sodium borohydride (1.13 g, 29.85 mmol) was added. The mixture was subjected to nitrogen purging for three times, and the reaction was carried out at 25°C for 18 hours. The reaction solution was poured into methanol (80 mL) for dilution and concentrated under reduced pressure. The residue was purified by silica gel column chromatography (eluent: system A) to obtain 2-bromo-3-(hydroxymethyl)phenol **38b** (1.78 g), with a yield of 88%.

MS m/z (ESI): 185.0/187.0 [M+1].

### Second step

### 2-bromo-3-(((tert-butyldimethylsilyl)oxy)methyl)phenol

At room temperature, 2-bromo-3-(hydroxymethyl)phenol **38b** (1.78 g, 8.77 mmol) was dissolved in dichloromethane (20 mL), and imidazole (1.19 g, 17.53 mmol) was added. Subsequently, a solution of tert-butyldimethylsilyl chloride (1.45 g, 9.64 mmol) in dichloromethane (2 mL) was added dropwise. The mixture was subjected to nitrogen purging for three times, and then the reaction was carried out at 25°C for 18 hours. The reaction solution was diluted with water (100 mL) and extracted with ethyl acetate (50 mL × 2). The combined organic phase was washed with saturated sodium chloride solution (50 mL), dried with anhydrous sodium sulfate, filtered, and concentrated under reduced pressure. The residue was purified by silica gel column chromatography (eluent: system A) to obtain 2-bromo-3-(((tert-butyldimethylsilyl)oxy)methyl)phenol **38c** (1.5 g), with a yield of 54%.

MS m/z (ESI): 317.0/319.0 [M+1].

### Third step

### methyl 5-bromo-1-(2-(2-bromo-3-(((tert-butyldimethylsilyl)oxy)methyl)phenoxy)ethyl)-1H-pyrrole-3-ca rboxylate

At room temperature, 2-bromo-3-(((tert-butyldimethylsilyl)oxy)methyl)phenol **38c** (959.27 mg, 3.02 mmol) and methyl 5-bromo-1-(2-hydroxyethyl)-1H-pyrrole-3-carboxylate **35a** (500.00 mg, 2.02 mmol) were dissolved in toluene (10 mL), and cyanomethylene tributyl phosphorane (1.46 g, 6.05 mmol, 1.58 mL) was added dropwise. The mixture was subjected to nitrogen purging for three times, and then the reaction was carried out at 80°C for 18 hours. The reaction solution was filtered and concentrated under reduced pressure. The residue was purified by silica gel column chromatography (eluent: system A) to obtain methyl 5-bromo-1-(2-(2-bromo-3-(((tert-butyldimethylsilyl)oxy)methyl)phenoxy)ethyl)-1H-pyrrole-3-ca rboxylate **38d** (580 mg), with a yield of 52%.

MS m/z (ESI): 568.0/570.0/572.0 [M+23].

### Fourth step

### methyl 11-(((tert-butyldimethylsilyl)oxy)methyl)-5,6-dihydrobenzo[f]pyrrolo[1,2-d][1,4]oxazepine-2-ca rboxylate

At room temperature, methyl 5-bromo-1-(2-(2-bromo-3-(((tert-butyldimethylsilyl)oxy)methyl)phenoxy)ethyl)-1H-pyrrole-3-ca rboxylate **38d** (580.00 mg, 1.06 mmol) was dissolved in a mixed solution of 1,2-dimethoxyethane (6 mL) and water (0.5 mL), and potassium carbonate (439.35 mg, 3.18 mmol), bis(pinacolato)diboron (807.25 mg, 3.18 mmol), palladium(II) acetate (47.58 mg, 0.21 mmol) and di(1-adamantyl)-n-butylphosphine (151.97 mg, 0.42 mmol) were added sequentially. The mixture was subjected to nitrogen purging for three times, and then the reaction was carried out at 80°C for 1 hour. The reaction solution was filtered and concentrated under reduced pressure. The residue was purified by silica gel column chromatography (eluent: system A) to obtain methyl 11-(((tert-butyldimethylsilyl)oxy)methyl)-5,6-dihydrobenzo[f]pyrrolo[1,2-d][1,4]oxazepine-2-ca rboxylate **38e** (327 mg), with a yield of 79%.

MS m/z (ESI): 388.2 [M+1].

### Fifth step

### methyl 11-(hydroxymethyl)-5,6-dihydrobenzo[f]pyrrolo[1,2-d][1,4]oxazepine-2-carboxylate

At room temperature, methyl 11-(((tert-butyldimethylsilyl)oxy)methyl)-5,6-dihydrobenzo[f]pyrrolo[1,2-d][1,4]oxazepine-2-ca rboxylate **38e** (277.00 mg, 0.71 mmol) was dissolved in dichloromethane (1 mL), and a solution of hydrogen chloride in ethyl acetate (4 M, 1.2 mL) was added dropwise. The reaction was carried out at 25°C for 1 hour. The reaction solution was concentrated under reduced pressure. The residue was purified by silica gel column chromatography (eluent: system A) to obtain methyl 11-(hydroxymethyl)-5,6-dihydrobenzo[f]pyrrolo[1,2-d][1,4]oxazepine-2-carboxylate **38f** (107 mg), with a yield of 55%.

MS m/z (ESI): 274.2 [M+1].

### Sixth step

### methyl 11-(phenoxymethyl)-5,6-dihydrobenzo[f]pyrrolo[1,2-d][1,4]oxazepine-2-carboxylate

At room temperature, methyl 11-(hydroxymethyl)-5,6-dihydrobenzo[f]pyrrolo[1,2-d][1,4]oxazepine-2-carboxylate **38f** (107.00 mg, 0.39 mmol) and phenol (55.27 mg, 0.59 mmol) were dissolved in a toluene (1 mL) solution, and cyanomethylene tributyl phosphorane (283.50 mg, 1.17 mmol, 0.31 mL) was added dropwise. The mixture was subjected to nitrogen purging for three times, and then the reaction was carried out at 80°C for 18 hours. The reaction solution was filtered and concentrated under reduced pressure. The residue was purified by silica gel column chromatography (eluent: system A) to obtain methyl 11-(phenoxymethyl)-5,6-dihydrobenzo[f]pyrrolo[1,2-d][1,4]oxazepine-2-carboxylate **38g** (97 mg), with a yield of 71%.

MS m/z (ESI): 350.2 [M+1].

### Seventh step

### N-(3-chloro-5-(methylsulfonamido)phenyl)-11-(phenoxymethyl)-5,6-dihydrobenzo[f]pyrrolo[1,2 -d][1,4]oxazepine-2-carboxamide

At room temperature, methyl 11-(phenoxymethyl)-5,6-dihydrobenzo[f]pyrrolo[1,2-d][1,4]oxazepine-2-carboxylate **38g** (97.00 mg, 0.28 mmol) and N-(3-amino-5-chlorophenyl)methanesulfonamide **1g** (61.27 mg, 0.28 mmol) were dissolved in a tetrahydrofuran (1 mL) solution, and trimethylaluminum (80.00 mg, 1.11 mmol, 2 M) was added dropwise. The reaction was carried out at 60°C for 18 hours. The reaction solution was poured into methanol (50 mL) for quenching and then concentrated under reduced pressure. The residue was separated by preparative liquid chromatography (column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-11-(phenoxymethyl)-5,6-dihydrobenzo[f]pyrrolo[1,2 -d][1,4]oxazepine-2-carboxamide **38** (57 mg), with a yield of 38%.

MS m/z (ESI): 538.2 [M+1].

1H NMR (400 MHz, DMSO-*d₆*) δ 10.00 (s, 1H), 9.84 (s, 1H), 7.84 (d, *J* = 1.6 Hz, 1H), 7.66 (t, *J* = 1.6 Hz, 1H), 7.60 (t, *J* = 1.6 Hz, 1H), 7.46 (dd, *J* = 7.8, 1.2 Hz, 1H), 7.39 (t, *J* = 7.8 Hz, 1H), 7.32 - 7.26 (m, 2H), 7.19 (dd, *J* = 7.8, 1.2 Hz, 1H), 6.98-6.89 (m, 5H), 5.16 (s, 2H), 4.43 (t, *J* = 5.8 Hz, 2H), 4.17 (t, *J* = 5.8 Hz, 2H), 3.05 (s, 3H).

Compounds of Examples 39-48 were prepared according to the preparation method of Example 9 of the present disclosure, and specific structures and structural characterizations were as follows:

| Serial number and structure of example | MS m/z (ESI) | ¹H NMR |
|---|---|---|
| 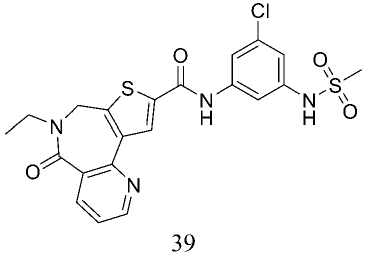 | 491.0[M+1] | ¹H NMR (400 MHz, DMSO-*d*₆) δ 10.63 (s, 1H), 10.08 (s, 1H), 8.83 - 8.80 (m, 1H), 8.62 (s, 1H), 8.31 - 8.27 (m, 1H), 7.71 (t, *J* = 2.0 Hz, 1H), 7.68 (t, *J* = 2.0 Hz, 1H), 7.56 - 7.51 (m, 1H), 6.96 (t, *J* = 2.0 Hz, 1H), 4.60 (s, 2H), 3.64 - 3.57 (m, 2H), 3.07 (s, 3H), 1.13 (t, *J* = 7.2 Hz, 3H). |
| 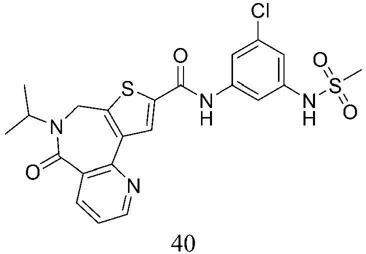 | 505.0 [M+1] | ¹H NMR (400 MHz, DMSO-*d*₆) δ 10.63 (s, 1H), 10.08 (s, 1H), 8.84 - 8.79 (m, 1H), 8.61 (s, 1H), 8.32 -8.27 (m, 1H), 7.71 - 7.65 (m, 2H), 7.55 - 7.50 (m, 1H), 6.96 (t, *J* = 1.6 Hz, 1H), 4.83 - 4.75 (m, 1H), 4.51 (s, 2H), 3.07 (s, 3H), 1.22 (s, 3H), 1.20 (s, 3H). |
| 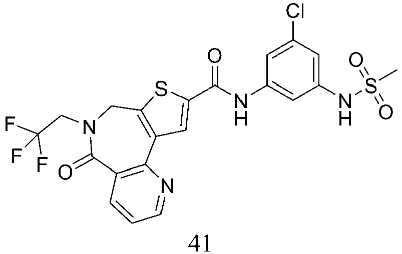 | 545.0 [M+1] | ¹H NMR (400 MHz, DMSO-*d*₆) δ 10.65 (s, 1H), 10.22 (s, 1H), 8.90 - 8.85 (m, 1H), 8.64 (s, 1H), 8.34 - 8.30 (m, 1H), 7.73 - 7.65 (m, 2H), 7.60 - 7.55 (m, 1H), 6.96 (t, *J* = 1.6 Hz, 1H), 4.75 (s, 2H), 4.54 - 4.43 (m, 2H), 3.07 (s, 3H). |
| 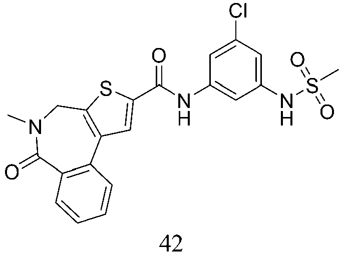 | 476.2 [M+1] | ¹H NMR (400 MHz, DMSO-*d₆*) δ 10.48 (s, 1H), 10.10 (s, 1H), 8.41 (s, 1H), 7.88 (d, *J* = 8.0 Hz, 1H), 7.69 - 7.64 (m, 3H), 7.61 (s, 1H), 7.54 - 7.47 (m, 1H), 6.96 (s, 1H), 4.46 (s, 2H), 3.11 (s, 3H), 3.07 (s, 3H). |
| 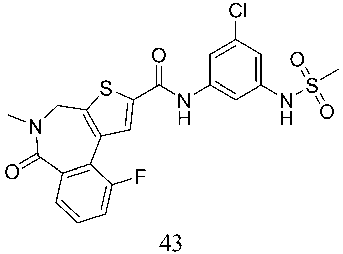 | 494.0 [M+1] | ¹H NMR (400 MHz, DMSO-*d*₆) δ 10.54 (s, 1H), 8.44 (d, *J* = 4.4 Hz, 1H), 7.71 - 7.66 (m, 2H), 7.63 (t, *J* = 2.0 Hz, 1H), 7.58 - 7.53 (m, 2H), 6.97 (t, *J* = 2.0 Hz, 1H), 4.61 - 4.35 (m, 2H), 3.09 (s, 3H), 3.07 (s, 3H). |
| 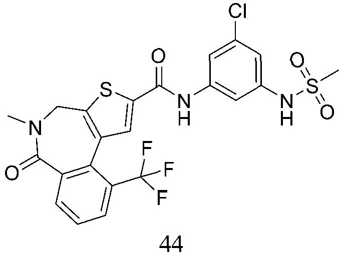 | 544.2 [M+1] | ¹H NMR (400 MHz, DMSO-*d*₆) δ 10.56 (s, 1H), 10.13 (s, 1H), 8.20 (s, 1H), 8.07 (dd, *J* = 8.4, 4.4 Hz, 2H), 7.74 (t, *J* = 8.0 Hz, 1H), 7.62 (t, *J* = 2.0 Hz, 1H), 7.57 (d, *J* = 2.0 Hz, 1H), 6.97 (d, *J* = 2.0 Hz, 1H), 4.55 - 4.37 (m, 2H), 3.06 (s, 3H), 3.05 (s, 3H). |
| 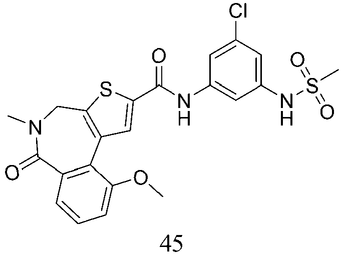 | 506.0 [M+1] | ¹H NMR (400 MHz, DMSO-*d₆*) δ 10.47 (s, 1H), 10.11 (s, 1H), 8.45 (s, 1H), 7.66 (t, *J* = 1.6 Hz, 1H), 7.61 (d, *J* = 1.6 Hz, 1H), 7.46 (t, *J* = 8.0 Hz, 1H), 7.38 (dd, *J* = 7.8, 0.8 Hz, 1H), 7.31 - 7.27 (m, 1H), 6.96 (t, *J* = 1.8 Hz, 1H), 4.47 (d, *J* = 15.6 Hz, 1H), 4.30 (d, *J* = 15.6 Hz, 1H), 3.90 (s, 3H), 3.06 (s, 3H). 3.05 (s, 3H). |
| 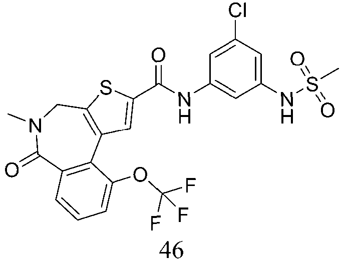 | 560.0 [M+1] | ¹H NMR (400 MHz, DMSO-*d₆*) δ 10.54 (s, 1H), 10.08 (s, 1H), 8.43 (s, 1H), 7.86 (dd, *J* = 7.6, 1.0 Hz, 1H), 7.74 - 7.69 (m, 1H), 7.67-7.60 (m, 3H), 6.98 (t, *J* = 1.8 Hz, 1H), 4.59 (d, *J* = 15.8 Hz, 1H), 4.38 (d, *J* = 15.8 Hz, 1H), 3.07 (s, 3H) , 3.06 (s, 3H). |
| 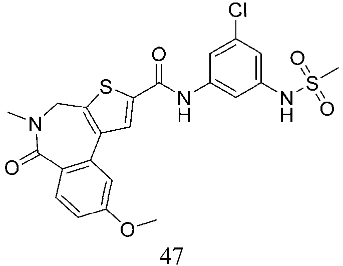 | 506.1 [M+1] | ¹H NMR (400 MHz, DMSO-*d₆*) δ 10.47 (s, 1H), 9.93 (s, 1H), 8.39 (s, 1H), 7.84 (d, *J* = 8.8 Hz, 1H), 7.67 (t, *J* = 1.6 Hz, 1H), 7.62 (t, *J* = 1.6 Hz, 1H), 7.15 (d, *J* = 2.4 Hz, 1H), 7.09 (dd, *J* = 8.8, 2.4 Hz, 1H), 6.97 (t, *J* = 2.0 Hz, 1H), 4.45 (s, 2H), 3.90 (s, 3H), 3.09 (s, 3H), 3.07 (s, 3H). |
| 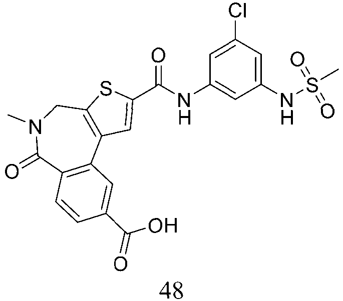 | 520.0 [M+1] | ¹H NMR (400 MHz, DMSO-*d₆*) δ 10.72 (s, 1H), 8.53 (s, 1H), 8.22 (s, 1H), 7.97 (d, J = 8.4 Hz, 1H), 7.89 (d, J = 7.6 Hz, 1H), 7.70 (s, 1H), 7.66 (s, 1H), 6.97 (s, 1H), 4.47 (s, 2H), 3.11 (s, 3H), 3.07 (s, 3H). |

### Example 49

### N-(3-chloro-5-(methylsulfonamido)phenyl)-10-hydroxy-5-methyl-6-oxo-5,6-dihydro-4H-benzo[ c]thieno[3,2-e]azepine-2-carboxamide

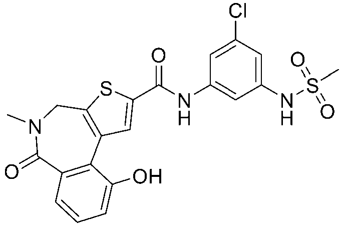

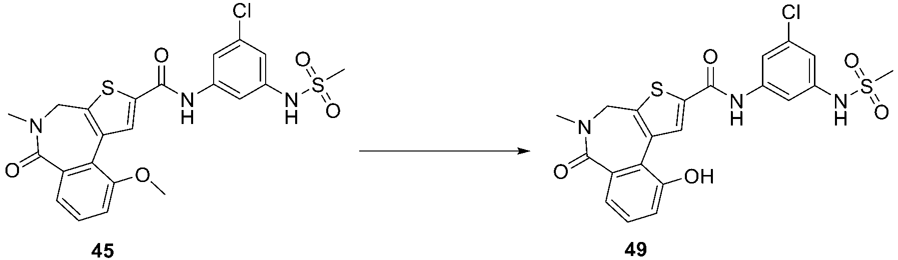

N-(3-chloro-5-(methanesulfonamido)phenyl)-10-methoxy-5-methyl-6-oxo-5,6-dihydro-4H-benzo[c]thieno[3,2-e]azepine-2-carboxamide **45** (125.00 mg, 0.12 mmol) was dissolved in dichloromethane (2 mL). The mixture was subjected to nitrogen purging for three times, and then boron tribromide (185.67 mg, 0.74 mmol) was added dropwise. The reaction was carried out at 25°C for 18 hours. The reaction solution was poured into methanol (50 mL) and stirred for 1 hour for quenching, and the mixture was filtered and concentrated under reduced pressure. The residue was separated by preparative liquid chromatography (column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-10-hydroxy-5-methyl-6-oxo-5,6-dihydro-4H-benzo[ c]thieno[3,2-e]azepine-2-carboxamide **49** (5 mg), with a yield of 8%.

MS m/z (ESI): 492.0 [M+1].

¹H NMR (400 MHz, DMSO-*d₆*) δ 10.51 (s, 1H), 10.20 (s, 1H), 10.06 (s, 1H), 8.53 (s, 1H), 7.68 (t, *J* = 1.6 Hz, 1H), 7.63 (t, *J* = 1.8 Hz, 1H), 7.31 - 7.22 (m, 2H), 7.12 (dd, *J* = 7.8, 1.6 Hz, 1H), 6.95 (t, *J* = 1.6 Hz, 1H), 4.47 - 4.28 (m, 2H), 3.06 (s, 3H) , 3.05 (s, 3H).

### Example 50

### 10-amino-N-(3-chloro-5-(methylsulfonamido)phenyl)-5-methyl-6-oxo-5,6-dihydro-4H-benzo[c]t hieno[3,2-e]azepine-2-carboxamide

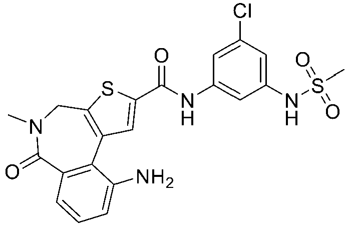

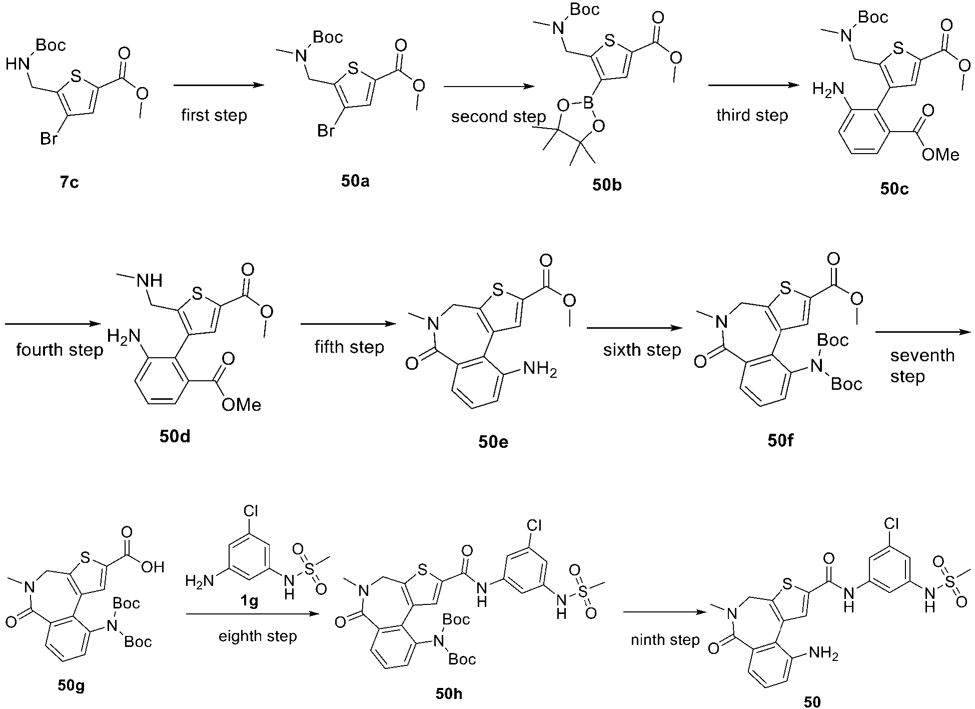

### First step

### methyl 4-bromo-5-(((tert-butoxycarbonyl)(methyl)amino)methyl)thiophene-2-carboxylate

Methyl 4-bromo-5-(((tert-butoxycarbonyl)amino)methyl)thiophene-2-carboxylate **7c** (5 g, 14.28 mmol) and cesium carbonate (9.30 g, 28.55 mmol) were dissolved in *N,N*-dimethylformamide (50 mL), methyl iodide (6.08 g, 42.83 mmol, 2.67 mL) was added to the resulting mixture, and the reaction solution was stirred at room temperature for 12 hours. The reaction solution was diluted with water (500 mL) and extracted with ethyl acetate (100 mL × 3). The extracts were dried with anhydrous sodium sulfate and concentrated. The residue was purified by silica gel column chromatography (eluent: system A) to obtain methyl 4-bromo-5-(((tert-butoxycarbonyl)(methyl)amino)methyl)thiophene-2-carboxylate **50a** (2.7 g), with a yield of 52%.

MS m/z (ESI): 386.0 [M+1].

### Second step

### methyl 5-(((tert-butoxycarbonyl)(methyl)amino)methyl)-4-(4,4,5,5-tetramethyl-1,3,2-dioxaborolan-2-yl) thiophene-2-carboxylate

Methyl 4-bromo-5-(((tert-butoxycarbonyl)(methyl)amino)methyl)thiophene-2-carboxylate **50a** (500 mg, 1.37 mmol) and bis(pinacolato)diboron (693.31 mg, 2.73 mmol) were dissolved in 1,4-dioxane (5 mL), and tris(dibenzylideneacetone)dipalladium (250.01 mg, 273.02 µmol), 2-dicyclohexylphosphino-2',4',6'-triisopropylbiphenyl (195.23 mg, 409.53 µmol) and potassium acetate (401.93 mg, 4.10 mmol) were added to the resulting mixture. The reaction solution was subjected to nitrogen purging for three times and stirred at 90°C under nitrogen protection for 2 hours. The reaction solution was filtered to obtain a solution of methyl 5-(((tert-butoxycarbonyl)(methyl)amino)methyl)-4-(4,4,5,5-tetramethyl-1,3,2-dioxaborolan-2-yl) thiophene-2-carboxylate **50b,** which was used directly in the next step.

MS m/z (ESI): 434.2 [M+1].

### Third step

### methyl 4-(2-amino-6-(methoxycarbonyl)phenyl)-5-(((tert-butoxycarbonyl)(methyl)amino)methyl)thioph ene-2-carboxylate

Methyl 3-amino-2-bromobenzoate (335.59 mg, 1.46 mmol), [1,1'-bis(diphenylphosphino)ferrocene]dichloropalladium(II) (177.89 mg, 243.12 µmol), potassium carbonate (504.02 mg, 3.65 mmol) and water (2 mL) were added to the solution of methyl 5-(((tert-butoxycarbonyl)(methyl)amino)methyl)-4-(4,4,5,5-tetramethyl-1,3,2-dioxaborolan-2-yl) thiophene-2-carboxylate **50b.** The reaction solution was subjected to nitrogen purging for three times and stirred at 90°C under nitrogen protection for 2 hours. The reaction solution was concentrated under reduced pressure, then diluted with water (20 mL), and extracted with ethyl acetate (30 mL × 3). The extracts were dried with anhydrous sodium sulfate and concentrated. The residue was purified by silica gel column chromatography (eluent: system A) to obtain methyl 4-(2-amino-6-(methoxycarbonyl)phenyl)-5-(((tert-butoxycarbonyl)(methyl)amino)methyl)thioph ene-2-carboxylate **50c** (154 mg), with a yield of 29%.

MS m/z (ESI): 335.0 [M+1].

### Fourth step

### methyl 4-(2-amino-6-(methoxycarbonyl)phenyl)-5-((methylamino)methyl)thiophene-2-carboxylate

Methyl 4-(2-amino-6-(methoxycarbonyl)phenyl)-5-(((tert-butoxycarbonyl)(methyl)amino)methyl)thioph ene-2-carboxylate **50c** (150 mg, 345.22 µmol) was dissolved in a solution of hydrogen chloride in ethyl acetate (2 mL, 4 M), and the reaction solution was stirred at room temperature for 6 hours. The reaction solution was concentrated under reduced pressure to obtain a residue of methyl
4-(2-amino-6-(methoxycarbonyl)phenyl)-5-((methylamino)methyl)thiophene-2-carboxylate **50d,** which was used directly in the next step.

MS m/z (ESI):335.0 [M+1].

### Fifth step

### methyl 10-amino-5-methyl-6-oxo-5,6-dihydro-4H-benzo[c]thieno[3,2-e]azepine-2-carboxylate

The methyl 4-(2-amino-6-(methoxycarbonyl)phenyl)-5-((methylamino)methyl)thiophene-2-carboxylate **50d** was dissolved in tetrahydrofuran (2 mL), and triethylamine was slowly added dropwise to adjust the pH of the solution to 7. The resulting mixture was subjected to nitrogen purging for three times, and trimethylaluminum (86.23 mg, 1.20 mmol, 106.45 µL) was slowly added dropwise to the reaction solution at 0°C. The reaction solution was stirred at 60°C under nitrogen protection for 12 hours. The reaction solution was added slowly to a methanol solution for quenching, and the mixture was concentrated. The residue was purified by silica gel column chromatography (eluent: system A) to obtain methyl 10-amino-5-methyl-6-oxo-5,6-dihydro-4H-benzo[c]thieno[3,2-e]azepine-2-carboxylate **50e** (60 mg), with a yield of 66%.

MS m/z (ESI): 303.0 [M+1].

### Sixth step

### methyl 10-[bis(tert-butoxycarbonyl)amino]-5-methyl-6-oxo-4H-thieno[2,3-d][2]benzazepine-2-carboxyl ate

Methyl 10-amino-5-methyl-6-oxo-5,6-dihydro-4H-benzo[c]thieno[3,2-e]azepine-2-carboxylate **50e** (60 mg, 198.45 µmol) was dissolved in tetrahydrofuran (2 mL), di-tert-butyl dicarbonate (86.62 mg, 396.89 µmol, 91.18 µL), 4-dimethylaminopyridine (2.42 mg, 19.84 µmol) and cesium carbonate (129.32 mg, 396.89 µmol) were added to the resulting mixture, and the reaction solution was stirred at 65°C for 1 hour. The reaction solution was filtered under reduced pressure, the filter cake was washed with ethyl acetate, and the filtrate was concentrated. The residue was purified by silica gel column chromatography (eluent: system A) to obtain methyl 10-[bis(tert-butoxycarbonyl)amino]-5-methyl-6-oxo-4H-thieno[2,3-d][2]benzazepine-2-carboxyl ate **50f** (69 mg), with a yield of 86%.

MS m/z (ESI): 503.2 [M+1].

### Seventh step

### 10-[bis(tert-butoxycarbonyl)amino]-5-methyl-6-oxo-4H-thieno[2,3-d][2]benzazepine-2-carboxyl ic acid

Methyl 10-[bis(tert-butoxycarbonyl)amino]-5-methyl-6-oxo-4H-thieno[2,3-d][2]benzazepine-2-carboxyl ate **50f** (69 mg, 137.29 µmol) was dissolved in tetrahydrofuran (2 mL) and water (1 mL), andlithium hydroxide monohydrate (11.52 mg, 274.58 µmol) was added to the resulting mixture. The reaction solution was stirred at room temperature for 2 hours. The reaction solution was concentrated to obtain 10-[bis(tert-butoxycarbonyl)amino]-5-methyl-6-oxo-4H-thieno[2,3-d][2]benzazepine-2-carboxyl ic acid **50g,** which was used directly in the next step.

MS m/z (ESI): 489.0 [M+1].

### Eighth step

### tert-butyl N-tert-butoxycarbonyl-N-[2-[[3-chloro-5-(methanesulfonamido)phenyl]carbamoyl]-5-methyl-6-oxo-4H-thieno[2,3-d][2]benzazepin-10-yl]carbamate

10-[bis(tert-butoxycarbonyl)amino]-5-methyl-6-oxo-4H-thieno[2,3-d][2]benzazepine-2-car boxylic acid **50g** and *N*-(3-amino-5-chlorophenyl)methanesulfonamide **1g** (42.81 mg, 194.00 µmol) were dissolved in N,N-dimethylformamide (1 mL), and (7-azabenzotriazol-1-yloxy)tripyrrolidinophosphonium hexafluorophosphate (202.29 mg, 388.00 µmol) and *N,N*--diisopropylethylamine (100.29 mg, 776.00 µmol, 137.57 µL) were added to the resulting mixture. The reaction solution was stirred at 65°C for 2 hours. The reaction solution was diluted with water (10 mL) and extracted with ethyl acetate (20 mL × 3). The extracts were dried with anhydrous sodium sulfate and concentrated under reduced pressure. The residue was purified by silica gel column chromatography (eluent: system A) to obtain tert-butyl N-tert-butoxycarbonyl-N-[2-[[3-chloro-5-(methanesulfonamido)phenyl]carbamoyl]-5-methyl-6-oxo-4H-thieno[2,3-d][2]benzazepin-10-yl]carbamate **50h** (80 mg), with a yield of 89%.

MS m/z (ESI): 691.1 [M+1].

### Ninth step

### 10-amino-N-(3-chloro-5-(methylsulfonamido)phenyl)-5-methyl-6-oxo-5,6-dihydro-4H-benzo[c]t hieno[3,2-e]azepine-2-carboxamide

The tert-butyl N-tert-butoxycarbonyl-N-[2-[[3-chloro-5-(methanesulfonamido)phenyl]carbamoyl]-5-methyl-6-oxo-4H-thieno[2,3-d][2]benzazepin-10-yl]carbamate **50h** (80 mg, 115.74 µmol) was dissolved in dichloromethane (2 mL), and trifluoroacetic acid (13.20 mg, 115.74 µmol, 1 mL) was added to the resulting mixture. The reaction solution was stirred at room temperature for 1 hour. The reaction solution was concentrated under reduced pressure. The residue was separated by preparative liquid chromatography (column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain 10-amino-N-(3-chloro-5-(methylsulfonamido)phenyl)-5-methyl-6-oxo-5,6-dihydro-4H-benzo[c]t hieno[3,2-e]azepine-2-carboxamide **50** (3 mg), with a yield of 5%.

MS m/z (ESI): 491.0 [M+1].

¹H NMR (400 MHz, DMSO-*d₆*) δ 10.46 (s, 1H), 10.08 (s, 1H), 8.46 (s, 1H), 7.97 (s, 1H), 7.16 (t, *J* = 8.0 Hz, 1H), 7.05 (dd, *J* = 8.4, 2.5 Hz, 1H), 6.97 (dd, *J* = 8.0, 1.2 Hz, 1H), 6.96 - 6.92 (m, 2H), 6.01 (s, 2H), 4.43 (d, *J* = 16.0 Hz, 1H), 4.31 (d, *J* = 16.0 Hz, 1H), 3.05 (s, 3H), 3.01 (s, 3H).

### Example 51

### N-(3-chloro-5-(methylsulfonamido)phenyl)-10-cyano-5-methyl-6-oxo-5,6-dihydro-4H-benzo[c]t hieno[3,2-e]azepine-2-carboxamide

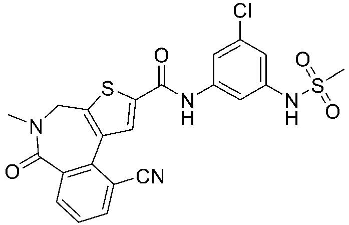

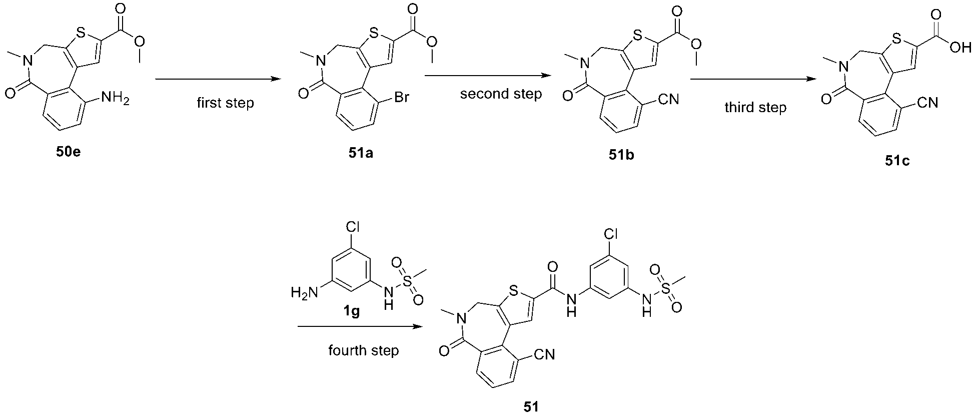

### First step

### methyl 10-bromo-5-methyl-6-oxo-5,6-dihydro-4H-benzo[c]thieno[3,2-e]azepine-2-carboxylate

Methyl 10-amino-5-methyl-6-oxo-5,6-dihydro-4H-benzo[c]thieno[3,2-e]azepine-2-carboxylate **50e** (330 mg, 1.09 mmol) was dissolved in dimethyl sulfoxide (5 mL), and tert-butyl nitrite (337.65 mg, 3.27 mmol, 389.45 µL) was added to the resulting solution. The reaction solution was subjected to nitrogen purging for three times and stirred at room temperature under nitrogen protection for 20 minutes. Cuprous bromide (469.71 mg, 3.27 mmol) was added to the reaction solution, and the mixture was stirred at room temperature for 2 hours. The reaction solution was diluted with water (100 mL) and extracted with ethyl acetate (30 mL × 3). The extracts were dried with anhydrous sodium sulfate and concentrated under reduced pressure. The residue was purified by silica gel column chromatography (eluent: system A) to obtain methyl 10-bromo-5-methyl-6-oxo-5,6-dihydro-4H-benzo[c]thieno[3,2-e]azepine-2-carboxylate **51a** (150 mg), with a yield of 37%.

MS m/z (ESI): 366.0 [M+1].

### Second step

### methyl 10-cyano-5-methyl-6-oxo-5,6-dihydro-4H-benzo[c]thieno[3,2-e]azepine-2-carboxylate

Methyl 10-bromo-5-methyl-6-oxo-5,6-dihydro-4H-benzo[c]thieno[3,2-e]azepine-2-carboxylate **51a** (80 mg, 218.44 µmol) was dissolved in *N,N-*dimethylformamide (2 mL), and zinc cyanide (76.95 mg, 655.33 µmol) and tetrakis(triphenylphosphine)palladium (50.48 mg, 43.69 µmol) were added to the resulting solution. The reaction solution was subjected to nitrogen purging for three times and stirred at 100°C under nitrogen protection for 3 hours. The reaction solution was diluted with water (20 mL) and extracted with ethyl acetate (15 mL × 3). The extracts were dried with anhydrous sodium sulfate and concentrated under reduced pressure. The residue was purified by silica gel column chromatography (eluent: system A) to obtain methyl 10-cyano-5-methyl-6-oxo-5,6-dihydro-4H-benzo[c]thieno[3,2-e]azepine-2-carboxylate **51b** (60 mg), with a yield of 88%.

### Third step

### 10-cyano-5-methyl-6-oxo-5,6-dihydro-4H-benzo[c]thieno[3,2-e]azepine-2-carboxylic acid

Methyl 10-cyano-5-methyl-6-oxo-5,6-dihydro-4H-benzo[c]thieno[3,2-e]azepine-2-carboxylate **51b** (70 mg, 224.11 µmol) was dissolved in tetrahydrofuran (2 mL), and lithium hydroxide monohydrate (18.81 mg, 448.23 µmol) and water (1 mL) were added to the resulting solution. The reaction solution was stirred at room temperature for 2 hours. The reaction solution was concentrated under reduced pressure to obtain 10-cyano-5-methyl-6-oxo-5,6-dihydro-4H-benzo[c]thieno[3,2-e]azepine-2-carboxylic acid **51c,** which was used directly in the next step.

MS m/z (ESI): 299.0 [M+1].

### Fourth step

### N-(3-chloro-5-(methylsulfonamido)phenyl)-10-cyano-5-methyl-6-oxo-5,6-dihydro-4H-benzo[c]t hieno[3,2-e]azepine-2-carboxamide

10-cyano-5-methyl-6-oxo-5,6-dihydro-4H-benzo[c]thieno[3,2-e]azepine-2-carboxylic acid **51c** and *N*-(3-amino-5-chlorophenyl)methanesulfonamide **1g** (76.56 mg, 346.95 µmol) were dissolved in *N,N*-dimethylformamide (2 mL). (7-azabenzotriazol-1-yloxy)tripyrrolidinophosphonium hexafluorophosphate (361.78 mg, 693.89 µmol) and *N,N*-diisopropylethylamine (179.36 mg, 1.39 mmol, 246.04 µL) were added to the resulting mixture. The reaction solution was stirred at 65°C for 2 hours. The reaction solution was diluted with water (20 mL) and extracted with ethyl acetate (30 mL × 3). The extracts were dried with anhydrous sodium sulfate and concentrated under reduced pressure. The residue was purified by silica gel column chromatography (eluent: system A) to obtain N-(3-chloro-5-(methylsulfonamido)phenyl)-10-cyano-5-methyl-6-oxo-5,6-dihydro-4H-benzo[c]t hieno[3,2-e]azepine-2-carboxamide **51** (46 mg), with a yield of 40%.

MS m/z (ESI): 501.0 [M+1].

¹H NMR (400 MHz, DMSO-*d₆*) δ 10.61 (s, 1H), 10.11 (s, 1H), 8.62 (s, 1H), 8.19 (dd, *J* = 8.0, 1.2 Hz, 1H), 8.13 (dd, *J* = 8.0, 1.2 Hz, 1H), 7.75 - 7.66 (m, 2H), 7.61 (t, *J* = 1.6 Hz, 1H), 6.99 (t, *J* = 2.0 Hz, 1H), 4.61 (d, *J* = 16.0 Hz, 1H), 4.45 (d, *J* = 16.0 Hz, 1H), 3.08 (s, 3H), 3.07 (s, 3H).

### Example 52

### 6-acetyl-10-(benzyloxy)-N-(3-chloro-5-(methylsulfonamido)phenyl)-5,6-dihydro-4H-benzo[b]th ieno[3,2-d]azepine-2-carboxamide

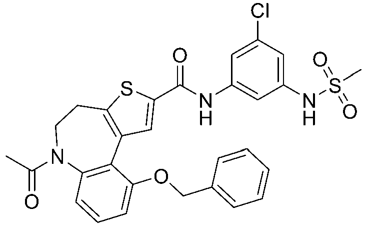

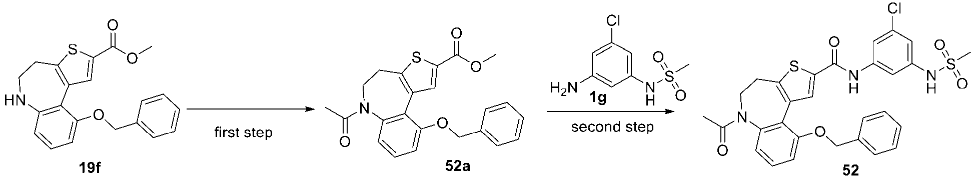

### First step

### methyl 6-acetyl-10-(benzyloxy)-5,6-dihydro-4H-benzo[b]thieno[3,2-d]azepine-2-carboxylate

Methyl 10-(benzyloxy)-5,6-dihydro-4H-benzo[b]thieno[3,2-d]azepine-2-carboxylate **19f** (40 mg, 109.46 µmol) was dissolved in dichloromethane (1 mL), and triethylamine (33.23 mg, 328.37 µmol, 45.64 µL) was added to the resulting solution. The reaction solution was cooled to 0°C in an ice bath, and then acetyl chloride (17.18 mg, 218.91 µmol, 15.57 µL) was added. The reaction solution was stirred at room temperature for 1 hour. The resulting mixture was concentrated under reduced pressure to obtain methyl 6-acetyl-10-(benzyloxy)-5,6-dihydro-4H-benzo[b]thieno[3,2-d]azepine-2-carboxylate **52a,** which was used directly in the next step.

MS m/z (ESI): 408.2 [M+1].

### Second step

### 6-acetyl-10-(benzyloxy)-N-(3-chloro-5-(methylsulfonamido)phenyl)-5,6-dihydro-4H-benzo[b]th ieno[3,2-d]azepine-2-carboxamide

Methyl 6-acetyl-10-(benzyloxy)-5,6-dihydro-4H-benzo[b]thieno[3,2-d]azepine-2-carboxylate **52a** and *N*-(3-amino-5-chlorophenyl)methanesulfonamide **1g** (64.99 mg, 294.49 µmol) were dissolved in tetrahydrofuran (2 mL), and a solution of trimethylaluminum in n-hexane (2 M, 736.23 µL) was slowly added dropwise to the resulting mixture. The reaction solution was stirred at 60°C for 1 hour. The reaction solution was slowly added dropwise to methanol (5 mL) for quenching, and the mixture was concentrated under reduced pressure. The residue was purified by silica gel column chromatography (eluent: system A), and further separated by preparative liquid chromatography (column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain 6-acetyl-10-(benzyloxy)-N-(3-chloro-5-(methylsulfonamido)phenyl)-5,6-dihydro-4H-benzo[b]th ieno[3,2-d]azepine-2-carboxamide **52** (30 mg), with a yield of 34%.

MS m/z (ESI): 596.1 [M+1].

¹H NMR (400 MHz, DMSO-*d₆*) δ 10.38 (s, 1H), 10.07 (s, 1H), 8.38 (s, 1H), 7.68 (t, *J* = 1.6 Hz, 1H), 7.62 (t, *J* = 1.6 Hz, 1H), 7.46 (dd, *J* = 14.8, 7.2 Hz, 3H), 7.34 - 7.24 (m, 4H), 7.10 (d, *J* = 7.6 Hz, 1H), 6.96 (t, *J* = 2.0 Hz, 1H), 5.31 (d, *J* = 12.0 Hz, 1H), 5.16 (d, *J* = 12.0 Hz, 1H), 4.84 (m, 1H), 3.48 (m, 1H), 3.12 (m, 1H), 3.08 (s, 3H), 2.78 - 2.69 (m, 1H), 1.55 (s, 3H).

Compounds of Examples 53-54 were prepared according to the preparation method of Example 52 of the present disclosure, and specific structures and structural characterizations were as follows:

| Serial number and structure of example | MS m/z (ESI) | ¹H NMR |
|---|---|---|
| 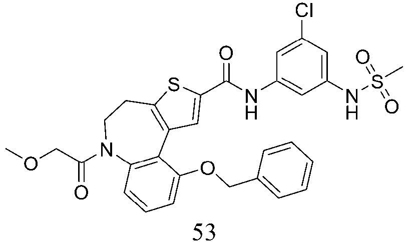 | 626.2[M+1] | ¹H NMR (400 MHz, DMSO-*d₆*) δ 10.35 (s, 1H), 8.38 (s, 1H), 7.65 (t, *J* = 1.6 Hz, 1H), 7.60 (t, *J* = 1.6 Hz, 1H), 7.45 (dd, *J* = 15.6, 7.2 Hz, 3H), 7.34 (d, *J* = 8.0 Hz, 1H), 7.30 - 7.24 (m, 3H), 7.13 (d, *J* = 7.2 Hz, 1H), 6.94 (t, *J* = 2.0 Hz, 1H), 5.31 (d, *J* = 12.4 Hz, 1H), 5.16 (d, *J* = 12.0 Hz, 1H), 4.85 (td, *J* = 12.4, 6.8 Hz, 1H), 3.73 (d, *J* = 14.8 Hz, 1H), 3.51 (dd, *J* = 12.0, 6.8 Hz, 1H), 3.24 (d, *J* = 15.2 Hz, 1H), 3.17 - 3.12 (m, 1H), 3.05 (s, 3H), 3.01 (s, 3H), 2.78 - 2.71 (m, 1H). |
| 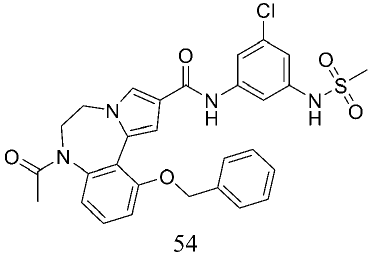 | 579.2 [M+1] | ¹H NMR (400 MHz, DMSO-*d₆*) δ 10.03 (s, 1H), 9.83 (s, 1H), 7.74 - 7.68 (m, 2H), 7.59 (t, *J* = 1.7 Hz, 1H), 7.46 - 7.28 (m, 7H), 7.09 - 7.05 (m, 1H), 7.00 (d, *J* = 1.8 Hz, 1H), 6.88 (t, *J* = 1.8 Hz, 1H), 5.31 (d,*J* = 12.4 Hz, 1H), 5.17 (d, *J* = 12.4 Hz, 1H), 4.87 (ddd, *J* = 15.6, 9.6, 3.4 Hz, 1H), 4.33 (dd, *J* = 14.2, 6.2 Hz, 1H), 3.71 (ddd, *J* = 14.0, 9.8, 4.0 Hz, 1H), 3.37 (d, *J* = 5.2 Hz, 1H), 3.05 (s, 3H), 1.52 (s, 3H). |

Compounds of Examples 55-57 were prepared according to the preparation method of Example 19 of the present disclosure and specific structures and structural characterizations were as follows:

| Serial number and structure of example | MS m/z (ESI) | ¹H NMR |
|---|---|---|
| 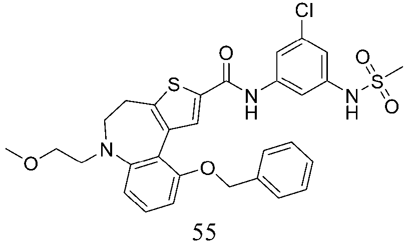 | 612.1[M+1] | ¹H NMR (400 MHz, DMSO-*d₆*) δ 10.30 (s, 1H), 10.08 (s, 1H), 8.29 (s, 1H), 7.65 (dt, *J* = 5.6, 2.0 Hz, 2H), 7.45 (dd, *J* = 7.6, 2.0 Hz, 2H), 7.30 - 7.17 (m, 4H), 6.96 (t, *J* = 2.0 Hz, 1H), 6.91 (d, *J* = 8.0 Hz, 1H), 6.87 (d, *J* = 8.0 Hz, 1H), 5.13 (s, 2H), 3.46 (t, *J* = 6.4 Hz, 2H), 3.35 (t, *J* = 6.0 Hz, 2H), 3.25 (t, *J* = 6.0 Hz, 2H), 3.13 (s, 3H), 3.08 (s, 3H), 2.87 (t, *J* = 6.4 Hz, 2H). |
| 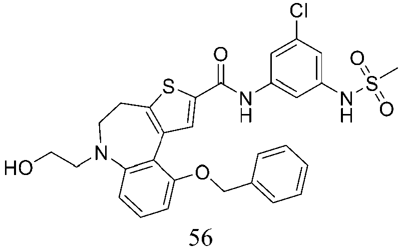 | 598.2[M+1] | ¹H NMR (400 MHz, DMSO-*d₆*) δ 10.29 (s, 1H), 8.29 (s, 1H), 7.64 - 7.61 (m, 2H), 7.45 (dd, *J* = 7.6, 1.6 Hz, 2H), 7.27 - 7.21 (m, 4H), 6.95 (t, *J* = 1.6 Hz, 1H), 6.89 (t, *J* = 8.0 Hz, 2H), 5.13 (s, 2H), 4.42 (s, 1H), 3.46 (t, *J* = 6.4 Hz, 2H), 3.41 (t, *J* = 6.4 Hz, 2H), 3.17 (t, *J* = 6.4 Hz, 2H), 3.06 (s, 3H), 2.87 (t, *J* = 6.4 Hz, 2H). |
| 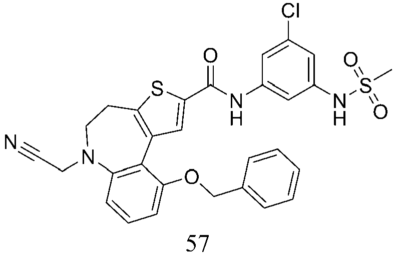 | 593.2[M+1] | ¹H NMR (400 MHz, DMSO-*d₆*) δ 10.29 (s, 2H), 8.27 (s, 1H), 7.61 (t, *J* = 2.0 Hz, 1H), 7.59 (t, *J* = 2.0 Hz, 1H), 7.48 - 7.42 (m, 2H), 7.37 (t, *J* = 8.0 Hz, 1H), 7.30 - 7.20 (m, 3H), 7.03 (d, *J* = 8.4 Hz, 1H), 6.98 (d, *J* = 8.0 Hz, 1H), 6.94 (t, *J* = 2.0 Hz, 1H), 5.16 (s, 2H), 4.26 (s, 2H), 3.56 (t, *J* = 6.4 Hz, 2H), 3.04 (s, 3H), 2.90 (t, *J* = 6.4 Hz, 2H). |

### Example 58

### N-(3-chloro-5-(methylsulfonamido)phenyl)-5'-oxo-5',6'-dihydrospiro[cyclopropane-1,7'-pyrido [3,2-c]thieno[3,2-e]azepine]-9'-carboxamide

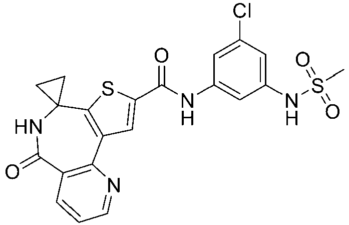

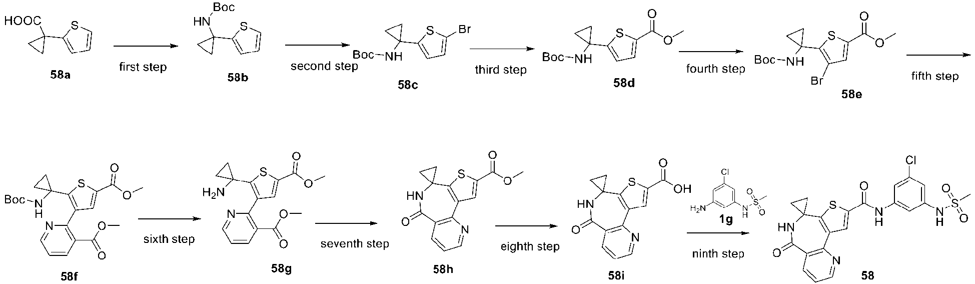

### First step

### tert-butyl (1-(thiophen-2-yl)cyclopropyl)carbamate

1-(thiophen-2-yl)cyclopropane-1-carboxylic acid **58a** (200 mg, 1.19 mmol, commercially available) and triethylamine (200 mg, 1.19 mmol) were dissolved in toluene (10 mL) and tert-butanol (2 mL), and then diphenyl phosphoraazidate (392.64 mg, 1.43 mmol) was added. Subsequently, the mixture was allowed to react at 80°C under a nitrogen atmosphere for 12 hours. The reaction solution was diluted with water and extracted with dichloromethane (20 mL) three times. The extracts were combined, washed with saturated sodium chloride solution, dried with anhydrous sodium sulfate, and concentrated under reduced pressure. The residue was purified by silica gel column chromatography (eluent: system A) to obtain tert-butyl (1-(thiophen-2-yl)cyclopropyl)carbamate **58b** (170 mg), with a yield of 59%.

MS m/z (ESI): 184.0 [M+1].

### Second step

### tert-butyl (1-(5-bromothiophen-2-yl)cyclopropyl)carbamate

Tert-butyl (1-(thiophen-2-yl)cyclopropyl)carbamate **58b** (1.1 g, 4.60 mmol) was dissolved in *N,N*-dimethylformamide (15 mL), and then *N*-bromosuccinimide (1.23 g, 6.89 mmol) was added. The mixture was allowed to react at room temperature for 2 hours. The reaction solution was diluted with water and extracted with ethyl acetate (50 mL) three times. The extracts were combined, washed with saturated sodium chloride solution, dried with anhydrous sodium sulfate, and concentrated under reduced pressure. The residue was purified by silica gel column chromatography (eluent: System A) to obtain tert-butyl (1-(5-bromothiophen-2-yl)cyclopropyl)carbamate **58c** (1.3 g), with a yield of 90%.

MS m/z (ESI): 263.9 [M+1].

### Third step

### methyl 5-(1-((tert-butoxycarbonyl)amino)cyclopropyl)thiophene-2-carboxylate

Tert-butyl (1-(5-bromothiophen-2-yl)cyclopropyl)carbamate **58c** (500 mg, 1.57 mmol) and triethylamine (476.97 mg, 4.71 mmol) were dissolved in methanol (10 mL), and then [1,1'-bis(diphenylphosphino)ferrocene]dichloropalladium(II) (233.10 mg, 314.24 µmol) was added. Subsequently, the mixture was allowed to react at 80°C under a carbon monoxide atmosphere for 2 hours. After the reaction was complete, the reaction solution was diluted with water and extracted with ethyl acetate (20 mL) three times. The extracts were combined, washed with saturated sodium chloride solution, dried with anhydrous sodium sulfate, and concentrated under reduced pressure. The residue was purified by silica gel column chromatography (eluent: system A) to obtain methyl 5-(1-((tert-butoxycarbonyl)amino)cyclopropyl)thiophene-2-carboxylate **58d** (180 mg), with a yield of 81%.

MS m/z (ESI): 242.0 [M+1].

### Fourth step

### methyl 4-bromo-5-(1-((tert-butoxycarbonyl)amino)cyclopropyl)thiophene-2-carboxylate

Methyl 5-(1-((tert-butoxycarbonyl)amino)cyclopropyl)thiophene-2-carboxylate **58d** (1.5 g, 4.13 mmol) was dissolved in tetrahydrofuran (20 mL), and trimethylaluminum (2 M, 12.38 mL) was added dropwise at 0°C. Subsequently, the mixture was allowed to react at 60°C for 12 hours. The reaction solution was quenched with methanol and concentrated under reduced pressure. The residue was purified by silica gel column chromatography (eluent: system A) to obtain methyl 4-bromo-5-(1-((tert-butoxycarbonyl)amino)cyclopropyl)thiophene-2-carboxylate **58e** (400 mg), with a yield of 29%.

MS m/z (ESI): 332.0 [M+1].

### Fifth step

### methyl 2-(2-(1-((tert-butoxycarbonyl)amino)cyclopropyl)-5-(methoxycarbonyl)thiophen-3-yl)nicotinate

Methyl 4-bromo-5-(1-((tert-butoxycarbonyl)amino)cyclopropyl)thiophene-2-carboxylate **58e** (250 mg, 664.42 µmol), methyl 2-bromonicotinate (287.07 mg, 1.33 mmol), bis(pinacolato)diboron (506.17 mg, 1.99 mmol), di(1-adamantyl)-butylphosphine (95.29 mg, 265.77 µmol) and potassium carbonate (459.14 mg, 3.32 mmol) were dissolved in 1,2-dimethoxyethane (10 mL) and water (2 mL). The reaction solution was subjected to nitrogen purging for three times. Under nitrogen protection, palladium(II) acetate (29.83 mg, 132.88 µmol) was added, and the reaction solution was allowed to react at 80°C for 1 hour. The reaction solution was diluted with water and extracted with ethyl acetate (50 mL) three times. The extracts were combined, washed with saturated sodium chloride solution, dried with anhydrous sodium sulfate, and concentrated under reduced pressure. The residue was purified by silica gel column chromatography (eluent: system A) to obtain methyl 2-(2-(1-((tert-butoxycarbonyl)amino)cyclopropyl)-5-(methoxycarbonyl)thiophen-3-yl)nicotinate **58f** (170 mg), with a yield of 59%.

MS m/z (ESI): 433.2 [M+1].

### Sixth step

### methyl 2-(2-(1-aminocyclopropyl)-5-(methoxycarbonyl)thiophen-3-yl)nicotinate Methyl

2-(2-(1-((tert-butoxycarbonyl)amino)cyclopropyl)-5-(methoxycarbonyl)thiophen-3-yl)nicotinate **58f** (180 mg, 416.20 µmol) was dissolved in 4 M hydrogen chloride in dioxane (10 mL), and then the mixture was allowed to react at room temperature for 1 hour. After the reaction was complete, the reaction solution was concentrated under reduced pressure to obtain methyl 2-(2-(1-aminocyclopropyl)-5-(methoxycarbonyl)thiophen-3-yl)nicotinate **58g,** which was used directly in the next step.

MS m/z (ESI): 333.2 [M+1].

### Seventh step

### methyl 5'-oxo-5',6'-dihydrospiro[cyclopropane-1,7'-pyrido[3,2-c]thieno[3,2-e]azepine]-9'-carboxylate

Methyl 2-(2-(1-aminocyclopropyl)-5-(methoxycarbonyl)thiophen-3-yl)nicotinate **58g** was dissolved in tetrahydrofuran (5 mL), and trimethylaluminum (2 M, 902.60 mL) was added dropwise at 0°C. Subsequently, the mixture was allowed to react at 25°C for 1 hour. After the reaction was complete, the trimethylaluminum was quenched with methanol. The residue was purified by silica gel column chromatography (eluent: system A) to obtain methyl 5'-oxo-5',6'-dihydrospiro[cyclopropane-1,7'-pyrido[3,2-c]thieno[3,2-e]azepine]-9'-carboxylate **58h** (55 mg), with a two-step yield of 40%.

MS m/z (ESI): 301.0 [M+1].

### Eighth step

### 5'-oxo-5',6'-dihydrospiro[cyclopropane-1,7'-pyrido[3,2-c]thieno[3,2-e]azepine]-9'-carboxylic acid

Methyl 5'-oxo-5',6'-dihydrospiro[cyclopropane-1,7'-pyrido[3,2-c]thieno[3,2-e]azepine]-9'-carboxylate **58h** (40 mg, 133.19 µmol) was dissolved in tetrahydrofuran (4 mL) and water (2 mL), and then lithium hydroxide monohydrate (11.18 mg, 266.37 µmol) was added. Subsequently, the mixture was allowed to react at room temperature for 1 hour. The reaction solution was concentrated under reduced pressure to obtain 5'-oxo-5',6'-dihydrospiro[cyclopropane-1,7'-pyrido[3,2-c]thieno[3,2-e]azepine]-9'-carboxylic acid **58i,** which was used directly in the next step.

MS m/z (ESI): 287.0 [M+1].

### Ninth step

### N-(3-chloro-5-(methylsulfonamido)phenyl)-5'-oxo-5',6'-dihydrospiro[cyclopropane-1,7'-pyrido [3,2-c]thieno[3,2-e]azepine]-9'-carboxamide

At room temperature, *O*-(7-azabenzotriazol-1-yl)-*N,N,N',N'*-tetramethyluronium hexafluorophosphate (136.58 mg, 261.96 µmol) and N,N-diisopropylethylamine (67.71 mg, 523.92 µmol) were added to a solution of 5'-oxo-5',6'-dihydrospiro[cyclopropane-1,7'-pyrido[3,2-c]thieno[3,2-e]azepine]-9'-carboxylic acid **58i** and N-(3-amino-5-chlorophenyl)methanesulfonamide **1g** (70.96 mg, 321.55 µmol) in N,N-dimethylformamide (1 mL), and the reaction solution was allowed to react at 65°C for 1 hour. The reaction solution was diluted with water and extracted with ethyl acetate (50 mL) three times. The extracts were combined, washed with saturated sodium chloride solution, dried with anhydrous sodium sulfate, and concentrated under reduced pressure. The residue was separated by preparative liquid chromatography (column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-5'-oxo-5',6'-dihydrospiro[cyclopropane-1,7'-pyrido [3,2-c]thieno[3,2-e]azepine]-9'-carboxamide **58** (8 mg), with a yield of 9%.

MS m/z (ESI): 489.0 [M+1].

¹H NMR (400 MHz, DMSO-*d₆*) δ 10.64 (s, 1H), 10.10 (s, 1H), 9.17 (s, 1H), 8.84 (dd, J = 4.8, 1.6 Hz, 1H), 8.62 (s, 1H), 8.28 (dd, J = 8.0, 1.6 Hz, 1H), 7.70 (s, 1H), 7.66 (s, 1H), 7.56 (dd, J = 8.0, 4.8 Hz, 1H), 6.96 (s, 1H), 3.07 (s, 3H), 1.29 (s, 2H), 1.12 (s, 2H).

Compounds of Examples 59-60 were prepared according to the preparation method of Example 58 of the present disclosure, and specific structures and structural characterizations were as follows:

| Serial number and structure of example | MS m/z (ESI) | ¹H NMR |
|---|---|---|
| 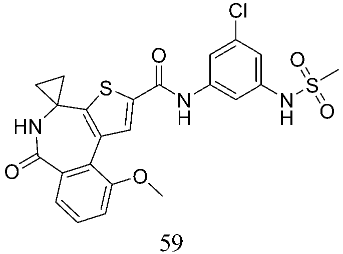 | 518.0[M+1] | ¹H NMR (400 MHz, DMSO-*d₆*) δ 10.45 (s, 1H), 10.24 (s, 1H), 9.00 (s, 1H), 8.41 (s, 1H), 7.64 (t, *J* = 1.6 Hz, 1H), 7.58 (t, *J* = 1.6 Hz, 1H), 7.46 (t, *J* = 8.0 Hz, 1H), 7.37 (dd, *J* = 7.6, 1.2 Hz, 1H), 7.31 (dd, *J* = 7.2 Hz, 1H), 6.95 (t, *J* = 2.0 Hz, 1H), 3.89 (s, 3H), 3.04 (s, 3H), 1.44 - 1.34 (m, 1H), 1.32 - 1.25 (m, 1H), 0.97 - 0.90 (m, 1H), 0.68 - 0.61 (m, 1H). |
| 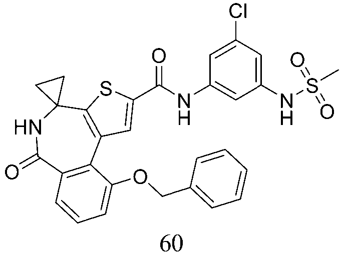 | 594.2[M+1] | ¹H NMR (400 MHz, DMSO-*d₆*) δ 10.43 (s, 1H), 10.12 (s, 1H), 9.00 (s, 1H), 8.52 (s, 1H), 7.66 - 7.61 (m, 2H), 7.48 - 7.39 (m, 5H), 7.27 - 7.18 (m, 3H), 6.98 (t, *J* = 1.8 Hz, 1H), 5.30 (d, *J* = 12.0 Hz, 1H), 5.18 (d, *J* = 12.0 Hz, 1H), 3.08 (s, 3H), 1.43 - 1.23 (m, 2H), 0.97 - 0.88 (m, 1H), 0.66 - 0.57 (m, 1H). |

### Example 61

### N-(3-chloro-5-(methylsulfonamido)phenyl)-6'-oxo-5',6'-dihydrospiro[cyclopropane-1,7'-pyrido [3,2-b]thieno[3,2-d]azepine]-9'-carboxamide

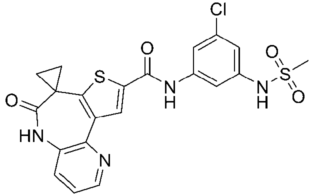

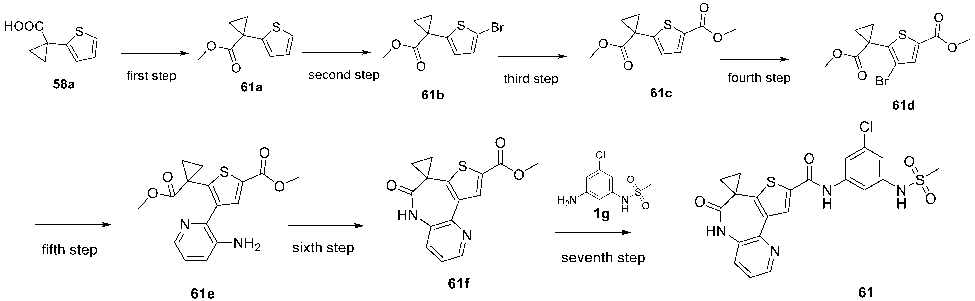

### First step

### methyl 1-(thiophen-2-yl)cyclopropane-1-carboxylate

1-(thiophen-2-yl)cyclopropane-1-carboxylic acid **58a** (2 g, 11.89 mmol) was dissolved in *N,N*-dimethylformamide (20 mL), and sodium hydride (1.43 g, 35.67 mmol, purity 60%) was added portionwise at 0°C. Subsequently, the mixture was stirred at room temperature for 15 minutes. Subsequently, methyl iodide was added dropwise to the reaction solution, and the mixture was continued to react at room temperature for 1 hour. The reaction solution was diluted with water and extracted with ethyl acetate (30 mL) three times. The extracts were combined, washed with saturated sodium chloride solution, dried with anhydrous sodium sulfate, and concentrated under reduced pressure. The residue was purified by silica gel column chromatography (eluent: system A) to obtain methyl 1-(thiophen-2-yl)cyclopropane-1-carboxylate **61a** (1.9 g), with a yield of 87%.

¹H NMR (400 MHz, DMSO-*d₆*) δ 7.19 (dd, J = 5.2, 1.2 Hz, 1H), 6.94 (dd, J = 3.6, 1.2 Hz, 1H), 6.91 (dd, J = 4.8, 3.6 Hz, 1H), 3.67 (s, 3H), 1.70 (q, J = 4.0 Hz, 2H), 1.32 (q, J = 4.0 Hz, 2H).

### Second step

### methyl 1-(5-bromothiophen-2-yl)cyclopropane-1-carboxylate

Methyl 1-(thiophen-2-yl)cyclopropane-1-carboxylate **61a** (500 mg, 2.74 mmol) was dissolved in *N,N*-dimethylformamide (5 mL), and then *N*-bromosuccinimide (976.65 mg, 5.49 mmol) was added. The mixture was allowed to react at room temperature for 1 hours. The reaction solution was diluted with water and extracted with ethyl acetate (50 mL) three times. The extracts were combined, washed with saturated sodium chloride solution, dried with anhydrous sodium sulfate, and concentrated under reduced pressure. The residue was purified by silica gel column chromatography (eluent: system A) to obtain methyl 1-(5-bromothiophen-2-yl)cyclopropane-1-carboxylate **61b** (460 mg), with a yield of 64%.

MS m/z (ESI): 261.0 [M+1].

### Third step

### methyl 5-(1-(methoxycarbonyl)cyclopropyl)thiophene-2-carboxylate

Methyl 1-(5-bromothiophen-2-yl)cyclopropane-1-carboxylate **61b** (1.3 g, 4.98 mmol) and triethylamine (2.01 g, 19.91 mmol, 2.77 mL) were dissolved in methanol (20 mL), and then [1,1'-bis(diphenylphosphino)ferrocene]dichloropalladium(II) (738.56 mg, 995.65 µmol) was added. Subsequently, the mixture was allowed to react at 70°C under a carbon monoxide atmosphere for 2 hours. After the reaction was complete, the reaction solution was diluted with water and extracted with dichloromethane (100 mL) three times. The extracts were combined, washed with saturated sodium chloride solution, dried with anhydrous sodium sulfate, and concentrated under reduced pressure. The residue was purified by silica gel column chromatography (eluent: system A) to obtain methyl 5-(1-(methoxycarbonyl)cyclopropyl)thiophene-2-carboxylate **61c** (300 mg), with a yield of 75%.

MS m/z (ESI): 241.0 [M+1].

### Fourth step

### methyl 4-bromo-5-(1-(methoxycarbonyl)cyclopropyl)thiophene-2-carboxylate

Methyl 5-(1-(methoxycarbonyl)cyclopropyl)thiophene-2-carboxylate **61c** (300 mg, 1.25 mmol) was dissolved in trifluoroacetic acid (3 mL) and sulfuric acid (0.3 mL), and then N-bromosuccinimide (288.89 mg, 1.62 mmol) was added. The mixture was allowed to react at room temperature for 1 hour. The reaction solution was adjusted to basic pH with saturated sodium bicarbonate solution and extracted with ethyl acetate (50 mL) three times. The extracts were combined, washed with saturated sodium chloride solution, dried with anhydrous sodium sulfate, and concentrated under reduced pressure. The residue was purified by silica gel column chromatography (eluent: system A) to obtain methyl 4-bromo-5-(1-(methoxycarbonyl)cyclopropyl)thiophene-2-carboxylate **61d** (300 mg), with a yield of 75%.

MS m/z (ESI): 321.0 [M+1].

### Fifth step

### methyl 4-(3-aminopyridin-2-yl)-5-(1-(methoxycarbonyl)cyclopropyl)thiophene-2-carboxylate

Methyl 4-bromo-5-(1-(methoxycarbonyl)cyclopropyl)thiophene-2-carboxylate **61d** (700 mg, 2.19 mmol), 2-bromo-3-aminopyridine (758.89 mg, 4.39 mmol), bis(pinacolato)diboron (1.67 g, 6.58 mmol), di(1-adamantyl)-butylphosphine (314.54 mg, 877.27 µmol) and potassium carbonate (1.52 g, 10.97 mmol) were dissolved in 1,2-dimethoxyethane (20 mL) and water (4 mL). The reaction solution was subjected to nitrogen purging for three times. Under nitrogen protection, palladium(II) acetate (98.48 mg, 438.64 µmol) was added, and the reaction solution was stirred at 70°C for 2 hours. The reaction solution was diluted with water and extracted with dichloromethane (100 mL) three times. The extracts were combined, washed with a saturated sodium chloride solution, dried with anhydrous sodium sulfate, and concentrated under reduced pressure. The residue was purified by silica gel column chromatography (eluent: system A) to obtain methyl 4-(3-aminopyridin-2-yl)-5-(1-(methoxycarbonyl)cyclopropyl)thiophene-2-carboxylate **61e** (590 mg), with a yield of 81%.

MS m/z (ESI): 333.1 [M+1].

### Sixth step

### methyl 6'-oxo-5',6'-dihydrospiro[cyclopropane-1,7'-pyrido[3,2-b]thieno[3,2-d]azepine]-9'-carboxylate Methyl

4-(3-aminopyridin-2-yl)-5-(1-(methoxycarbonyl)cyclopropyl)thiophene-2-carboxylate **61e** (700 mg, 2.11 mmol) was dissolved in tetrahydrofuran (10 mL), and trimethylaluminum (2 M, 8.42 mL) was added dropwise at 0°C. Subsequently, the mixture was allowed to react at 60°C for 1 hours. After the reaction was complete, the trimethylaluminum was quenched with methanol. The residue was purified by silica gel column chromatography (eluent: system A) to obtain methyl 6'-oxo-5',6'-dihydrospiro[cyclopropane-1,7'-pyrido[3,2-b]thieno[3,2-d]azepine]-9'-carboxylate **61f** (400 mg), with a yield of 63%.

MS m/z (ESI): 301.0 [M+1].

### Seventh step

### N-(3-chloro-5-(methylsulfonamido)phenyl)-6'-oxo-5',6'-dihydrospiro[cyclopropane-1,7'-pyrido [3,2-b]thieno[3,2-d]azepine]-9'-carboxamide

Methyl 6'-oxo-5',6'-dihydrospiro[cyclopropane-1,7'-pyrido[3,2-b]thieno[3,2-d]azepine]-9'-carboxylate **61f** (30 mg, 99.89 µmol) and N-(3-amino-5-chlorophenyl)methanesulfonamide **1g** (44.09 mg, 199.78 µmol) were dissolved in tetrahydrofuran (2 mL), and trimethylaluminum (2 M, 399.56 µL) was added dropwise at 0°C. Subsequently, the mixture was stirred at 60°C for 1 hour. After the reaction was complete, the trimethylaluminum was quenched with methanol. The residue was purified by silica gel column chromatography (eluent: system A), and further separated by preparative liquid chromatography (column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-6'-oxo-5',6'-dihydrospiro[cyclopropane-1,7'-pyrido [3,2-b]thieno[3,2-d]azepine]-9'-carboxamide **61** (5 mg), with a yield of 10%.

MS m/z (ESI): 489.0 [M+1].

¹H NMR (400 MHz, DMSO-*d₆*) δ 10.61 (s, 1H), 10.42 (s, 1H), 10.08 (s, 1H), 8.55 (s, 1H), 8.49 (d, J = 3.6 Hz, 1H), 7.70 (s, 1H), 7.67 (s, 1H), 7.63 (d, J = 7.6 Hz, 1H), 7.46 (dd, J = 8.1, 4.5 Hz, 1H), 6.96 (s, 1H), 3.07 (s, 3H), 1.44 (d, J = 6.8 Hz, 2H), 1.12 (d, J = 6.4 Hz, 2H).

A compound of Example 62 was prepared according to the preparation method of Example 7 of the present disclosure, and a specific structure and a structural characterization were as follows:

| Serial number and structure of example | MS m/z (ESI) | ¹H NMR |
|---|---|---|
| 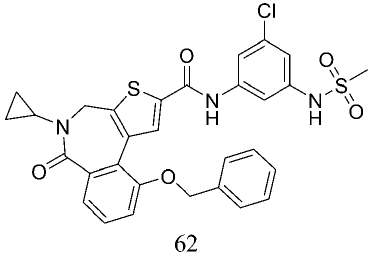 | 608.2[M+1] | ¹H NMR (400 MHz, DMSO-*d₆*) δ 10.43 (s, 1H), 8.52 (s, 1H), 7.63 - 7.59 (m, 2H), 7.53 - 7.42 (m, 3H), 7.41 - 7.34 (m, 2H), 7.32 - 7.19 (m, 3H), 6.97 (t, *J* = 2.0 Hz, 1H), 5.33 (d, *J* = 12.0 Hz, 1H), 5.15 (d, *J* = 12.0 Hz, 1H), 4.41 (d, *J* = 16.0 Hz, 1H), 4.21 (d, *J* = 16.0 Hz, 1H), 3.07 (s, 3H), 2.94 - 2.84 (m, 1H), 0.91 - 0.73 (m, 3H), 0.73 - 0.59 (m, 1H). |

### Example 63

### 10-(benzyloxy)-N-(3-chloro-5-(methylsulfonamido)phenyl)-4,6-dihydrobenzo[c]thieno[3,2-e]ox epine-2-carboxamide

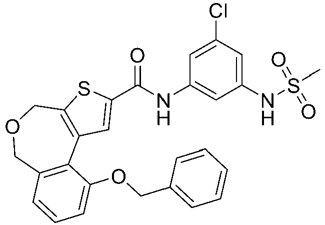

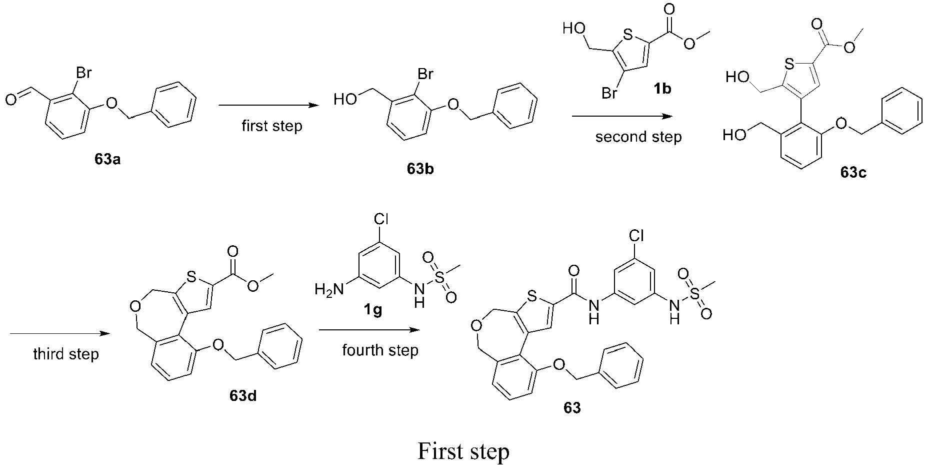

### (3-(benzyloxy)-2-bromophenyl)methanol

Sodium borohydride (390 mg, 10.3 mmol) was added to a solution of 3-(benzyloxy)-2-bromobenzaldehyde **63a** (1.00 g, 3.43 mmol) in methanol (20 mL) at 0°C, and the mixture was allowed to react at 25°C for 18 hours. The mixture was diluted with methanol (50 mL) and then concentrated under reduced pressure. The crude product was purified by silica gel column chromatography (eluent: system A) to obtain (3-(benzyloxy)-2-bromophenyl)methanol **63b** (900 mg), with a yield of 89%.

MS m/z (ESI): 292/294.0 [M+1].

### Second step

### methyl 4-(2-(benzyloxy)-6-(hydroxymethyl)phenyl)-5-(hydroxymethyl)thiophene-2-carboxylate

(3-(benzyloxy)-2-bromophenyl)methanol **63b** (900 mg, 3.07 mmol) was dissolved in 1,4-dioxane (10 mL), and methyl 4-bromo-5-(hydroxymethyl)thiophene-2-carboxylate **1b** (1.36 g, 5.18 mmol), bis(pinacolato)diboron (3.03 g, 11.95 mmol), di(1-adamantyl)-n-butylphosphine (571 mg, 1.59 mmol), potassium carbonate (1.65 g, 12.0 mmol), palladium(II) acetate (179 mg, 0.797 mmol) and water (1 mL) were added to the resulting solution. The reaction solution was subjected to nitrogen purging for three times and stirred at 80°C under nitrogen protection for 2 hours. The reaction solution was filtered and then concentrated under reduced pressure. The crude product was purified by silica gel column chromatography (eluent: system A) to obtain methyl 4-(2-(benzyloxy)-6-(hydroxymethyl)phenyl)-5-(hydroxymethyl)thiophene-2-carboxylate **63c** (1.20 g, purity 60%). The yield was 64%.

MS m/z (ESI): 385.2 [M+1].

### Third step

### methyl 10-(benzyloxy)-4,6-dihydrobenzo[c]thieno[3,2-e]oxepine-2-carboxylate Methyl

4-(2-(benzyloxy)-6-(hydroxymethyl)phenyl)-5-(hydroxymethyl)thiophene-2-carboxylate **63c** (500 mg, purity 60%, 0.78 mmol) was dissolved in 1,4-dioxane (5 mL), and an aqueous hydrobromic acid solution (2 mL, 24% w/w) was added to the resulting solution. The mixture was stirred at 100°C for 20 minutes to obtain a yellow solution. The mixture was poured into water (60 mL) and extracted with ethyl acetate (60 mL). The combined organic phase was washed with a saturated sodium chloride solution (50 mL), dried with anhydrous sodium sulfate, filtered, and concentrated under reduced pressure. The crude product was purified by silica gel column chromatography (eluent: system A) to obtain methyl 10-(benzyloxy)-4,6-dihydrobenzo[c]thieno[3,2-e]oxepine-2-carboxylate **63d** (195 mg), with a yield of 68.3%.

MS m/z (ESI): 367.2 [M+1].

### Fourth step

### 10-(benzyloxy)-N-(3-chloro-5-(methylsulfonamido)phenyl)-4,6-dihydrobenzo[c]thieno[3,2-e]ox epine-2-carboxamide

Methyl 10-(benzyloxy)-4,6-dihydrobenzo[c]thieno[3,2-e]oxepine-2-carboxylate **63d** (60.0 mg, 0.164 mmol) and N-(3-amino-5-chlorophenyl)methanesulfonamide **1g** (72.3 mg, 0.327 mmol) were dissolved in tetrahydrofuran (5.0 mL), and trimethylaluminum (2 M, 0.82 mL) was slowly added dropwise to the resulting mixture. The reaction solution was stirred at 65°C for 2 hours. The reaction solution was slowly added dropwise to methanol (50 mL) to quench the reaction. The resulting mixture was concentrated under reduced pressure. Subsequently, the crude product was purified by silica gel column chromatography (eluent: system A), and further separated by preparative liquid chromatography (separation column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain 10-(benzyloxy)-N-(3-chloro-5-(methylsulfonamido)phenyl)-4,6-dihydrobenzo[c]thieno[3,2-e]ox epine-2-carboxamide **63** (28.0 mg), with a yield of 31%.

MS m/z (ESI): 555.2 [M+1].

¹H NMR (400 MHz, DMSO-*d*₆) δ 10.49 (s, 1H), 8.49 (s, 1H), 7.67 - 7.58 (m, 2H), 7.53 - 7.46 (m, 2H), 7.44 - 7.36 (m, 1H), 7.34 - 7.22 (m, 4H), 7.17 (d, *J* = 7.2 Hz, 1H), 6.97 (t, *J* = 2.0 Hz, 1H), 5.25 (s, 2H), 4.49 (s, 2H), 4.29 (s, 2H), 3.06 (s, 3H).

### Example 64

### 10-(benzyloxy)-N-(3-chloro-5-(methylsulfonamido)phenyl)-4,5-dihydrobenzo[b]thieno[3,2-d]ox epine-2-carboxamide

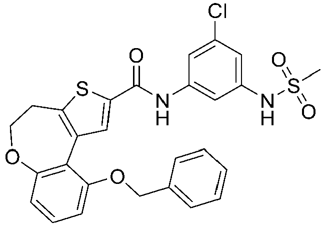

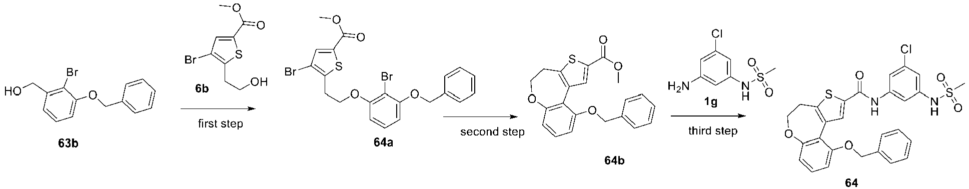

### First step

### methyl 5-(2-(3-(benzyloxy)-2-bromophenoxy)ethyl)-4-bromothiophene-2-carboxylate

(3-(benzyloxy)-2-bromophenyl)methanol **63b** (70.0 mg, 0.264 mmol) was added to toluene (5 mL) at 25°C, and 3-(benzyloxy)-2-bromophenol **6b** (111 mg, 0.396 mmol) and cyanomethylenetri-n-butylphosphorane (191 mg, 0.792 mmol) were added to the above reaction solution. The mixture was allowed to react at 90°C under nitrogen protection for 18 hours. The system was concentrated under reduced pressure. The crude product was purified by silica gel column chromatography (eluent: system A) to obtain methyl 5-(2-(3-(benzyloxy)-2-bromophenoxy)ethyl)-4-bromothiophene-2-carboxylate **64a** (220 mg, purity 40%). The yield was 63.6%.

MS m/z (ESI): 525.0 [M+1].

### Second step

### methyl 10-(benzyloxy)-4,5-dihydrobenzo[b]thieno[3,2-d]oxepine-2-carboxylate

Methyl 5-(2-(3-(benzyloxy)-2-bromophenoxy)ethyl)-4-bromothiophene-2-carboxylate **64a** (200.0 mg, purity 40%, 0.152 mmol) was dissolved in 1,4-dioxane (5 mL), and bis(pinacolato)diboron (116 mg, 0.456 mmol), di(1-adamantyl)-n-butylphosphine (21.8 mg, 0.0608 mmol), potassium carbonate (63.0 mg, 0.456 mmol), palladium(II) acetate (6.83 mg, 0.0304 mmol) and water (1 mL) were added to the resulting solution. The reaction solution was subjected to nitrogen purging for three times and stirred at 80°C under nitrogen protection for 18 hours. The resulting mixture was filtered and then concentrated under reduced pressure. The crude product was purified by silica gel column chromatography (eluent: system A) to obtain methyl 10-(benzyloxy)-4,5-dihydrobenzo[b]thieno[3,2-d]oxepine-2-carboxylate **64b** (20.0 mg), with a yield of 36%.

MS m/z (ESI): 367.2 [M+1].

### Third step

### 10-(benzyloxy)-N-(3-chloro-5-(methylsulfonamido)phenyl)-4,5-dihydrobenzo[b]thieno[3,2-d]ox epine-2-carboxamide

Methyl 10-(benzyloxy)-4,5-dihydrobenzo[b]thieno[3,2-d]oxepine-2-carboxylate **64b** (20.0 mg, 0.0546 mmol) and *N*-(3-amino-5-chlorophenyl)methanesulfonamide **1g** (24.1 mg, 0.109 mmol) were dissolved in tetrahydrofuran (3.0 mL), and trimethylaluminum (2 M, 0.27 mL) was slowly added dropwise to the resulting mixture. The reaction solution was stirred at 65°C for 2 hours. The reaction solution was slowly added dropwise to methanol (50 mL) to quench the reaction. The resulting mixture was concentrated under reduced pressure. The crude product was purified by silica gel column chromatography (eluent: system A), and further separated by preparative liquid chromatography (separation column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain 10-(benzyloxy)-N-(3-chloro-5-(methylsulfonamido)phenyl)-4,5-dihydrobenzo[b]thieno[3,2-d]ox epine-2-carboxamide **64** (5.0 mg), with a yield of 17%.

MS m/z (ESI): 555.0 [M+1].

¹H NMR (400 MHz, DMSO-*d*₆) δ 10.32 (s, 1H), 8.39 (s, 1H), 7.59 - 7.51 (m, 2H), 7.50 - 7.45 (m, 2H), 7.32 - 7.22 (m, 4H), 7.04 (d, *J* = 8.4 Hz, 1H), 6.91 (t, *J* = 2.0 Hz, 1H), 6.83 (d, *J* = 8.0 Hz, 1H), 5.20 (s, 2H), 4.51 (t, *J* = 6.4 Hz, 2H), 3.02 (t, *J* = 6.4 Hz, 2H), 2.99 (s, 3H).

### Example 65

### 10-(benzyloxy)-N-(3-chloro-5-(methylsulfonamido)phenyl)-5,6-dihydro-4H-benzo[c]thieno[3,2-e]azepine-2-carboxamide

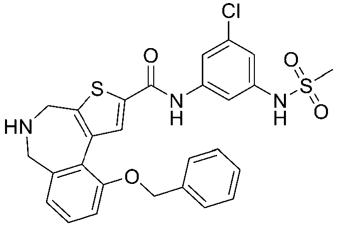

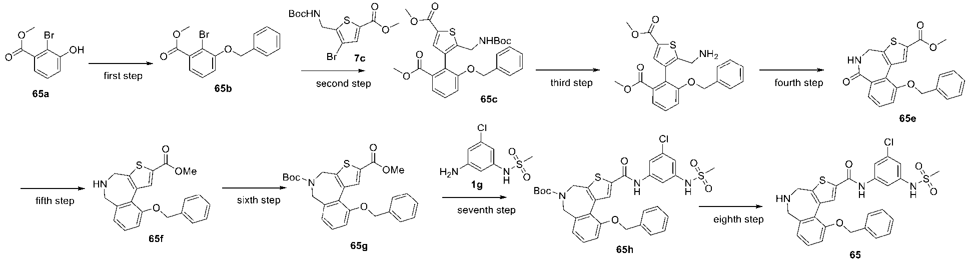

### First step

### methyl 3-(benzyloxy)-2-bromobenzoate

Methyl 2-bromo-3-hydroxybenzoate **65a** (1.00 g, 4.33 mmol) was dissolved in an N,N-dimethylformamide (1 mL) solution, and potassium carbonate (1.20 g, 8.66 mmol) and benzyl bromide (740.27 mg, 4.33 mmol) were added sequentially. The mixture was subjected to nitrogen purging for three times, and then the reaction was carried out at 25°C for 3 hours. The reaction solution was diluted with water (100 mL) and extracted with ethyl acetate (50 mL×2), and the combined organic phase was washed with brine (50 mL), dried with anhydrous sodium sulfate, filtered, and concentrated under reduced pressure. The crude product was purified by silica gel column chromatography (eluent: system A) to obtain methyl 3-(benzyloxy)-2-bromobenzoate **65b** (950.00 mg), with a yield of 68.3%.

MS m/z (ESI): 321/323 [M+1].

### Second step

### methyl 4-(2-(benzyloxy)-6-(methoxycarbonyl)phenyl)-5-(((tert-butoxycarbonyl)amino)methyl)thiophen e-2-carboxylate

Methyl 4-bromo-5-(((tert-butoxycarbonyl)amino)methyl)thiophene-2-carboxylate **7c** (1.5 g, 4.28 mmol) and methyl 3-(benzyloxy)-2-bromobenzoate **65b** (1.58 g, 4.92 mmol) were dissolved in 1,4-dioxane (15 mL), and bis(pinacolato)diboron (3.26 g, 12.85 mmol), di(1-adamantyl)-n-butylphosphine (614.24 mg, 1.71 mmol), potassium carbonate (1.78 g, 12.85 mmol), palladium(II) acetate (192.31 mg, 856.58 µmol) and water (3 mL) were added to the resulting solution. The reaction solution was subjected to nitrogen purging for three times and stirred at 80°C for 12 hours. The resulting mixture was concentrated under reduced pressure. The crude product was purified by silica gel column chromatography (eluent: system A) to obtain methyl
4-(2-(benzyloxy)-6-(methoxycarbonyl)phenyl)-5-(((tert-butoxycarbonyl)amino)methyl)thiophen e-2-carboxylate **65c** (800 mg), with a yield of 36.5%.

MS m/z (ESI): 512.2 [M+1].

### Third step

### methyl 5-(aminomethyl)-4-(2-(benzyloxy)-6-(methoxycarbonyl)phenyl)thiophene-2-carboxylate

Methyl 4-(2-(benzyloxy)-6-(methoxycarbonyl)phenyl)-5-(((tert-butoxycarbonyl)amino)methyl)thiophen e-2-carboxylate **65c** (1.6 g, 3.13 mmol) was dissolved in dichloromethane (20 mL), and trifluoroacetic acid (356.61 mg, 3.13 mmol, 5 mL) was added to the resulting solution. The reaction solution was stirred at room temperature for 1 hour. The resulting mixture was concentrated under reduced pressure to obtain methyl 5-(aminomethyl)-4-(2-(benzyloxy)-6-(methoxycarbonyl)phenyl)thiophene-2-carboxylate **65d,** which was used directly in the next step.

MS m/z (ESI): 412.2 [M+1].

### Fourth step

### methyl 10-(benzyloxy)-6-oxo-5,6-dihydro-4H-benzo[c]thieno[3,2-e]azepine-2-carboxylate

The methyl 5-(aminomethyl)-4-(2-(benzyloxy)-6-(methoxycarbonyl)phenyl)thiophene-2-carboxylate **65d** crude product was dissolved in toluene (13 mL), and N,N-diisopropylethylamine (6.03 g, 46.65 mmol, 8.13 mL) was added to the resulting solution. The reaction solution was stirred at 70°C for 12 hours. The resulting mixture was concentrated under reduced pressure. The crude product was purified by silica gel column chromatography (eluent: system A) to obtain methyl 10-(benzyloxy)-6-oxo-5,6-dihydro-4H-benzo[c]thieno[3,2-e]azepine-2-carboxylate **65e** (960 mg), with a yield of 80.1%.

MS m/z (ESI): 380.2 [M+1].

### Fifth step

### methyl 10-(benzyloxy)-5,6-dihydro-4H-benzo[c]thieno[3,2-e]azepine-2-carboxylate

Methyl 10-(benzyloxy)-6-oxo-5,6-dihydro-4H-benzo[c]thieno[3,2-e]azepine-2-carboxylate **65e** (100 mg, 0.264 mmol) was dissolved in a borane in tetrahydrofuran solution (5 mL). The reaction solution was allowed to react at 60°C for 2 hours. The mixture was added dropwise to methanol (20 mL) to quench the reaction, and the solution was concentrated under reduced pressure. The crude product was purified by silica gel column chromatography (eluent: system A) to obtain methyl 10-(benzyloxy)-5,6-dihydro-4H-benzo[c]thieno[3,2-e]azepine-2-carboxylate **65f** (70.0 mg), with a yield of 62%.

MS m/z (ESI): 366.2 [M+1].

### Sixth step

### 5-(tert-butyl) 2-methyl 10-(benzyloxy)-4,6-dihydro-5H-benzo[c]thieno[3,2-e]azepine-2,5-dicarboxylate

Methyl 10-(benzyloxy)-5,6-dihydro-4H-benzo[c]thieno[3,2-e]azepine-2-carboxylate **65f** (90.0 mg, 0.246 mmol) was dissolved in dichloromethane (5 mL), and di-tert-butyl dicarbonate (108 mg, 0.493 mmol), 4-dimethylaminopyridine (3.01 mg, 0.0246 mmol) and N,N-diisopropylethylamine (95.5 mg, 0.739 mmol) were added to the above reaction solution. The reaction solution was allowed to react at 25°C for 18 hours. The reaction solution was diluted with water (100 mL), and the above aqueous solution was extracted with ethyl acetate (100 mL). The organic phase was dried with anhydrous sodium sulfate and then filtered, and the filtrate was concentrated. Subsequently, the crude product was purified by silica gel column chromatography (eluent: system A) to obtain 5-(tert-butyl) 2-methyl 10-(benzyloxy)-4,6-dihydro-5H-benzo[c]thieno[3,2-e]azepine-2,5-dicarboxylate **65g** (80.0 mg), with a yield of 70%.

MS m/z (ESI): 366.2 [M+1-100].

### Seventh step

### tert-butyl 10-(benzyloxy)-2-((3-chloro-5-(methylsulfonamido)phenyl)carbamoyl)-4,6-dihydro-5H-benzo[c ]thieno[3,2-e]azepine-5-carboxylate

5-(tert-butyl) 2-methyl 10-(benzyloxy)-4,6-dihydro-5H-benzo[c]thieno[3,2-e]azepine-2,5-dicarboxylate **65g** (60.0 mg, 0.129 mmol) and *N*-(3-amino-5-chlorophenyl)methanesulfonamide **1g** (56.9 mg, 0.258 mmol) were dissolved in tetrahydrofuran (7.0 mL), and trimethylaluminum (2 M, 0.64 mL) was slowly added dropwise to the resulting mixture. The reaction solution was stirred at 60°C for 2 hours. The reaction solution was slowly added dropwise to methanol (50 mL) to quench the reaction. The resulting mixture was concentrated under reduced pressure. Subsequently, the crude product was purified by silica gel column chromatography (eluent: system A) to obtain tert-butyl 10-(benzyloxy)-2-((3-chloro-5-(methylsulfonamido)phenyl)carbamoyl)-4,6-dihydro-5H-benzo[c ]thieno[3,2-e]azepine-5-carboxylate **65h** (60.0 mg), with a yield of 71%.

### Eighth step

### 10-(benzyloxy)-N-(3-chloro-5-(methylsulfonamido)phenyl)-5,6-dihydro-4H-benzo[c]thieno[3,2-e]azepine-2-carboxamide

Tert-butyl 10-(benzyloxy)-2-((3-chloro-5-(methylsulfonamido)phenyl)carbamoyl)-4,6-dihydro-5H-benzo[c ]thieno[3,2-e]azepine-5-carboxylate **65h** (60.0 mg, 0.0917 mmol) was dissolved in dichloromethane (4.0 mL), and trifluoroacetic acid (1.0 mL) was slowly added dropwise to the resulting mixture. The mixture was allowed to react at 25°C for 1 hour. The reaction solution was concentrated under reduced pressure, and then separated by preparative liquid chromatography (column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain 10-(benzyloxy)-N-(3-chloro-5-(methylsulfonamido)phenyl)-5,6-dihydro-4H-benzo[c]thieno[3,2-e]azepine-2-carboxamide **65** (32.0 mg), with a yield of 63%.

MS m/z (ESI): 554.2 [M+1].

¹H NMR (400 MHz, DMSO-*d*₆) δ 10.44 (s, 1H), 8.42 (s, 1H), 8.18 (s, 1H), 7.66 (dt, *J* = 8.0, 2.0 Hz, 2H), 7.50 - 7.43 (m, 2H), 7.37 - 7.19 (m, 5H), 7.07 (dd, *J* = 7.6, 1.2 Hz, 1H), 6.97 (t, *J* = 2.0 Hz, 1H), 5.21 (s, 2H), 3.74 (s, 2H), 3.54 (s, 2H), 3.08 (s, 3H).

### Example 66

### 10-(benzyloxy)-N-(3-chloro-5-(methylsulfonamido)phenyl)-5-methyl-5,6-dihydro-4H-benzo[c]t hieno[3,2-e]azepine-2-carboxamide

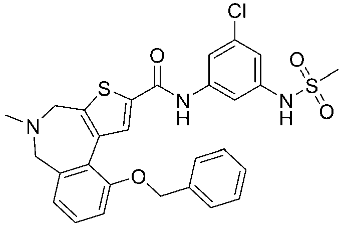

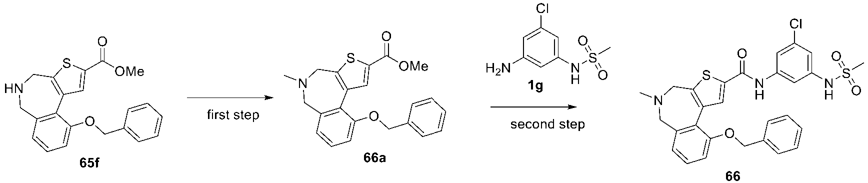

### First step

### methyl 10-(benzyloxy)-5-methyl-5,6-dihydro-4H-benzo[c]thieno[3,2-e]azepine-2-carboxylate

Methyl 10-(benzyloxy)-5,6-dihydro-4H-benzo[c]thieno[3,2-e]azepine-2-carboxylate **65f** (70 mg, 0.192 mmol) was dissolved in 1,2-dichloroethane (5 mL), and an aqueous formaldehyde solution (77.7 mg, 0.958 mmol) and sodium triacetoxyborohydride (122 mg, 0.575 mmol) were added to the resulting solution. The reaction solution was stirred at 25°C for 2 hours. The mixture was added to methanol for quenching, and then the solution was concentrated under reduced pressure. The crude product was purified by silica gel column chromatography (eluent: system A) to obtain methyl 10-(benzyloxy)-5-methyl-5,6-dihydro-4H-benzo[c]thieno[3,2-e]azepine-2-carboxylate **66a** (50.0 mg), with a yield of 69%.

MS m/z (ESI): 380.2 [M+1].

### Second step

### 10-(benzyloxy)-N-(3-chloro-5-(methylsulfonamido)phenyl)-5-methyl-5,6-dihydro-4H-benzo[c]t hieno[3,2-e]azepine-2-carboxamide

Methyl 10-(benzyloxy)-5-methyl-5,6-dihydro-4H-benzo[c]thieno[3,2-e]azepine-2-carboxylate **66a** (60.0 mg, 0.156 mmol) and *N*-(3-amino-5-chlorophenyl)methanesulfonamide **1g** (68.7 mg, 0.311 mmol) were dissolved in tetrahydrofuran (5.0 mL), and trimethylaluminum (2 M, 0.78 mL) was slowly added dropwise to the resulting mixture. The reaction solution was stirred at 60°C for 2 hours. The reaction solution was slowly added dropwise to methanol (50 mL) to quench the reaction. The resulting mixture was concentrated under reduced pressure. Subsequently, the crude product was purified by silica gel column chromatography (eluent: system A), and further separated by preparative liquid chromatography (separation column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain 10-(benzyloxy)-N-(3-chloro-5-(methylsulfonamido)phenyl)-5-methyl-5,6-dihydro-4H-benzo[c]t hieno[3,2-e]azepine-2-carboxamide **66** (7.0 mg), with a yield of 8%.

MS m/z (ESI): 568.2 [M+1].

¹H NMR (400 MHz, DMSO-*d*₆) δ 10.39 (s, 1H), 10.01 (s, 1H), 8.43 (s, 1H), 7.59 (d, *J* = 2.0 Hz, 1H), 7.56 (s, 1H), 7.49 - 7.44 (m, 2H), 7.37 - 7.27 (m, 3H), 7.26 - 7.18 (m, 2H), 7.08 (d, *J* = 7.6 Hz, 1H), 6.93 (t, *J* = 2.0 Hz, 1H), 5.21 (s, 2H), 3.46 (s, 2H), 3.22 (s, 2H), 3.02 (s, 3H), 2.33 (s, 3H).

### Example 67

### 11-benzamido-N-(3-chloro-5-(methylsulfonamido)phenyl)-5,6-dihydrobenzo[f]pyrrolo[1,2-d][1, 4]oxazepine-2-carboxamide

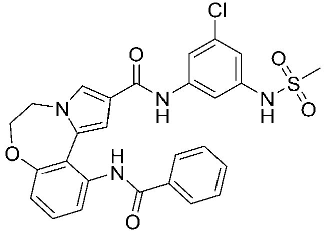

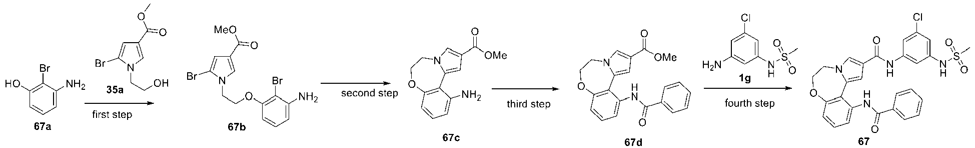

### First step

### methyl 1-(2-(3-amino-2-bromophenoxy)ethyl)-5-bromo-1H-pyrrole-3-carboxylate

3-amino-2-bromophenol **67a** (1.8 g, 9.57 mmol, commercially available) and methyl 5-bromo-1-(2-hydroxyethyl)-1H-pyrrole-3-carboxylate **35a** (4.75 g, 19.15 mmol) were dissolved in toluene (20 mL), and cyanomethylene tri-n-butyl phosphorane (6.93 g, 28.72 mmol, 7.62 mL) was added to the resulting solution. The reaction solution was stirred at 80°C for 2 hours. The resulting mixture was concentrated under reduced pressure. Subsequently, the crude product was purified by silica gel column chromatography (eluent: system A) to obtain methyl 1-(2-(3-amino-2-bromophenoxy)ethyl)-5-bromo-1H-pyrrole-3-carboxylate **67b** (1.88 g), with a yield of 46.9%.

MS m/z (ESI): 417.0 [M+1].

### Second step

### methyl 11-amino-5,6-dihydrobenzo[f]pyrrolo[1,2-d][1,4]oxazepine-2-carboxylate

Methyl 1-(2-(3-amino-2-bromophenoxy)ethyl)-5-bromo-1H-pyrrole-3-carboxylate **67b** (1.88 g, 4.50 mmol) was dissolved in dimethoxyethane (30 mL) and water (6.0 mL), and bis(pinacolato)diboron (3.43 g, 13.49 mmol), di(1-adamantyl)-n-butylphosphine (644.9 mg, 1.80 mmol), potassium carbonate (1.86 g, 13.49 mmol) and palladium(II) acetate (201.91 mg, 899.35 µmol) were added to the resulting solution. The reaction solution was subjected to nitrogen purging for three times, and heated to 70°C and stirred for 18 hours. The resulting mixture was filtered under reduced pressure, the filter cake was washed with dichloromethane (30 mL × 3), and the resulting filtrates were concentrated under reduced pressure and then purified by silica gel column chromatography (eluent: system A) to obtain methyl 11-amino-5,6-dihydrobenzo[f]pyrrolo[1,2-d][1,4]oxazepine-2-carboxylate **67c** (250 mg, purity 71.7%), with a yield of 15.4%.

MS m/z (ESI): 259.1 [M+1].

### Third step

### methyl 11-benzamido-5,6-dihydrobenzo[f]pyrrolo[1,2-d][1,4]oxazepine-2-carboxylate

Methyl 11-amino-5,6-dihydrobenzo[f]pyrrolo[1,2-d][1,4]oxazepine-2-carboxylate **67c** (111.6 mg, purity 71.7%, 309.751 µmol) was dissolved in dichloromethane (40 mL), and triethylamine (94.03 mg, 129.16 µL) and benzoyl chloride (43.54 mg, 309.75 µmol, 35.98 µL) were added to the resulting solution. The reaction solution was stirred at 25°C for 18 hours. The mixture was concentrated under reduced pressure. The crude product was purified by silica gel column chromatography (eluent: system A) to obtain methyl 11-benzamido-5,6-dihydrobenzo[f]pyrrolo[1,2-d][1,4]oxazepine-2-carboxylate **67d** (80 mg), with a yield of 71.2%.

MS m/z (ESI): 363.1 [M+1].

### Fourth step

### 11-benzamido-N-(3-chloro-5-(methylsulfonamido)phenyl)-5,6-dihydrobenzo[f]pyrrolo[1,2-d][1, 4]oxazepine-2-carboxamide

Methyl 11-benzamido-5,6-dihydrobenzo[f]pyrrolo[1,2-d][1,4]oxazepine-2-carboxylate **67d** (20 mg, 55.19 µmol) and N-(3-amino-5-chlorophenyl)methanesulfonamide **1g** (24.36 mg, 110.38 µmol) were dissolved in tetrahydrofuran (1 mL), and trimethylaluminum (2 M, 275.95 µL) was slowly added dropwise to the resulting mixture. The reaction solution was stirred at 60°C for 2 hours. The reaction solution was slowly added dropwise to methanol (10 mL) to quench the reaction. The resulting mixture was concentrated under reduced pressure. The crude product was purified by silica gel column chromatography (eluent: system A), and further separated by preparative liquid chromatography (separation column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain 11-benzamido-N-(3-chloro-5-(methylsulfonamido)phenyl)-5,6-dihydrobenzo[f]pyrrolo[1,2-d][1, 4]oxazepine-2-carboxamide **67** (12.56 mg), with a yield of 41.3%.

MS m/z (ESI): 551.1 [M+1].

¹H NMR (400 MHz, DMSO-*d₆*) δ 9.98 (s, 1H), 9.86 (s, 1H), 9.80 (s, 1H), 7.90 (d, *J* = 7.4 Hz, 2H), 7.81 (d, *J* = 1.7 Hz, 1H), 7.65 (t, *J* = 1.8 Hz, 1H), 7.59 - 7.54 (m, 2H), 7.51 (dt, *J* = 10.7, 5.5 Hz, 3H), 7.38 (t, *J* = 8.0 Hz, 1H), 7.09 (dd, *J* = 8.0, 1.0 Hz, 1H), 6.92 (d, *J* = 1.7 Hz, 1H), 6.89 (t, *J* = 1.9 Hz, 1H), 4.46 (t, *J* = 5.9 Hz, 2H), 4.21 (d, *J* = 5.1 Hz, 2H), 3.05 (s, 3H).

### Example 68

### N-(3-chloro-5-(methylsulfonamido)phenyl)-11-((3,5-difluorobenzyl)oxy)-6,7-dihydro-5H-benzo [f]pyrrolo[1,2-d][1,4]diazepine-2-carboxamide

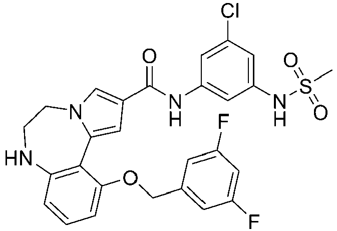

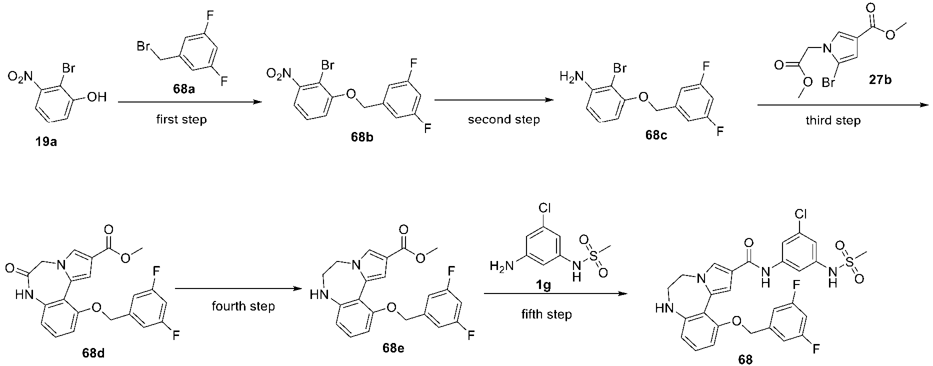

### First step

### 2-bromo-1-((3,5-difluorobenzyl)oxy)-3-nitrobenzene

At room temperature, potassium carbonate (24.88 g, 180.00 mmol) and 1-(bromomethyl)-3,5-difluorobenzene **68a** (12.55 g, 60.60 mmol) were added to a solution of 2-bromo-3-nitrophenol **19a** (13.08 g, 60 mmol) in acetonitrile (150 mL), and the mixture was heated to 70°C and stirred for 4 hours. The reaction solution was filtered, and the filtrate was concentrated under reduced pressure to obtain 2-bromo-1-((3,5-difluorobenzyl)oxy)-3-nitrobenzene **68b** (19.5 g), with a yield of 94.5%.

1H NMR (400 MHz, DMSO-*d₆*) δ 7.67-7.53 (m, 2H), 7.51-7.44 (m, 1H), 7.30-7.18 (m, 3H), 5.35 (s, 2H).

### Second step

### 2-bromo-3-((3,5-difluorobenzyl)oxy)aniline

Iron powder (11.68 g, 209.24 mmol) and ammonium chloride (11.19 g, 209.24 mmol) were added to a mixed solution of 2-bromo-1-((3,5-difluorobenzyl)oxy)-3-nitrobenzene **68b** (18 g, 52.31 mmol) in water (100 mL) and ethanol (200 mL), and the mixture was heated to 80°C and stirred for 2 hours. The reaction solution was filtered, and the filtrate was extracted with ethyl acetate (100 mL×3). The organic phases were combined, washed with saturated sodium chloride solution (100 mL×2), dried with anhydrous sodium sulfate, and filtered, and the filtrate was concentrated under reduced pressure to obtain 2-bromo-3-((3,5-difluorobenzyl)oxy)aniline **68c** (13.00 g), with a yield of 79.1%.

MS m/z (ESI): 316.0 [M+1].

### Third step

### methyl 11-((3,5-difluorobenzyl)oxy)-6-oxo-6,7-dihydro-5H-benzo[f]pyrrolo[1,2-d][1,4]diazepine-2-carb oxylate

At room temperature, palladium(II) acetate (571 mg, 2.55 mmol) and di(1-adamantyl)-n-butylphosphine (1.83 g, 5.09 mmol) were added to dioxane (200 mL). The mixture was subjected to argon purging and then stirred for 10 minutes. Subsequently, the 2-bromo-3-((3,5-difluorobenzyl)oxy)aniline **68c** (4.00 g, 12.73 mmol), methyl 5-bromo-1-(2-methoxy-2-oxoethyl)-1H-pyrrole-3-carboxylate **27b** (4.22 g, 15.28 mmol), bis(pinacolato)diboron (6.47 g, 25.47 mmol), potassium carbonate (5.28 g, 38.20 mmol) and water (20 mL) were added. The mixture was subjected to argon purging, then heated to 100°C and stirred for 16 hours. The reaction solution was filtered, and the filtrate was concentrated under reduced pressure. Water (50 mL), ethyl acetate (100 mL) and petroleum ether (50 mL) were added, the mixture was stirred for 30 minutes and filtered, and the filter cake was dried under vacuum to obtain methyl 11-((3,5-difluorobenzyl)oxy)-6-oxo-6,7-dihydro-5H-benzo[f]pyrrolo[1,2-d][1,4]diazepine-2-carb oxylate **68d** (1.80 g), with a yield of 35.5%.

MS m/z (ESI): 399.1 [M+1].

### Fourth step

### methyl 11-((3,5-difluorobenzyl)oxy)-6,7-dihydro-5H-benzo[f]pyrrolo[1,2-d][1,4]diazepine-2-carboxylat e

Borane dimethyl sulfide complex (312.56 mg, 22.59 mmol, 2 M tetrahydrofuran solution) was added to a solution of methyl 11-((3,5-difluorobenzyl)oxy)-6-oxo-6,7-dihydro-5H-benzo[f]pyrrolo[1,2-d][1,4]diazepine-2-carb oxylate **68d** (4.5 g, 11.30 mmol) in tetrahydrofuran (80 mL), and the mixture was heated to 60°C and stirred for 1 hour. Methanol (10 mL) was slowly added dropwise to quench the reaction, and the mixture was concentrated under reduced pressure. The residue was purified by silica gel column chromatography (eluent: system A) to obtain methyl 11-((3,5-difluorobenzyl)oxy)-6,7-dihydro-5H-benzo[f]pyrrolo[1,2-d][1,4]diazepine-2-carboxylat e **68e** (3.5 g), with a yield of 80.6%.

MS m/z (ESI): 385.1 [M+1].

### Fifth step

### N-(3-chloro-5-(methylsulfonamido)phenyl)-11-((3,5-difluorobenzyl)oxy)-6,7-dihydro-5H-benzo [f]pyrrolo[1,2-d][1,4]diazepine-2-carboxamide

At room temperature, trimethylaluminum (1.59 g, 22.11 mmol, 2 M tetrahydrofuran) was added dropwise to a solution of N-(3-amino-5-chlorophenyl)methanesulfonamide **1g** (585 mg, 2.65 mmol) and methyl 11-((3,5-difluorobenzyl)oxy)-6,7-dihydro-5H-benzo[f]pyrrolo[1,2-d][1,4]diazepine-2-carboxylat e **68e** (850 mg, 2.21 mmol) in tetrahydrofuran (20 mL). The mixture was subjected to argon purging, and then heated, refluxed and stirred for 2 hours. Methanol (10 mL) was slowly added dropwise to quench the reaction, and the mixture was concentrated under reduced pressure. The residue was purified by silica gel column chromatography (eluent: system A) to obtain N-(3-chloro-5-(methylsulfonamido)phenyl)-11-((3,5-difluorobenzyl)oxy)-6,7-dihydro-5H-benzo [f]pyrrolo[1,2-d][1,4]diazepine-2-carboxamide **68** (596 mg), with a yield of 45.9%.

MS m/z (ESI): 573.2 [M+1].

1H NMR (400 MHz, DMSO-d6) δ 9.96 (s, 1H), 9.74 (s, 1H), 7.69 (s, 1H), 7.64 (s, 1H), 7.62 (s, 1H), 7.19 - 7.07 (m, 3H), 7.06 - 6.98 (m, 2H), 6.89 (s, 1H), 6.50 (t, J = 8.3 Hz, 2H), 5.73 (s, 1H), 5.13 (s, 2H), 4.23 - 4.03 (m, 2H), 3.54-3.45 (m, 2H), 3.06 (s, 3H).

### Example 69

### N-(3-chloro-5-(methylsulfonamido)phenyl)-11-((3,5-difluorobenzyl)oxy)-7-formyl-6,7-dihydro-5H-benzo[f]pyrrolo[1,2-d][1,4]diazepine-2-carboxamide

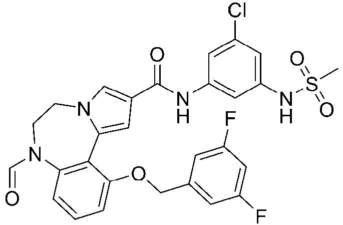

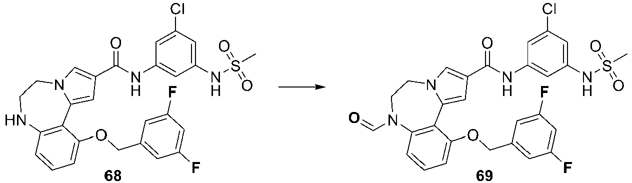

At room temperature, N-(3-chloro-5-(methylsulfonamido)phenyl)-11-((3,5-difluorobenzyl)oxy)-6,7-dihydro-5H-benzo [f]pyrrolo[1,2-d][1,4]diazepine-2-carboxamide **68** (50 mg, 87.26 µmol) was added to a formic acid (1 mL) solution, and the mixture was heated to 100°C and stirred for 4 hours. The reaction solution was concentrated under reduced pressure. The resulting residue was separated and purified by preparative liquid chromatography (column: AKZONOBEL Kromasil; 250×21.2 mm I.D.; 5 µm, 20 mL/min; mobile phase A: 0.05% TFA+H₂O, and mobile phase B: CH₃CN) to obtain N-(3-chloro-5-(methylsulfonamido)phenyl)-11-((3,5-difluorobenzyl)oxy)-7-formyl-6,7-dihydro-5H-benzo[f]pyrrolo[1,2-d][1,4]diazepine-2-carboxamide **69** (14 mg), with a yield of 26.4%.

MS m/z (ESI): 603.0 [M+1].

¹H NMR (400 MHz, DMSO-*d*₆) δ 10.00 (s, 1H), 9.85 (s, 1H), 8.09 (s, 1H), 7.77 (s, 1H), 7.67 (s, 1H), 7.63 (s, 1H), 7.44 (t, *J =* 8.1 Hz, 1H), 7.28 (d, *J =* 8.5 Hz, 1H), 7.15 (d, *J =* 7.0 Hz, 3H), 7.10 - 7.00 (m, 2H), 6.90 (s, 1H), 5.27 (s, 2H), 4.20 (s, 2H), 4.02 (s, 2H), 3.06 (s, 3H).

### Example 70

### 7-acetyl-N-(3-chloro-5-(methylsulfonamido)phenyl)-11-((3,5-difluorobenzyl)oxy)-6,7-dihydro-5 H-benzo[f]pyrrolo[1,2-d][1,4]diazepine-2-carboxamide

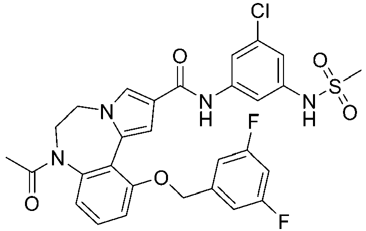

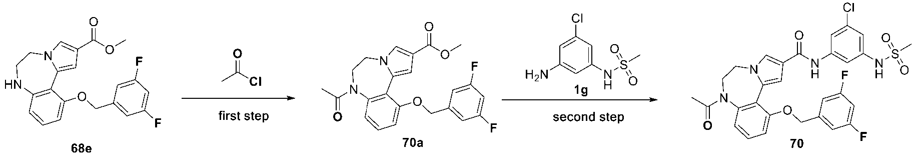

### First step

### methyl 7-acetyl-11-((3,5-difluorobenzyl)oxy)-6,7-dihydro-5H-benzo[f]pyrrolo[1,2-d][1,4]diazepine-2-ca rboxylate

In an ice bath, acetyl chloride (15 mg, 195.12 µmol) was added dropwise to a solution of methyl
11-((3,5-difluorobenzyl)oxy)-6,7-dihydro-5H-benzo[f]pyrrolo[1,2-d][1,4]diazepine-2-carboxylat e **68e** (50 mg, 130.08 µmol) and N,N-diisopropylethylamine (50 mg, 390.24 µmol) in dichloromethane (2 mL), and the mixture was stirred at room temperature for 2 hours. The reaction solution was concentrated under reduced pressure. The residue was purified by silica gel column chromatography (eluent: system A) to obtain methyl 7-acetyl-11-((3,5-difluorobenzyl)oxy)-6,7-dihydro-5H-benzo[f]pyrrolo[1,2-d][1,4]diazepine-2-ca rboxylate **70a** (50 mg), with a yield of 90.9%.

MS m/z (ESI): 427.1 [M+1].

### Second step

### 7-acetyl-N-(3-chloro-5-(methylsulfonamido)phenyl)-11-((3,5-difluorobenzyl)oxy)-6,7-dihydro-5 H-benzo[f]pyrrolo[1,2-d][1,4]diazepine-2-carboxamide

At room temperature, trimethylaluminum (85 mg, 1.17 mmol) was added dropwise to a solution of N-(3-amino-5-chlorophenyl)methanesulfonamide **1g** (31 mg, 140.71 µmol) and methyl
7-acetyl-11-((3,5-difluorobenzyl)oxy)-6,7-dihydro-5H-benzo[f]pyrrolo[1,2-d][1,4]diazepine-2-ca rboxylate **70a** (50 mg, 117.26 µmol) in tetrahydrofuran (3 mL). The mixture was subjected to argon purging, and then heated, refluxed and stirred for 2 hours. A dilute hydrochloric acid solution (2 mL, 1 M) was slowly added dropwise to the reaction solution to quench the reaction, and ethyl acetate was added for extraction (5 mL×3). The organic phases were combined, washed with a saturated sodium chloride solution (5 mL×2), dried with anhydrous sodium sulfate, filtered, and concentrated under reduced pressure. The resulting residue was separated and purified by preparative liquid chromatography (column: AKZONOBEL Kromasil; 250×21.2 mm I.D.; 5 µm, 20 mL/min; mobile phase A: 0.05% TFA+H₂O, and mobile phase B: CH₃CN) to obtain
7-acetyl-N-(3-chloro-5-(methylsulfonamido)phenyl)-11-((3,5-difluorobenzyl)oxy)-6,7-dihydro-5 H-benzo[f]pyrrolo[1,2-d][1,4]diazepine-2-carboxamide **70** (35 mg), with a yield of 47.1%.

MS m/z (ESI): 615.1 [M+1].

¹H NMR (400 MHz, DMSO-*d*₆) δ 9.98 (s, 1H), 9.83 (s, 1H), 7.74 (d, *J =* 1.5 Hz, 1H), 7.69 (t, *J* = 1.6 Hz, 1H), 7.65 - 7.58 (m, 1H), 7.45 (t, *J =* 8.1 Hz, 1H), 7.28 (d, *J =* 8.4 Hz, 1H), 7.18 - 7.06 (m, 5H), 6.89 (t, *J* = 1.8 Hz, 1H), 5.35 (d, *J* = 13.3 Hz, 1H), 5.21 (d, *J* = 13.3 Hz, 1H), 4.88 (td, *J* = 12.8, 6.0 Hz, 1H), 4.43 - 4.26 (m, 1H), 3.82 - 3.65 (m, 1H), 3.36 (dd, *J =* 13.1, 5.0 Hz, 1H), 3.06 (s, 3H), 1.53 (s, 3H).

### Example 71

### N-(3-chloro-5-(methylsulfonamido)phenyl)-11-((3,5-difluorobenzyl)oxy)-7-methyl-6,7-dih ydro-5H-benzo[f]pyrrolo[1,2-d][1,4]diazepine-2-carboxamide

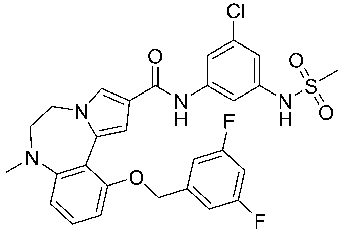

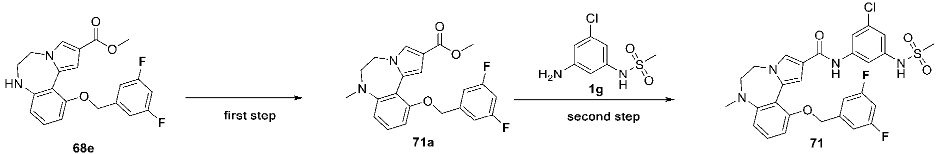

### First step

### methyl 11-((3,5-difluorobenzyl)oxy)-7-methyl-6,7-dihydro-5H-benzo[f]pyrrolo[1,2-d][1,4]diazepine-2-c arboxylate

At room temperature, iodomethane (52 mg, 364.23 µmol) was added dropwise to a solution of methyl 11-((3,5-difluorobenzyl)oxy)-6,7-dihydro-5H-benzo[f]pyrrolo[1,2-d][1,4]diazepine-2-carboxylat e **68e** (70 mg, 182.11 µmol) and potassium carbonate (75 mg, 546.34 µmol) in N,N-dimethylformamide (3 mL), and the mixture was stirred at room temperature for 6 hours. The reaction solution was filtered, and the filtrate was concentrated under reduced pressure. The residue was purified by silica gel column chromatography (eluent: system A) to obtain methyl 11-((3,5-difluorobenzyl)oxy)-7-methyl-6,7-dihydro-5H-benzo[f]pyrrolo[1,2-d][1,4]diazepine-2-c arboxylate **71a** (61 mg), with a yield of 84.1%.

MS m/z (ESI): 399.1 [M+1].

### Second step

### N-(3-chloro-5-(methylsulfonamido)phenyl)-11-((3,5-difluorobenzyl)oxy)-7-methyl-6,7-dihydro-5H-benzo[f]pyrrolo[1,2-d][1,4]diazepine-2-carboxamide

At room temperature, trimethylaluminum (127 mg, 1.76 mmol) was added dropwise to a solution of N-(3-amino-5-chlorophenyl)methanesulfonamide **1g** (47mg, 210.84 µmol) and methyl
11-((3,5-difluorobenzyl)oxy)-7-methyl-6,7-dihydro-5H-benzo[f]pyrrolo[1,2-d][1,4]diazepine-2-c arboxylate **71a** (70 mg, 175.70 µmol) in tetrahydrofuran (10 mL). The mixture was subjected to argon purging, and then heated, refluxed and stirred for 2 hours. A dilute hydrochloric acid solution (2 mL, 1 M) was slowly added dropwise to the reaction solution to quench the reaction, and ethyl acetate was added for extraction (10 mL×3). The organic phases were combined, washed with saturated sodium chloride solution (10 mL×2), dried with anhydrous sodium sulfate, filtered, and concentrated under reduced pressure. The resulting residue was separated and purified by preparative liquid chromatography (column: AKZONOBEL Kromasil; 250×21.2 mm I.D.; 5 µm, 20 mL/min; mobile phase A: 0.05% TFA+H₂O, and mobile phase B: CH₃CN) to obtain
N-(3-chloro-5-(methylsulfonamido)phenyl)-11-((3,5-difluorobenzyl)oxy)-7-methyl-6,7-dihydro-5H-benzo[f]pyrrolo[1,2-d][1,4]diazepine-2-carboxamide **71** (25 mg), with a yield of 24.2%.

MS m/z (ESI): 587.1 [M+1].

¹H NMR (400 MHz, DMSO-*d*₆) δ 9.97 (s, 1H), 9.76 (s, 1H), 7.74 - 7.68 (m, 2H), 7.67 - 7.62 (m, 1H), 7.27 (t, *J* = 8.2 Hz, 1H), 7.12 (t, *J* = 7.3 Hz, 3H), 6.94 (d, *J =* 1.6 Hz, 1H), 6.89 (t, *J* = 1.7 Hz, 1H), 6.84 (d, *J =* 8.3 Hz, 1H), 6.78 (d, *J* = 8.1 Hz, 1H), 5.17 (s, 2H), 4.02 (t, *J =* 5.8 Hz, 2H), 3.37 (t, *J* = 5.9 Hz, 2H), 3.06 (s, 3H), 2.71 (s, 3H).

### Example 72

### N-(3-chloro-5-(methylsulfonamido)phenyl)-7-cyclopropyl-11-((3,5-difluorobenzyl)oxy)-6,7-dih ydro-5H-benzo[f]pyrrolo[1,2-d][1,4]diazepine-2-carboxamide

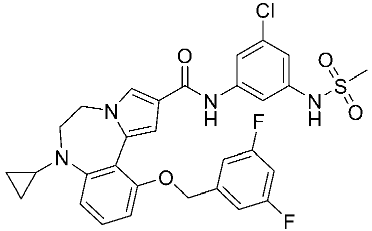

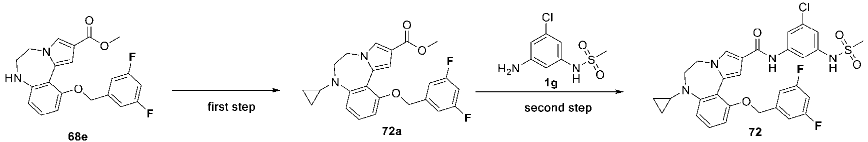

### First step

### methyl 7-cyclopropyl-11-((3,5-difluorobenzyl)oxy)-6,7-dihydro-5H-benzo[f]pyrrolo[1,2-d][1,4]diazepin e-2-carboxylate

At room temperature, (1-ethoxycyclopropoxy)-trimethylsilane (79 mg, 455.28 µmol) was added to a solution of methyl 11-((3,5-difluorobenzyl)oxy)-6,7-dihydro-5H-benzo[f]pyrrolo[1,2-d][1,4]diazepine-2-carboxylat e **68e** (50 mg, 130.08 µmol) and acetic acid (78 mg, 1.30 mmol) in methanol (3 mL), the mixture was stirred at room temperature for 10 minutes, and then sodium borohydride (41 mg, 650.41 µmol) was added. The mixture was stirred at room temperature for 30 minutes, and then heated to 60°C and stirred for 16 hours. The reaction solution was concentrated under reduced pressure. The residue was purified by silica gel column chromatography (eluent: system A) to obtain methyl
7-cyclopropyl-11-((3,5-difluorobenzyl)oxy)-6,7-dihydro-5H-benzo[f]pyrrolo[1,2-d][1,4]diazepin e-2-carboxylate **72a** (48 mg), with a yield of 86.9%.

MS m/z (ESI): 425.1 [M+1].

### Second step

### N-(3-chloro-5-(methylsulfonamido)phenyl)-7-cyclopropyl-11-((3,5-difluorobenzyl)oxy)-6,7-dih ydro-5H-benzo[f]pyrrolo[1,2-d][1,4]diazepine-2-carboxamide

At room temperature, trimethylaluminum (87 mg, 1.21 mmol) was added dropwise to a solution of N-(3-amino-5-chlorophenyl)methanesulfonamide **1g** (32 mg, 145.49 µmol) and methyl
7-cyclopropyl-11-((3,5-difluorobenzyl)oxy)-6,7-dihydro-5H-benzo[f]pyrrolo[1,2-d][1,4]diazepin e-2-carboxylate **72a** (51.46 mg, 121.25 µmol) in tetrahydrofuran (3 mL). The mixture was subjected to argon purging, and then heated, refluxed and stirred for 2 hours. The reaction solution was slowly added dropwise to a dilute hydrochloric acid solution (2 mL, 1 M) to quench the reaction, and ethyl acetate was added for extraction (5 mL×3). The organic phases were combined, washed with a saturated sodium chloride solution (5 mL×2), dried with anhydrous sodium sulfate, filtered, and concentrated under reduced pressure. The resulting residue was separated and purified by preparative liquid chromatography (column: AKZONOBEL Kromasil; 250×21.2 mm I.D.; 5 µm, 20 mL/min; mobile phase A: 0.05% TFA+H₂O, and mobile phase B: CH₃CN) to obtain N-(3-chloro-5-(methylsulfonamido)phenyl)-7-cyclopropyl-11-((3,5-difluorobenzyl)oxy)-6,7-dih ydro-5H-benzo[f]pyrrolo[1,2-d][1,4]diazepine-2-carboxamide **72** (25 mg), with a yield of 28.2%.

MS m/z (ESI): 613.2 [M+1].

¹H NMR (400 MHz, DMSO-*d*₆) δ 9.99 (s, 1H), 9.77 (s, 1H), 7.66 (d, *J* = 19.8 Hz, 3H), 7.27 (t, *J* = 7.6 Hz, 1H), 7.11 (s, 4H), 6.90 (s, 2H), 6.82 (d, *J* = 7.9 Hz, 1H), 5.15 (s, 2H), 4.68 (s, 1H), 4.03 (s, 2H), 3.52 (s, 2H), 3.06 (s, 3H), 0.63 (s, 2H), 0.11 (s, 2H).

### Example 73

### N-(3-chloro-5-(methylsulfonamido)phenyl)-11-((3,5-difluorobenzyl)oxy)-5,6-dihydrobenzo[f]py rrolo[1,2-d][1,4]oxazepine-2-carboxamide

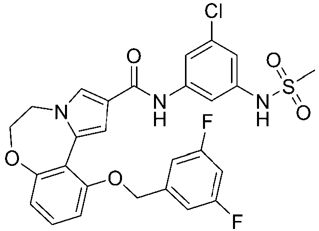

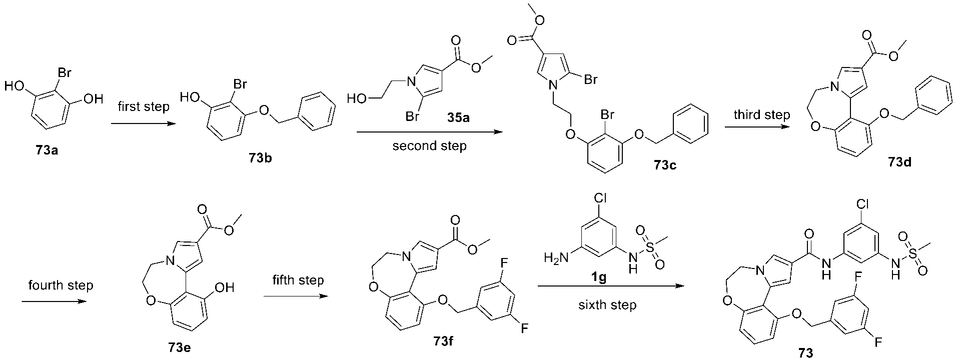

### First step

### 3-(benzyloxy)-2-bromophenol

At room temperature, 2-bromobenzene-1,3-diol **73a** (1.00 g, 5.29 mmol) was dissolved in acetonitrile (10 mL), then potassium carbonate (1.46 g, 10.58 mmol) and benzyl bromide (542.95 mg, 3.17 mmol) were added sequentially, and the mixture was allowed to react at 25°C for 2 hours. The reaction solution was filtered and concentrated under reduced pressure. The residue was purified by silica gel column chromatography (eluent: system A) to obtain 3-(benzyloxy)-2-bromophenol **73b** (533 mg), with a yield of 36.1%.

MS m/z (ESI): 279.0/281.0 [M+1].

### Second step

### methyl 1-(2-(3-(benzyloxy)-2-bromophenoxy)ethyl)-5-bromo-1H-pyrrole-3-carboxylate

At room temperature, methyl 5-bromo-1-(2-hydroxyethyl)-1H-pyrrole-3-carboxylate **35a** (200.0 mg, 0.81 mmol) was dissolved in a toluene (2 mL) solution, and 3-(benzyloxy)-2-bromophenol **73b** (247.5 mg, 0.89 mmol) and cyanomethylene tri-n-butyl phosphorane (583.7 mg, 2.42 mmol) were added sequentially. The mixture was subjected to nitrogen purging for three times and then allowed to react at 80°C for 18 hours. The reaction solution was filtered and concentrated under reduced pressure. The residue was purified by silica gel column chromatography (eluent: system A) to obtain methyl 1-(2-(3-(benzyloxy)-2-bromophenoxy)ethyl)-5-bromo-1H-pyrrole-3-carboxylate 73c (150 mg), with a yield of 36.5%.

MS m/z (ESI): 508.0/510.0/512.0 [M+1].

### Third step

### methyl 11-(benzyloxy)-5,6-dihydrobenzo[f]pyrrolo[1,2-d][1,4]oxazepine-2-carboxylate

At room temperature, compound **73c** (150.0 mg, 0.29 mmol) was dissolved in a solution of 1,2-dimethoxyethane (4 mL) and water (0.5 mL), and potassium carbonate (122.0 mg, 0.88 mmol), bis(pinacolato)diboron (224.4 mg, 0.88 mmol), palladium(II) acetate (13.3 mg, 0.06 mmol) and di(1-adamantyl)-n-butylphosphine (42.2 mg, 0.12 mmol) were added sequentially. The mixture was subjected to nitrogen purging for three times and then allowed to react at 80°C for 18 hours. The reaction solution was filtered and concentrated under reduced pressure. The residue was purified by silica gel column chromatography (eluent: system A) to obtain methyl 11-(benzyloxy)-5,6-dihydrobenzo[f]pyrrolo[1,2-d][1,4]oxazepine-2-carboxylate **73d** (72 mg), with a yield of 69.9%.

MS m/z (ESI): 350.0 [M+1].

### Fourth step

### methyl 11-hydroxy-5,6-dihydrobenzo[f]pyrrolo[1,2-d][1,4]oxazepine-2-carboxylate

Methyl 11-(benzyloxy)-5,6-dihydrobenzo[f]pyrrolo[1,2-d][1,4]oxazepine-2-carboxylate **73d** (800 mg, 2.29 mmol) was dissolved in methanol (20 mL), and palladium on carbon (10%) (1.00 g, 9.40 mmol) was added to the resulting solution. The reaction solution was subjected to hydrogen purging for three times and stirred at room temperature under a hydrogen atmosphere for 12 hours. The resulting mixture was filtered under reduced pressure, and the filter cake was washed with methanol (50 mL × 3). The resulting filtrates were concentrated under reduced pressure, then petroleum ether/ethyl acetate (30 mL, 3: 1) were added, and the mixture was stirred for 30 minutes and filtered. The filter cake was dried to obtain methyl 11-hydroxy-5,6-dihydrobenzo[f]pyrrolo[1,2-d][1,4]oxazepine-2-carboxylate **73e** (300 mg), with a yield of 50.5%.

MS m/z (ESI): 260.2 [M+1].

### Fifth step

### methyl 11-((3,5-difluorobenzyl)oxy)-5,6-dihydrobenzo[f]pyrrolo[1,2-d][1,4]oxazepine-2-carboxylate

Methyl 11-hydroxy-5,6-dihydrobenzo[f]pyrrolo[1,2-d][1,4]oxazepine-2-carboxylate **73e** (2.0 g, 7.71 mmol) was dissolved in tetrahydrofuran (40 mL), and cesium carbonate (5.0 g, 15.43 mmol) and 1-(chloromethyl)-3,5-difluorobenzene (2.5 g, 15.43 mmol, 1.94 mL) were added to the resulting solution. The reaction solution was stirred at 60°C for 12 hours. The resulting mixture was filtered under reduced pressure, and the filter cake was washed with methanol (5 mL × 3). Petroleum ether/ethyl acetate (30 mL, 10: 1) were added, and the mixture was stirred for 30 minutes and filtered. The filter cake was dried to obtain methyl 11-((3,5-difluorobenzyl)oxy)-5,6-dihydrobenzo[f]pyrrolo[1,2-d][1,4]oxazepine-2-carboxylate **73f** (2.8 g), with a yield of 94.2%.

MS m/z (ESI): 386.2 [M+1].

### Sixth step

### N-(3-chloro-5-(methylsulfonamido)phenyl)-11-((3,5-difluorobenzyl)oxy)-5,6-dihydrobenzo[f]py rrolo[1,2-d][1,4]oxazepine-2-carboxamide

Methyl 11-((3,5-difluorobenzyl)oxy)-5,6-dihydrobenzo[f]pyrrolo[1,2-d][1,4]oxazepine-2-carboxylate **73f** (200 mg, 519.00 µmol) and N-(3-amino-5-chlorophenyl)methanesulfonamide **1g** (229.1 mg, 1.04 mmol) were dissolved in tetrahydrofuran (4 mL), and trimethylaluminum (2 M, 2.08 µL) was slowly added dropwise to the resulting mixture. The reaction solution was stirred at 60°C for 2 hours. The reaction solution was slowly added dropwise to methanol (10 mL) to quench the reaction. The resulting mixture was concentrated under reduced pressure. The crude product was purified by silica gel column chromatography (eluent: system A), and further separated by preparative liquid chromatography (separation column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-11-((3,5-difluorobenzyl)oxy)-5,6-dihydrobenzo[f]py rrolo[1,2-d][1,4]oxazepine-2-carboxamide **73** (80 mg), with a yield of 26.8%.

MS m/z (ESI): 574.2 [M+1].

¹H NMR (400 MHz, DMSO-*d₆*) δ 10.00 (s, 1H), 9.84 (s, 1H), 7.77 (d, *J* = 1.6 Hz, 1H), 7.70 (t, *J* = 1.6 Hz, 1H), 7.65 (t, *J* = 1.6 Hz, 1H), 7.29 (t, *J* = 8.0 Hz, 1H), 7.18 - 7.13 (m, 3H), 7.08 (d, *J* = 1.6 Hz, 1H), 6.99 (dd, *J* = 8.0, 1.2 Hz, 1H), 6.90 (t, *J* = 2.0 Hz, 1H), 6.83 (dd, *J =* 8.0, 0.4 Hz, 1H), 5.24 (s, 2H), 4.44 (t, *J* = 5.6 Hz, 2H), 4.16 (t, *J* = 5.6 Hz, 2H), 3.06 (s, 3H).

### Example 74

### N-(3-chloro-5-(methylsulfonamido)phenyl)-11-((3-cyano-5-fluorobenzyl)oxy)-5,6-dihydrobenzo [f]pyrrolo[1,2-d][1,4]oxazepine-2-carboxamide

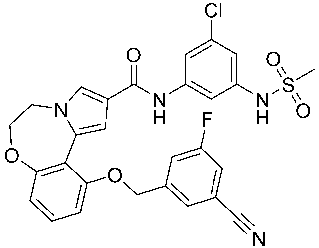

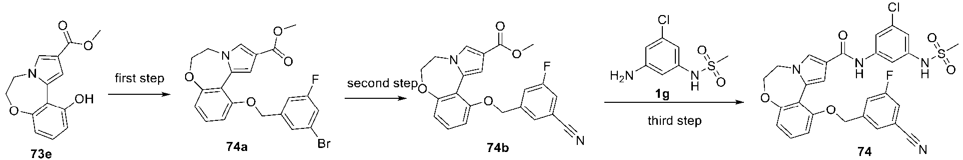

### First step

### methyl 11-((3-bromo-5-fluorobenzyl)oxy)-5,6-dihydrobenzo[f]pyrrolo[1,2-d][1,4]oxazepine-2-carboxyl ate

Methyl 11-hydroxy-5,6-dihydrobenzo[f]pyrrolo[1,2-d][1,4]oxazepine-2-carboxylate **73e** (100 mg, 385.72 µmol) was dissolved in tetrahydrofuran (3 mL), and cesium carbonate (251.35 mg, 771.44 µmol) and 3-bromo-5-fluorobenzyl bromide (206.68 mg, 771.44 µmol, commercially available) were added to the resulting solution. The reaction solution was stirred at 60°C for 3 hours. The resulting mixture was filtered under reduced pressure, and the filter cake was washed with dichloromethane (20 mL × 3). Petroleum ether/ethyl acetate (30 mL, 3: 1) were added to the resulting filtrates, and the mixture was stirred for 30 minutes and filtered. The filter cake was dried to obtain methyl 11-((3-bromo-5-fluorobenzyl)oxy)-5,6-dihydrobenzo[f]pyrrolo[1,2-d][1,4]oxazepine-2-carboxyl ate **74a** (160 mg), with a yield of 92.9%.

MS m/z (ESI): 446.0/448.0 [M+1].

### Second step

### methyl 11-((3-cyano-5-fluorobenzyl)oxy)-5,6-dihydrobenzo[f]pyrrolo[1,2-d][1,4]oxazepine-2-carboxyla te

Methyl 11-((3-bromo-5-fluorobenzyl)oxy)-5,6-dihydrobenzo[f]pyrrolo[1,2-d][1,4]oxazepine-2-carboxyl ate **74a** (80 mg, 179.27 µmol) was dissolved in *N*,*N*-dimethylformamide (2 mL), and zinc cyanide (63.15 mg, 537.80 µmol) and tetrakis(triphenylphosphine)palladium (41.43 mg, 35.85 µmol) were added to the resulting solution. The reaction solution was subjected to nitrogen purging three times and stirred at 90°C under nitrogen protection for 3 hours. The resulting mixture was diluted with water (20 mL) and extracted with ethyl acetate (15 mL × 2). The extracts were allowed to stand, and then a solid was precipitated, filtered and dried to obtain methyl
11-((3-cyano-5-fluorobenzyl)oxy)-5,6-dihydrobenzo[f]pyrrolo[1,2-d][1,4]oxazepine-2-carboxyla te **74b** (50 mg), with a yield of 71.1%.

MS m/z (ESI): 393.2 [M+1].

### Third step

### N-(3-chloro-5-(methylsulfonamido)phenyl)-11-((3-cyano-5-fluorobenzyl)oxy)-5,6-dihydrobenzo [f]pyrrolo[1,2-d][1,4]oxazepine-2-carboxamide

Methyl
11-((3-cyano-5-fluorobenzyl)oxy)-5,6-dihydrobenzo[f]pyrrolo[1,2-d][1,4]oxazepine-2-carboxyla te **74b** (40 mg, 101.94 µmol) and *N*-(3-amino-5-chlorophenyl)methanesulfonamide **1g** (44.99 mg, 203.88 µmol) were dissolved in tetrahydrofuran (1 mL), and trimethylaluminum (2 M, 407.77 µL) was slowly added dropwise to the resulting mixture. The reaction solution was stirred at 60°C for 2 hours. The reaction solution was slowly added dropwise to methanol (10 mL) to quench the reaction. The resulting mixture was concentrated under reduced pressure. The crude product was purified by silica gel column chromatography (eluent: system A), and further separated by preparative liquid chromatography (separation column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-11-((3-cyano-5-fluorobenzyl)oxy)-5,6-dihydrobenzo [f]pyrrolo[1,2-d][1,4]oxazepine-2-carboxamide **74** (6 mg), with a yield of 10.1%.

MS m/z (ESI): 581.1 [M+1].

¹H NMR (400 MHz, DMSO-*d₆*) δ 9.95 (s, 1H), 9.81 (s, 1H), 7.80 - 7.77 (m, 1H), 7.76 (d, *J* = 1.6 Hz, 1H), 7.73 (t, *J* = 1.6 Hz, 1H), 7.69 (t, *J =* 1.6 Hz, 1H), 7.67 - 7.64 (m, 1H), 7.61 (t, *J* = 1.6 Hz, 1H), 7.30 (t, *J* = 8.0 Hz, 1H), 7.09 (d, *J* = 1.6 Hz, 1H), 7.00 (dd, *J =* 8.0, 0.8 Hz, 1H), 6.88 (t, *J* = 2.0 Hz, 1H), 6.84 (dd, *J* = 8.0, 0.8 Hz, 1H), 5.28 (s, 2H), 4.44 (t, *J* = 6.0 Hz, 2H), 4.16 (t, *J* = 6.0 Hz, 2H), 3.04 (s, 3H).

### Example 75

### N-(3-chloro-5-(methylsulfonamido)phenyl)-11-((3-(dimethylamino)-5-fluorobenzyl)oxy)-5,6-dih ydrobenzo[f]pyrrolo[1,2-d][1,4]oxazepine-2-carboxamide

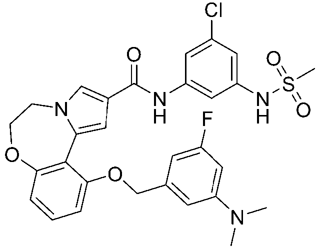

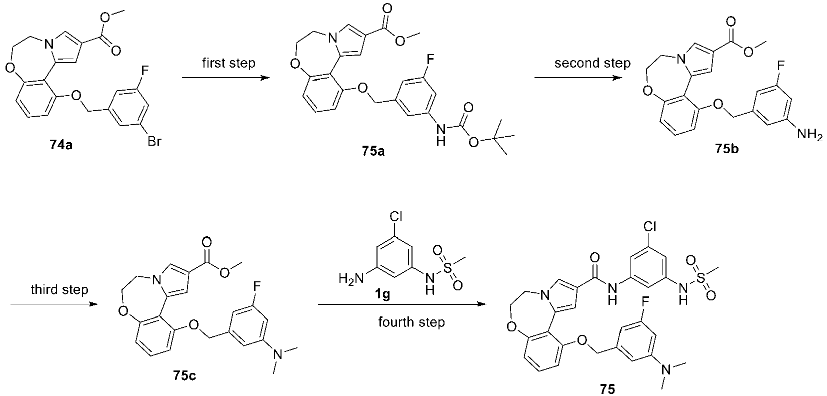

### First step

### methyl 11-((3-((tert-butoxycarbonyl)amino)-5-fluorobenzyl)oxy)-5,6-dihydrobenzo[f]pyrrolo[1,2-d][1,4 ]oxazepine-2-carboxylate

Methyl 11-((3-bromo-5-fluorobenzyl)oxy)-5,6-dihydrobenzo[f]pyrrolo[1,2-d][1,4]oxazepine-2-carboxyl ate **74a** (60 mg, 134.45 µmol) and tert-butyl carbamate (47.25 mg, 403.35 µmol) were dissolved in 1,4-dioxane (2 mL), and cesium carbonate (131.42 mg, 403.35 µmol), tris(dibenzylideneacetone)dipalladium (12.31 mg, 13.44 µmol) and 4,5-bis(diphenylphosphino)-9,9-dimethylxanthene (15.56 mg, 26.89 µmol) were added to the resulting solution. The reaction solution was subjected to nitrogen purging for three times and stirred at 100°C under nitrogen protection for 3 hours. The resulting mixture was filtered under reduced pressure, and the filter cake was washed with dichloromethane (10 mL × 2). The filtrates were concentrated under reduced pressure. The residue was purified by silica gel column chromatography (eluent: system A) to obtain methyl 11-((3-((tert-butoxycarbonyl)amino)-5-fluorobenzyl)oxy)-5,6-dihydrobenzo[f]pyrrolo[1,2-d][1,4 ]oxazepine-2-carboxylate **75a** (60 mg), with a yield of 92.5%.

MS m/z (ESI): 483.2 [M+1].

### Second step

### methyl 11-((3-amino-5-fluorobenzyl)oxy)-5,6-dihydrobenzo[f]pyrrolo[1,2-d][1,4]oxazepine-2-carboxyla te

Methyl 11-((3-((tert-butoxycarbonyl)amino)-5-fluorobenzyl)oxy)-5,6-dihydrobenzo[f]pyrrolo[1,2-d][1,4 ]oxazepine-2-carboxylate **75a** (60 mg, 124.35 µmol) was dissolved in dichloromethane (1 mL), and trifluoroacetic acid (14.18 mg, 124.35 µmol, 0.5 mL) was added to the resulting solution. The reaction solution was stirred at 25°C for 1 hour. The resulting mixture was concentrated under reduced pressure to obtain methyl 11-((3-amino-5-fluorobenzyl)oxy)-5,6-dihydrobenzo[f]pyrrolo[1,2-d][1,4]oxazepine-2-carboxyla te **75b,** which was used directly in the next step of synthesis.

MS m/z (ESI): 383.2 [M+1].

### Third step

### methyl 11-((3-(dimethylamino)-5-fluorobenzyl)oxy)-5,6-dihydrobenzo[f]pyrrolo[1,2-d][1,4]oxazepine-2 -carboxylate

Methyl 11-((3-amino-5-fluorobenzyl)oxy)-5,6-dihydrobenzo[f]pyrrolo[1,2-d][1,4]oxazepine-2-carboxyla te **75b** was dissolved in *N*,*N*-dimethylformamide (2 mL), and potassium carbonate (65.06 mg, 470.73 µmol) and iodomethane (222.72 mg, 1.57 mmol, 97.68 µL) were added to the resulting solution. The reaction solution was stirred at 25°C for 12 hours. The resulting mixture was filtered under reduced pressure, and the filter cake was washed with dichloromethane (5 mL × 2). The filtrates were concentrated under reduced pressure. The residue was separated and purified by silica gel column chromatography (eluent: system A) to obtain methyl 11-((3-(dimethylamino)-5-fluorobenzyl)oxy)-5,6-dihydrobenzo[f]pyrrolo[1,2-d][1,4]oxazepine-2 -carboxylate **75c** (20 mg), with a yield of 39.2%.

MS m/z (ESI): 411.2 [M+1].

### Fourth step

### N-(3-chloro-5-(methylsulfonamido)phenyl)-11-((3-(dimethylamino)-5-fluorobenzyl)oxy)-5,6-dih ydrobenzo[f]pyrrolo[1,2-d][1,4]oxazepine-2-carboxamide

Methyl 11-((3-(dimethylamino)-5-fluorobenzyl)oxy)-5,6-dihydrobenzo[f]pyrrolo[1,2-d][1,4]oxazepine-2 -carboxylate **75c** (20 mg, 48.73 µmol) and *N*-(3-amino-5-chlorophenyl)methanesulfonamide **1g** (21.51 mg, 97.46 µmol) were dissolved in tetrahydrofuran (1 mL), and trimethylaluminum (2 M, 194.91 µL) was slowly added dropwise to the resulting mixture. The reaction solution was stirred at 60°C for 2 hours. The reaction solution was slowly added dropwise to methanol (5 mL) to quench the reaction. The resulting solution was concentrated under reduced pressure. The residue was purified by silica gel column chromatography (eluent: system A) to obtain N-(3-chloro-5-(methylsulfonamido)phenyl)-11-((3-(dimethylamino)-5-fluorobenzyl)oxy)-5,6-dih ydrobenzo[f]pyrrolo[1,2-d][1,4]oxazepine-2-carboxamide 75 (9 mg), with a yield of 30.8%.

MS m/z (ESI): 599.1 [M+1].

¹H NMR (400 MHz, DMSO-*d₆*) δ 10.00 (s, 1H), 9.84 (s, 1H), 7.76 (d, *J =* 1.6 Hz, 1H), 7.69 (t, *J* = 1.6 Hz, 1H), 7.64 (t, *J* = 1.6 Hz, 1H), 7.28 (t, *J =* 8.0 Hz, 1H), 7.06 (d, *J* = 1.6 Hz, 1H), 7.01 (d, *J* = 8.4 Hz, 1H), 6.89 (t, *J =* 2.0 Hz, 1H), 6.81 (d, *J* = 8.0 Hz, 1H), 6.63 (s, 1H), 6.45 (d, *J* = 8.8 Hz, 1H), 6.37 (dt, *J* = 12.8, 2.0 Hz, 1H), 5.14 (s, 2H), 4.43 (t, *J* = 6.0 Hz, 2H), 4.16 (t, *J* = 6.0 Hz, 2H), 3.06 (s, 3H), 2.84 (s, 6H).

### Example 76

### 11-((3-amino-5-fluorobenzyl)oxy)-N-(3-chloro-5-(methylsulfonamido)phenyl)-5,6-dihydrobenzo [f]pyrrolo[1,2-d][1,4]oxazepine-2-carboxamide

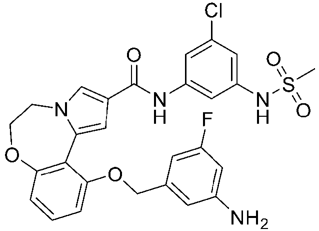

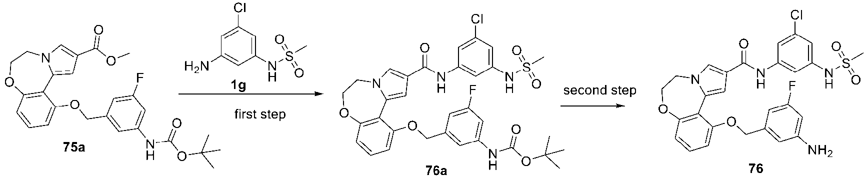

### First step

### tert-butyl (3-(((2-((3-chloro-5-(methylsulfonamido)phenyl)carbamoyl)-5,6-dihydrobenzo[f]pyrrolo[1,2-d][ 1,4]oxazepin-11-yl)oxy)methyl)-5-fluorophenyl)carbamate

Methyl 11-((3-((tert-butoxycarbonyl)amino)-5-fluorobenzyl)oxy)-5,6-dihydrobenzo[f]pyrrolo[1,2-d][1,4 ]oxazepine-2-carboxylate **75a** (60 mg, 124.35 µmol) and N-(3-amino-5-chlorophenyl)methanesulfonamide **1g** (54.88 mg, 248.70 µmol) were dissolved in tetrahydrofuran (1.5 mL), and trimethylaluminum (2 M, 497.41 µL) was slowly added dropwise to the resulting mixture. The reaction solution was stirred at 60°C for 2 hours. The reaction solution was slowly added dropwise to methanol (10 mL) to quench the reaction. The resulting solution was concentrated under reduced pressure. The residue was purified by silica gel column chromatography (eluent: system A) to obtain tert-butyl (3-(((2-((3-chloro-5-(methylsulfonamido)phenyl)carbamoyl)-5,6-dihydrobenzo[f]pyrrolo[1,2-d][ 1,4]oxazepin-11-yl)oxy)methyl)-5-fluorophenyl)carbamate **76a** (30 mg), with a yield of 35.9%. MS m/z (ESI): 693.2 [M+23].

### Second step

### 11-((3-amino-5-fluorobenzyl)oxy)-N-(3-chloro-5-(methylsulfonamido)phenyl)-5,6-dihydrobenzo [f]pyrrolo[1,2-d][1,4]oxazepine-2-carboxamide

Tert-butyl (3-(((2-((3-chloro-5-(methylsulfonamido)phenyl)carbamoyl)-5,6-dihydrobenzo[f]pyrrolo[1,2-d][ 1,4]oxazepin-11-yl)oxy)methyl)-5-fluorophenyl)carbamate **76a** (40 mg, 59.60 µmol) was dissolved in dichloromethane (1 mL), and trifluoroacetic acid (6.80 mg, 59.60 µmol, 0.5 mL) was added to the resulting solution. The reaction solution was stirred at 25°C for 1 hour. The resulting mixture was concentrated under reduced pressure, and then separated by preparative liquid chromatography (separation column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain 11-((3-amino-5-fluorobenzyl)oxy)-N-(3-chloro-5-(methylsulfonamido)phenyl)-5,6-dihydrobenzo [f]pyrrolo[1,2-d][1,4]oxazepine-2-carboxamide **76** (10 mg), with a yield of 29.3%.

MS m/z (ESI): 571.1 [M+1].

¹H NMR (400 MHz, DMSO-*d₆*) δ 10.00 (s, 1H), 9.85 (s, 1H), 7.76 (d, *J =* 1.6 Hz, 1H), 7.71 (t, *J* = 1.6 Hz, 1H), 7.63 (t, *J =* 1.6 Hz, 1H), 7.25 (t, *J =* 8.0 Hz, 1H), 7.03 (d, *J =* 1.6 Hz, 1H), 6.94 (dd, *J =* 8.0, 0.4 Hz, 1H), 6.89 (t, *J =* 2.0 Hz, 1H), 6.79 (dd, *J =* 8.0, 0.4 Hz, 1H), 6.46 (t, *J =* 1.6 Hz, 1H), 6.33 (dt, *J =* 8.8, 2.4 Hz, 1H), 6.22 (dt, *J=* 11.6, 2.0 Hz, 1H), 5.41 (s, 2H), 5.06 (s, 2H), 4.43 (t, *J=* 5.6 Hz, 2H), 4.16 (t, *J =* 5.6 Hz, 2H), 3.05 (s, 3H).

### Example 77

### N-(3-chloro-5-(methylsulfonamido)phenyl)-11-((3,3-difluorocyclopentyl)methoxy)-5,6-dihydrob enzo[f]pyrrolo[1,2-d][1,4]oxazepine-2-carboxamide

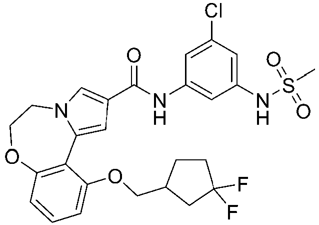

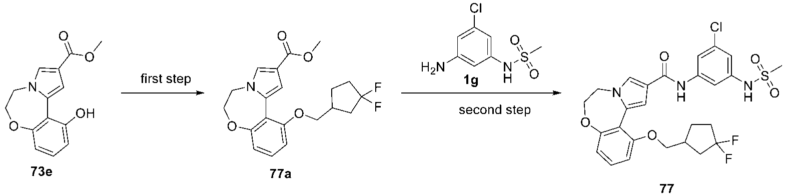

### First step

### methyl 11-((3,3-difluorocyclopentyl)methoxy)-5,6-dihydrobenzo[f]pyrrolo[1,2-d][1,4]oxazepine-2-carb oxylate

Methyl 11-hydroxy-5,6-dihydrobenzo[f]pyrrolo[1,2-d][1,4]oxazepine-2-carboxylate 73e (30 mg, 115.72 µmol) and (3,3-difluorocyclopentyl)methanol (31.51 mg, 231.43 µmol) were dissolved in toluene (1 mL), and cyanomethylene tri-n-butyl phosphorane (83.78 mg, 347.15 µmol, 90.97 µL) was added to the resulting solution. The reaction solution was stirred at 80°C for 2 hours. The resulting mixture was concentrated under reduced pressure. The residue was purified by silica gel column chromatography (eluent: system A) to obtain methyl 11-((3,3-difluorocyclopentyl)methoxy)-5,6-dihydrobenzo[f]pyrrolo[1,2-d][1,4]oxazepine-2-carb oxylate **77a** (35 mg), with a yield of 80.1%.

MS m/z (ESI): 378.2 [M+1].

### Second step

### N-(3-chloro-5-(methylsulfonamido)phenyl)-11-((3,3-difluorocyclopentyl)methoxy)-5,6-dihydrob enzo[f]pyrrolo[1,2-d][1,4]oxazepine-2-carboxamide

Methyl 11-((3,3-difluorocyclopentyl)methoxy)-5,6-dihydrobenzo[f]pyrrolo[1,2-d][1,4]oxazepine-2-carb oxylate **77a** (60 mg, 158.99 µmol) and N-(3-amino-5-chlorophenyl)methanesulfonamide **1g** (70.17 mg, 317.98 µmol) were dissolved in tetrahydrofuran (2 mL), and trimethylaluminum (2 M, 635.96 µL) was slowly added dropwise to the resulting mixture. The reaction solution was stirred at 60°C for 2 hours. The reaction solution was slowly added dropwise to methanol (10 mL) to quench the reaction. The resulting mixture was concentrated under reduced pressure. The crude product was purified by silica gel column chromatography (eluent: system A), and further separated by preparative liquid chromatography (separation column: AKZONOBEL Kromasil; 250×21.2 mm I.D.; 5 µm, 20 mL/min; mobile phase A: 0.05% NH₄HCO₃+H₂O, and mobile phase B: CH₃CN) to obtain N-(3-chloro-5-(methylsulfonamido)phenyl)-11-((3,3-difluorocyclopentyl)methoxy)-5,6-dihydrob enzo[f]pyrrolo[1,2-d][1,4]oxazepine-2-carboxamide 77 (25 mg), with a yield of 27.7%.

MS m/z (ESI): 566.1 [M+1].

¹H NMR (400 MHz, DMSO-*d₆*) δ 10.00 (s, 1H), 9.82 (s, 1H), 7.75 (d, *J =* 1.6 Hz, 1H), 7.69 (t, *J* = 1.6 Hz, 1H), 7.63 (t, *J =* 1.6 Hz, 1H), 7.26 (t, *J =* 8.0 Hz, 1H), 6.95 - 6.91 (m, 2H), 6.89 (t, *J =* 2.0 Hz, 1H), 6.79 (dd, *J =* 8.0, 0.8 Hz, 1H), 4.43 (t, *J* = 6.0 Hz, 2H), 4.15 (t, *J* = 6.0 Hz, 2H), 3.99 (d, *J* = 6.4 Hz, 2H), 3.05 (s, 3H), 2.62-2.54 (m, 1H), 2.29 - 1.93 (m, 5H), 1.70-1.60 (m, 1H).

Compounds of Examples 78-85 were prepared according to the preparation method of Example 77 of the present disclosure, and specific structures and structural characterizations were as follows:

| Serial number and structure of example | MS m/z (ESI) | ¹H NMR |
|---|---|---|
| 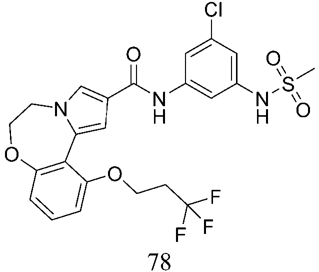 | 544.1[M+1] | ¹H NMR (400 MHz, DMSO-*d₆*) δ 9.99 (s, 1H), 9.78 (s, 1H), 7.75 (d, *J* = 1.7 Hz, 1H), 7.68 (s, 1H), 7.61 (s, 1H), 7.28 (t, *J =* 8.2 Hz, 1H), 6.99 (d, *J* = 8.1 Hz, 1H), 6.94 (d, *J* = 1.7 Hz, 1H), 6.88 (t, *J =* 1.8 Hz, 1H), 6.82 (d, *J* = 8.1 Hz, 1H), 4.43 (t*, J =* 5.8 Hz, 2H), 4.25 (t, *J* = 6.0 Hz, 2H), 4.15 (t, *J* = 5.7 Hz, 2H), 3.04 (s, 3H), 2.90 - 2.75 (m, 2H). |
| 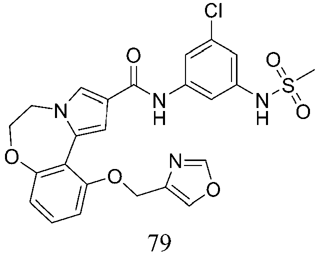 | 529.1[M+1] | ¹H NMR (400 MHz, DMSO-*d₆*) δ 10.00 (s, 1H), 9.83 (s, 1H), 8.40 (d, *J* = 0.8 Hz, 1H), 8.15 (d, *J =* 0.8 Hz, 1H), 7.75 (d, *J* = 1.6 Hz, 1H), 7.71 (t, *J* = 1.6 Hz, 1H), 7.62 (t, *J* = 1.6 Hz, 1H), 7.28 (t*, J =* 8.0 Hz, 1H), 7.12 (d, *J* = 8.0 Hz, 1H), 6.92 (d, *J* = 2.0 Hz, 1H), 6.89 (t, *J* = 2.0 Hz, 1H), 6.81 (dd, *J =* 8.0, 0.8 Hz, 1H), 5.10 (s, 2H), 4.42 (t, *J =* 6.0 Hz, 2H), 4.15 (t, *J =* 6.0 Hz, 2H), 3.06 (s, 3H). |
| 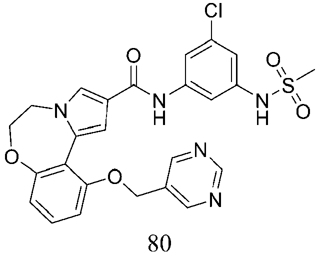 | 540.2[M+1] | ¹H NMR (400 MHz, DMSO-*d₆*) δ 9.80 (s, 1H), 9.13 (s, 1H), 8.88 (s, 2H), 7.75 (d, *J =* 1.6 Hz, 1H), 7.68 (t, *J* = 1.6 Hz, 1H), 7.62 (t, *J =* 1.6 Hz, 1H), 7.31 (t, *J =* 8.0 Hz, 1H), 7.08 (d, *J= 8.0* Hz, 1H), 6.99 (d, *J* = 2.0 Hz, 1H), 6.89 (t, *J =* 2.0 Hz, 1H), 6.84 (d, *J* = 7.6 Hz, 1H), 5.28 (s, 2H), 4.43 (t, *J =* 5.6 Hz, 2H), 4.14 (t, *J =* 5.6 Hz, 2H), 3.05 (s, 3H). |
| 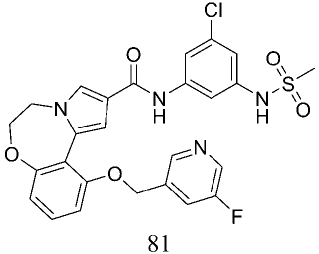 | 557.2[M+1] | ¹H NMR (400 MHz, DMSO-*d₆*) δ 9.82 (s, 1H), 8.54 (t, *J* = 1.6 Hz, 1H), 8.52 (d, *J* = 2.8 Hz, 1H), 7.81 - 7.77 (m, 1H), 7.76 (d, *J =* 1.6 Hz, 1H), 7.69 (t, *J* = 1.6 Hz, 1H), 7.64 (t, *J =* 1.6 Hz, 1H), 7.31 (t, *J =* 8.0 Hz, 1H), 7.06 - 7.03 (m, 2H), 6.89 (t, *J =* 2.0 Hz, 1H), 6.84 (dd, *J =* 8.0, 0.4 Hz, 1H), 5.29 (s, 2H), 4.44 (t, *J =* 6.0 Hz, 2H), 4.15 (t, *J =* 6.0 Hz, 2H), 3.06 (s, 3H). |
| 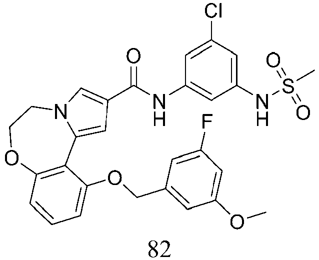 | 586.1[M+1] | ¹H NMR (400 MHz, DMSO-*d₆*) δ 9.95 (s, 1H), 9.84 (s, 1H), 7.77 (d, *J* = 1.6 Hz, 1H), 7.69 (t, *J =* 1.6 Hz, 1H), 7.64 (t, *J =* 1.6 Hz, 1H), 7.28 (t, *J=* 8.0 Hz, 1H), 7.07 (d, *J=* 1.6 Hz, 1H), 7.00 (d, *J =* 8.0 Hz, 1H), 6.90 (t, *J =* 1.6 Hz, 2H), 6.83 (dd, *J =* 6.4, 4.4 Hz, 2H), 6.72 (dt, *J =* 12.0, 2.4 Hz, 1H), 5.19 (s, 2H), 4.44 (t, *J =* 5.6 Hz, 2H), 4.16 (t, *J =* 5.6 Hz, 2H), 3.71 (s, 3H), 3.06 (s, 3H). |
| 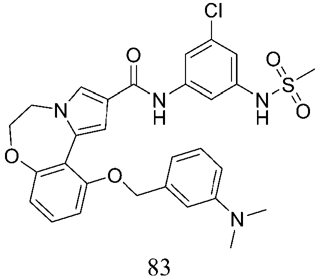 | 581.1[M+1] | ¹H NMR (400 MHz, DMSO-*d₆*) δ 10.00 (s, 1H), 9.83 (s, 1H), 7.75 (d, *J* = 1.6 Hz, 1H), 7.69 (t, *J =* 1.6 Hz, 1H), 7.63 (t*, J =* 1.6 Hz, 1H), 7.27 (t, *J* = 8.0 Hz, 1H), 7.14 (t, *J* = 8.0 Hz, 1H), 7.05-7.02 (m, 2H), 6.89 (t, *J =* 2.0 Hz, 1H), 6.83 (t, *J* = 2.8 Hz, 1H), 6.79 (dd, *J =* 8.0, 0.8 Hz, 1H), 6.69 (d, *J* = 7.6 Hz, 1H), 6.62 (dd, *J =* 8.0, 2.4 Hz, 1H), 5.13 (s, 2H), 4.43 (t, *J =* 6.0 Hz, 2H), 4.16 (t, *J =* 6.0 Hz, 2H), 3.06 (s, 3H), 2.82 (s, 6H). |
| 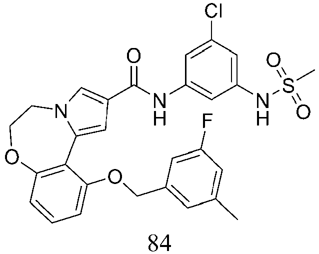 | 570.2[M+1] | ¹H NMR (400 MHz, DMSO-*d₆*) δ 10.02 (s, 1H), 9.87 (s, 1H), 7.75 (s, 1H), 7.70 (s, 1H), 7.63 (s, 1H), 7.27 (t, J = 8.4 Hz, 1H), 7.13 (s, 1H), 7.08 (s, 1H), 7.05-7.00 (m, 1H), 7.00-6.96 (m, 1H), 6.92 (d, J = 9.9 Hz, 1H), 6.89 - 6.85 (m, 1H), 6.80 (d, J = 8.0 Hz, 1H), 5.16 (s, 2H), 4.41 (t, J *=* 6.1 Hz, 2H), 4.13 (t, J = 6.3 Hz, 2H), 3.05 (s, 3H), 2.25 (s, 3H). |
| 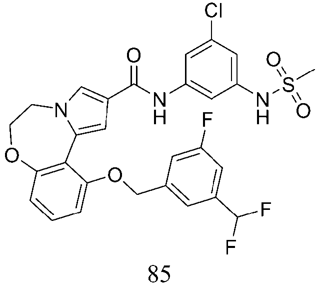 | 606.1 [M+1] | ¹H NMR (400 MHz, DMSO-*d₆*) δ 10.02 (s, 1H), 9.84 (s, 1H), 7.75 (s, 1H), 7.71 (s, 1H), 7.62 (d, J = 2.7 Hz, 1H), 7.50 (s, 1H), 7.44 (d, J = 9.7 Hz, 1H), 7.34 (d, J = 9.0 Hz, 1H), 7.27 (t, J = 16.0 Hz, 1H), 7.08 (s, 1H), 7.03-7.00 (m, 1H), 7.00-6.97 (m, 1H), 6.87 (d, J = 2.5 Hz, 1H), 6.82 (d, J = 7.7 Hz, 1H), 5.28 (s, 2H), 4.41 (s, 2H), 4.15 (s, 2H), 3.04 (s, 3H). |

Compounds of Examples 86-99 were prepared according to the preparation method of Example 73 of the present disclosure, and specific structures and structural characterizations were as follows:

| Serial number and structure of example | MS m/z (ESI) | ¹H NMR |
|---|---|---|
| 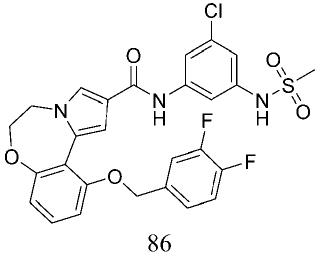 | 574.1[M+1] | ¹H NMR (400 MHz, DMSO-*d₆*) δ 10.00 (s, 1H), 9.82 (s, 1H), 7.76 (d, *J* = 1.6 Hz, 1H), 7.70 (s, 1H), 7.63 (s, 1H), 7.55 - 7.48 (m, 1H), 7.42 (d, *J =* 10.7 Hz, 1H), 7.28 (t, *J* = 8.2 Hz, 2H), 7.01 (dd, *J =* 9.6, 5.1 Hz, 2H), 6.89 (s, 1H), 6.82 (d, *J =* 8.0 Hz, 1H), 5.19 (s, 2H), 4.43 (t, *J =* 5.6 Hz, 2H), 4.15 (t, *J =* 5.6 Hz, 2H), 3.05 (s, 3H). |
| 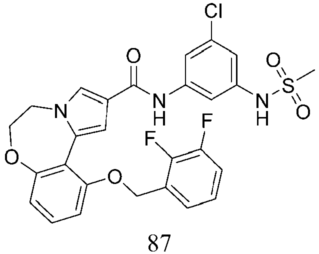 | 574.1[M+1] | ¹H NMR (400 MHz, DMSO-*d₆*) δ 9.99 (s, 1H), 9.81 (s, 1H), 7.75 (d, *J* = 1.8 Hz, 1H), 7.70 (t, *J =* 1.8 Hz, 1H), 7.60 (t, *J =* 1.8 Hz, 1H), 7.44 - 7.34 (m, 2H), 7.29 (t, *J =* 8.2 Hz, 1H), 7.23 - 7.17 (m, 1H), 7.06 (d, *J* = 8.0 Hz, 1H), 6.93 (d, *J* = 1.8 Hz, 1H), 6.89 (t, *J* = 1.9 Hz, 1H), 6.83 (d, *J =* 7.4 Hz, 1H), 5.27 (s, 2H), 4.43 (t, *J =* 5.8 Hz, 2H), 4.14 (t, *J =* 5.8 Hz, 2H), 3.06 (s, 3H). |
| 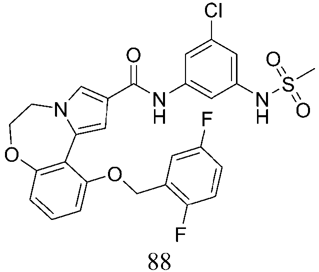 | 573.6[M+1] | ¹H NMR (400 MHz, DMSO-*d₆*) δ 10.10 (s, 1H), 9.80 (s, 1H), 7.76 (d, J = 1.6 Hz, 1H), 7.67 (s, 1H), 7.59 (s, 1H), 7.41 - 7.34 (m, 1H), 7.36 - 7.26 (m, 2H), 7.25 - 7.17 (m, 1H), 7.07 (d, J = 8.4 Hz, 1H), 6.99 (d, J = 1.6 Hz, 1H), 6.88 (t, J = 2.0 Hz, 1H), 6.84 (d, J = 8.0 Hz, 1H), 5.23 (s, 2H), 4.43 (t, J = 5.6 Hz, 2H), 4.15 (t, J = 5.6 Hz, 2H), 3.03 (s, 3H). |
| 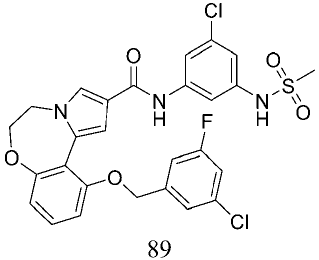 | 590.1 [M+1] | ¹H NMR (400 MHz, DMSO-*d₆*) δ 9.99 (s, 1H), 9.84 (s, 1H), 7.76 (d, *J* = 1.6 Hz, 1H), 7.70 (t, *J =* 1.6 Hz, 1H), 7.65 (t*, J =* 1.6 Hz, 1H), 7.38 (s, 1H), 7.34 (dt, *J =* 8.8, 2.0 Hz, 1H), 7.28 (dd, *J* = 16.0, 8.8 Hz, 2H), 7.10 (d, *J =* 1.6 Hz, 1H), 6.99 (d, *J =* 8.0 Hz, 1H), 6.89 (t, *J = 2.0* Hz, 1H), 6.83 (d, *J =* 7.6 Hz, 1H), 5.24 (s, 2H), 4.44 (t, *J =* 5.6 Hz, 2H), 4.16 (t, *J* = 5.6 Hz, 2H), 3.06 (s, 3H). |
| 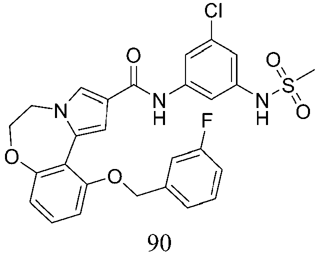 | 555.8[M+1] | ¹H NMR (400 MHz, DMSO-*d₆*) δ 10.02 (s, 1H), 9.84 (s, 1H), 7.76 (d, J = 1.6 Hz, 1H), 7.71 (t, J = 1.6 Hz, 1H), 7.64 (d, J = 1.6 Hz, 1H), 7.45 - 7.37 (m, 1H), 7.32 - 7.24 (m, 3H), 7.15 - 7.08 (m, 1H), 7.05 (d, J = 1.6 Hz, 1H), 7.00 (d, J = 8.0 Hz, 1H), 6.90 (t, J = 1.6 Hz, 1H), 6.81 (d, J = 8.0 Hz, 1H), 5.23 (s, 2H), 4.43 (t, J = 5.6 Hz, 2H), 4.16 (t, J = 5.6 Hz, 2H), 3.06 (s, 3H). |
| 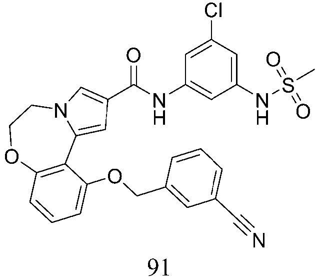 | 563.1[M+1] | ¹H NMR (400 MHz, DMSO-*d₆*) δ 10.01 (s, 1H), 9.85 (s, 1H), 7.88 (t, *J* = 1.6 Hz, 1H), 7.81-7.77 (m, 3H), 7.74 (t, *J* = 1.6 Hz, 1H), 7.64 (t, *J* = 1.6 Hz, 1H), 7.60 (t, *J =* 7.6 Hz, 1H), 7.30 (t, *J =* 8.0 Hz, 1H), 7.06 (d, *J =* 1.6 Hz, 1H), 7.03 (d, *J =* 8.0 Hz, 1H), 6.91 (t, *J =* 2.0 Hz, 1H), 6.84 (d, *J =* 7.6 Hz, 1H), 5.29 (s, 2H), 4.45 (t, *J =* 6.0 Hz, 2H), 4.17 (t, *J =* 6.0 Hz, 2H), 3.08 (s, 3H). |
| 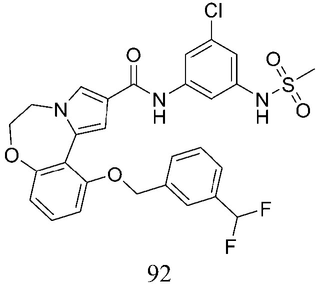 | 588.1[M+1] | ¹H NMR (400 MHz, DMSO-*d₆*) δ9.99 (s, 1H), 9.83 (s, 1H), 7.75 (d, *J* = 1.6 Hz, 1H), 7.72 (t, *J* = 1.6 Hz, 1H), 7.66 (s, 1H), 7.62 (dd, *J =* 4.8, 3.2 Hz, 2H), 7.53 - 7.47 (m, 2H), 7.28 (t, *J* = 8.0 Hz, 1H), 7.06 (d, *J* = 1.6 Hz, 1H), 7.03 (dd, *J =* 8.0, 0.4 Hz, 1H), 6.99 (t, *J =* 56.0 Hz, 1H), 6.89 (t, *J* = 2.0 Hz, 1H), 6.81 (dd, *J =* 7.2, 0.8 Hz, 1H), 5.28 (s, 2H), 4.43 (t, *J* = 5.6 Hz, 2H), 4.15 (t, *J* = 5.6 Hz, 2H), 3.06 (s, 3H). |
| 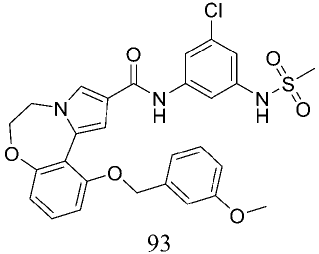 | 568.1[M+1] | ¹H NMR (400 MHz, DMSO-*d₆*) δ 10.00 (s, 1H), 9.84 (s, 1H), 7.76 (d, *J* = 1.6 Hz, 1H), 7.70 (t, *J =* 1.6 Hz, 1H), 7.63 (t*, J =* 1.6 Hz, 1H), 7.29 - 7.24 (m, 2H), 7.05 (d, *J =* 2.0 Hz, 2H), 7.01 (dd, *J =* 10.0, 8.0 Hz, 2H), 6.90 (t, *J* = 2.0 Hz, 1H), 6.83 (dd, *J =* 8.0, 2.0 Hz, 1H), 6.80 (dd, *J =* 8.0, 0.8 Hz, 1H), 5.18 (s, 2H), 4.43 (t, *J* = 6.0 Hz, 2H), 4.16 (t, *J =* 6.0 Hz, 2H), 3.70 (s, 3H), 3.06 (s, 3H). |
| 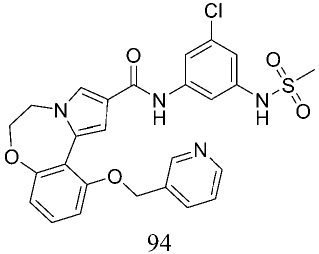 | 539.2[M+1] | ¹H NMR (400 MHz, DMSO-*d₆*) δ 9.99 (s, 1H), 9.82 (s, 1H), 8.69 - 8.64 (m, 1H), 8.51 (dd, *J* = 4.8*,* 1.6 Hz, 1H), 7.86 (dt, *J =* 8.0, 2.0 Hz, 1H), 7.75 (d, *J = 2.0* Hz, 1H), 7.71 (t, *J =* 2.0 Hz, 1H), 7.62 (t, *J* = 2.0 Hz, 1H), 7.39 (dd, *J* = 8.0*,* 4.8, 1H), 7.29 (t, *J* = 8.0 Hz, 1H), 7.05 (dd, *J =* 8.4, 1.2 Hz, 1H), 6.99 (d, *J =* 2.0 Hz, 1H), 6.90 (t, *J =* 2.0 Hz, 1H), 6.82 (dd, *J =* 8.0, 1.2 Hz, 1H), 5.25 (s, 2H), 4.43 (t, *J =* 6.0 Hz, 2H), 4.14 (t, *J =* 6.0 Hz, 2H), 3.07 (s, 3H). |
| 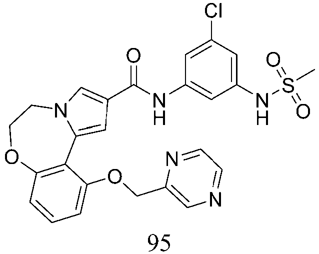 | 540.1[M+1] | ¹H NMR (400 MHz, DMSO-*d₆*) δ 10.00 (s, 1H), 9.81 (s, 1H), 8.80 (d, *J* = 1.2 Hz, 1H), 8.67 - 8.65 (m, 1H), 8.60 (d, *J* = 2.4 Hz, 1H), 7.77 (d, *J =* 1.6 Hz, 1H), 7.69 (t, *J =* 1.6 Hz, 1H), 7.61 (t, *J =* 1.6 Hz, 1H), 7.29 (t, *J =* 8.0 Hz, 1H), 7.08-7.04 (m, 2H), 6.89 (t, *J =* 2.0 Hz, 1H), 6.84 (d, *J =* 7.6 Hz, 1H), 5.35 (s, 2H), 4.44 (t, *J =* 5.6 Hz, 2H), 4.17 (t, *J =* 5.6 Hz, 2H), 3.05 (s, 3H). |
| 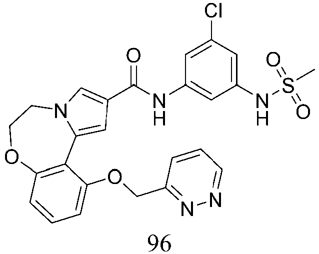 | 540.2[M+1] | ¹H NMR (400 MHz, DMSO-*d₆*) δ 10.11 (s, 1H), 9.82 (s, 1H), 9.21 (dd, *J* = 4.9, 1.6 Hz, 1H), 7.82 (dd, *J =* 8.5, 1.6 Hz, 1H), 7.78 (d, *J =* 1.8 Hz, 1H), 7.73 (dd, *J =* 8.1, 5.3 Hz, 2H), 7.61 (t, *J* = 1.8 Hz, 1H), 7.31 (t, *J =* 8.2 Hz, 1H), 7.09 (d, *J* = 7.9 Hz, 1H), 7.04 (d, *J =* 1.8 Hz, 1H), 6.90 (t*, J =* 1.9 Hz, 1H), 6.85 (d, *J =* 8.0 Hz, 1H), 5.51 (s, 2H), 4.46 (t*, J =* 5.8 Hz, 2H), 4.18 (t, *J* = 5.7 Hz, 2H), 3.07 (s, 3H). |
| 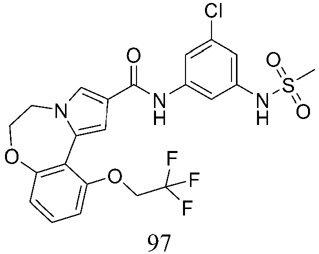 | 530.2[M+1] | ¹H NMR (400 MHz, DMSO-*d₆*) δ 10.07 (s, 1H), 9.86 (s, 1H), 7.79 (d, *J* = 1.6 Hz, 1H), 7.69 (t, *J* = 1.6 Hz, 1H), 7.60 (t, *J =* 1.6 Hz, 1H), 7.31 (t, *J* = 8.4 Hz, 1H), 7.04 (d, *J =* 8.4 Hz, 1H), 6.89- 6.87(m, 3H), 4.79 (q, *J =* 8.8 Hz, 2H), 4.45 (t, *J =* 6.0 Hz, 2H), 4.18 (t, *J* = 6.0 Hz, 2H), 3.04 (s, 3H). |
| 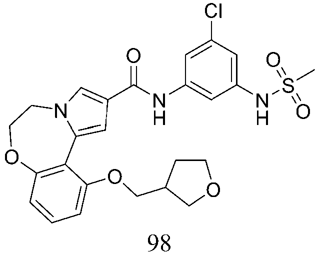 | 532.2[M+1] | ¹H NMR (400 MHz, DMSO-*d₆*) δ 9.85 (s, 1H), 8.28 (s, 1H), 7.73 (d, *J* = 17.8 Hz, 2H), 7.63 (s, 1H), 7.25 (t, *J* = 8.1 Hz, 1H), 6.91 (d*, J =* 17.3 Hz, 3H), 6.77 (d, *J* = 7.9 Hz, 1H), 4.43 (t, *J =* 6.0 Hz, 2H), 4.15 (t, *J =* 6.0 Hz, 2H), 3.93 (d, *J* = 6.4 Hz, 2H), 3.05 (s, 3H), 2.67 - 2.55 (m, 2H), 2.41-2.26 (m, 3H), 1.93 (m, 1H), 1.55 (m, 1H). |
| 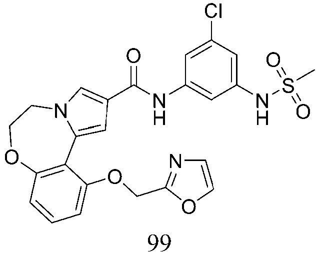 | 529.2[M+1] | ¹H NMR (400 MHz, DMSO-*d₆*) δ 9.99 (s, 1H), 9.84 (s, 1H), 8.15 (d, *J* = 0.8 Hz, 1H), 7.75 (d, *J* = 1.6 Hz, 1H), 7.72 (t, *J =* 1.6 Hz, 1H), 7.60 (t, *J* = 1.6 Hz, 1H), 7.29 (d, *J =* 8.4 Hz, 1H), 7.26 - 7.26 (m, 1H), 7.08 (dd, *J* = 8.0, 0.8 Hz, 1H), 6.91 (d, *J* = 2.0 Hz, 1H), 6.88 (t, *J =* 2.0 Hz, 1H), 6.83 (dd, *J =* 8.0, 0.8 Hz, 1H), 5.31 (s, 2H), 4.42 (t, *J =* 6.0 Hz, 2H), 4.15 (t, *J* = 6.0 Hz, 2H), 3.06 (s, 3H). |
| 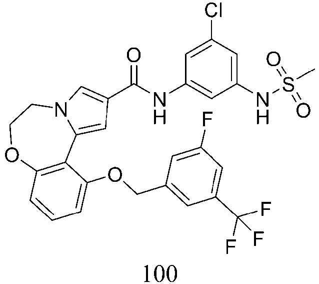 | 624.2[M+1] | ¹H NMR (400 MHz, DMSO-*d₆*) δ 10.01 (s, 1H), 9.82 (s, 1H), 7.74 (d, *J* = 1.9 Hz, 1H), 7.69 (s, 1H), 7.65 (s, 1H), 7.62 (s, 1H), 7.60 (s, 1H), 7.58 (s, 1H), 7.30 (t, *J =* 8.1 Hz, 1H), 7.09 (d, *J =* 1.8 Hz, 1H), 7.01 (d, *J* = 8.5 Hz, 1H), 6.87 (s, 1H), 6.83 (d, *J =* 8.0 Hz, 1H), 5.31 (s, 2H), 4.41 (t, *J* = 6.1 Hz, 2H), 4.13 (t, *J =* 6.1 Hz, 2H), 3.04 (s, 3H). |

A compound of Example 101 was prepared according to the preparation method of Example 69 of the present disclosure, and a specific structure and a structural characterization were as follows:

| Serial number and structure of example | MS m/z (ESI) | ¹H NMR |
|---|---|---|
| 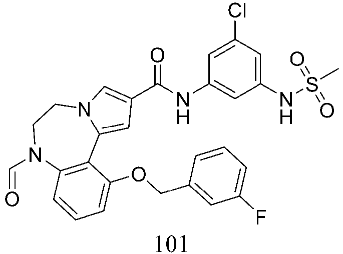 | 583.0[M+1] | ¹H NMR (400 MHz, DMSO-*d₆*) δ10.02 (s, 1H), 9.86 (s, 1H), 8.08 (s, 1H), 7.79- 7.75 (m, 1H), 7.70 - 7.67 (m, 1H), 7.63 - 7.60 (m, 1H), 7.45 - 7.42 (m, 1H), 7.41 - 7.37 (m, 1H), 7.29 (d, J = 4.7 Hz, 1H), 7.28 - 7.24 (m, 1H), 7.25 (dd, J = 12.8, 7.1 Hz, 1H), 7.13 (t, J = 8.3 Hz, 1H), 7.04 (d, J = 1.8 Hz, 1H), 7.01 (d, J = 7.9 Hz, 1H), 6.90 (d, J = 2.4 Hz, 1H), 5.27 (s, 2H), 4.19 (m, 2H), 4.06 - 3.96 (m, 2H), 3.06 (s, 3H). |

Compounds of Examples 102-105 were prepared according to the preparation method of Example 70 of the present disclosure, and specific structures and structural characterizations were as follows:

| Serial number and structure of example | MS m/z (ESI) | ¹H NMR |
|---|---|---|
| 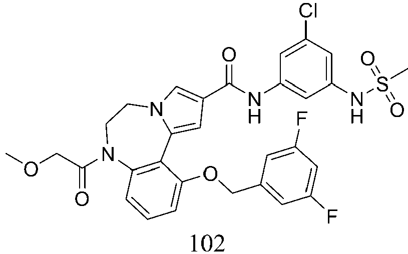 | 645.2[M+1] | ¹H NMR (400 MHz, DMSO-*d₆*) δ 9.99 (s, 1H), 9.84 (s, 1H), 7.76 (s, 1H), 7.69 (s, 1H), 7.64 (s, 1H), 7.43 (d, J = 7.9 Hz, 1H), 7.30 (d, J = 8.5 Hz, 1H), 7.15 (s, 4H), 7.08 (s, 1H), 6.90 (s, 1H), 5.36 (d, J = 13.1 Hz, 1H), 5.21 (d, J = 13.2 Hz, 1H), 4.98 - 4.81 (m, 1H), 4.45 - 4.30 (m, 1H), 3.73 (d, J = 15.1 Hz, 2H), 3.19 (d, J = 15.1 Hz, 2H), 3.06 (s, 3H), 3.01 (s, 3H). |
| 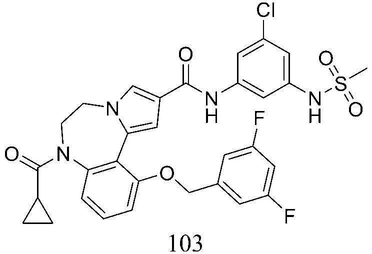 | 641.1[M+1] | ¹H NMR (400 MHz, DMSO-*d₆*) δ 10.00 (s, 1H), 9.86 (s, 1H), 7.75 (s, 1H), 7.70 (s, 1H), 7.65 (s, 1H), 7.46 (t, *J* = 7.9 Hz, 1H), 7.28 (d, *J* = 8.2 Hz, 1H), 7.13 (dd, *J =* 20.2, 9.0 Hz, 5H), 6.91 (s, 1H), 5.35 (d, *J =* 13.3 Hz, 1H), 5.21 (d, *J =* 13.2 Hz, 1H), 4.89 - 4.71 (m, 1H), 4.37 (dd, *J* = 14.0, 5.4 Hz, 1H), 3.89 - 3.71 (m, 1H), 3.44 (d, *J =* 16.0 Hz, 1H), 3.07 (s, 3H), 0.97 (m, 1H), 0.78 - 0.52 (m, 3H), 0.46 (m, 1H). |
| 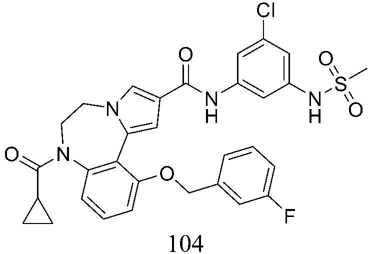 | 623.1[M+1] | ¹H NMR (400 MHz, DMSO-*d₆*) δ 10.03 (s, 1H), 9.86 (s, 1H), 7.74 (s, 1H), 7.71 (s, 1H), 7.63 (s, 1H), 7.46 - 7.41 (m, 2H), 7.30 - 7.26 (m, 3H),7.14 - 7.05 (m, 3H), 6.90 (s, 1H), 5.34 (d, J = 12.9 Hz, 1H), 5.21 (d, J = 12.9 Hz, 1H), 4.82-4.73 (m,1H), 4.40-4.32 (m, 1H), 3.84 - 3.74 (m, 1H), 3.45 (m, 1H), 3.07 (s, 3H), 1.01 - 0.93 (m, 1H), 0.68 - 0.54(m, 3H), 0.49 - 0.41 (m, 1H). |
| 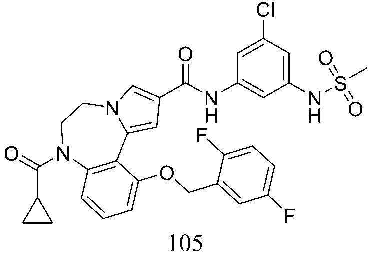 | 641.2[M+1] | ¹H NMR (400 MHz, DMSO-*d₆*) δ 10.02 (s, 1H), 9.84 (s, 1H), 7.72 (s, 1H), 7.67 (s, 1H), 7.59 (s, 1H), 7.45 (t, *J =* 8.1 Hz, 1H), 7.35 - 7.33 (m, 2H), 7.31 - 7.27 (m, 1H), 7.25 - 7.20 (m, 1H), 7.10 (d, *J =* 7.9 Hz, H), 6.97 (s, 1H), 6.87 (s, 1H), 5.32 (d, *J* = 12.8 Hz, 1H), 5.19 (d*, J =* 12.8 Hz, 1H), 4.78 - 4.70 (m, 1H), 4.42 - 4.30 (m, 1H), 3.85 - 3.70 (m, 2H), 3.04 (s, 3H), 1.01 - 0.90 (m, 1H), 0.66 - 0.60 (m, 2H), 0.58 - 0.49 (m, 1H), 0.44 - 0.39 (m, 1H). |

Compounds of Examples 106-111 were prepared according to the preparation method of Example 68 of the present disclosure, and specific structures and structural characterizations were as follows:

| Serial number and structure of example | MS m/z (ESI) | ¹H NMR |
|---|---|---|
| 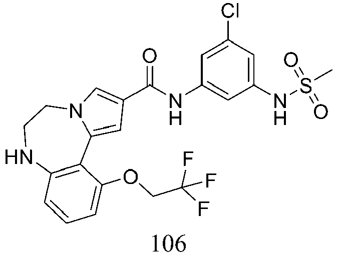 | 529.0[M+1] | ¹H NMR (400 MHz, DMSO-*d₆*) δ 10.05 (s, 1H), 9.76 (s, 1H), 7.68 (t, *J* = 2.0 Hz, 1H), 7.64 (d, *J* = 2.0 Hz, 1H), 7.60 (t, *J* = 2.0 Hz, 1H), 7.02 (t, *J* = 8.0 Hz, 1H), 6.88 (t, *J* = 2.0 Hz, 1H), 6.84 (d, *J* = 2.0 Hz, 1H), 6.55 (dd, *J* = 8.4, 1.2 Hz, 1H), 6.50 (d, *J*= 8.0 Hz, 1H), 5.83 (t, *J =* 4.0 Hz, 1H), 4.63 (q, *J* = 8.8 Hz, 2H), 4.16 (t, *J* = 4.8 Hz, 2H), 3.59 - 3.49 (m, 2H), 3.04 (s, 3H). |
| 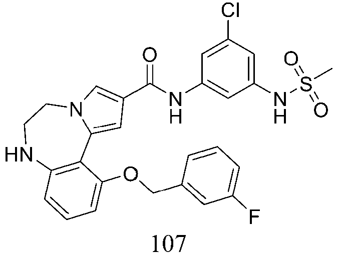 | 555.1[M+1] | ¹H NMR (400 MHz, DMSO-*d₆*) δ 10.00 (s, 1H), 9.77 (s, 1H), 7.71 (s, 1H), 7.67 - 7.61 (m, 2H), 7.40 - 7.35 (m, 1H), 7.27 (d, J = 9.2 Hz, 2H), 7.11 - 7.01 (m, 3H), 6.89 (d, J = 1.8 Hz, 1H), 6.64 - 6.50 (m, 2H), 5.14 (s, 2H), 4.15 (t, J = 5.1 Hz, 2H), 4.08 - 3.82(m, 1H), 3.55 (m, 1H), 3.06 (s, 3H). |
| 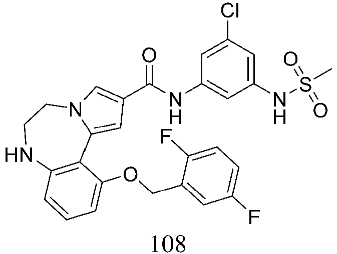 | 573.1[M+1] | ¹H NMR (400 MHz, DMSO-*d₆*) δ 10.01 (s, 1H), 9.77 (s, 1H), 7.70 (s, 1H), 7.66 (s, 1H), 7.63 (s, 1H), 7.40 - 7.35 (m, 1H), 7.30 - 7.25 (m, 1H), 7.21 - 7.16 (m, 1H), 7.09 (t, J = 8.2 Hz, 1H), 7.01 (s, 1H), 6.89 (s, 1H), 6.68 (d, J = 8.1 Hz, 1H), 6.63 (d, J = 8.1 Hz, 1H), 5.15 (s, 2H), 4.19 - 4.13 (m, 2H), 4.04 (s, 1H), 3.57 (m, 2H), 3.06 (s, 3H). |
| 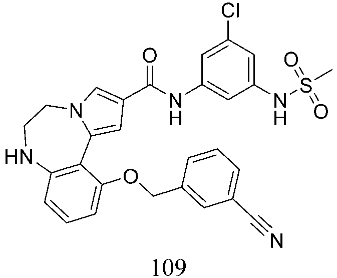 | 562.2[M+1] | ¹H NMR (400 MHz, DMSO-*d₆*) δ 10.00 (s, 1H), 9.78 (s, 1H), 7.85 (s, 1H), 7.77 - 7.67 (m, 4H), 7.67 (s, 1H), 7.62 (s, 1H), 7.55 (t, *J* = 7.6 Hz, 1H), 7.17 - 7.02 (m, 2H), 6.88 (s, 1H), 6.75 - 6.59 (m, 2H), 5.20 (s, 2H), 4.15 (s, 2H), 3.59 (s, 2H), 3.06 (s, 3H). |
| 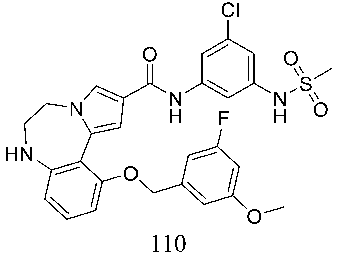 | 585.1[M+1] | ¹H NMR (400 MHz, DMSO-*d₆*) δ 10.01 (s, 1H), 9.79 (s, 1H), 7.68 - 7.61 (m, 3H), 7.10 - 7.06 (m, 3H), 6.87 (s, 2H), 6.81 (d, *J* = 9.5 Hz, 1H), 6.72 - 6.61 (m, 2H), 6.60 - 6.53 (m, 1H), 5.10 (s, 2H), 4.16 - 4.12 (m, 2H), 3.67 (s, 3H), 3.65 - 3.52 (m, 2H), 3.05 (s, 3H). |
| 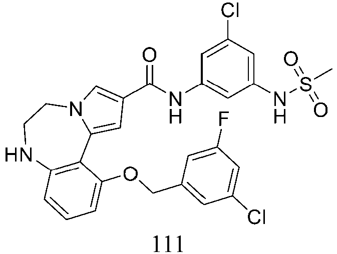 | 589.2[M+1] | ¹H NMR (400 MHz, DMSO-*d₆*) δ 10.00 (s, 1H), 9.79 (s, 1H), 7.70 (t, *J* = 1.8 Hz, 1H), 7.65 (t, *J* = 1.8 Hz, 2H), 7.38 - 7.36 (m, 1H), 7.32 (dt, *J =* 9.6, 2.0 Hz,1H), 7.11 - 7.06 (m, 2H), 6.89 (t, *J =* 2.0 Hz, 1H), 6.60 (d, *J* = 8.2 Hz, 2H), 5.16 (s, 2H), 4.16 (t, *J* = 5.1 Hz, 2H), 3.72 - 3.64 (m, 3H), 3.06 (s, 3H). |

Compounds of Examples 112-133 were prepared according to the preparation method of Example 71 of the present disclosure, and specific structures and structural characterizations were as follows:

| Serial number and structure of example | MS m/z (ESI) | ¹H NMR |
|---|---|---|
| 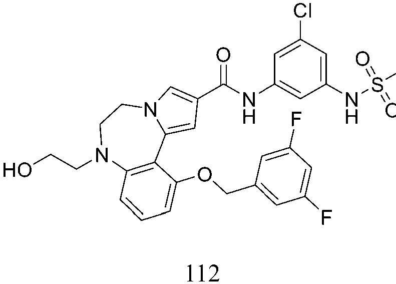 I | 617.2[M+1] | ¹H NMR (400 MHz, DMSO-*d₆*) δ 9.99 (s, 1H), 9.78 (s, 1H), 7.69 (s, 2H), 7.64 (s, 1H), 7.27 (t*, J =* 8.2 Hz, 1H), 7.11 (d, *J =* 8.1 Hz, 3H), 6.95 (s, 1H), 6.92 - 6.80 (m, 3H), 5.17 (s, 2H), 4.01 (t, *J* = 6.1 Hz, 2H), 3.43 (q, *J* = 6.2 Hz, 4H), 3.15 (t, *J* = 6.4 Hz, 2H), 3.06 (s, 3H). |
| 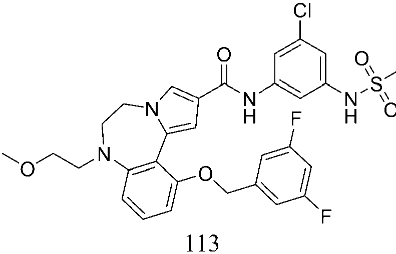 | 631.2[M+1] | ¹H NMR (400 MHz, DMSO-*d₆*) δ 9.98 (s, 1H), 9.78 (s, 1H), 7.69 (d, *J* = 1.7 Hz, 2H), 7.65 (t, *J* = 1.6 Hz, 1H), 7.27 (t, *J* = 8.2 Hz, 1H), 7.16 - 7.05 (m, 3H), 6.95 (d, *J* = 1.7 Hz, 1H), 6.89 (t, *J* = 1.8 Hz, 1H), 6.85 (dd, *J* = 8.1, 5.3 Hz, 2H), 5.17 (s, 2H), 4.00 (t, *J =* 5.7 Hz, 2H), 3.44 (t, *J* = 5.7 Hz, 2H), 3.36 (t, *J* = 5.8 Hz, 2H), 3.24 (t, *J =* 5.8 Hz, 2H), 3.14 (s, 3H), 3.06 (s, 3H). |
| 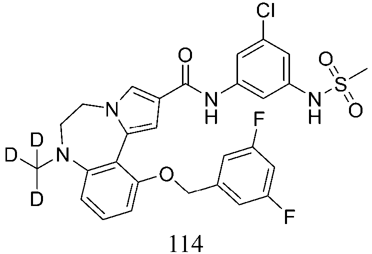 | 590.2[M+1] | ¹H NMR (400 MHz, DMSO-*d₆*) δ 10.00 (s, 1H), 9.79 (s, 1H), 7.71 (d, *J* = 5.3 Hz, 2H), 7.66 (s, 1H), 7.28 (t, *J* = 8.2 Hz, 1H), 7.11 (d, *J* = 7.4 Hz, 3H), 6.96 (s, 1H), 6.90 (s, 1H), 6.85 (d, *J* = 8.3 Hz, 1H), 6.80 (d, *J* = 8.1 Hz, 1H), 5.18 (s, 2H), 4.03 (t, *J =* 5.4 Hz, 2H), 3.38 (t, *J =* 5.2 Hz, 2H), 3.06 (s, 3H). |
| 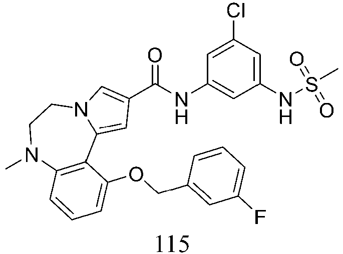 | 569.2[M+1] | ¹H NMR (400 MHz, DMSO-*d₆*) δ 10.00 (s, 1H), 9.78 (s, 1H), 7.71 (d, J = 2.7 Hz, 2H), 7.64 (d, J = 2.0 Hz, 1H), 7.41 - 7.35 (m, 1H), 7.27 (d, J = 9.0 Hz, 1H), 7.26-7.24 (m, 1H), 7.22 - 7.16 (m, 1H), 7.10 (t, J = 9.0 Hz, 1H), 6.91 (d, J = 1.8 Hz, 1H), 6.89 (d, J = 2.0 Hz, 1H), 6.85 (d, J = 8.3 Hz, 1H), 6.77 (d, J = 8.2 Hz, 1H), 5.17 (s, 2H), 4.02 (t, J = 6.0 Hz, 2H), 3.36 (t, J = 6.1 Hz, 2H), 3.06 (s, 3H), 2.71 (s, 3H). |
| 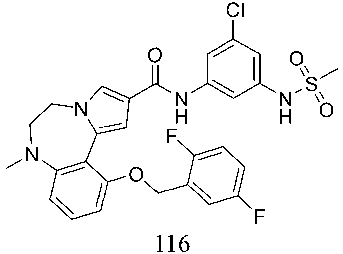 | 587.1[M+1] | ¹H NMR (400 MHz, DMSO-*d₆*) δ 10.00 (s, 1H), 9.77 (s, 1H), 7.70 (s, 2H), 7.63 (s, 1H), 7.34 - 7.31 (m, 1H), 7.30 - 7.24 (m, 2H), 7.22 - 7.15(m, 1H), 6.93 - 6.86 (m, 3H), 6.80 (d, J = 8.1 Hz, 1H), 5.16 (s, 2H), 4.02 (t, J = 6.1 Hz, 2H), 3.36 (t, J = 12.2 Hz, 2H), 3.06 (s, 3H), 2.71 (s, 3H). |
| 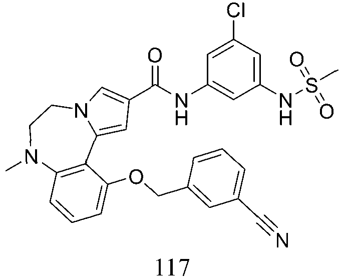 | 576.2[M+1] | ¹H NMR (400 MHz, DMSO-*d₆*) δ 10.00 (s, 1H), 9.77 (s, 1H), 7.80 (s, 1H), 7.77 - 7.67 (m, 4H), 7.60 (s, 1H), 7.54 (t, *J =* 7.8 Hz, 1H), 7.25 (t, *J* = 8.1 Hz, 1H), 6.90 (d, *J* = 1.8 Hz, 1H), 6.88 - 6.81 (m, 2H), 6.76 (d, *J =* 8.2 Hz, 1H), 5.19 (s, 2H), 3.99 (t, *J =* 5.5 Hz, 2H), 3.35 (t, *J =* 5.5 Hz, 2H), 3.05 (s, 3H), 2.69 (s, 3H). |
| 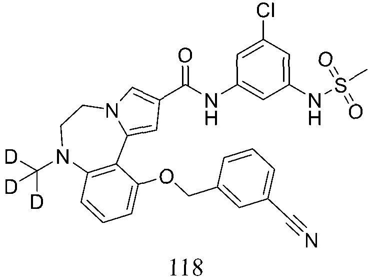 | 579.2[M+1] | ¹H NMR (400 MHz, DMSO-*d₆*) δ 10.00 (s, 1H), 9.77 (s, 1H), 7.80 (s, 1H), 7.77 - 7.67 (m, 4H), 7.60 (s, 1H), 7.54 (t, *J* = 7.6 Hz, 1H), 7.25 (t, *J* = 8.4 Hz, 1H), 6.90 (s, 1H), 6.88 - 6.80 (m, 2H), 6.76 (d, *J =* 8.3 Hz, 1H), 5.19 (s, 2H), 3.99 (t, *J* = 6.1 Hz, 2H), 3.34 (t, *J* = 6.1 Hz, 2H), 3.05 (s, 3H). |
| 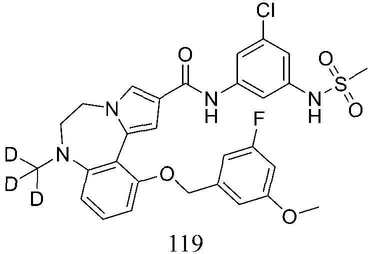 | 602.1[M+1] | ¹H NMR (400 MHz, DMSO-*d₆*) δ 10.01 (s, 1H), 9.78 (s, 1H), 7.70 - 7.66 (m, 2H), 7.62 (s, 1H), 7.25 (t, J = 8.1 Hz, 1H), 6.92 (d, J = 2.0 Hz, 1H), 6.87 - 6.81 (m, 3H), 6.76 (t, J = 9.8 Hz, 2H), 6.68 (d, J = 11.1 Hz, 1H), 5.11 (s, 2H), 4.05-3.95 (m, 2H), 3.67 (s, 3H), 3.35-3.20 (m, 2H), 3.05 (s, 3H). |
| 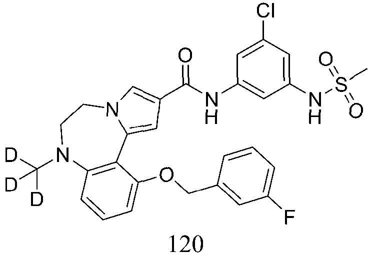 | 572.1[M+1] | ¹H NMR (400 MHz, DMSO-*d₆*) δ 10.01 (s, 1H), 9.78 (s, 1H), 7.71 - 7.68 (m, 2H), 7.62 (s, 1H), 7.40 - 7.32 (m, 1H), 7.25 - 7.20 (m, 3H), 7.08 (t, *J* = 9.1 Hz, 1H), 6.90 (d, *J* = 1.8 Hz, 1H), 6.87 (d, *J* = 2.1 Hz, 1H), 6.83 (d, *J* = 8.3 Hz, 1H), 6.74 (d, *J* = 8.2 Hz, 1H), 5.15 (s, 2H), 4.00 (t, *J =* 6.0 Hz, 2H), 3.34 (t, *J =* 5.9 Hz, 2H), 3.05 (s, 3H). |
| 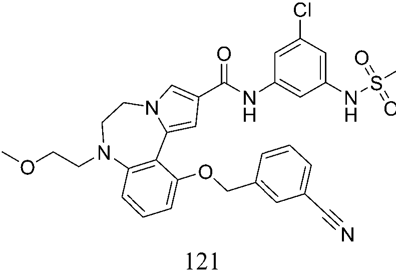 | 620.2[M+1] | ¹H NMR (400 MHz, DMSO-*d₆*) δ 10.00 (s, 1H), 9.78 (s, 1H), 7.79 (s, 1H), 7.77-7.67 (m, 4H), 7.60 (s, 1H), 7.54 (t, *J* = 7.8 Hz, 1H), 7.25 (t, *J=* 8.2 Hz, 1H), 6.90 (s, 1H), 6.88 - 6.81 (m, 3H), 5.18 (s, 2H), 3.97 (t*, J =* 6.2 Hz, 2H), 3.40 (d, *J =* 6.2 Hz, 2H), 3.33 (t, *J* = 5.8 Hz, 2H), 3.21 (t, *J =* 6.0 Hz, 2H), 3.11 (s, 3H), 3.05 (s, 3H). |
| 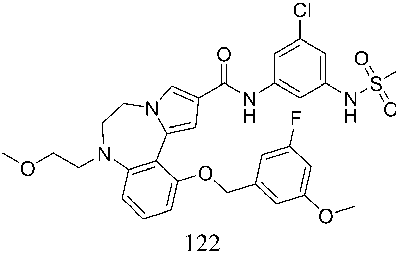 | 643.0[M+1] | ¹H NMR (400 MHz, DMSO-*d₆*) δ 10.02 (s, 1H), 9.79 (s, 1H), 7.69 - 7.66 (m, 2H), 7.62 (s, 1H), 7.25 (t, J = 8.1 Hz, 1H), 6.93 - 6.91 (m, 1H), 6.88 - 6.86 (m, 1H), 6.84 (s, 2H), 6.82 (s, 1H), 6.77 (d, J = 9.4 Hz, 1H), 6.68 (d, J = 10.9 Hz, 1H), 5.10 (s, 2H), 3.40 - 3.96 (m, 2H), 3.67 (s, 3H), 3.40 - 3.96 (m, 2H), 3.33 (t, J = 5.9 Hz, 2H), 3.21 (t, J = 5.9 Hz, 2H), 3.11 (s, 3H), 3.05 (s, 3H). |
| 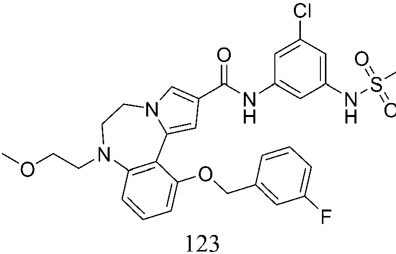 | 613.0[M+1] | ¹H NMR (400 MHz, DMSO-*d₆*) δ 10.02 (s, 1H), 9.80 (s, 1H), 7.70 - 7.67 (m, 2H), 7.63 (s, 1H), 7.40 - 7.34 (m, 1H), 7.26 - 7.19 (m, 3H), 7.08 (t, J = 8.4 Hz, 1H), 6.91 (d, J = 1.8 Hz, 1H), 6.87 - 6.81 (m, 3H), 5.14 (s, 2H), 4.02 - 3.95 (m, 2H), 3.45 - 3.38 (m, 2H), 3.33 (t, J = 5.9 Hz, 2H), 3.21 (t, J = 6.0 Hz, 2H), 3.11 (s, 3H), 3.05 (s, 3H). |
| 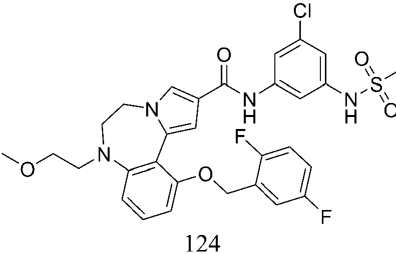 | 631.1[M+1] | ¹H NMR (400 MHz, DMSO-*d₆*) δ 10.01 (s, 1H), 9.78 (s, 1H), 7.70 - 7.66 (m, 2H), 7.63 - 7.58 (m, 1H), 7.32 - 7.23 (m, 3H), 7.20 - 7.13 (m, 1H), 6.90 (d, *J* = 8.4 Hz, 1H), 6.86 - 6.84 (m, 3H), 5.14 (s, 2H), 4.05 - 3.93 (m, 2H), 3.40 - 3.30 (m, 2H), 3.33 (t, *J* = 5.8 Hz, 2H), 3.21 (t, *J* = 6.0 Hz, 2H), 3.11 (s, 3H), 3.05 (s, 3H). |
| 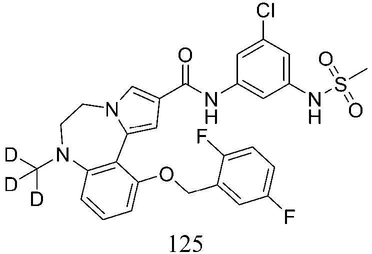 | 590.1[M+1] | ¹H NMR (400 MHz, DMSO-*d₆*) δ 10.01 (s, 1H), 9.77 (s, 1H), 7.72 - 7.70 (m, 2H), 7.64 (t, J = 1.9 Hz, 1H), 7.34 - 7.26 (m, 3H), 7.22 - 7.16 (m, 1H), 6.94 - 6.89 (m, 3H), 6.80 (d, J = 8.1 Hz, 1H), 5.17 (t, J = 10.0 Hz, 2H), 4.02 (s, 2H), 3.37 (t, J = 12.0 Hz, 2H), 3.07 (s, 3H). |
| 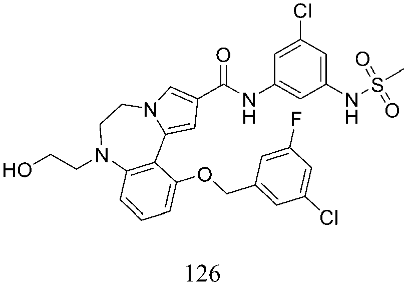 | 633.1[M+1] | ¹H NMR (400 MHz, DMSO-*d₆*) δ9.99 (s, 1H), 9.79 (s, 1H), 7.69 (t, J = 1.8 Hz, 2H), 7.64 (t, J = 1.9 Hz, 1H), 7.34 - 7.29 (m, 2H), 7.24 (dd, J = 17.9, 8.8 Hz, 2H), 6.97 (d, J = 1.8 Hz, 1H), 6.90 - 6.82 (m, 3H), 5.17 (s, 2H), 4.47 (t, J = 5.3 Hz, 1H), 4.01 (t, J = 6.0 Hz, 2H), 3.45 (t, J = 6.0 Hz, 2H), 3.42 (t, J = 5.9 Hz, 2H), 3.15 (t, J = 6.5 Hz, 2H), 3.06 (s, 3H). |
| 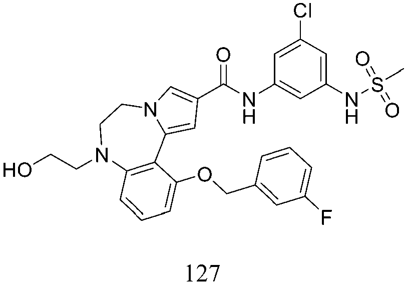 | 599.1[M+1] | 1H NMR (400 MHz, DMSO-*d₆*) δ 10.01 (s, 1H), 9.79 (s, 1H), 7.72 - 7.68 (m, 2H), 7.64 (t, J = 1.9 Hz, 1H), 7.40 -7.35 (m, 1H), 7.26 - 7.22 (m, 3H), 7.12 - 7.04 (m, 1H), 6.92 (t, J = 1.9 Hz, 1H), 6.90 (t, J = 2.0 Hz, 1H), 6.86 (dd, J = 8.3, 3.1 Hz, 2H), 5.16 (s, 2H), 4.00 (t, J = 5.9 Hz, 2H), 3.42 (m, 4H), 3.14 (t, J = 6.4 Hz, 2H), 3.07 (s, 3H). |
| 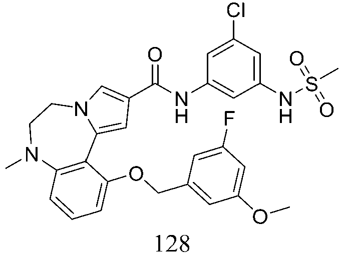 | 599.3[M+1] | 1H NMR (400 MHz, DMSO-*d₆*) δ 10.01 (s, 1H), 9.78 (s, 1H), 7.71 (d, J = 1.9 Hz, 1H), 7.69 (t, J = 1.9 Hz, 1H), 7.65 (t, J = 1.9 Hz, 1H), 7.28 (t, J = 8.3 Hz, 1H), 6.94 (d, J = 1.9 Hz, 1H), 6.90 (t, J = 1.9 Hz, 1H), 6.87 - 6.84 (m, 2H), 6.81 - 6.77 (m, 2H), 6.70 (dt, J = 11.0, 2.4 Hz, 1H), 5.13 (s, 2H), 4.03 (t, J = 5.9 Hz, 2H), 3.74 (s, 3H), 3.37 (t, J = 6.0 Hz, 2H), 3.07 (s, 3H), 2.72 (s, 3H). |
| 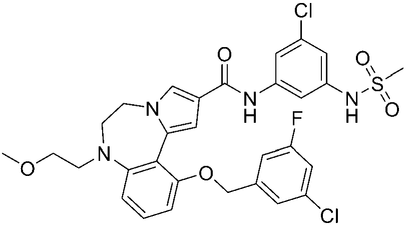 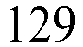 | 647.2[M+1] | ¹H NMR (400 MHz, DMSO-*d₆*) δ10.00 (s, 1H), 9.79 (s, 1H), 7.70 (t, J = 1.9 Hz, 2H), 7.65 (t, J = 2.0 Hz, 1H), 7.35 - 7.30 (m, 2H), 7.29 - 7.24 (m, 1H), 7.24 - 7.20 (m, 1H), 6.97 (d, J = 1.8 Hz, 1H), 6.90 - 6.85 (m, 3H), 5.18 (s, 2H), 4.01 (t, J = 6.0 Hz, 2H), 3.44 (t, J = 6.0 Hz, 2H), 3.37 (t, J = 5.8 Hz, 2H), 3.24 (t, J = 5.8 Hz, 2H), 3.14 (s, 3H), 3.02 (s, 3H). |
| 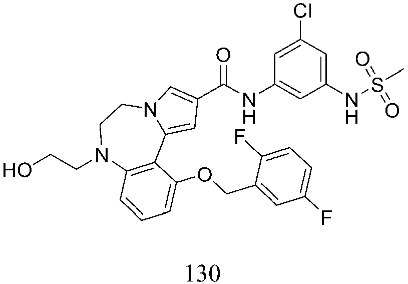 | 617.2[M+1] | ¹H NMR (400 MHz, DMSO-*d₆*) δ 10.00 (s, 1H), 9.77 (s, 1H), 7.69 (t, J = 1.9 Hz, 2H), 7.62 (t, J = 1.9 Hz, 1H), 7.33 - 7.25 (m, 3H), 7.22 - 7.16 (m, 1H), 6.92 - 6.87 (m, 4H), 5.16 (s, 2H), 4.00 (t, J = 5.9 Hz, 2H), 3.45 - 3.41 (m, 5H), 3.15 (t, J = 6.5 Hz, 2H), 3.07 (s, 3H). |
| 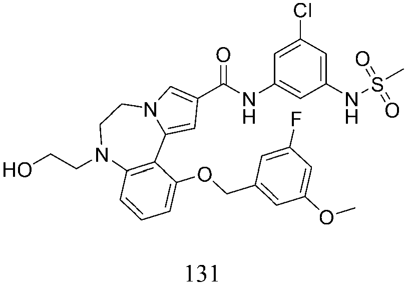 | 629.2[M+1] | ¹H NMR (400 MHz, DMSO-*d₆*) δ 10.01 (s, 1H), 9.80 (s, 1H), 7.70 (s, 1H), 7.68 (s, 1H), 7.65 (s, 1H), 7.27 (t, *J* = 8.0 Hz, 1H), 6.94 (s, 1H), 6.92 - 6.83 (m, 4H), 6.79 (d, *J* = 8.9 Hz, 1H), 6.70 (d, *J* = 10.9 Hz, 1H), 5.12 (s, 2H), 4.87-4.86 (m, 1H), 4.01 (t, *J* = 5.6 Hz, 2H), 3.70 (s, 3H), 3.48-3.38 (m, 4H), 3.15 (t, *J* = 6.3 Hz, 2H), 3.07 (s, 3H). |
| 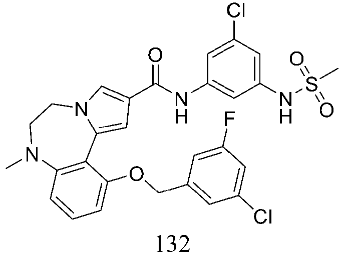 | 603.2[M+1] | ¹H NMR (400 MHz, DMSO-*d₆*) δ10.00 (s, 1H), 9.79 (s, 1H), 7.72-7.68 (m, 2H), 7.66 (s, 1H), 7.37 - 7.19 (m, 4H), 6.97 (s, 1H), 6.90 (s, 1H), 6.85 (d, *J =* 8.3 Hz, 1H), 6.80 (d, *J* = 8.1 Hz, 1H), 5.18 (s, 2H), 4.03 (t, *J* = 5.6 Hz, 2H), 3.38 (t, *J =* 5.6 Hz, 2H), 3.07 (s, 3H), 2.72 (s, 3H). |
| 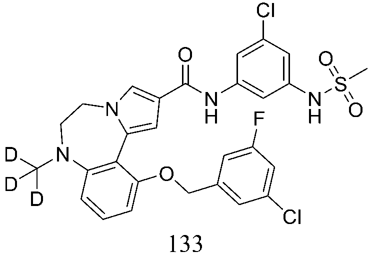 | 606.2[M+1] | ¹H NMR (400 MHz, DMSO-*d₆*) δ 10.00 (s, 1H), 9.79 (s, 1H), 7.72-7.68 (m, 2H), 7.66 (s, 1H), 7.37 - 7.19 (m, 4H), 6.98 (s, 1H), 6.90 (t*, J =* 1.7 Hz, 1H), 6.85 (d, *J =* 8.3 Hz, 1H), 6.79 (d, *J* = 8.1 Hz, 1H), 5.18 (s, 2H), 4.06-4.02(m, 2H), 3.38 (t, *J =* 5.8 Hz, 2H), 3.07 (s, 3H). |

Compounds of Examples 134-135 were prepared according to the preparation method of Example 67 of the present disclosure, and specific structures and structural characterizations were as follows:

| Serial number and structure of example | MS m/z (ESI) | ¹H NMR |
|---|---|---|
| 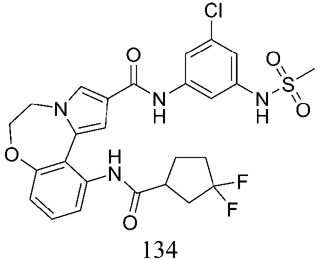 | 579.1[M+1] | ¹H NMR (400 MHz, DMSO-*d₆*) δ 10.00 (s, 1H), 9.82 (s, 1H), 9.48 (s, 1H), 7.79 (d, *J* = 1.6 Hz, 1H), 7.70 (t, *J* = 1.6 Hz, 1H), 7.60 (t*, J =* 1.6 Hz, 1H), 7.35-7.28 (m, 2H), 7.01 (dd, *J =* 7.6, 0.8 Hz, 1H), 6.89 (t, *J* = 2.0 Hz, 1H), 6.85 (d, *J* = 1.6 Hz, 1H), 4.42 (t, *J* = 5.6 Hz, 2H), 4.17 (t, *J* = 5.6 Hz, 2H), 3.08 - 3.03 (m, 4H), 2.34-2.25 (m, 2H), 2.19 - 2.10 (m, 1H), 2.07 - 1.98 (m, 2H), 1.96 - 1.88 (m, 1H). |
| 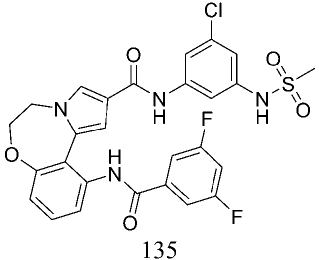 | 587.1[M+1] | ¹H NMR (400 MHz, DMSO-*d₆*) δ 10.06 (s, 1H), 9.97 (s, 1H), 9.78 (s, 1H), 7.80 (d, *J* = 1.6 Hz, 1H), 7.62 (t, *J* = 1.6 Hz, 1H), 7.59-7.54 (m, 3H), 7.51-7.45 (m, 1H), 7.43 (d, *J* = 7.2 Hz, 1H), 7.38 (t, *J =* 8.0 Hz, 1H), 7.10 (dd, *J =* 8.0, 0.8 Hz, 1H), 6.90 (d, *J* = 1.6 Hz, 1H), 6.87 (t, *J* = 1.6 Hz, 1H), 4.45 (t, *J =* 5.6 Hz, 2H), 4.19 (t, *J* = 5.2 Hz, 2H), 3.03 (s, 3H). |

Compounds of Examples 136-138 were prepared according to the preparation method of Example 63 of the present disclosure, and specific structures and structural characterizations were as follows:

| Serial number and structure of example | MS m/z (ESI) | ¹H NMR |
|---|---|---|
| 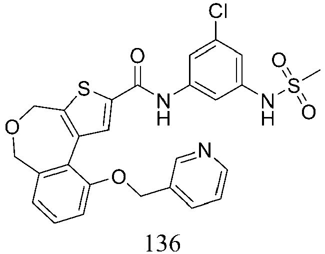 | 556.2[M+1] | ¹H NMR (400 MHz, DMSO-*d₆*) δ 10.47 (s, 1H), 10.10 (s, 1H), 8.69 (s, 1H), 8.49 (s, 2H), 7.89 (d, *J* = 8.0 Hz, 1H), 7.75 - 7.58 (m, 2H), 7.49 - 7.37 (m, 1H), 7.37 - 7.32 (m, 2H), 7.19 (d, *J* = 7.2 Hz, 1H), 7.00 (s, 1H), 5.30 (s, 2H), 4.48 (s, 2H), 4.29 (s, 2H), 3.09 (s, 3H). |
| 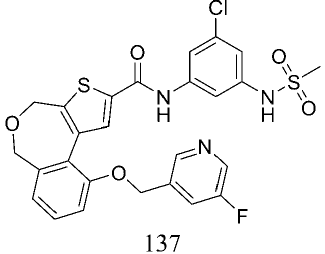 | 574.0[M+1] | ¹H NMR (400 MHz, DMSO-*d₆*) δ 10.47 (s, 1H), 10.22 (s, 1H), 8.54 (s, 1H), 8.52 (s, 1H), 8.48 (d, *J =* 2.6 Hz, 1H), 7.83 (d, *J* = 9.2 Hz, 1H), 7.70 - 7.58 (m, 2H), 7.50 - 7.40 (m, 1H), 7.33 (d, *J* = 8.4 Hz, 1H), 7.21 (d, *J =* 7.2 Hz, 1H), 6.96 (t, *J =* 2.0 Hz, 1H), 5.35 (s, 2H), 4.48 (s, 2H), 4.30 (s, 2H), 3.06 (s, 3H). |
| 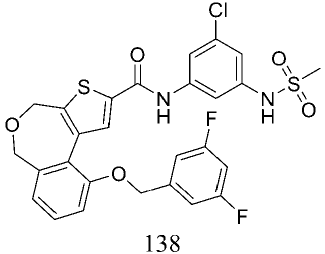 | 591.2[M+1] | ¹H NMR (400 MHz, DMSO-*d₆*) δ 10.50 (s, 1H), 10.18 (s, 1H), 8.56 (s, 1H), 7.65 - 7.59 (m, 2H), 7.43 (t, *J =* 8.0 Hz, 1H), 7.27 (d, *J =* 8.0 Hz, 1H), 7.23 - 7.16 (m, 3H), 7.11 (t, *J* = 9.2 Hz, 1H), 6.96 (s, 1H), 5.28 (s, 2H), 4.50 (s, 2H), 4.30 (s, 2H), 3.05 (s, 3H). |

Compounds of Examples 139-142 were prepared according to the preparation method of Example 64 of the present disclosure, and specific structures and structural characterizations were as follows:

| Serial number and structure of example | MS m/z (ESI) | ¹H NMR |
|---|---|---|
| 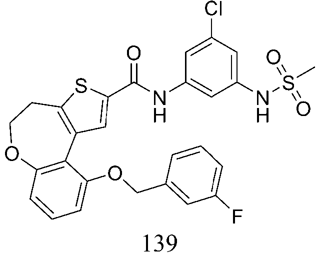 | 573.2[M+1] | ¹H NMR (400 MHz, DMSO-*d₆*) δ 10.38 (s, 1H), 10.06 (s, 1H), 8.42 (s, 1H), 7.65 (s, 2H), 7.35 - 7.27 (m, 4H), 7.06 (mz, 2H), 6.96 (t, *J =* 2.0 Hz, 1H), 6.85 (d, *J =* 8.0 Hz, 1H), 5.23 (s, 2H), 4.52 (t, *J* = 6.4 Hz, 2H), 3.07 (s, 3H), 3.02 (t, *J =* 6.4 Hz, 2H). |
| 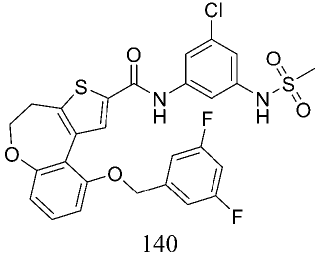 | 591.1[M+1] | ¹H NMR (400 MHz, DMSO-*d₆*) δ 10.41 (s, 1H), 10.08 (s, 1H), 8.47 (s, 1H), 7.68 (t, *J =* 1.6 Hz, 1H), 7.65 (t, *J* = 1.6 Hz, 1H), 7.35 (t, *J =* 8.4 Hz, 1H), 7.20 (d, *J =* 6.4 Hz, 2H), 7.14-7.09 (m, 1H), 7.04 (d, *J* = 8.4 Hz, 1H), 6.97 (t, *J* = 2.0 Hz, 1H), 6.89 (d, *J =* 8.4 Hz, 1H), 5.25 (s, 2H), 4.55 (t, *J =* 6.4 Hz, 2H), 3.08 (s, 3H), 3.04 (t, *J =* 6.4 Hz, 2H). |
| 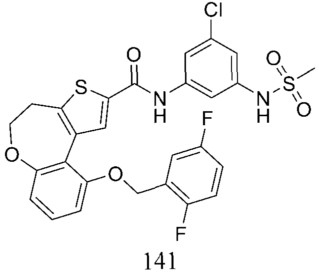 | 591.2[M+1] | ¹H NMR (400 MHz, DMSO-*d₆*) δ10.35 (s, 1H), 10.06 (s, 1H), 8.37 (s, 1H), 7.63 (m, 2H), 7.39 - 7.31 (m, 2H), 7.24 (m, 1H), 7.18 - 7.09 (m, 2H), 6.95 (t, *J =* 2.0 Hz, 1H), 6.88 (d, *J* = 7.8 Hz, 1H), 5.23 (s, 2H), 4.52 (t, *J* = 6.4 Hz, 2H), 3.07 (s, 3H), 3.02 (t, *J* = 6.4 Hz, 2H). |
| 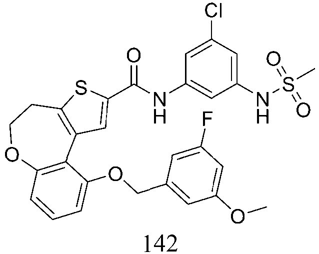 | 603.2[M+1] | ¹H NMR (400 MHz, DMSO-*d₆*) δ 10.38 (s, 1H), 10.06 (s, 1H), 8.42 (s, 1H), 7.64 (m, 2H), 7.32 (t, *J* = 8.0 Hz, 1H), 7.03 (d, *J* = 8.4 Hz, 1H), 6.96 (t, *J* = 2.0 Hz, 1H), 6.85 (m, 3H), 6.68 (m, 1H), 5.18 (s, 2H), 4.52 (t, *J* = 6.4 Hz, 2H), 3.61 (s, 3H), 3.07 (s, 3H), 3.02 (t, *J =* 6.4 Hz, 2H). |

Compounds of Examples 143-146 were prepared according to the preparation method of Example 65 of the present disclosure, and specific structures and structural characterizations were as follows:

| Serial number and structure of example | MS m/z (ESI) | ¹H NMR |
|---|---|---|
| 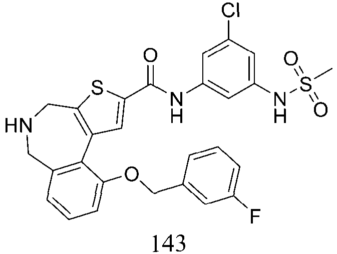 | 572.1[M+1] | ¹H NMR (400 MHz, DMSO-*d₆*) δ 10.44 (s, 1H), 8.45 (s, 1H), 8.16 (s, 1H), 7.65 (d, *J =* 2.0 Hz, 2H), 7.40 - 7.24 (m, 4H), 7.20 (d, *J =* 8.4 Hz, 1H), 7.12 - 7.03 (m, 2H), 6.96 (t, *J =* 2.0 Hz, 1H), 5.24 (s, 2H), 3.75 (s, 2H), 3.55 (s, 2H), 3.07 (s, 3H). |
| 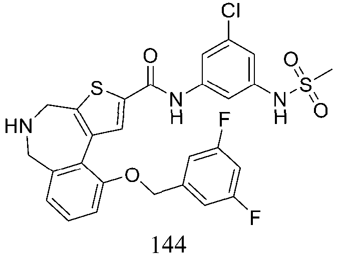 | 590.2[M+1] | ¹H NMR (400 MHz, DMSO-*d₆*) δ 10.42 (s, 1H), 8.47 (s, 1H), 8.17 (s, 1H), 7.65 (d, *J* = 1.6 Hz, 1H), 7.63 (d, *J* = 1.6 Hz, 1H), 7.36 (t, *J =* 8.0 Hz, 1H), 7.20 - 7.05 (m, 5H), 6.96 (t, *J* = 2.0 Hz, 1H), 5.24 (s, 2H), 3.84 - 3.63 (m, 2H), 3.55 - 3.49 (m, 2H), 3.06 (s, 3H). |
| 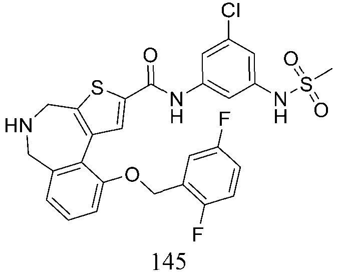 | 590.0[M+1] | ¹H NMR (400 MHz, DMSO-*d₆*) δ 10.45 (s, 1H), 8.43 (s, 1H), 8.17 (s, 1H), 7.69 - 7.60 (m, 2H), 7.46 - 7.39 (m, 1H), 7.39 - 7.30 (m, 2H), 7.29 - 7.22 (m, 1H), 7.21 - 7.12 (m, 2H), 6.98 (t, *J* = 2.0 Hz, 1H), 5.27 (s, 2H), 3.82 (s, 2H), 3.63 (s, 2H), 3.09 (s, 3H). |
| 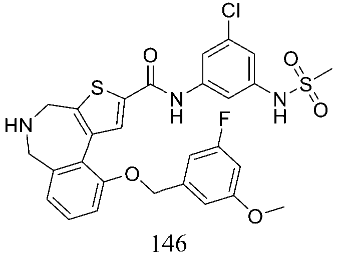 | 602.2[M+1] | ¹H NMR (400 MHz, DMSO-*d₆*) δ 10.43 (s, 1H), 8.45 (s, 1H), 8.16 (s, 1H),7.65 (t, *J* = 2.0 Hz, 1H), 7.62 (t, *J* = 2.0 Hz, 1H), 7.40 - 7.32 (m, 1H), 7.19 (d, *J* = 8.0 Hz, 1H), 7.08 (d, *J* = 8.0 Hz, 1H), 6.96 (t, *J =* 2.0 Hz, 1H), 6.88 - 6.79 (m, 2H), 6.68 (dt, *J =* 11.2, 2.4 Hz, 1H), 5.19 (s, 2H), 3.73 (s, 2H), 3.62 (s, 3H), 3.53 (s, 2H), 3.07 (s, 3H). |

Compounds of Examples 147-150 were prepared according to the preparation method of Example 66 of the present disclosure, and specific structures and structural characterizations were as follows:

| Serial number and structure of example | MS m/z (ESI) | ¹H NMR |
|---|---|---|
| 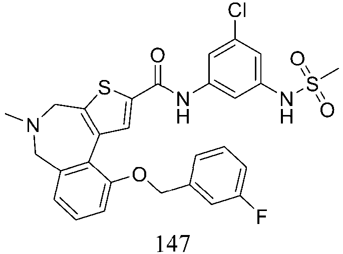 | 586.0[M+1] | ¹H NMR (400 MHz, DMSO-*d₆*) δ 10.46 (s, 1H), 8.49 (s, 1H), 8.13 (s, 1H), 7.65 (d, *J* = 2.0 Hz, 2H), 7.40 - 7.32 (m, 2H), 7.31 - 7.26 (m, 2H), 7.22 (d, *J* = 8.4 Hz, 1H), 7.14 - 7.04 (m, 2H), 6.97 (t, *J =* 2.0 Hz, 1H), 5.25 (s, 2H), 3.54 (s, 2H), 3.30 (s, 2H), 3.08 (s, 3H), 2.39 (s, 3H). |
| 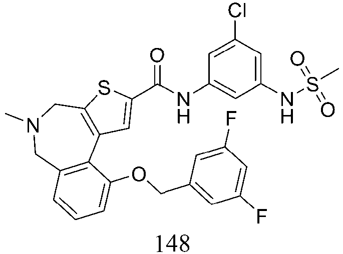 | 604.2[M+1] | ¹H NMR (400 MHz, DMSO-*d₆*) δ 10.46 (s, 1H), 8.52 (s, 1H), 8.13 (s, 1H), 7.65 (d, *J =* 2.0 Hz, 1H), 7.64 (d, *J =* 2.0 Hz, 1H), 7.39 (t, *J =* 8.0 Hz, 1H), 7.23 - 7.08 (m, 5H), 6.96 (t, *J* = 2.0 Hz, 1H), 5.25 (s, 2H), 3.54 (s, 2H), 3.23 (s, 2H), 3.07 (s, 3H), 2.39 (s, 3H). |
| 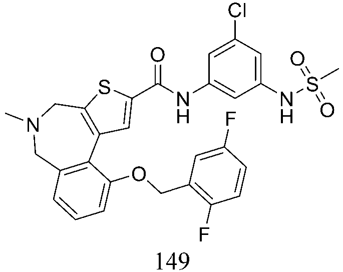 | 604.1[M+1] | ¹H NMR (400 MHz, DMSO-*d₆*) δ 10.41 (s, 1H), 10.08 (s, 1H), 8.42 (s, 1H), 7.63 (d, *J =* 2.0 Hz, 2H), 7.40 - 7.21 (m, 4H), 7.19 - 7.10 (m, 2H), 6.96 (t, *J =* 2.0 Hz, 1H), 5.25 (s, 2H), 3.46 (s, 2H), 3.23 (s, 2H), 3.07 (s, 3H), 2.34 (s, 3H). |
| 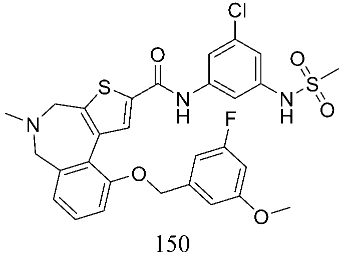 | 616.1[M+1] | ¹H NMR (400 MHz, DMSO-*d₆*) δ 10.45 (s, 1H), 10.08 (s, 1H), 8.49 (s, 1H), 7.65 (t, *J =* 2.0 Hz, 1H), 7.63 (t, *J =* 2.0 Hz, 1H), 7.37 (t, *J* = 8.0 Hz, 1H), 7.21 (d*, J =* 8.4 Hz, 1H), 7.11 (d, *J =* 7.6 Hz, 1H), 6.97 (t, *J* = 2.0 Hz, 1H), 6.88 - 6.80 (m, 2H), 6.69 (dt, *J* = 11.2, 2.4 Hz, 1H), 5.20 (s, 2H), 3.62 (s, 3H), 3.51 (s, 2H), 3.26 (s, 2H), 3.07 (s, 3H), 2.37 (s, 3H). |

Compounds of Examples 151-154 were prepared according to the preparation method of Example 68 of the present disclosure, and specific structures and structural characterizations were as follows:

| Serial number and structure of example | MS m/z (ESI) | ¹H NMR |
|---|---|---|
| 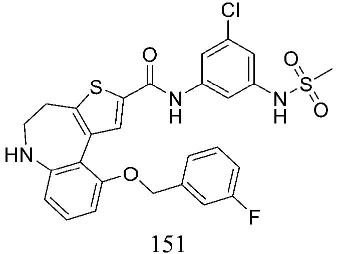 | 572.1[M+1] | ¹H NMR (400 MHz, DMSO-*d₆*) δ 10.26 (s, 1H), 8.39 (s, 1H), 7.64 - 7.58 (m, 2H), 7.34 - 7.25 (m, 3H), 7.08 - 7.00 (m, 2H), 6.93 (t, *J* = 2.0 Hz, 1H), 6.59 (dd, *J* = 15.2, 8.0 Hz, 2H), 5.57 (s, 1H), 5.12 (s, 2H), 3.57 (t, *J* = 5.6 Hz, 2H), 3.04 (s, 3H), 2.98 (t, *J =* 5.6 Hz, 2H). |
| 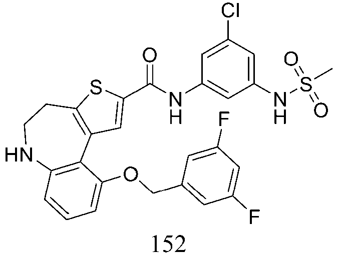 | 590.2[M+1] | ¹H NMR (400 MHz, DMSO-*d₆*) δ 10.27 (s, 1H), 10.14 (s, 1H), 8.41 (s, 1H), 7.63 (d, *J =* 1.6 Hz, 1H), 7.60 (d, *J* = 1.6 Hz, 1H), 7.18 (d, *J* = 6.4 Hz, 2H), 7.06 (t, *J =* 8.0 Hz, 2H), 6.93 (t, *J* = 2.0 Hz, 1H), 6.58 (dd, *J =* 8.0, 2.0 Hz, 2H), 5.60 (s, 1H), 5.13 (s, 2H), 3.57 (t, *J =* 4.4 Hz, 2H), 3.04 (s, 3H), 2.98 (t, *J =* 5.6 Hz, 2H). |
| 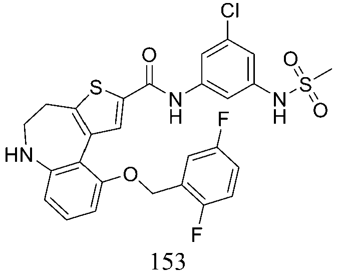 | 590.1[M+1 | ¹H NMR (400 MHz, DMSO-*d₆*) δ 10.22 (s, 1H), 9.93 (s, 1H), 8.33 (s, 1H), 7.61 (d, *J* = 2.0 Hz, 1H), 7.58 (t, *J* = 2.0 Hz, 1H), 7.40 - 7.33 (m, 1H), 7.23 -7.17 (m, 1H), 7.15 - 7.09 (m, 1H), 7.06 (t, *J* = 8.0 Hz, 1H), 6.93 (t, *J* = 2.0 Hz, 1H), 6.68 - 6.63 (m, 1H), 6.61 - 6.56 (m, 1H), 5.60 (t, *J* = 3.6 Hz, 1H), 5.13 (s, 2H), 3.57 (td, *J =* 5.6, 3.6 Hz, 2H), 3.05 (s, 3H), 2.98 (t, *J =* 5.6 Hz, 2H). |
| 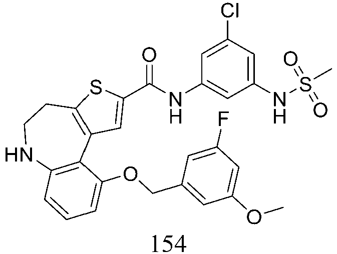 | 602.1[M+1 | ¹H NMR (400 MHz, DMSO-*d₆*) δ 10.27 (s, 1H), 10.01 (s, 1H), 8.39 (s, 1H), 7.64 (t, *J =* 2.0 Hz, 1H), 7.59 (t, *J* = 2.0 Hz, 1H), 7.06 (t, *J =* 8.0 Hz, 1H), 6.94 (t, *J =* 2.0 Hz, 1H), 6.89 - 6.83 (m, 2H), 6.67 - 6.62 (m, 1H), 6.61 - 6.55 (m, 2H), 5.56 (t, *J =* 3.6 Hz, 1H), 5.08 (s, 2H), 3.66 - 3.52 (m, 5H), 3.05 (s, 3H), 2.98 (t, *J =* 5.6 Hz, 2H). |

Compounds of Examples 155-158 were prepared according to the preparation method of Example 19 of the present disclosure, and specific structures and structural characterizations were as follows:

| Serial number and structure of example | MS m/z (ESI) | ¹H NMR |
|---|---|---|
| 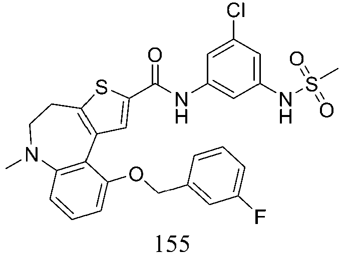 | 586.2[M+1] | ¹H NMR (400 MHz, DMSO-*d₆*) δ 10.29 (s, 1H), 10.06 (s, 1H), 8.29 (s, 1H), 7.64 (d, *J =* 1.2 Hz, 2H), 7.33 - 7.23 (m, 4H), 7.05 (t, *J =* 8.8 Hz, 1H), 6.95 (t, *J* = 2.0 Hz, 1H), 6.89 (d, *J* = 8.0 Hz, 1H), 6.83 (d, *J* = 8.0 Hz, 1H), 5.16 (s, 2H), 3.44 (t*, J =* 6.4 Hz, 2H), 3.07 (s, 3H), 2.84 (t, *J =* 6.4 Hz, 2H), 2.71 (s, 3H). |
| 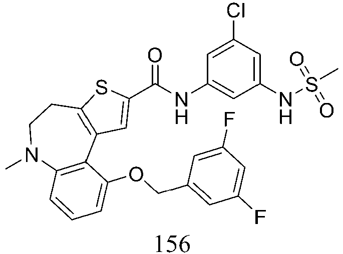 | 604.1[M+1] | ¹H NMR (400 MHz, DMSO-*d₆*) δ 10.29 (s, 1H), 10.05 (s, 1H), 8.32 (s, 1H), 7.64 (t, *J =* 2.0 Hz, 1H), 7.62 (t, *J* = 2.0 Hz, 1H), 7.30 (t, *J =* 8.0 Hz, 1H), 7.16 - 7.05 (m, 3H), 6.95 (t, *J =* 2.0 Hz, 1H), 6.88 (d, *J* = 8.4 Hz, 1H), 6.85 (d, *J= 8.4* Hz, 1H), 5.17 (s, 2H), 3.44 (t, *J* = 6.4 Hz, 2H), 3.06 (s, 3H), 2.85 (t, *J=* 6.4 Hz, 2H), 2.72 (s, 3H). |
| 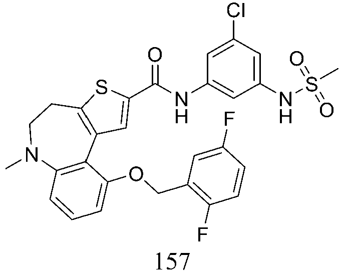 | 604.2[M+1] | ¹H NMR (400 MHz, DMSO-*d₆*) δ 10.26 (s, 1H), 10.05 (s, 1H), 8.23 (s, 1H), 7.64 - 7.60 (m, 2H), 7.33 - 7.26 (m, 2H), 7.25 - 7.17 (m, 1H), 7.17 - 7.09 (m, 1H), 7.01 - 6.92 (m, 2H), 6.88 - 6.82 (m, 1H), 5.17 (s, 2H), 3.44 (t, *J =* 6.4 Hz, 2H), 3.06 (s, 3H), 2.84 (t, *J =* 6.4 Hz, 2H), 2.71 (s, 3H). |
| 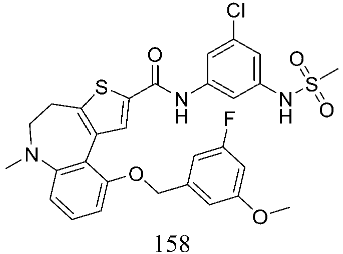 | 616.2[M+1] | ¹H NMR (400 MHz, DMSO-*d₆*) δ 10.28 (s, 1H), 10.05 (s, 1H), 8.29 (s, 1H), 7.64 (t, *J =* 2.0 Hz, 1H), 7.60 (t, *J =* 2.0 Hz, 1H), 7.30 (t, *J =* 8.0 Hz, 1H), 6.95 (t, *J =* 2.0 Hz, 1H), 6.89 (d, *J =* 8.4 Hz, 1H), 6.86 - 6.78 (m, 3H), 6.69 - 6.63 (m, 1H), 5.12 (s, 2H), 3.60 (s, 3H), 3.44 (t, *J =* 6.4 Hz, 2H), 3.06 (s, 3H), 2.85 (t, *J =* 6.4 Hz, 2H), 2.71 (s, 3H). |

### Biological evaluation

### Test Example 1. Determination of inhibitory effects of the compounds of the present disclosure on CCRF- CEM cell proliferation

Influences of the compounds of the present disclosure on CCRF-CEM cell proliferation were determined by the following method. CCRF-CEM cells (MSI-H cells) were purchased from the Cell Bank of the Chinese Academy of Sciences, and cultured in a RPMI-1640 medium containing 10% fetal bovine serum, 100 U/mL penicillin and 100 µg/mL streptomycin. Cell viability was determined with a CellTiter-Glo^{®} Luminescent Cell Viability Assay kit (Promega, Cat. No. G7573).

An experimental method was operated according to steps in the instruction of the kit, which was briefly described as follows: a test compound was dissolved in DMSO first to prepare a 10 mM stock solution, then the stock solution was diluted with the above medium to prepare a test sample, and a final concentration of the compound was in a range of 10000 nM-1.52 nM. Cells in a logarithmic growth phase were seeded into a 96-well cell culture plate at a density of 1000 cells per well. Subsequently, the test compound was added, and then the cells were incubated at 37°C in an air incubator for 120 hours. After the culture was complete, 50 µL of CellTiter-Glo assay reagent was added to each well, the plate was shaken for 5 minutes and then allowed to stand for 10 minutes, and subsequently, a luminescence value of each well of the sample was read by a microplate reader in a Luminescence mode. By comparing with a numerical value of a control group (0.1% DMSO), a percentage inhibition rate of the compound at each concentration was calculated, and then nonlinear regression analysis was carried out based on concentration logarithm-inhibition rate of the compound in GraphPad Prism 9 software, so as to obtain an IC₅₀ value of the compound in inhibition of cell proliferation, which can be seen in Table 1.

**Table 1 IC₅₀ data of the compounds of the present disclosure in the inhibition of CCRF-CEM cell proliferation**

| Serial number of example | IC₅₀ (nM) | Serial number of example | IC₅₀ (nM) | Serial number of example | IC₅₀ (nM) |
|---|---|---|---|---|---|
| 5 | 18.6 | 59 | 7.2 | 96 | 80.4 |
| 7 | 12.5 | 60 | 4.1 | 97 | 54.2 |
| 8 | 12.2 | 61 | 64.1 | 99 | 76.8 |
| 9 | 19.7 | 62 | 5.3 | 100 | 18 |
| 10 | 16.4 | 64 | 25.1 | 101 | 1.6 |
| 11 | 62.9 | 65 | 6.2 | 102 | 4.6 |
| 12 | 36.9 | 66 | 37.3 | 103 | 4.5 |
| 13 | 11.6 | 67 | 1.2 | 104 | 13.4 |
| 14 | 27.6 | 68 | 12.4 | 105 | 25.2 |
| 17 | 76.9 | 69 | 1.9 | 107 | 4.9 |
| 22 | 53 | 70 | 2.6 | 108 | 17.2 |
| 23 | 79 | 71 | 11.96 | 109 | 29 |
| 29 | 51.6 | 72 | 11.2 | 112 | 4.0 |
| 30 | 65.9 | 73 | 6.5 | 113 | 6.9 |
| 31 | 34.1 | 74 | 7.0 | 114 | 3.7 |
| 32 | 49.3 | 75 | 7.3 | 115 | 20.5 |
| 35 | 99.6 | 76 | 2.0 | 116 | 61.1 |
| 36 | 65.8 | 79 | 63.1 | 117 | 51.2 |
| 39 | 21.8 | 80 | 12.5 | 135 | 0.64 |
| 41 | 32.5 | 81 | 2.3 | 137 | 14.7 |
| 42 | 63.6 | 82 | 9.1 | 138 | 20.6 |
| 43 | 41.8 | 83 | 64.7 | 139 | 7.4 |
| 45 | 14.9 | 86 | 24.8 | 140 | 3.0 |
| 48 | 18.1 | 87 | 76.1 | 141 | 14 |
| 50 | 22.1 | 88 | 7.7 | 142 | 8.3 |
| 51 | 40.1 | 89 | 13.2 | 143 | 0.96 |
| 52 | 61.6 | 90 | 4.6 | 144 | 0.61 |
| 53 | 88.3 | 91 | 10.3 | 147 | 11.3 |
| 54 | 67.9 | 92 | 23.4 | 148 | 5.7 |
| 56 | 28.4 | 93 | 50.3 | 152 | 28.9 |
| 57 | 10.7 | 94 | 26.5 | 156 | 28.7 |
| 58 | 10.4 | 95 | 28.8 | ATX-968 | 104 |

Conclusion: the compounds of the present disclosure had relatively good inhibitory effects on CCRF-CEM cell proliferation IC₅₀<100 nM.

Note: the structure of ATX-968 (prepared in Example 31 according to published patent WO2023154519A1) was as follows:

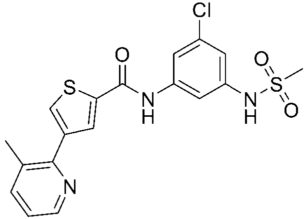

### Test Example 2. Determination of inhibitory effects of the compounds of the present disclosure on LS411N cell proliferation

Influences of the compounds of the present disclosure on LS411N cell proliferation were determined by the following method. LS411N cells (MSI-H cells) were purchased from the American Type Culture Collection (ATCC), and cultured in a RPMI-1640 medium containing 10% fetal bovine serum, 100 U/mL penicillin and 100 µg/mL streptomycin. Cell viability was determined with a CellTiter-Glo^{®} Luminescent Cell Viability Assay kit (Promega, Cat. No. G7573).

An experimental method was operated according to steps in the instruction of the kit, which was briefly described as follows: a test compound was dissolved in DMSO first to prepare a 10 mM stock solution, then the stock solution was diluted with the above medium to prepare a test sample, and a final concentration of the compound was in a range of 10000 nM-0.152 nM. Cells in a logarithmic growth phase were seeded into a 96-well cell culture plate at a density of 1000 cells per well. Subsequently, the test compound was added, and then the cells were incubated at 37°C in an air incubator for 120 hours. After the culture was complete, 50 µL of CellTiter-Glo assay reagent was added to each well, the plate was shaken for 5 minutes and then allowed to stand for 10 minutes, and subsequently, a luminescence value of each well of the sample was read by a microplate reader in a Luminescence mode. By comparing with a numerical value of a control group (0.1% DMSO), a percentage inhibition rate of the compound at each concentration was calculated, and then nonlinear regression analysis was carried out based on concentration logarithm-inhibition rate of the compound in GraphPad Prism 9 software, so as to obtain an IC₅₀ value of the compound in inhibition of cell proliferation, which can be seen in Table 2.

**Table 2 IC₅₀ data of the compounds of the present disclosure in the inhibition of LS411N cell proliferation**

| Serial number of example | IC₅₀ (nM) | Serial number of example | IC₅₀ (nM) | Serial number of example | IC₅₀ (nM) |
|---|---|---|---|---|---|
| 8 | 79 | 64 | 16.1 | 88 | 4.0 |
| 10 | 84.9 | 65 | 6.0 | 89 | 13.4 |
| 12 | 61.5 | 66 | 23.6 | 90 | 1.9 |
| 13 | 88.8 | 67 | 17.1 | 91 | 21.6 |
| 14 | 18.7 | 68 | 38.2 | 92 | 16.2 |
| 17 | 54.5 | 69 | 33.3 | 93 | 74.3 |
| 22 | 39.6 | 70 | 25.6 | 100 | 35.4 |
| 32 | 67 | 71 | 11.1 | 102 | 12.5 |
| 37 | 26.9 | 72 | 10.9 | 103 | 3.9 |
| 48 | 93.5 | 73 | 3.3 | 107 | 10.4 |
| 52 | 19.3 | 74 | 44.7 | 108 | 11.1 |
| 53 | 23.6 | 75 | 8.3 | 112 | 36.2 |
| 54 | 22 | 76 | 16.2 | 113 | 23.7 |
| 55 | 70.9 | 80 | 131 | 115 | 24.7 |
| 56 | 64.4 | 81 | 5.2 | 135 | 25.1 |
| 57 | 17.4 | 82 | 6.1 | 137 | 21.7 |
| 58 | 57.5 | 83 | 75.3 | 138 | 27 |
| 60 | 8.8 | 86 | 29.7 | 140 | 4.7 |
| 62 | 25 | 87 | 66.1 | ATX-968 | 130 |

Conclusion: the compounds of the present disclosure had relatively good inhibitory effects on LS411N cell proliferation IC₅₀<100 nM.

### Test Example 3. Testing of inhibitory effects of the compounds of the present disclosure on hDHX9 enzyme (ATPase)

Inhibition degrees of the compounds of the present disclosure against recombinant human DExH-box helicase 9 (hDHX9, ATPase) activity in vitro were determined by the following method. An ADP-Glo^{™} Kinase Assay kit (Cat. No. V9102) from Promega Company was used in this method. Detailed experimental operation referred to the instruction of the kit.

An experimental procedure was briefly described as follows: a test compound was first dissolved in DMSO to prepare a stock solution, then serial dilution was carried out with a reaction buffer (40 mM HEPES, pH7.5, 20 mM MgCl₂, 0.01% Tween-20, 0.01% BSA, 1 mM DTT, 0.004 U/ml RNaseOUT^{™} Recombinant Ribonuclease Inhibitor), and a final concentration of the test compound in the reaction system ranged from 10000 nM to 0.04 nM; and a hDHX9 protein (entrusted to Nanjing GenScript Biotechnology Co., Ltd. for expression and purification) was prepared using the reaction buffer, and a double-stranded RNA substrate for DHX9 was entrusted to Suzhou GENEWIZ Biotechnology Co., Ltd. for synthesis and annealing (with sequence 1: 5'-GAAUUAACCAAGGAAAAUAACAAGGACAGGGACCAGG-3' and sequence 2: 5'-GCCUGGUCCCUGUCCUUGUUAUUUUCCUUGGUUAAUU-3'). The reaction was carried out in a 384-well microplate. The test compound and the recombinant human DHX9 protein (final concentration 6.3 nM) were added to the wells and incubated at room temperature for 15 minutes. Subsequently, a double-stranded RNA solution (final concentration 18.8 nM) and an ATP solution (final concentration 30 µM, derived from Component V915A of the ADP-Glo^{™} Kinase Assay Kit) were added to the reaction solution, and the mixture was incubated at room temperature for 45 minutes. Subsequently, 5 µL of ADP-Glo Reagent was added to the reaction system, and the mixture was incubated at room temperature for 50 minutes. Then, 10 µL of Kinase Detection Reagent was added to the reaction system, and the mixture was incubated at room temperature for 30 minutes. After the incubation was complete, a chemiluminiscence intensity value of each well was determined by a microplate reader in a Luminescence mode. By comparing with a luminescence intensity ratio of a control group (0.1% DMSO), a percentage inhibition rate of the compound at each concentration was calculated, and nonlinear regression analysis was carried out based on concentration logarithm value-inhibition rate of the compound in GraphPad Prism 9 software, so as to obtain an IC₅₀ value of the compound, which can be seen in Table 3.

**Table 3 IC₅₀ data of the compounds of the present disclosure in the inhibition of hDHX9 enzyme (ATPase) activity**

| Serial number of example | IC₅₀ (nM) | Serial number of example | IC₅₀ (nM) | Serial number of example | IC₅₀ (nM) |
|---|---|---|---|---|---|
| 1 | 7.7 | 49 | 1.1 | 93 | 92.9 |
| 3 | 78.2 | 51 | 12.9 | 94 | 26.5 |
| 5 | 31 | 52 | 6.1 | 95 | 4.0 |
| 7 | 1.7 | 53 | 2.4 | 96 | 6.3 |
| 8 | 10.8 | 54 | 1.2 | 97 | 5.8 |
| 10 | 9.4 | 55 | 57.5 | 98 | 10.9 |
| 11 | 2.1 | 56 | 8.9 | 99 | 3.6 |
| 12 | 4.7 | 57 | 31.8 | 102 | 2.4 |
| 13 | 6.0 | 58 | 1.6 | 103 | 10.6 |
| 14 | 1.6 | 60 | 1.8 | 106 | 17.2 |
| 15 | 13.3 | 61 | 4.8 | 112 | 4.4 |
| 17 | 11.6 | 62 | 5.9 | 113 | 4.4 |
| 18 | 13.4 | 63 | 26.3 | 114 | 29.5 |
| 19 | 58.5 | 64 | 26.1 | 115 | 32.9 |
| 21 | 22.7 | 66 | 2.1 | 134 | 2.6 |
| 22 | 1.1 | 67 | 1.2 | 135 | 0.9 |
| 23 | 5.7 | 68 | 50.6 | 136 | 1.6 |
| 24 | 75.9 | 69 | 4.3 | 137 | 1.6 |
| 26 | 28.1 | 70 | 2.3 | 138 | 25.1 |
| 27 | 3.5 | 71 | 5.8 | | |
| 28 | 11.3 | 72 | 47.2 | | |
| 29 | 12.6 | 73 | 8.3 | | |
| 30 | 14.1 | 74 | 3.6 | | |
| 31 | 1.9 | 75 | 47.5 | | |
| 32 | 26.7 | 76 | 6.1 | | |
| 33 | 14.4 | 77 | 68.8 | | |
| 34 | 34.5 | 78 | 16.6 | | |
| 35 | 6.6 | 79 | 2.9 | | |
| 36 | 8.3 | 80 | 33.6 | | |
| 37 | 24.6 | 81 | 0.16 | | |
| 39 | 4.0 | 82 | 12.2 | | |
| 40 | 5.9 | 83 | 30.4 | | |
| 41 | 3.2 | 86 | 10 | | |
| 42 | 3.6 | 87 | 45 | | |
| 43 | 4.2 | 88 | 9.2 | | |
| 44 | 6.0 | 89 | 28 | | |
| 45 | 2.4 | 90 | 6.4 | | |
| 46 | 6.2 | 91 | 2.6 | | |
| 47 | 3.1 | 92 | 17.6 | | |

Conclusion: the compounds of the present disclosure had better inhibitory effects on the hDHX9 enzyme (ATPase) activity IC₅₀<100 nM.

### Test Example 4. Pharmacokinetic testing of the compounds of the present disclosure in mice

### 1. Experimental purpose

ICR mice were taken as test animals, and intragastrically administered with a control compound ATX-968 and the compounds of the present disclosure, and then drug concentrations in plasma at different time points were determined by a LC/MS/MS method, so as to investigate in-vivo pharmacokinetic profiles of the compounds of the present disclosure in the mice.

### 2. Experimental scheme

### 2.1 Experimental drugs and animals

Control compound ATX-968, and Compounds 68 and 73.

ICR mice, male, 32.8-38.0 g, purchased from Vital River Laboratory Animal Technology Co., Ltd.

### 2.2 Preparation of drugs

An appropriate amount of the test compound was weighed, and DMA (N,N-dimethylacetamide), 30% Solutol HS15 (30% polyethylene glycol-15 hydroxystearate) and Saline were added sequentially. The mixture was mixed by ultrasonication and vortexing to prepare a 1 mg/mL dosing formulation, wherein DMA:30% Solutol HS15:Saline = 10:10:80 (v/v/v).

### 2.3 Administration

The ICR mice in each to-be-tested compound group (9 mice in each group) were fasted overnight and then intragastrically administered with the compound (PO, a dose of the compound was 10 mg/kg and a volume of the compound was 10 mL/kg), and food was provided 4 hours after administration.

### 3. Operation

Before administration, and at 0.25, 0.5, 1, 2, 4, 8, 10 and 24 hours after administration, approximately 0.1 mL of blood was collected via the orbital vein, and whole blood samples were transferred into anticoagulant tubes containing EDTA-K2. The collected blood samples were placed on ice, and plasma was separated by centrifugation (centrifugation conditions: 1500 g, 10 minutes). The collected plasma was stored at -40°C to -20°C before analysis.

A content of the to-be-tested compound in the plasma of the mice after intragastric administration was determined by LC-MS/MS.

### 4. Results of pharmacokinetic parameters

Pharmacokinetic parameters of to-be-tested compounds can be seen in Table 4 below.

**Table 4 Pharmacokinetic parameters of to-be-tested compounds in mice**

| Serial number of example | Pharmacokinetic experiment | | | |
|---|---|---|---|---|
| | Administration mode (Administration dosage) | Plasma concentration Cmax (ng/mL) | Area under the curve AUC₀₋ₜ (ng·h/mL) | Half-life period T_{1/2} (h) |
| ATX-968 | Oral administration (10 mg/kg) | 1930 | 1510 | 1.22 |
| 68 | Oral administration (10 mg/kg) | 12322 | 37339 | 2.35 |
| 73 | Oral administration (10 mg/kg) | 3230 | 11500 | 1.27 |

### Test Example 5. Pharmacodynamic testing of the compounds of the present disclosure in mice

### 1. Experimental purpose

Anti-tumor effects of the control compound ATX-968 and the Compounds 68 and 73 of the present disclosure in a BALB/c nude mouse model bearing LS411N human cecal cancer subcutaneous xenografts.

### 2. Experimental animals

BALB/c nude mice, female, 6-7 weeks old, purchased from Jiangsu Gempharmatech Biotechnology Co., Ltd. Animal production license number: SCXK (Su) 2023-0009, and animal certificate number: No. B202409230909.

### 3. Preparation of test compounds

A vehicle control group was administered with DMA:Solutol HS 15:20% HP-β-CD = 10:10:80 (v/v/v).

ATX-968: an appropriate amount of ATX-968 was weighed, appropriate amounts of DMA, Solutol HS 15 and 20% HP-β-CD were added sequentially, and the mixture was mixed by ultrasonication and vortexing to prepare a 20 mg/mL dosing formulation, wherein DMA, Solutol HS 15 and 20% HP-β-CD =10:10:80 (v: v: v)

Compound 68: an appropriate amount of Compound 68 was weighed, appropriate amounts of DMA, Solutol HS 15 and 20% HP-β-CD were added sequentially, and the mixture was mixed by ultrasonication and vortexing to prepare a 10 mg/mL dosing formulation, wherein DMA, Solutol HS 15 and 20% HP-β-CD =10:10:80 (v: v: v)

Compound 73: an appropriate amount of Compound 73 was weighed, appropriate amounts of DMA, Solutol HS 15 and 20% HP-β-CD were added sequentially, and the mixture was mixed by ultrasonication and vortexing to prepare a 10 mg/mL dosing formulation, wherein DMA, Solutol HS 15 and 20% HP-β-CD =10:10:80 (v: v: v)

### 4. LS411N cell culture

LS411N human cecal cancer cells were cultured in a RPMI Medium 1640 medium containing 10% fetal bovine serum and a 1% penicillin-streptomycin-amphotericin B solution. The LS411N cells in the exponential growth phase were collected and resuspended in a mixture of PBS and Matrigel (1: 1). The cell suspension was adjusted to an appropriate concentration for subcutaneous tumor inoculation on the right dorsal side of the BALB/c nude mice.

### 5. Animal inoculation and grouping

Female BALB/c nude mice were subcutaneously inoculated with approximately 4.0×10⁶ LS411N cells on the right dorsal side. When an average tumor volume reached about 100-250 mm³, the mice were randomly divided into groups according to tumor size, with 6 mice in each group.

### 6. Animal administration and observation

The animals in each group were given the test compound twice per day according to body weights of the animals at a fixed time point every day by oral administration (po), the animals started to be administered with the test compound for the first time on the day of grouping for 21 consecutive days, and the body weights the animals were recorded every day.
Group 1 (G1): vehicle control group;
Group 2 (G2): oral administration of ATX-968 at a dose of 200 mg/kg, twice daily (BID);
Group 3 (G3): oral administration of Compound 68 at a dose of 100 mg/kg, twice daily (BID);
Group 4 (G4): oral administration of Compound 73 at a dose of 100 mg/kg, twice daily (BID).

Tumor formulation at an inoculation site of each animal in each group was observed, and a tumor volume was measured twice a week. Calculation formulas for tumor volume (TV), relative tumor volume (RTV), relative tumor proliferation rate (T/C) and relative tumor growth inhibition rate (TGI) were as follows:
(1) $TV \left(tumor volume\right) = 1 / 2 \times a \times {b}^{2} ,$ wherein a and b respectively represented a tumor length and a tumor width;
(2) $RTV \left(relative tumor volume\right) = {V}_{t} / {V}_{0} ,$ wherein V₀ was a tumor volume measured at grouping, and Vₜ was a tumor volume at each measurement;
(3) $T / C \left(%\right) = {T}_{RTV} / {C}_{RTV} \times 100 % ,$ wherein T_{RTV} was RTV of the treatment group, and C_{RTV} was RTV of the solvent control group; and
(4) $TGI % = \left(1-T/C\right) \times 100 % ,$ wherein T and C were relative tumor volumes of the treatment group and the solvent control group at a specific time point respectively.

### 7. Results

The results can be seen in Table 5.

**Table 5 Pharmacodynamic analysis table of various groups of to-be-tested compounds in LS411N human cecal cancer subcutaneous xenograft model**

| Group | Day 21 after administration | | |
|---|---|---|---|
| | Tumor volume (x±S) | Relative tumor volume (x±S) | TGI% |
| Group 1 Solvent control group | 1001±131 | 4.80±0.17 | - |
| Group 2 ATX-968 (200 mg/kg) | 603±83 | 2.90±0.24 | 39.6% |
| Group 3 Example 68 (100 mg/kg) | 572±75 | 2.72±0.13 | 43.3% |
| Group 4 Example 73 (100 mg/kg) | 396±74 | 1.85±0.25 | 61.4% |

Note: the data were presented as "mean ± standard error".

Conclusion: under the conditions set in this experiment, in the mouse model bearing the LS411N human cecal cancer subcutaneous xenografts, compared with the vehicle and the ATX-968 at the dose of 200 mg/kg, Compounds 68 and 73 of the present disclosure exerted a more significant tumor growth inhibitory effect at a lower dose (100 mg/kg).

## Claims

1. A compound as shown in formula (I), or a stereoisomer thereof, a tautomer thereof or a pharmaceutically acceptable salt thereof: 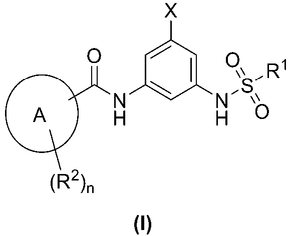 wherein:
a ring A is selected from tricyclic heteroaryl or a tricyclic fused ring;
X is selected from halogen;
R¹ is selected from C₁₋₆ alkyl or 3-5 membered cycloalkyl; wherein the alkyl or cycloalkyl is optionally further substituted with one or more substituents selected from halogen, hydroxyl, cyano, C₁₋₆ alkyl or C₁₋₆ alkoxy;
each R² is independently selected from a hydrogen atom, cyano, halogen, alkyl, alkenyl, alkynyl, cycloalkyl, heterocyclyl, aryl, heteroaryl, SF₅, -O(CH₂)tR^{A}, -C(O)R³, -C(O)OR³, -NHC(O)R³, -NHC(O)OR³, -NR⁴R⁵, -C(O)NR⁴R⁵, -S(O)₂N_{R}⁴R⁵, -CH₂NHC(O)OR³, -CH₂NR⁴R⁵ or -S(O)ᵣR³; wherein the alkyl, alkenyl, alkynyl, cycloalkyl, heterocyclyl, aryl or heteroaryl is optionally further substituted with one or more substituents selected from a deuterium atom, halogen, nitro, cyano, alkyl, haloalkyl, cycloalkyl, heterocyclyl, aryl, heteroaryl, =O, -OR^{A}, -C(O)R⁶, -C(O)OR⁶, -OC(O)R⁶, -NR⁷R⁸, -C(O)NR⁷R⁸, -SO₂NR⁷R⁸ or -NR⁷C(O)R⁸;
or, two R², together with the same carbon atom to which the groups are attached, form a -C(O) or cyclopropyl;
each R³ is independently selected from a hydrogen atom, alkyl, cycloalkyl, heterocyclyl, aryl or heteroaryl, wherein the alkyl, cycloalkyl, heterocyclyl, aryl or heteroaryl is optionally further substituted with one or more substituents selected from hydroxyl, halogen, nitro, cyano, alkyl, alkoxy, haloalkyl, haloalkoxy, cycloalkyl, heterocyclyl, aryl, heteroaryl, =O, -C(O)R⁶, -C(O)OR⁶, -OC(O)R⁶, -NR⁷R⁸, -C(O)NR⁷R⁸, -SO₂NR⁷R⁸ or -NR⁷C(O)R⁸;
R^{A} is selected from a hydrogen atom, alkyl, cycloalkyl, heterocyclyl, aryl or heteroaryl, wherein the alkyl, cycloalkyl, heterocyclyl, aryl or heteroaryl is optionally further substituted with one or more substituents selected from hydroxyl, halogen, nitro, cyano, alkyl, alkoxy, haloalkyl, haloalkoxy, cycloalkyl, heterocyclyl, aryl, heteroaryl, =O, -C(O)R⁶, -C(O)OR⁶, -OC(O)R⁶, -NR⁷R⁸, -C(O)NR⁷R⁸, -SO₂NR⁷R⁸ or -NR⁷C(O)R⁸;
R⁴ and R⁵ are each independently selected from a hydrogen atom, hydroxyl, alkyl, alkoxy, cycloalkyl, heterocyclyl, aryl or heteroaryl, wherein the alkyl, alkoxy, cycloalkyl, heterocyclyl, aryl or heteroaryl is optionally further substituted with one or more substituents selected from hydroxyl, halogen, nitro, cyano, alkyl, alkoxy, cycloalkyl, heterocyclyl, aryl, heteroaryl, =O, -C(O)R⁶, -C(O)OR⁶, -OC(O)R⁶, -NR⁷R⁸, -C(O)NR⁷R⁸, -SO₂NR⁷R⁸ or -NR⁷C(O)R⁸;
or, R⁴ and R⁵, together with atoms to which the groups are attached, form a 4-8 membered heterocyclyl, wherein the 4-8 membered heterocyclyl contains one or more N, O or S(O)ᵣ, and the 4-8 membered heterocyclyl is optionally further substituted with one or more substituents selected from hydroxyl, halogen, nitro, cyano, alkyl, alkoxy, cycloalkyl, heterocyclyl, aryl, heteroaryl, =O, -C(O)R⁶, -C(O)OR⁶, -OC(O)R⁶, -NR⁷R⁸, -C(O)NR⁷R⁸, -SO₂NR⁷R⁸ or -NR⁷C(O)R⁸.
R⁶, R⁷ and R⁸ are each independently selected from a hydrogen atom, alkyl, amino, cycloalkyl, heterocyclyl, aryl or heteroaryl, wherein the alkyl, cycloalkyl, heterocyclyl, aryl or heteroaryl is optionally further substituted with one or more substituents selected from hydroxyl, halogen, nitro, amino, cyano, alkyl, alkoxy, cycloalkyl, heterocyclyl, aryl, heteroaryl, carboxyl or a carboxylate group;
t is selected from 0 or 1;
n is selected from 0, 1, 2, 3, 4, 5, 6, 7 or 8; and
each r is independently 0, 1 or 2.

2. The compound, or the stereoisomer thereof, the tautomer thereof or the pharmaceutically acceptable salt thereof according to claim 1, wherein ring A is selected from a 12-18 membered tricyclic fused ring or 12-18 membered tricyclic heteroaryl, preferably a 12-16 membered tricyclic fused ring or 12-14 membered tricyclic heteroaryl.

3. The compound, or the stereoisomer thereof, the tautomer thereof or the pharmaceutically acceptable salt thereof according to claim 1 or 2, wherein R² is selected from a hydrogen atom, halogen, cyano, C₁₋₆ alkyl, amino, SF₅, -O(CH₂)ₜR^{A}, -C(O)OR³, -NHC(O)R³ or -C(O)R³, wherein the C₁₋₆ alkyl is optionally further substituted with one or more substituents selected from a deuterium atom, halogen, cyano, C₁₋₆ alkyl, -C(O)OR⁶ or -OR^{A};
or, two R², together with the same carbon atom to which the groups are attached, form a -C(O) or cyclopropyl;
R^{A} is selected from a hydrogen atom, C₁₋₆ alkyl, C₃₋₈ cycloalkyl, C₆₋₁₀ aryl, 5-6 membered heteroaryl or 5-6 membered heterocyclyl, wherein the C₁₋₆ alkyl, C₃₋₈ cycloalkyl, C₆₋₁₀ aryl, 5-6 membered heteroaryl or 5-6 membered heterocyclyl is optionally further substituted with one or more substituents selected from hydroxyl, halogen, cyano, -NR⁷R⁸, C₁₋₆ alkyl, C₁₋₆ haloalkyl or C₁₋₆ alkoxy;
R³ is selected from a hydrogen atom, C₃₋₈ cycloalkyl, C₆₋₁₀ aryl or C₁₋₆ alkyl, wherein the C₃₋₈ cycloalkyl, C₆₋₁₀ aryl or C₁₋₆ alkyl is optionally further substituted with one or more substituents selected from halogen, hydroxyl, cyano, C₁₋₆ alkyl or C₁₋₆ alkoxy;
R⁶ is selected from a hydrogen atom or C₁₋₆ alkyl;
R⁷ and R⁸ are each independently selected from a hydrogen atom or C₁₋₆ alkyl; and
t is selected from 0 or 1.

4. The compound, or the stereoisomer thereof, the tautomer thereof or the pharmaceutically acceptable salt thereof according to any one of claims 1 to 3, wherein R² is selected from a hydrogen atom, halogen, C₁₋₆ alkyl, hydroxyl, cyano, C₁₋₆ alkoxy, SF₅, C₁₋₆ haloalkyl or C₁₋₆ haloalkoxy;
or, two R², together with the same carbon atom to which the groups are attached, form a -C(O) group.

5. The compound, or the stereoisomer thereof, the tautomer thereof or the pharmaceutically acceptable salt thereof according to any one of claims 1 to 4, which is a compound as shown in formula (IIA), or a stereoisomer thereof, a tautomer thereof or a pharmaceutically acceptable salt thereof: 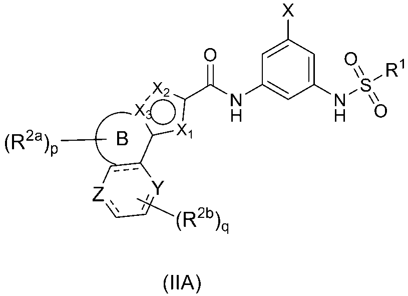 wherein: 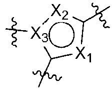
is 5-membered heteroaryl;
X₁ and X₂ are each independently selected from CH, S or N, and X₁ and X₂ are not S at the same time;
X₃ is selected from C or N; provided that when X₃ is C, X₁ and X₂ are not CH at the same time;
ring B is selected from 5-8 membered heterocyclyl, 6-10 membered aryl or 5-6 membered heteroaryl, wherein the heterocyclyl contains 1 or 2 double bonds;
-̅ -̅ -̅ is selected from a single bond or a double bond as required, so that each atom thereof is present in a normal valence state;
Y and Z are each independently selected from CR^{a}, CHR^{a}, O, N or NR^{b};
R^{2a} is selected from a hydrogen atom, C₃₋₈ cycloalkyl, C₁₋₆ alkyl or -C(O)R³, wherein the C₁₋₆ alkyl is optionally further substituted with one or more substituents selected from a deuterium atom, halogen, hydroxyl, cyano, C₁₋₆ alkyl, C₁₋₆ alkoxy or -C(O)OR⁶;
or, two R^{2a}, together with the same carbon atom to which the groups are attached, form a -C(O) or cyclopropyl;
each R^{2b} is independently selected from a hydrogen atom, C₁₋₆ alkyl, C₁₋₆ alkoxy or -C(O)OR³; wherein the C₁₋₆ alkyl or C₁₋₆ alkoxy is optionally further substituted with one or more substituents selected from hydroxyl, halogen or cyano;
R^{a} and R^{b} are each independently selected from a hydrogen atom, halogen, cyano, C₁₋₆ alkyl, amino, -NHC(O)R³ or -O(CH₂)ₜR^{A}, wherein the C₁₋₆ alkyl is optionally further substituted with one or more substituents selected from halogen, cyano, C₁₋₆ alkyl or -OR^{A};
each R^{A} is independently selected from a hydrogen atom, C₁₋₆ alkyl, C₃₋₈ cycloalkyl, C₆₋₁₀ aryl, 5-6 membered heteroaryl or 5-6 membered heterocyclyl, wherein the C₁₋₆ alkyl, C₃₋₈ cycloalkyl, C₆₋₁₀ aryl, 5-6 membered heteroaryl or 5-6 membered heterocyclyl is optionally further substituted with one or more substituents selected from hydroxyl, halogen, cyano, -NR⁷R⁸, C₁₋₆ alkyl, C₁₋₆ haloalkyl or C₁₋₆ alkoxy;
R³ is selected from a hydrogen atom, C₆₋₁₀ aryl, C₃₋₈ cycloalkyl or C₁₋₆ alkyl, wherein the C₆₋₁₀ aryl, C₃₋₈ cycloalkyl or C₁₋₆ alkyl is optionally further substituted with one or more substituents selected from halogen, hydroxyl, cyano, C₁₋₆ alkyl or C₁₋₆ alkoxy;
R⁶ is selected from a hydrogen atom or C₁₋₆ alkyl;
R⁷ and R⁸ are each independently selected from a hydrogen atom or C₁₋₆ alkyl;
t is selected from 0 or 1;
each p is independently selected from 0, 1, 2, 3, 4 or 5;
each q is independently selected from 0, 1 or 2; and
R¹ and X are as defined in claim 1.

6. The compound, or the stereoisomer thereof, the tautomer thereof or the pharmaceutically acceptable salt thereof according to claim 5, which is a compound as shown in formula (IIIA) or (IIIB), or a stereoisomer thereof, a tautomer thereof or a pharmaceutically acceptable salt thereof: 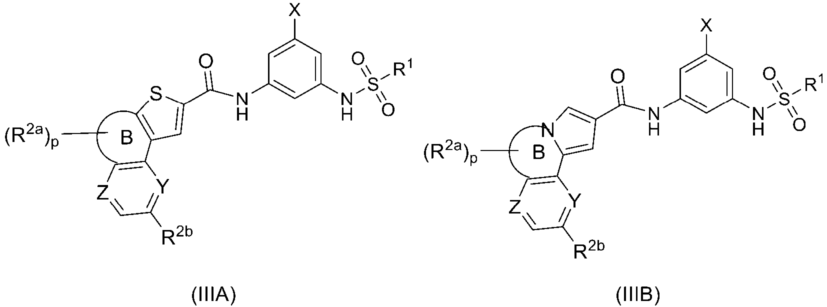 wherein:
ring B is selected from 5-8 membered heterocyclyl, wherein the heterocyclyl contains 1 or 2 double bonds;
Y and Z are each independently selected from CR^{a} or N;
R^{2a} is selected from a hydrogen atom, C₁₋₆ alkyl or -C(O)R³; wherein the C₁₋₆ alkyl is optionally further substituted with one or more substituents selected from halogen, hydroxyl, cyano, C₁₋₆alkyl, C₁₋₆ alkoxy or -C(O)OR⁶;
or, two R^{2a}, together with the same carbon atom to which the groups are attached, form a -C(O) or cyclopropyl;
each R^{2b} is independently selected from a hydrogen atom, C₁₋₆ alkyl, C₁₋₆ alkoxy or -C(O)OR³;
each R^{a} is independently selected from a hydrogen atom, halogen, cyano, C₁₋₆ alkyl, amino, -NHC(O)R³ or -O(CH₂)ₜR^{A}; wherein the C₁₋₆ alkyl is optionally further substituted with one or more substituents selected from halogen, cyano, C₁₋₆ alkyl or -OR^{A};
each R^{A} is independently selected from a hydrogen atom, C₁₋₆ alkyl, C₃₋₈ cycloalkyl, C₆₋₁₀ aryl, 5-6 membered heteroaryl or 5-6 membered heterocyclyl, wherein the C₁₋₆ alkyl, C₃₋₈ cycloalkyl, C₆₋₁₀ aryl, 5-6 membered heteroaryl or 5-6 membered heterocyclyl is optionally further substituted with one or more substituents selected from hydroxyl, halogen, cyano, -NR⁷R⁸, C₁₋₆ alkyl, C₁₋₆ haloalkyl or C₁₋₆ alkoxy;
R³ is selected from a hydrogen atom, C₆₋₁₀ aryl or C₁₋₆ alkyl, wherein the C₆₋₁₀ aryl or C₁₋₆ alkyl is optionally further substituted with one or more substituents selected from halogen, hydroxyl, cyano, C₁₋₆ alkyl or C₁₋₆ alkoxy;
R⁶ is selected from a hydrogen atom or C₁₋₆ alkyl;
R⁷ and R⁸ are each independently selected from a hydrogen atom or C₁₋₆ alkyl;
t is selected from 0 or 1;
each p is independently selected from 0, 1, 2, 3, 4 or 5; and
R¹ and X are as defined in claim 5.

7. The compound, or the stereoisomer thereof, the tautomer thereof or the pharmaceutically acceptable salt thereof according to claim 5, which is a compound as shown in formula (IIIC), or a stereoisomer thereof, a tautomer thereof or a pharmaceutically acceptable salt thereof: 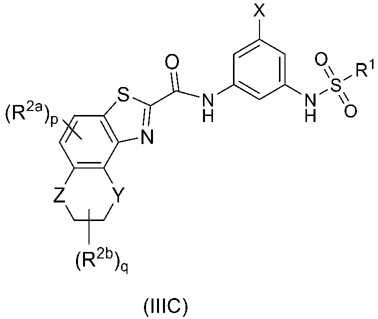 wherein:
Y and Z are each independently selected from CHR^{a}, O or NR^{b};
R^{2a} is selected from a hydrogen atom or C₁₋₆ alkyl, wherein the C₁₋₆ alkyl is optionally further substituted with one or more substituents selected from halogen, hydroxyl, cyano, C₁₋₆ alkyl or C₁₋₆ alkoxy;
each R^{2b} is independently selected from a hydrogen atom or C₁₋₆ alkyl;
R^{a} and R^{b} are each independently selected from a hydrogen atom, halogen, hydroxyl, cyano or C₁₋₆ alkyl, wherein the C₁₋₆ alkyl is optionally further substituted with one or more substituents selected from halogen, hydroxyl, cyano, C₁₋₆ alkyl or C₁₋₆ alkoxy;
each p is independently selected from 0, 1 or 2;
each q is independently selected from 0, 1 or 2; and
R¹ and X are as defined in claim 5.

8. The compound, or the stereoisomer thereof, the tautomer thereof or the pharmaceutically acceptable salt thereof according to any one of claims 1 to 4, which is a compound as shown in formula (II), or a stereoisomer thereof, a tautomer thereof or a pharmaceutically acceptable salt thereof: 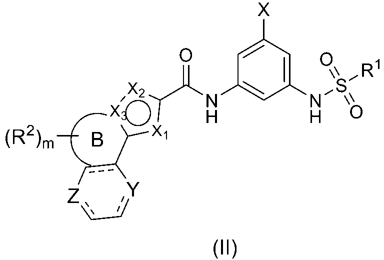 wherein: 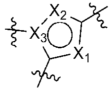
is 5-membered heteroaryl;
X₁ and X₂ are each independently selected from CH, S or N, and X₁ and X₂ are not S at the same time;
X₃ is selected from C or N; provided that when X₃ is C, X₁ and X₂ are not CH at the same time;
ring B is selected from 5-8 membered heterocyclyl, 6-10 membered aryl or 5-6 membered heteroaryl, wherein the heterocyclyl contains 1 or 2 double bonds; 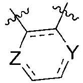
is 6-membered heteroaryl or 6-membered heterocyclyl;
-̅ -̅ -̅ is selected from a single bond or a double bond as required, so that each atom thereof is present in a normal valence state;
Y and Z are each independently selected from CR^{a}, O or NR^{b};
R^{a} is selected from a hydrogen atom, halogen, hydroxyl, cyano, C₁₋₆ alkyl or C₁₋₆ alkoxy, wherein the alkyl or alkoxy is optionally further substituted with one or more substituents selected from halogen, hydroxyl, cyano, C₁₋₆ alkyl or C₁₋₆ alkoxy;
R^{b} is selected from a hydrogen atom or C₁₋₆ alkyl, wherein the alkyl is optionally further substituted with one or more substituents selected from halogen, hydroxyl, cyano, C₁₋₆ alkyl or C₁₋₆ alkoxy;
m is selected from 0, 1, 2 or 3; and
R¹, R² and X are as defined in claim 1.

9. The compound, or the stereoisomer thereof, the tautomer thereof or the pharmaceutically acceptable salt thereof according to any one of claims 1 to 4, which is a compound as shown in formula (III), or a stereoisomer thereof, a tautomer thereof or a pharmaceutically acceptable salt thereof: 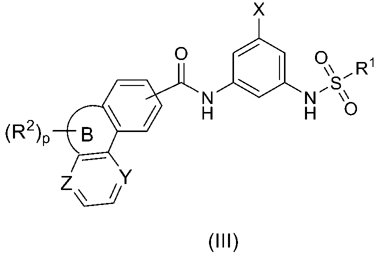 wherein:
ring B is selected from 5-8 membered heterocyclyl or 5-6 membered heteroaryl, wherein the heterocyclyl contains 1 or 2 double bonds;
Y and Z are each independently selected from CR^{a} or N;
R^{a} is selected from a hydrogen atom, halogen, hydroxyl, cyano, C₁₋₆ alkyl or C₁₋₆ alkoxy, wherein the C₁₋₆ alkyl or C₁₋₆ alkoxy is optionally further substituted with one or more substituents selected from halogen, hydroxyl, cyano, C₁₋₆ alkyl or C₁₋₆ alkoxy;
p is selected from 0, 1, 2 or 3; and
R¹, R² and X are as defined in claim 1.

10. The compound, or the stereoisomer thereof, the tautomer thereof or the pharmaceutically acceptable salt thereof according to any one of claims 1 to 4, wherein 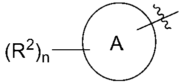 is selected from the following groups: 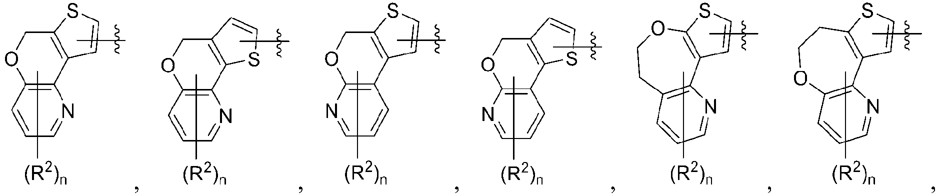 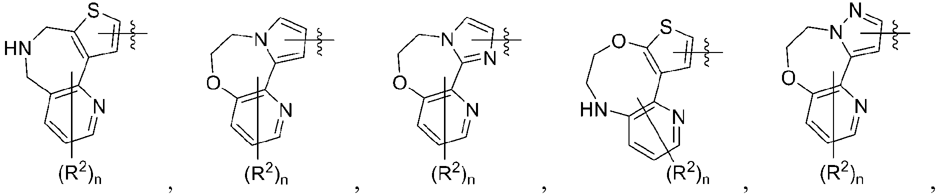 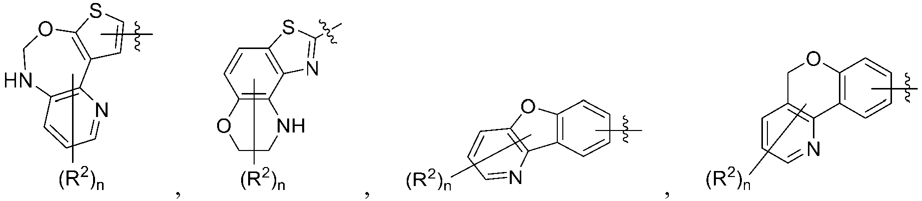 or 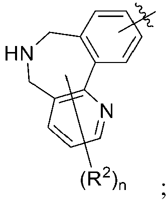 and
R² and n are as defined in claim 1.

11. The compound, or the stereoisomer thereof, the tautomer thereof or the pharmaceutically acceptable salt thereof according to claim 10, wherein 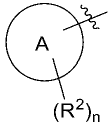 is selected from the following groups:

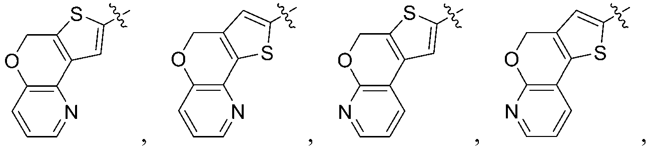

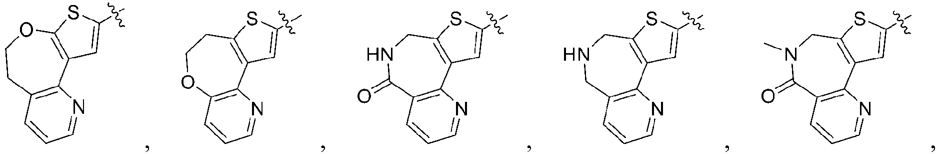

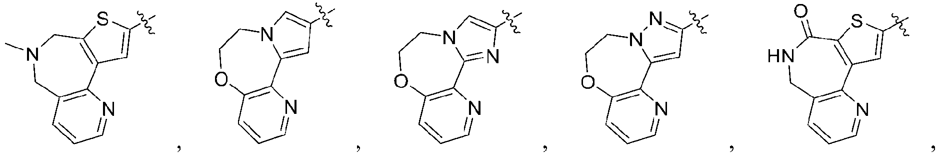

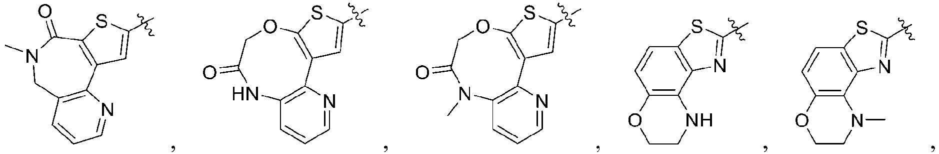

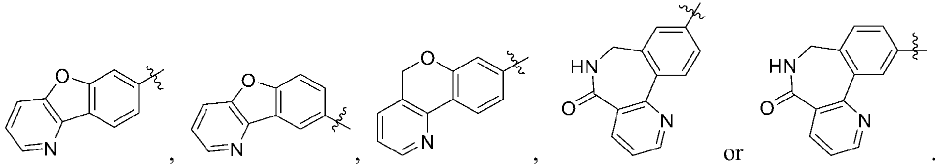

12. The compound, or the stereoisomer thereof, the tautomer thereof or the pharmaceutically acceptable salt thereof according to claim 5, wherein 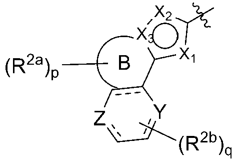 is selected from the following groups: 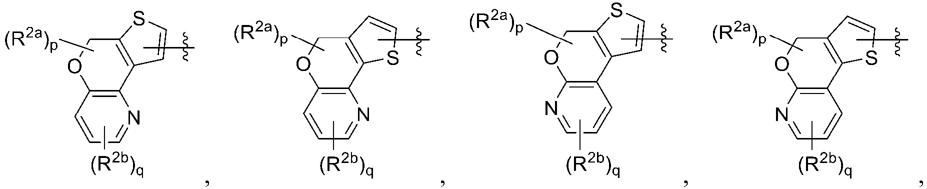 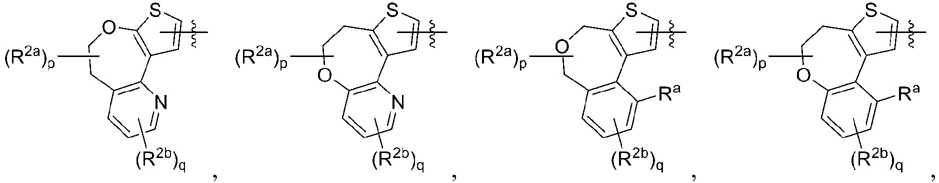 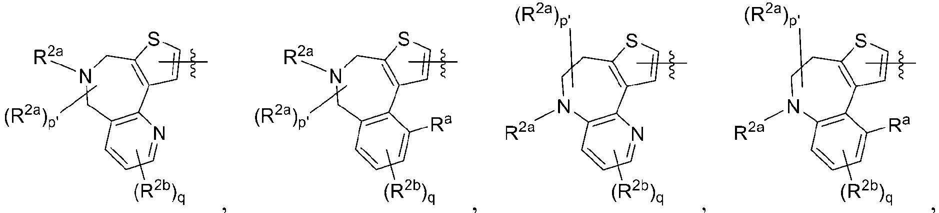 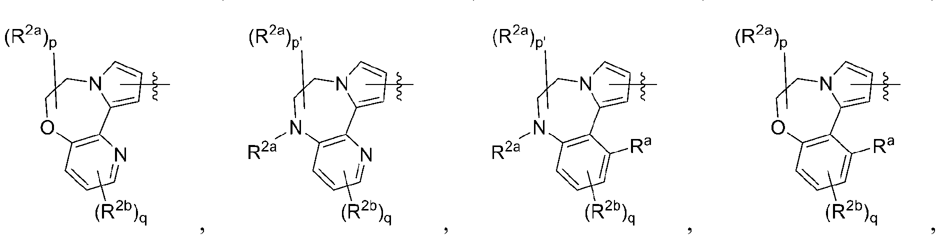 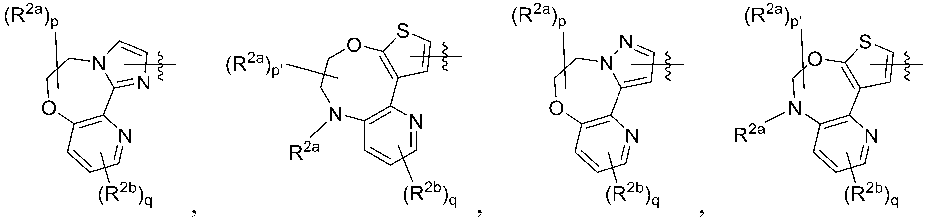 or 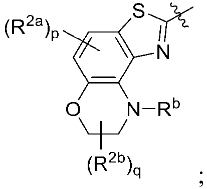
R^{2a} is selected from a hydrogen atom, C₁₋₆ alkyl, C₃₋₈ cycloalkyl or -C(O)R³, wherein the C₁₋₆ alkyl is optionally further substituted with one or more substituents selected from a deuterium atom, halogen, hydroxyl, cyano, C₁₋₆ alkyl, C₁₋₆ alkoxy or -C(O)OR⁶;
or, two R^{2a}, together with the same carbon atom to which the groups are attached, form a -C(O) or cyclopropyl;
each R^{2b} is independently selected from a hydrogen atom, C₁₋₆ alkyl, C₁₋₆ alkoxy or -C(O)OR³;
each R^{a} is independently selected from a hydrogen atom, halogen, cyano, C₁₋₆ alkyl, amino, -NHC(O)R³ or -O(CH₂)ₜR^{A}, wherein the C₁₋₆ alkyl is optionally further substituted with one or more substituents selected from halogen, cyano, C₁₋₆ alkyl or -OR^{A};
R^{b} is selected from a hydrogen atom or C₁₋₆ alkyl, wherein the C₁₋₆ alkyl is optionally further substituted with one or more halogens;
R^{A} is selected from a hydrogen atom, C₁₋₆ alkyl, C₃₋₈ cycloalkyl, C₆₋₁₀ aryl, 5-6 membered heteroaryl or 5-6 membered heterocyclyl, wherein the C₁₋₆ alkyl, C₃₋₈ cycloalkyl, C₆₋₁₀ aryl, 5-6 membered heteroaryl or 5-6 membered heterocyclyl is optionally further substituted with one or more substituents selected from hydroxyl, halogen, cyano, -NR⁷R⁸, C₁₋₆ alkyl, C₁₋₆ haloalkyl or C₁₋₆ alkoxy;
R³ is selected from a hydrogen atom, C₆₋₁₀ aryl, C₃₋₈ cycloalkyl or C₁₋₆ alkyl, wherein the C₆₋₁₀ aryl, C₃₋₈ cycloalkyl or C₁₋₆ alkyl is optionally further substituted with one or more substituents selected from halogen, hydroxyl, cyano, C₁₋₆ alkyl or C₁₋₆ alkoxy;
R⁶ is selected from a hydrogen atom or C₁₋₆ alkyl;
R⁷ and R⁸ are each independently selected from a hydrogen atom or C₁₋₆ alkyl;
t is selected from 0 or 1;
each p is independently selected from 0, 1, 2, 3, 4 or 5;
each p' is independently selected from 0, 1, 2, 3 or 4; and
each q is independently selected from 0, 1 or 2.

13. The compound, or the stereoisomer thereof, the tautomer thereof or the pharmaceutically acceptable salt thereof according to claim 12, wherein R^{2a} is selected from a hydrogen atom, methyl, ethyl, isopropyl, 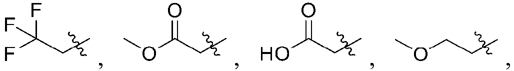 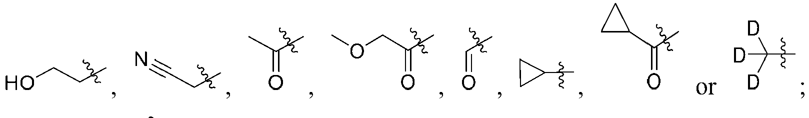 or, two R^{2a}, together with the same carbon atom to which the groups are attached, form a -C(O) or cyclopropyl.

14. The compound, or the stereoisomer thereof, the tautomer thereof or the pharmaceutically acceptable salt thereof according to claim 12, wherein R^{a} is selected from a hydrogen atom, hydroxyl, cyano, methyl, methoxy, trifluoromethyl, trifluoromethoxy, amino, 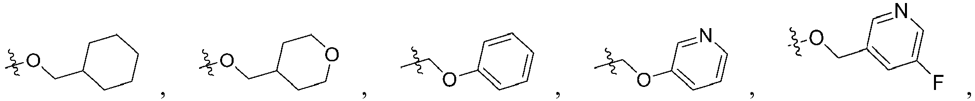 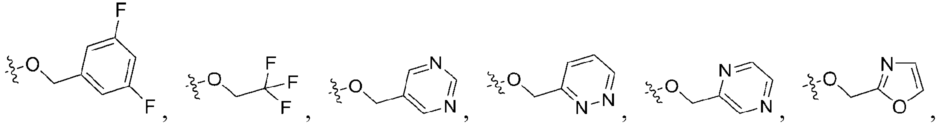 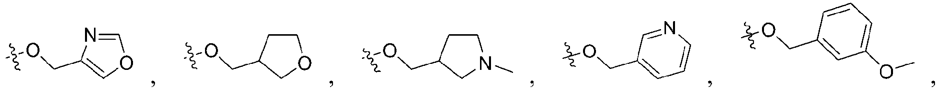 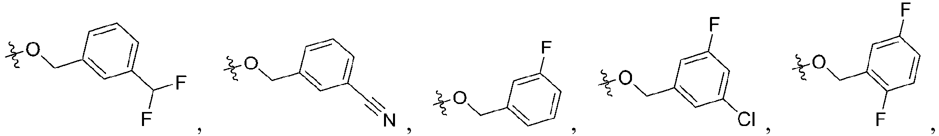 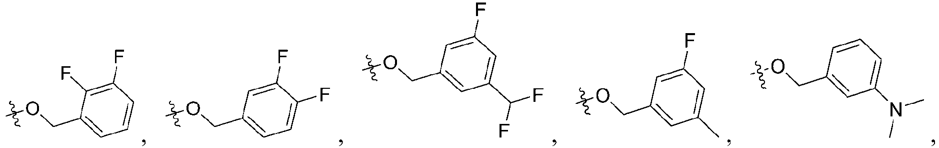 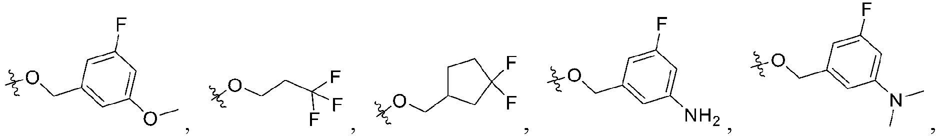 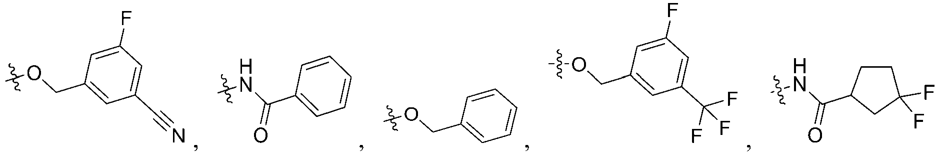 or

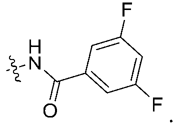

15. The compound, or the stereoisomer thereof, the tautomer thereof or the pharmaceutically acceptable salt thereof according to claim 12, wherein q is 0.

16. The compound, or the stereoisomer thereof, the tautomer thereof or the pharmaceutically acceptable salt thereof according to any one of claims 12 to 15, wherein 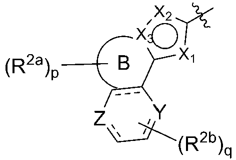 is selected from the following groups:

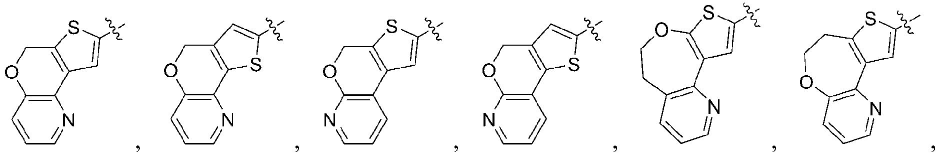

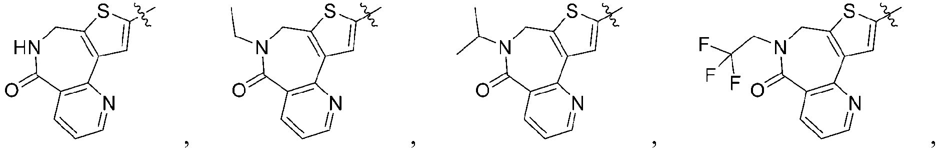

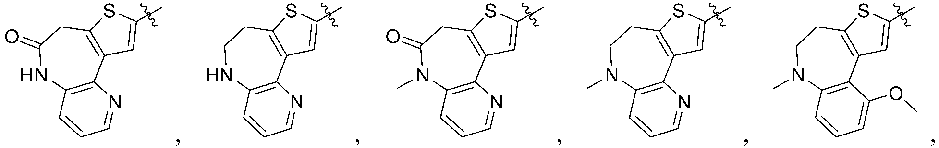

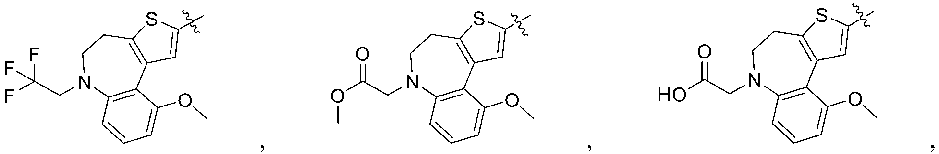

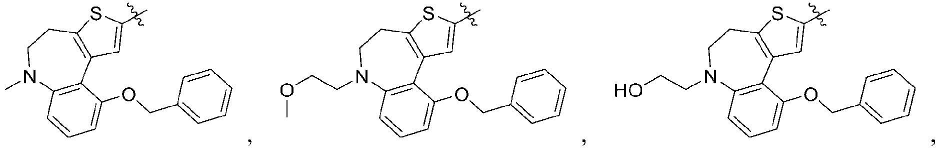

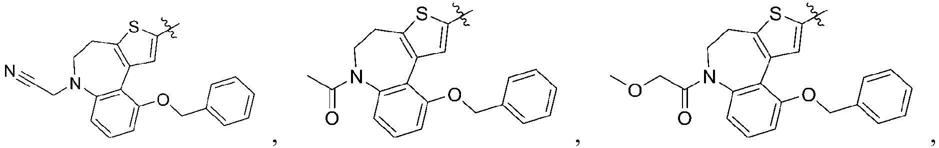

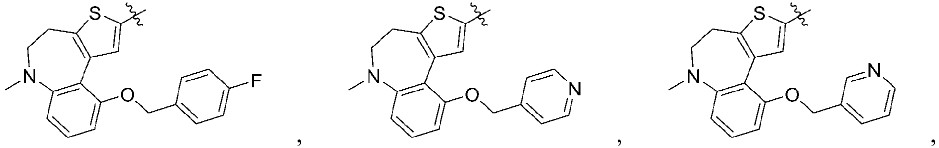

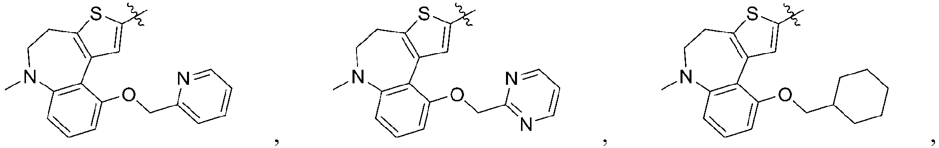

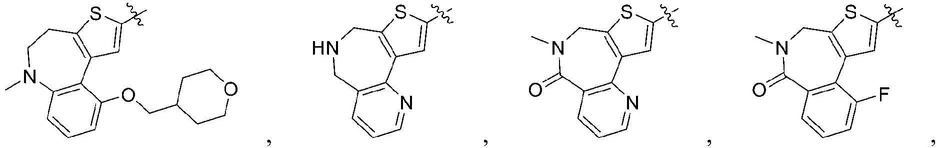

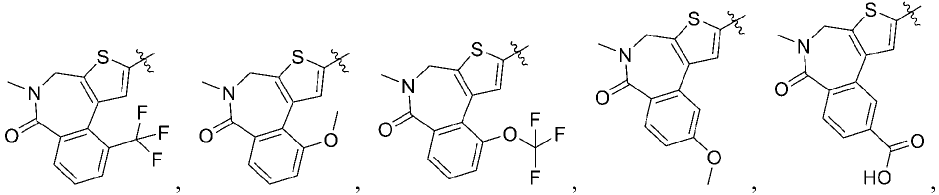

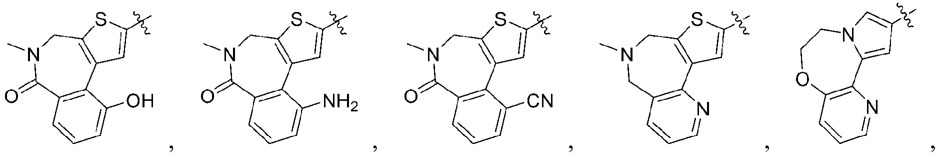

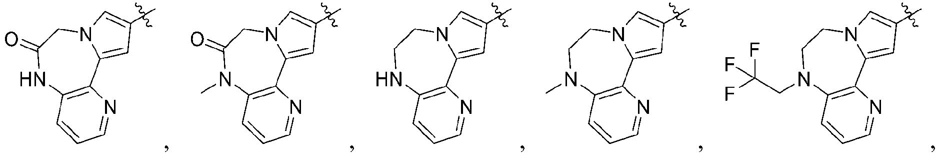

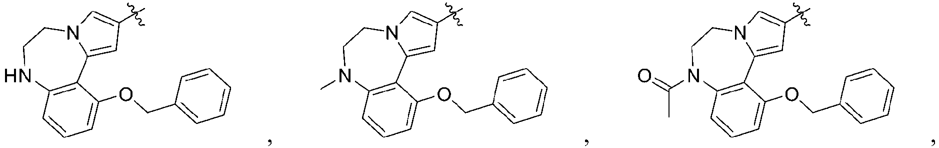

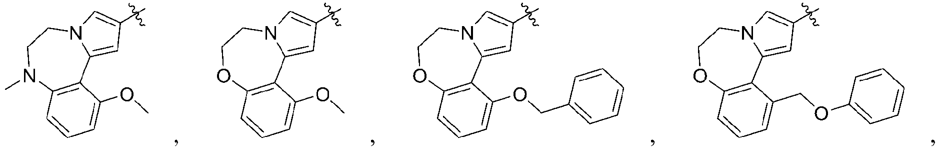

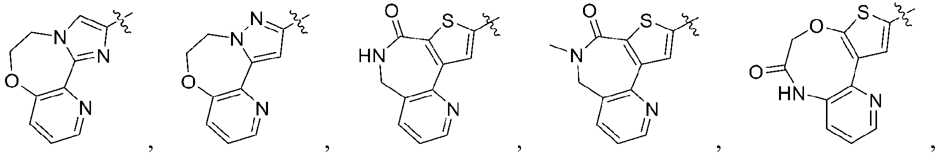

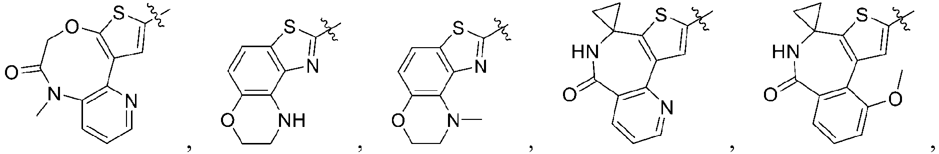

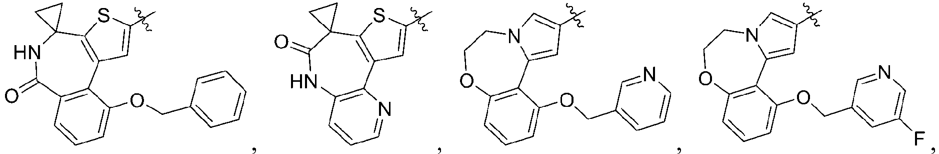

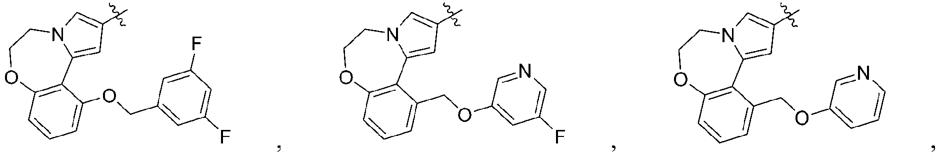

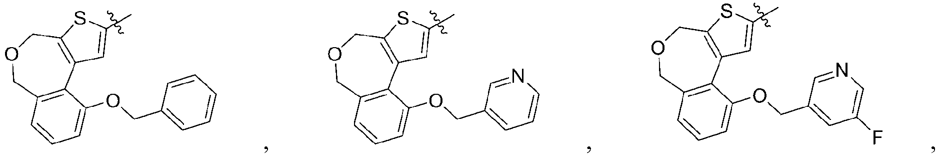

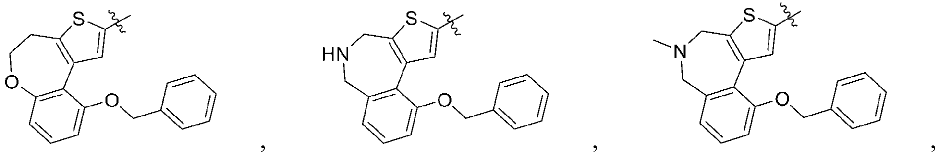

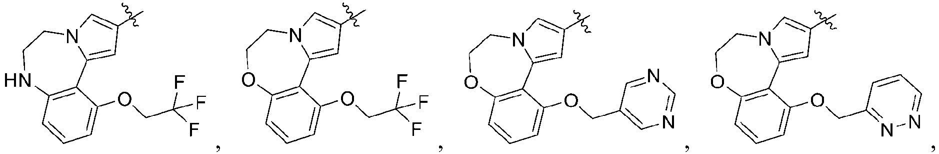

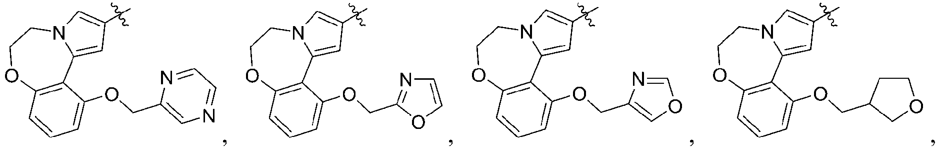

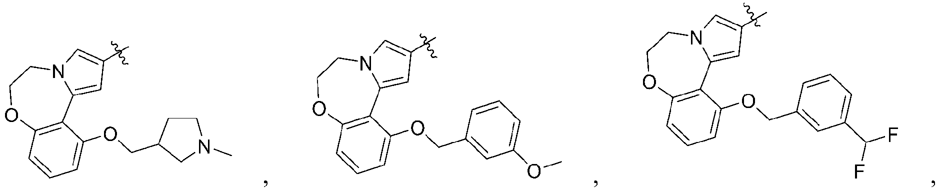

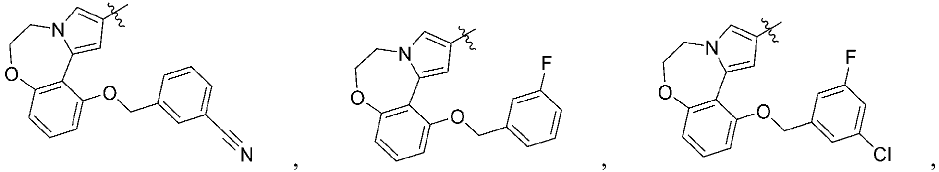

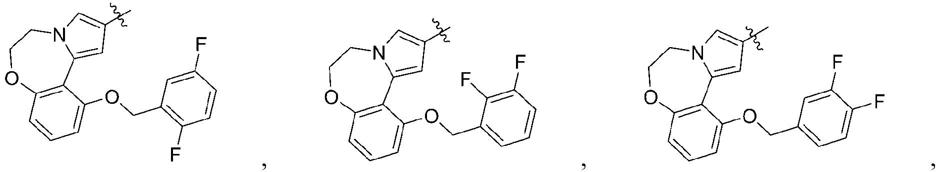

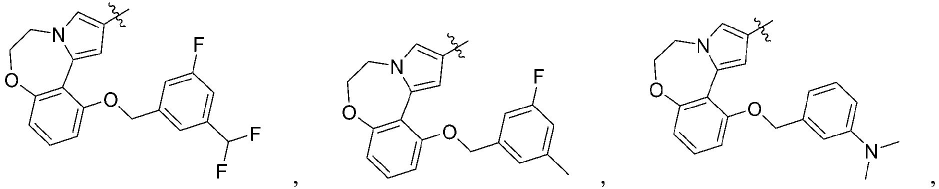

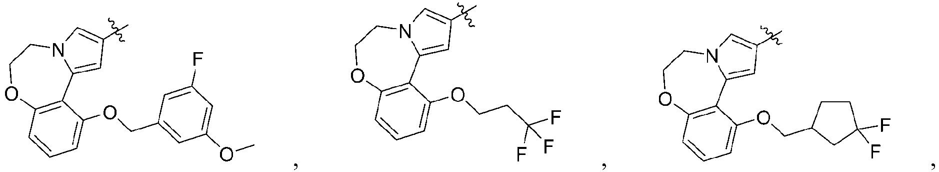

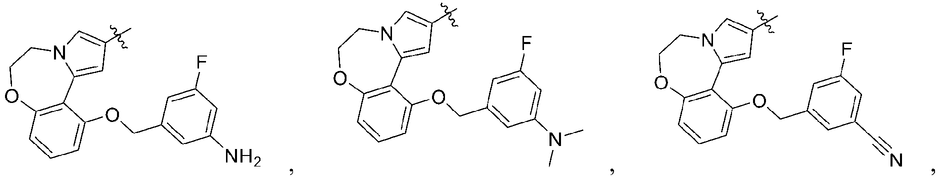

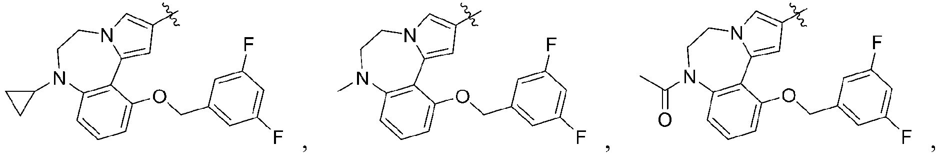

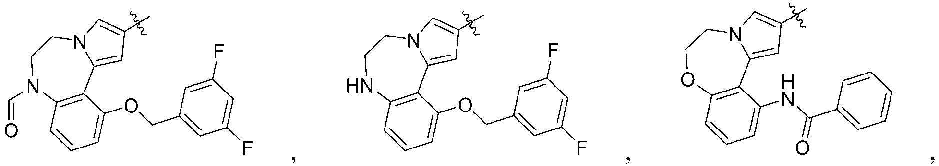

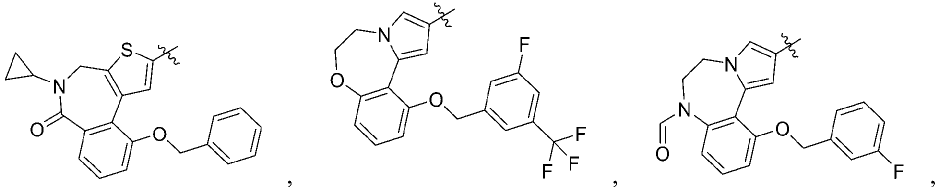

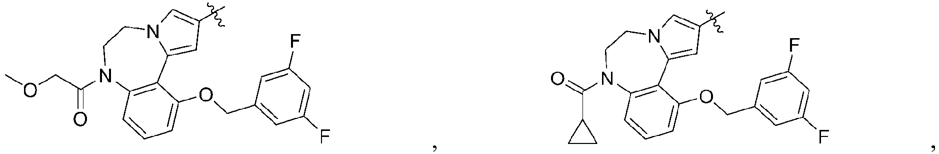

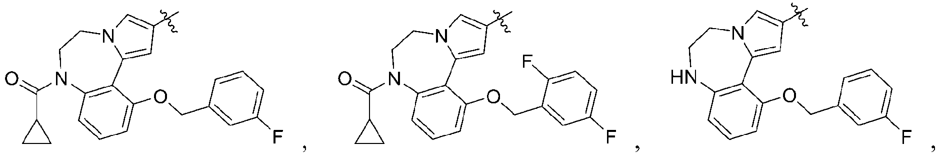

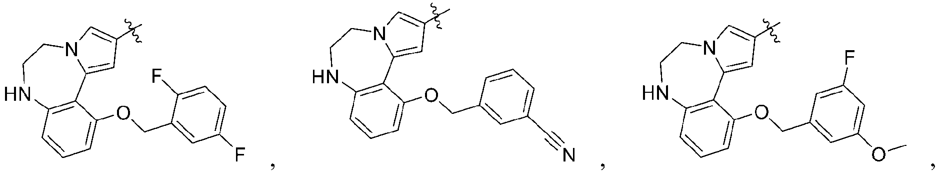

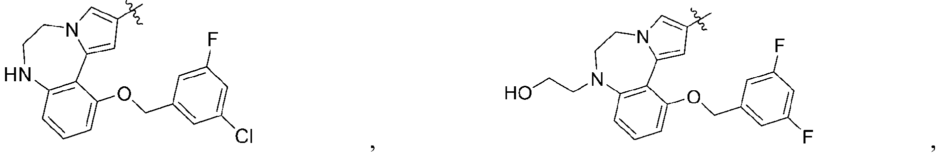

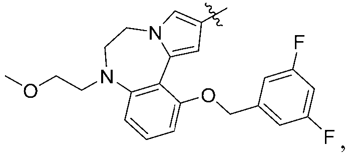

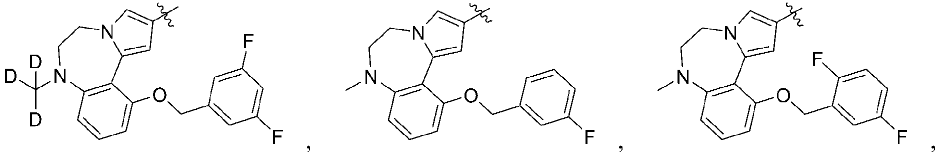

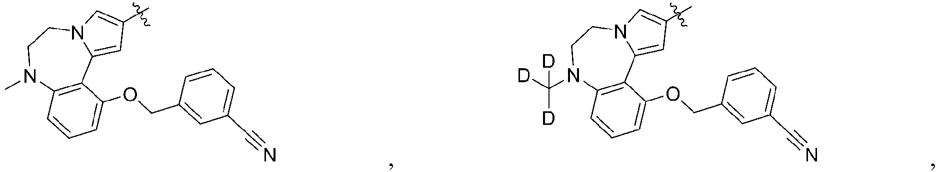

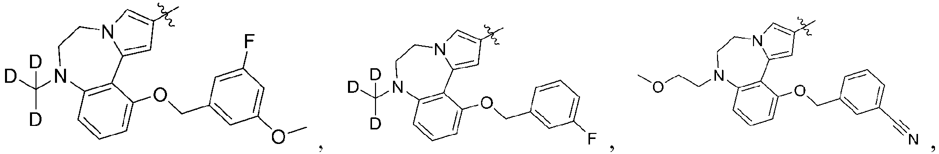

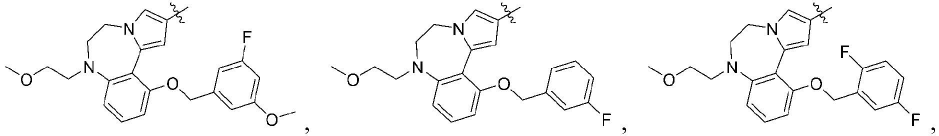

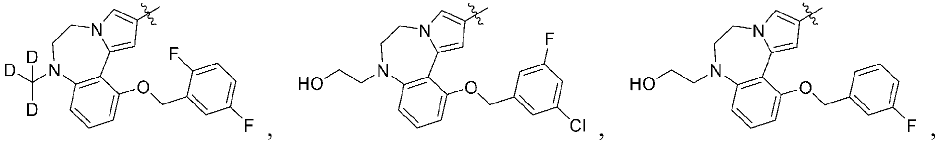

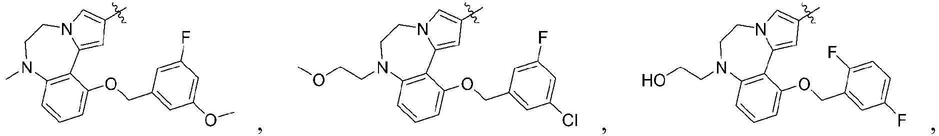

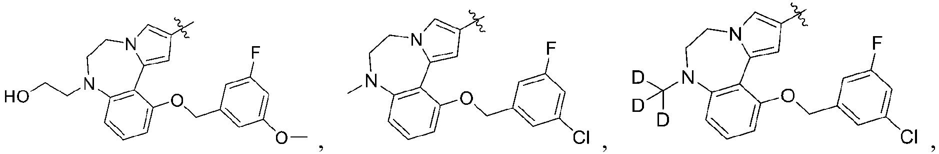

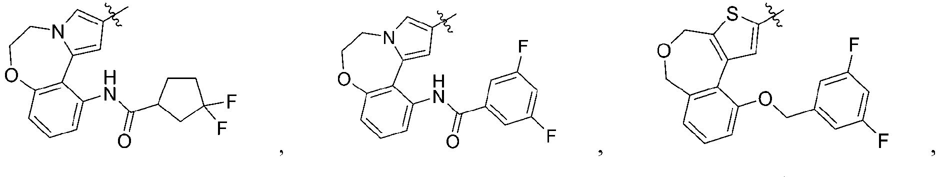

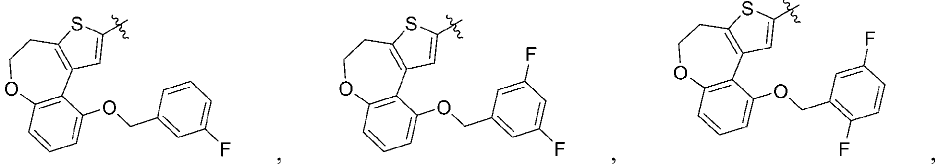

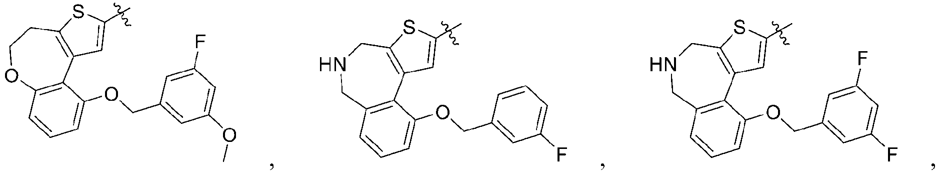

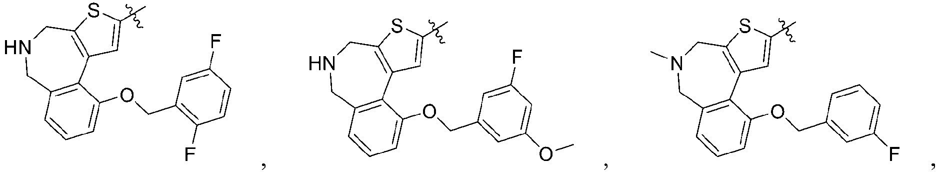

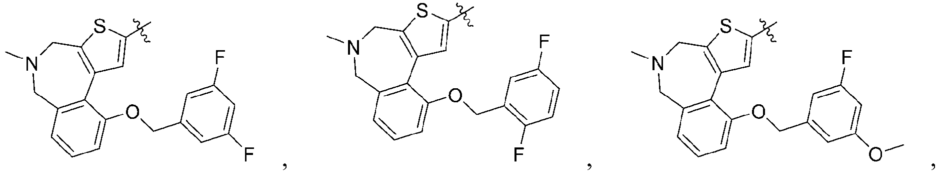

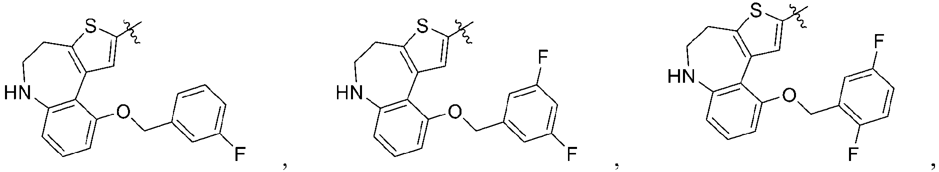

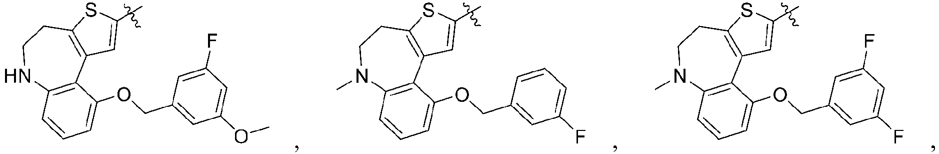

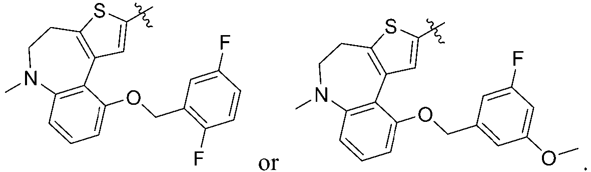

17. The compound, or the stereoisomer thereof, the tautomer thereof or the pharmaceutically acceptable salt thereof according to any one of claims 1 to 16, wherein X is selected from Cl or Br, preferably Cl.

18. The compound, or the stereoisomer thereof, the tautomer thereof or the pharmaceutically acceptable salt thereof according to any one of claims 1 to 17, wherein R¹ is selected from methyl or ethyl.

19. The compound, or the stereoisomer thereof, the tautomer thereof or the pharmaceutically acceptable salt thereof according to any one of claims 1 to 18, wherein the compound is:

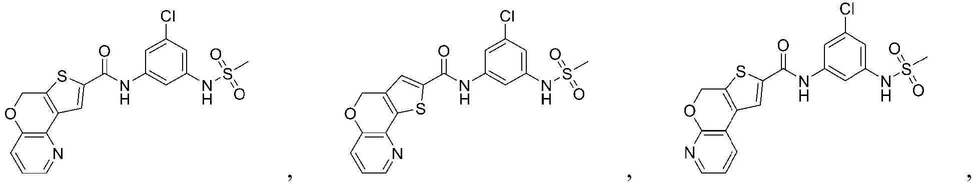

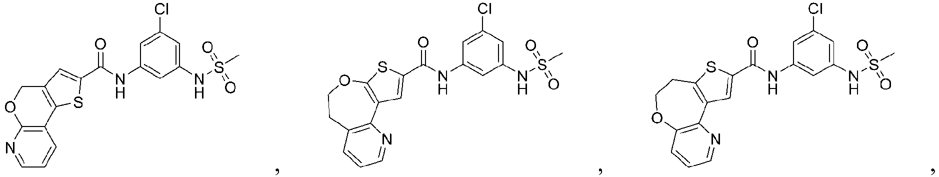

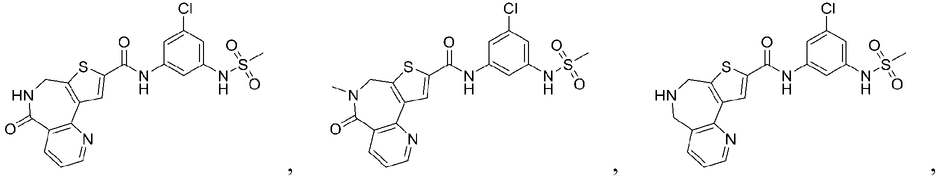

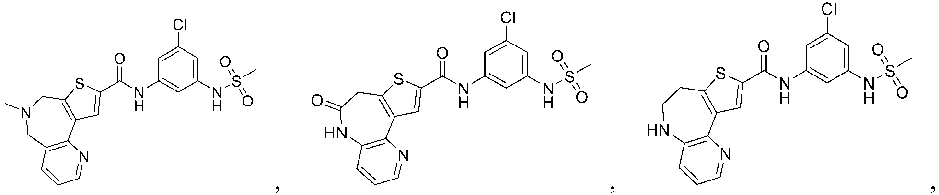

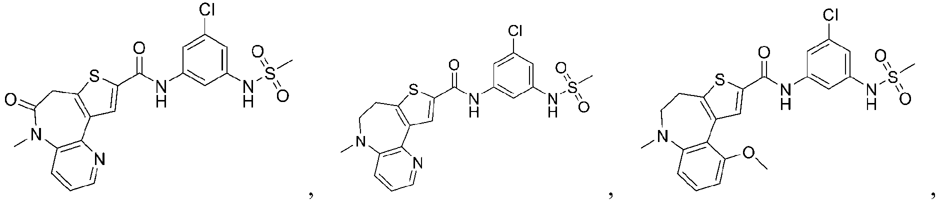

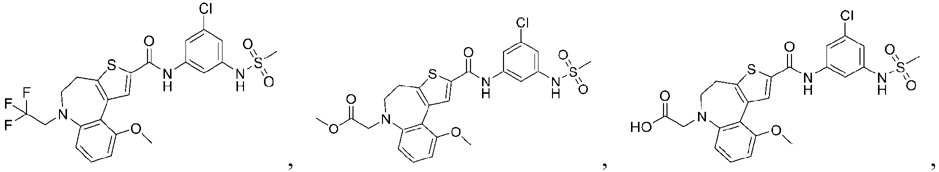

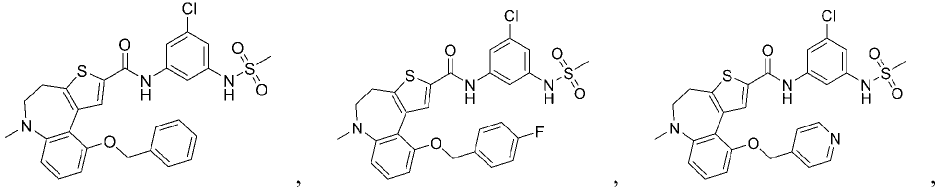

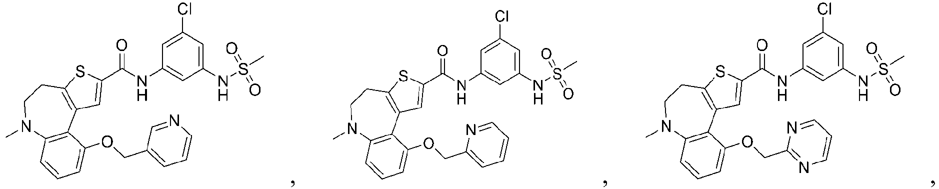

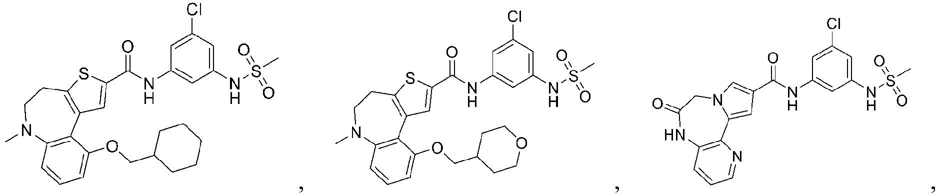

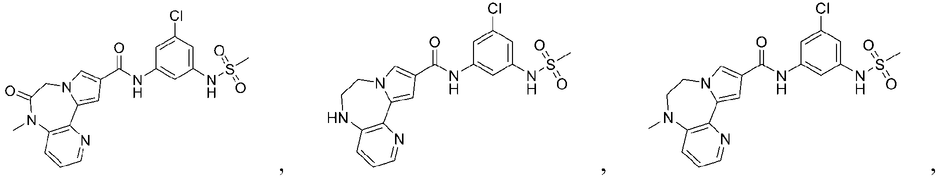

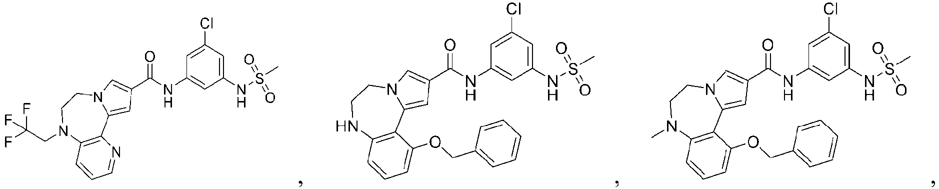

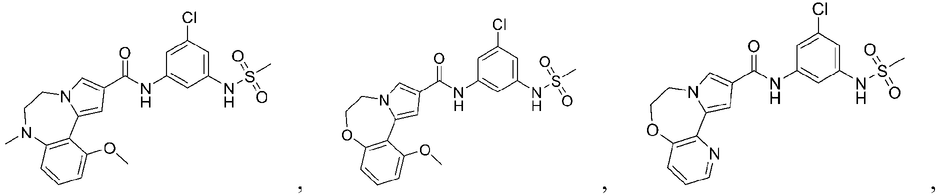

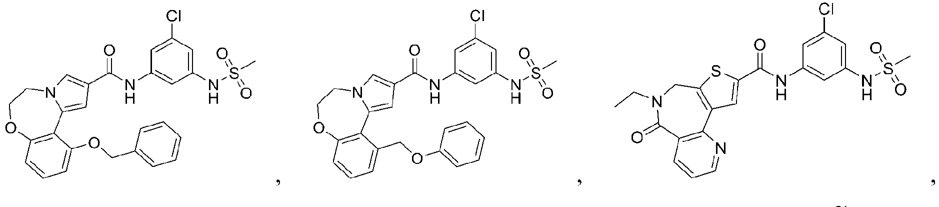

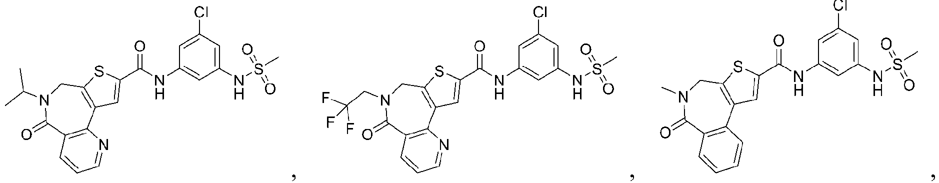

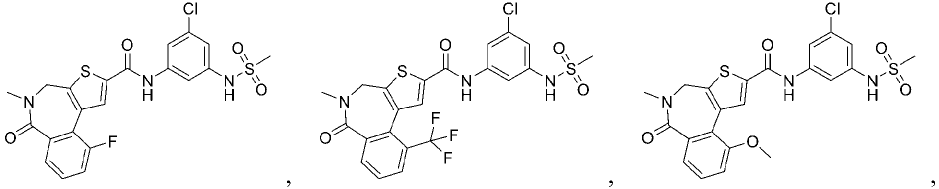

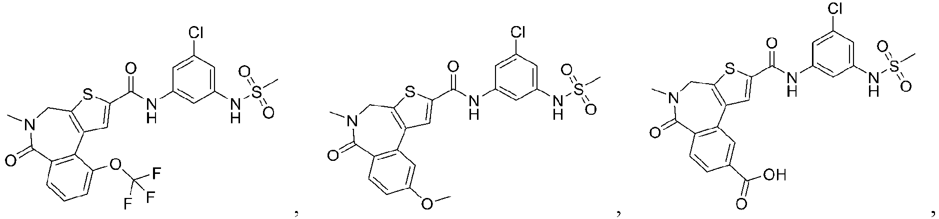

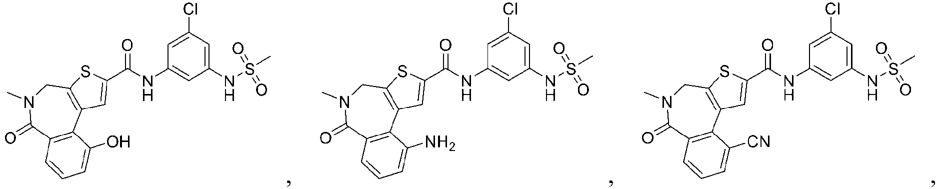

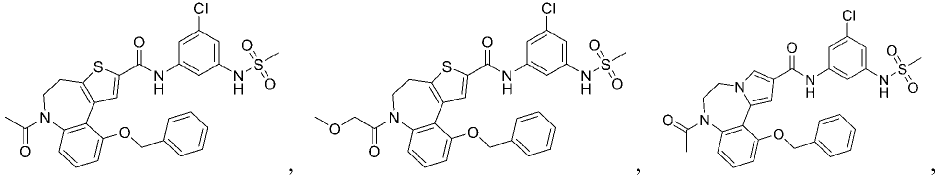

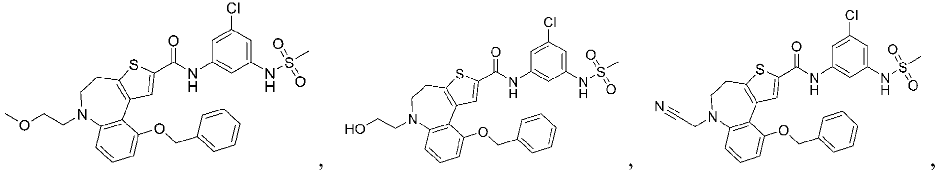

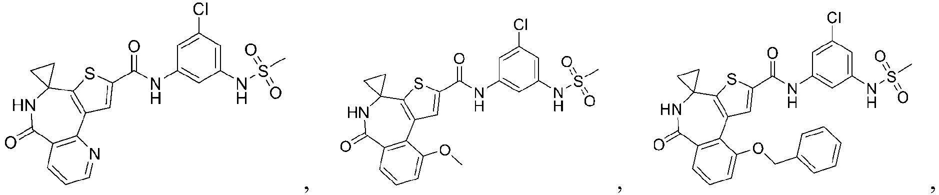

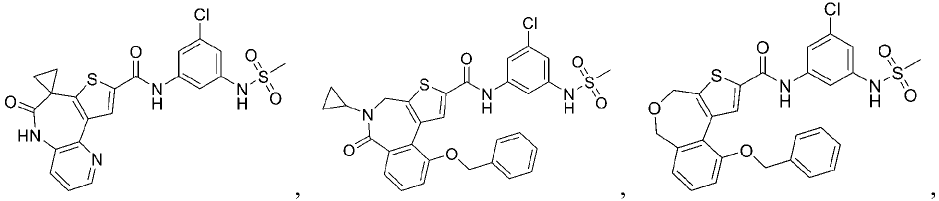

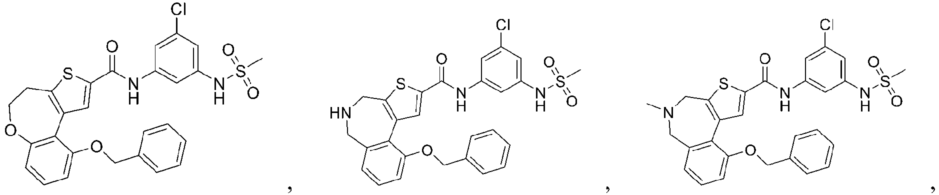

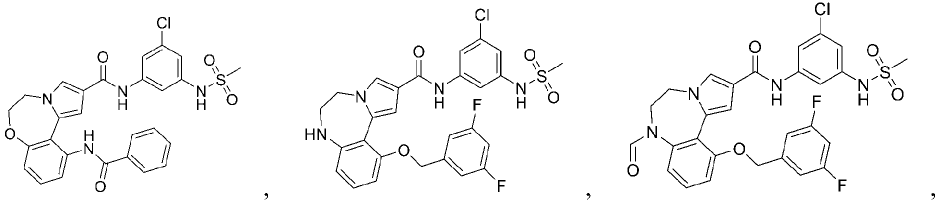

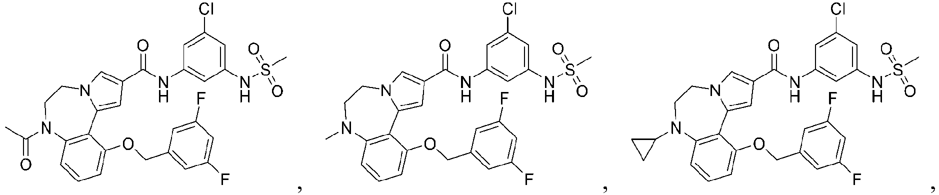

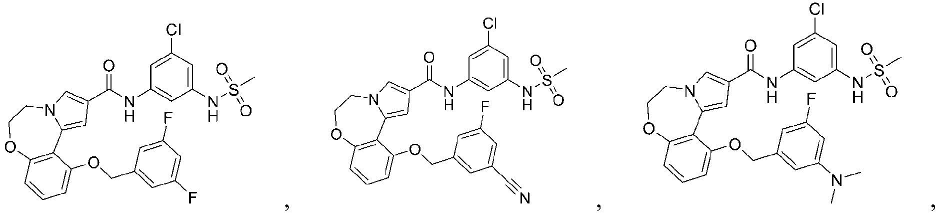

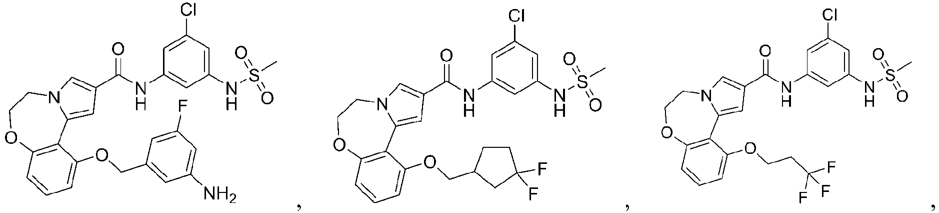

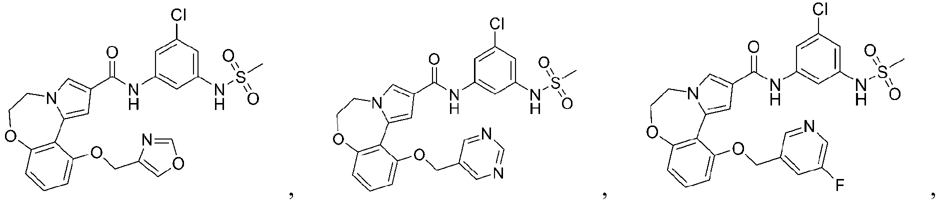

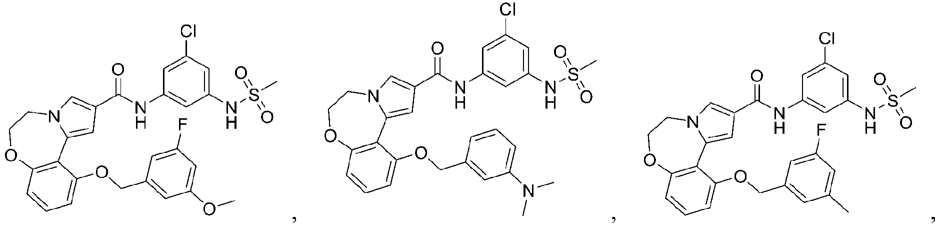

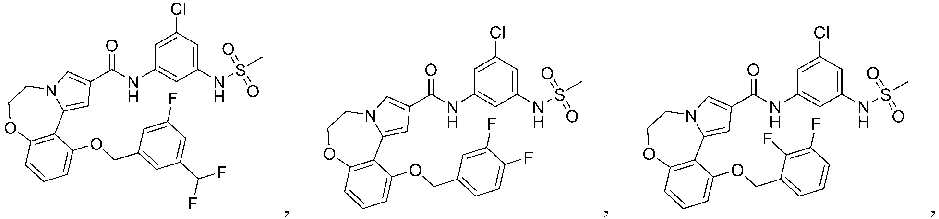

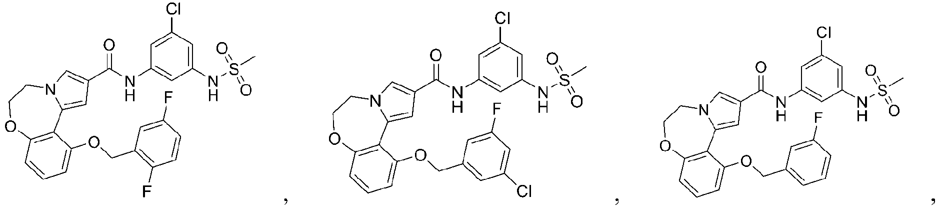

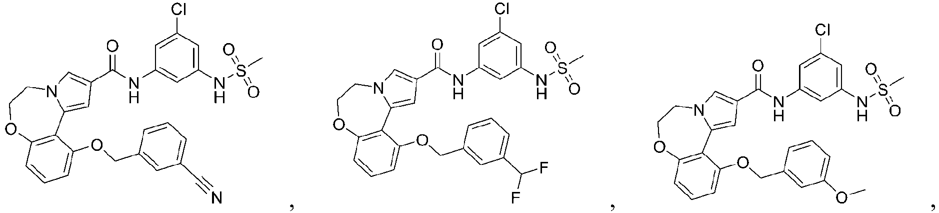

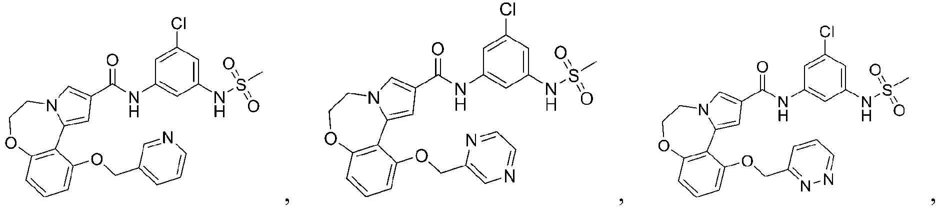

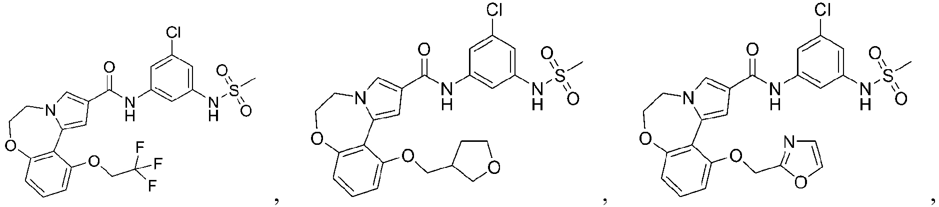

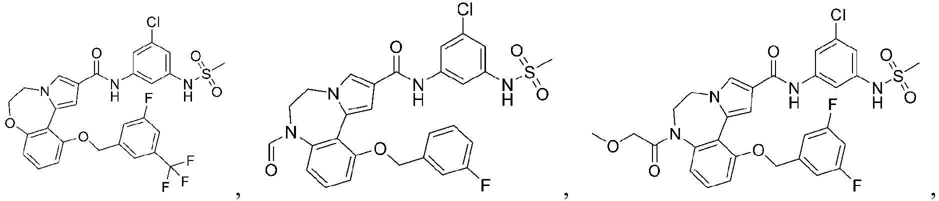

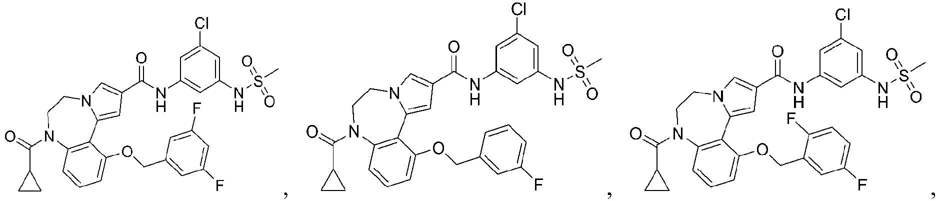

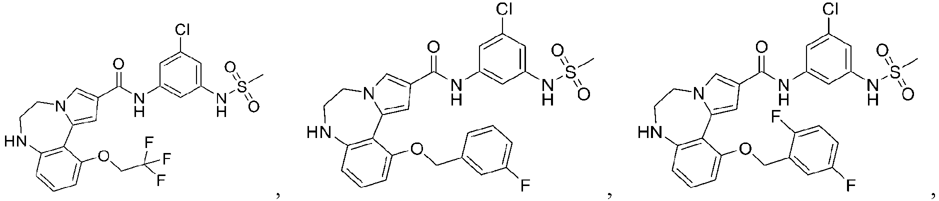

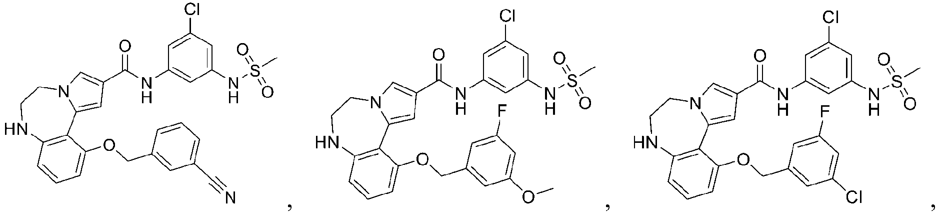

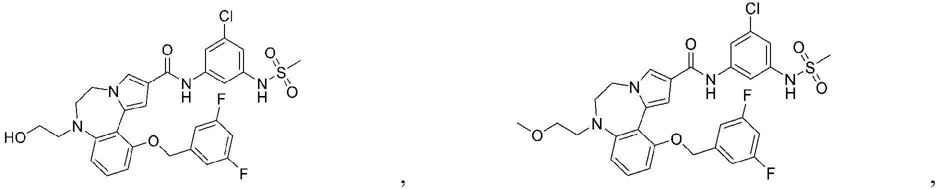

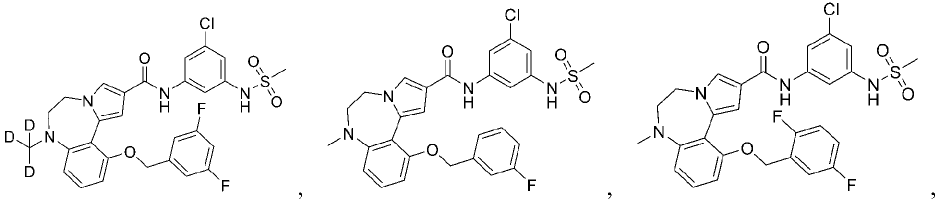

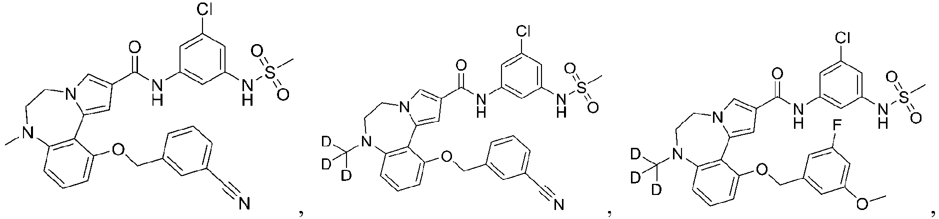

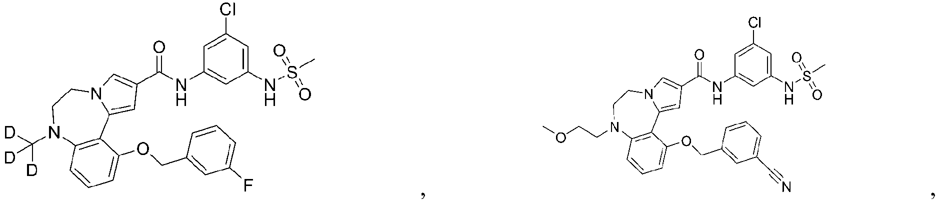

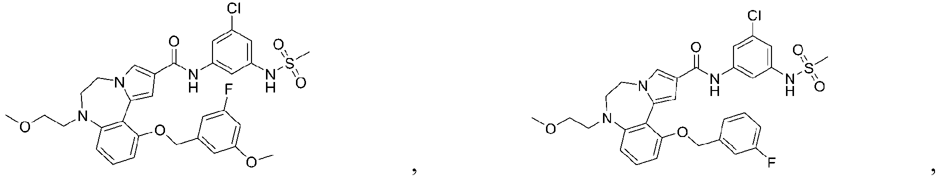

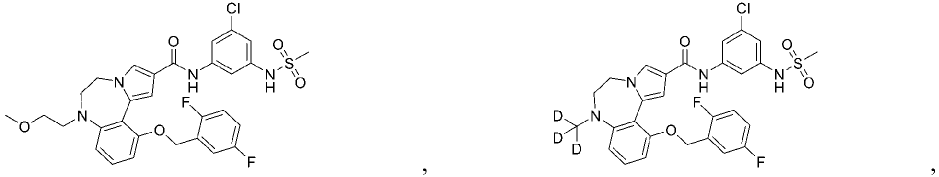

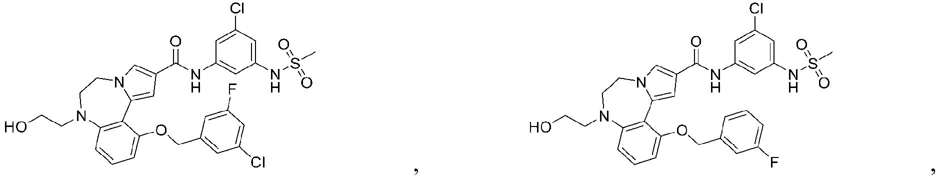

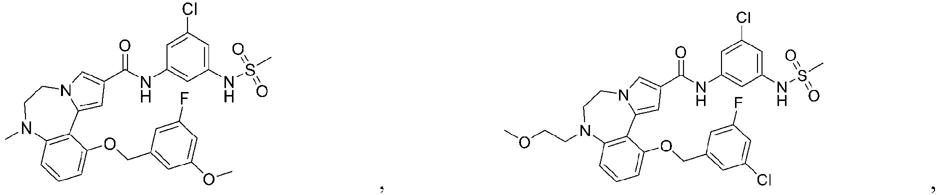

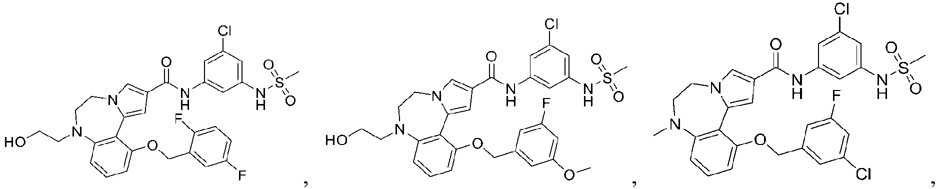

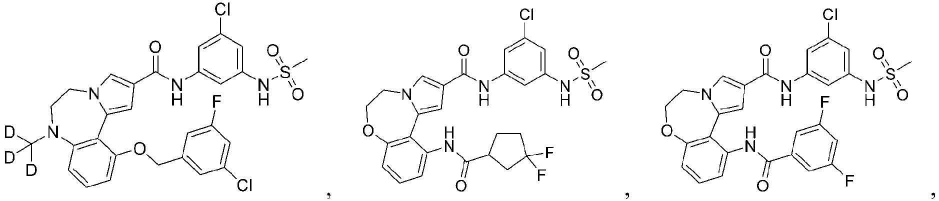

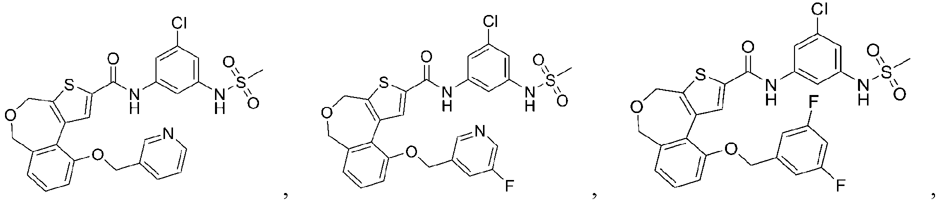

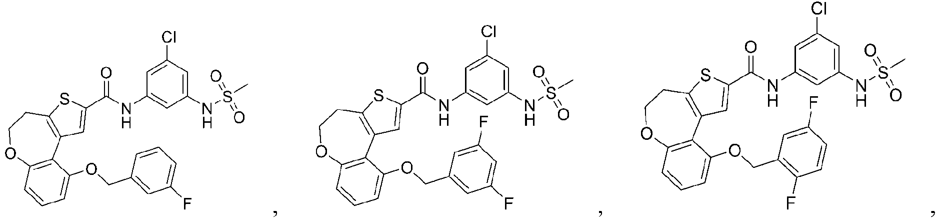

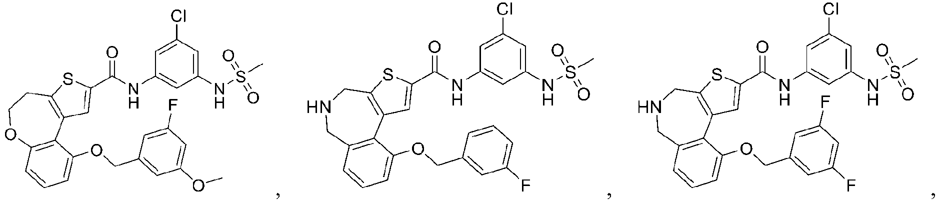

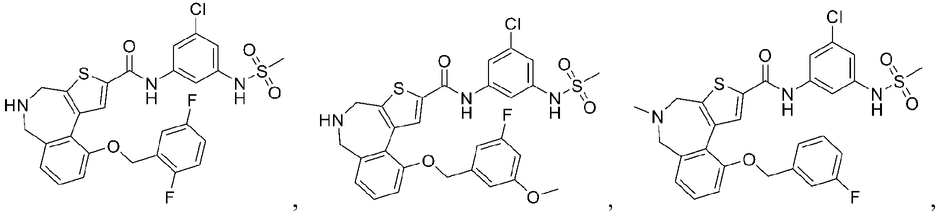

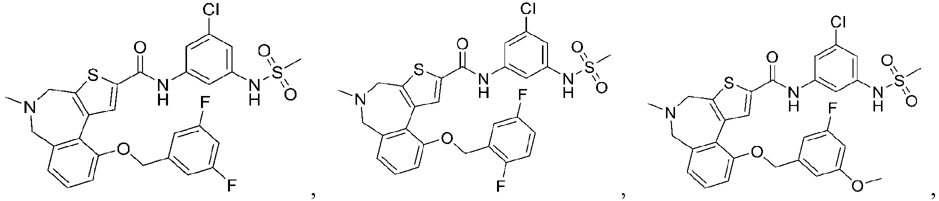

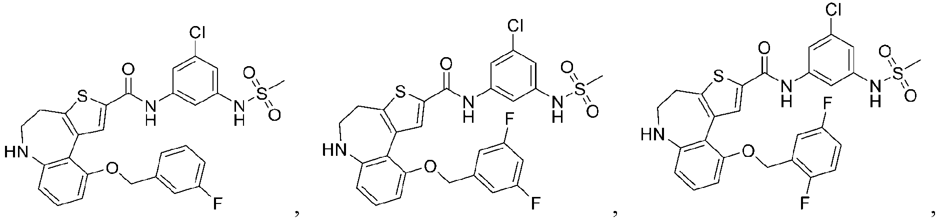

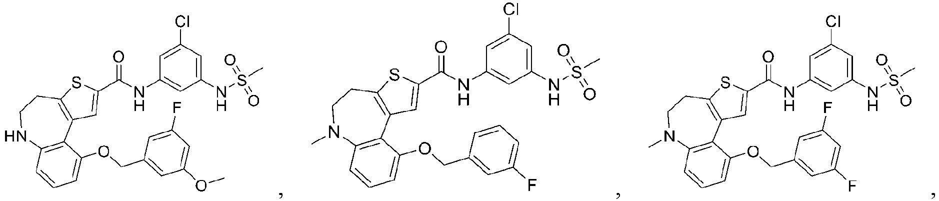

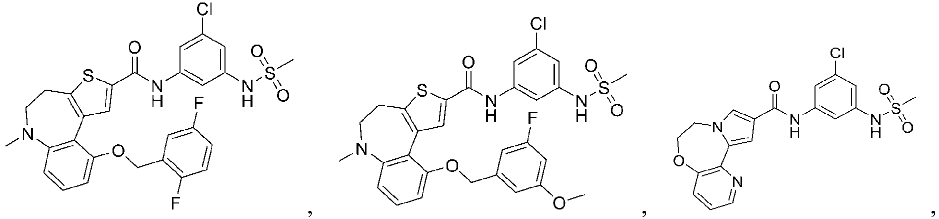

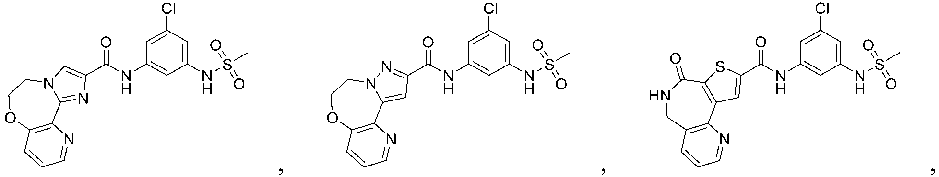

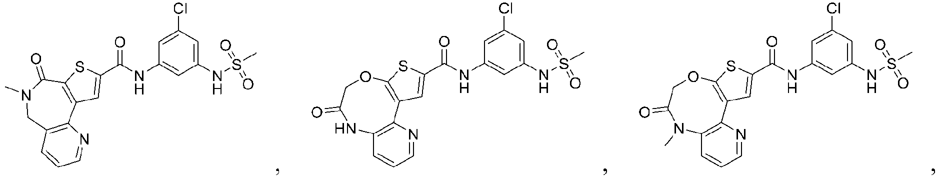

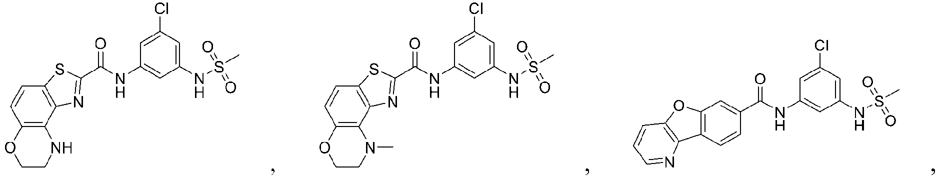

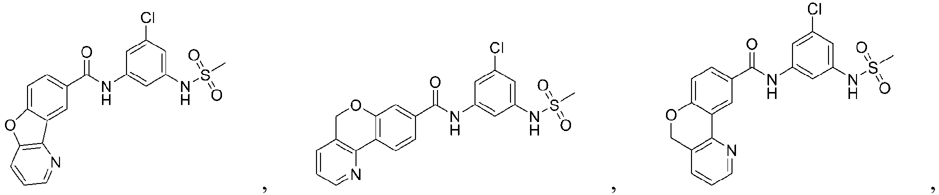

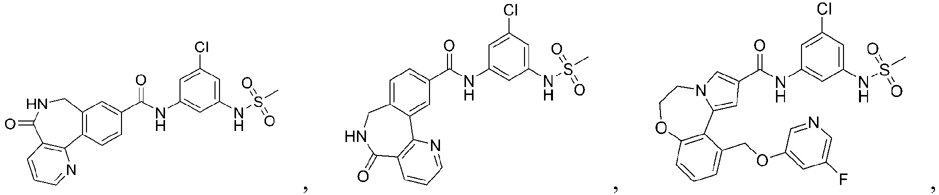

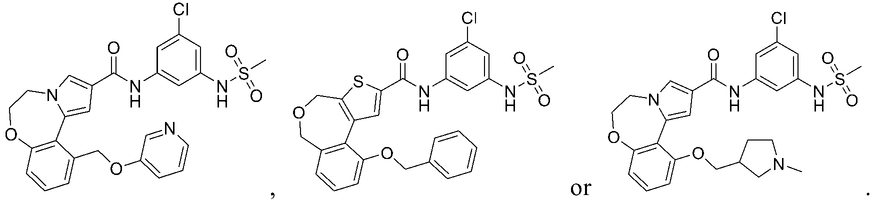

20. A pharmaceutical composition, wherein the pharmaceutical composition contains an effective dose of the compound, or the stereoisomer thereof, the tautomer thereof or the pharmaceutically acceptable salt thereof according to any one of claims 1 to 19, and a pharmaceutically acceptable carrier, an excipient or a combination thereof.

21. Use of the compound, or the stereoisomer thereof, the tautomer thereof or the pharmaceutically acceptable salt thereof according to any one of claims 1 to 19, or the pharmaceutical composition according to claim 20, in the preparation of a DHX9 inhibitor.

22. Use of the compound, or the stereoisomer thereof, the tautomer thereof or the pharmaceutically acceptable salt thereof according to any one of claims 1 to 19, or the pharmaceutical composition according to claim 20, in the manufacture of a medicament for the treatment of a DHX9-mediated disease, wherein the DHX9-mediated disease is preferably cancer, viral infection or autoimmune disease, more preferably cancer; and still further preferably, the cancer is selected from colorectal cancer, endometrial cancer, ovarian cancer, gastric cancer, hematopoietic system cancer, breast cancer, brain cancer, skin cancer, lung cancer, blood cancer, prostate cancer, head and neck cancer, pancreatic cancer, bladder cancer, bone cancer, soft tissue cancer, renal cancer and liver cancer, even more preferably colorectal cancer, endometrial cancer, ovarian cancer, hematopoietic system cancer and gastric cancer.

23. The use according to claim 22, wherein the cancer is microsatellite instability cancer, preferably high microsatellite instability cancer, and further preferably high microsatellite instability colorectal cancer.

24. Use of the compound, or the stereoisomer thereof, the tautomer thereof or the pharmaceutically acceptable salt thereof according to any one of claims 1 to 19, or the pharmaceutical composition according to claim 20, in the manufacture of a medicament for the treatment of cancer, viral infection or autoimmune disease, wherein, preferably, the cancer is selected from colorectal cancer, endometrial cancer, ovarian cancer, gastric cancer, hematopoietic system cancer, breast cancer, brain cancer, skin cancer, lung cancer, blood cancer, prostate cancer, head and neck cancer, pancreatic cancer, bladder cancer, bone cancer, soft tissue cancer, renal cancer and liver cancer, and further preferably, the cancer is selected from colorectal cancer, endometrial cancer, ovarian cancer, hematopoietic system cancer and gastric cancer.

25. The use according to claim 24, wherein the cancer is microsatellite instability cancer, preferably high microsatellite instability cancer, and further preferably high microsatellite instability colorectal cancer.
